(19) Europäisches Patentamt
European Patent Office
Office européen des brevets

(11) EP 4 570 813 A1

(12) **EUROPEAN PATENT APPLICATION**
published in accordance with Art. 153(4) EPC

(43) Date of publication:
**18.06.2025 Bulletin 2025/25**

(21) Application number: **23877334.5**

(22) Date of filing: **12.10.2023**

(51) International Patent Classification (IPC):
***C07K 7/64*** *(2006.01)* ***C07K 1/02*** *(2006.01)*

(52) Cooperative Patent Classification (CPC):
**C07K 1/02; C07K 7/64**

(86) International application number:
**PCT/JP2023/037071**

(87) International publication number:
**WO 2024/080333 (18.04.2024 Gazette 2024/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.10.2022 JP 2022164961**

(71) Applicant: **CHUGAI SEIYAKU KABUSHIKI KAISHA**
**Tokyo 115-8543 (JP)**

(72) Inventors:
- **HOSHINO Yuki**
  **Tokyo 115-8543 (JP)**
- **SERIZAWA Hiroki**
  **Tokyo 115-8543 (JP)**
- **NOGI Keisuke**
  **Tokyo 115-8543 (JP)**
- **MATSUDA Masaaki**
  **Tokyo 115-8543 (JP)**
- **TAMURA Miho**
  **Tokyo 115-8543 (JP)**

(74) Representative: **Vossius & Partner Patentanwälte Rechtsanwälte mbB**
**Siebertstrasse 3**
**81675 München (DE)**

(54) **METHOD FOR PRODUCING N-SUBSTITUTED AMINO ACID RESIDUE-CONTAINING CYCLIC PEPTIDE COMPOUND**

(57) It has been found that, by linking an N-terminal amino acid residue of a peptide compound with a C-terminal amino acid residue of the peptide compound in which at least one of the N-terminal amino acid residue and the C-terminal amino acid residue is a cyclic amino acid residue in a solvent containing one or more selected from the group consisting of acetonitrile, dimethyl carbonate, 2-MeTHF and anisole, epimerization can be suppressed and formation of oligomers can be reduced, thereby a cyclic peptide compound of interest can be efficiently produced.



Processed by Luminess, 75001 PARIS (FR)

## Description

[Technical Field]

The present invention relates to a method for producing a cyclic peptide compound, comprising a cyclization step of a peptide compound that comprises a C-terminal amino acid residue having a side chain on the α-carbon and has an N-substituted amino acid residue on either one or both of the N-terminal amino acid residue and the C-terminal amino acid residue.

[Background Art]

Peptides are molecules in which a large number of amino acids are linked. Peptides play an indispensable role in the life activities of the organisms. These peptides can be extended to a desired peptide sequence by reacting an amino acid or a peptide in which the C-terminal carboxyl group is activated with an amino acid or an amino group of a peptide to form an amide bond. However, the production of a peptide comprising non-natural amino acid in the sequence, especially a peptide comprising an N-methylamino acid, has had a problem in that the yield of target material is lowered due to the low reactivity of the condensation reaction caused by the steric hindrance of the N-methyl group, epimerization of the amino acid residue at position α, and the like (Non-Patent Literature 1).

Removing the byproducts produced by side reactions such as epimerization typically requires a purification step. However, in peptide synthesis on an industrial scale, the peptide purification step causes problems of the necessity of a longer production time and a higher cost therefor. Thus, there is a need of a method capable of synthesizing a peptide of interest without purification by column chromatography or cumbersome purification step while minimizing byproducts.

The problem of epimerization can also occur in linking an N-terminal amino acid residue of a linear peptide with a C-terminal amino acid residue of the linear peptide to synthesize a cyclic peptide. Thus, the cyclization reaction of a linear peptide also requires avoiding the epimerization of the C-terminal amino acid residue at position α, as in the elongation reaction of an amino acid or peptide.

To prevent such epimerization of the C-terminal amino acid residue of the peptide at position α, methods may be employed in which a carboxyl group at the C-terminus is activated to bind to the other amino acid or peptide where the C-terminal amino acid residue does not have a side chain on the α-carbon or have di-substituents (α,α-disubstituted) (Non-Patent Literature 2).

Meanwhile, it is known in the reaction of activating and cyclizing the carboxyl group of the linear peptide having a side chain on the α-carbon of the C-terminal amino acid residue that, when at least one of the amino acid residues at the reaction point is an N-substituted amino acid residue, epimerization actually occurs in the synthesis of the cyclic peptide by the cyclization reaction (Non-Patent Literature 3). However, reaction conditions that allow suppression of epimerization using readily available and inexpensive reagents and the reactive amino acid residues have not been cleared.

Furthermore, during the cyclization reaction, an intermolecular reaction may also proceed competitively, thus leading to the formation of oligomers such as dimers and trimers of the peptide (Non-Patent Literature 4). Either for such formation of oligomer, reaction conditions that allow suppression of oligomer formation using readily available and inexpensive reagents and solvents and the reactive amino acid residues have not been cleared. Furthermore, these epimerized peptides and oligomers of peptides need to be separated and removed from a cyclic peptide of interest, because they are byproducts. When such byproducts are produced in large quantities, cumbersome purification steps using large amounts of solvents such as column chromatography have to be incorporated into the production process, resulting in complex production processes and increased costs. Thus, a method is required in which formation of byproducts can be suppressed.

To improve the efficiency of the synthesis of the cyclic peptide, it is thus considered that cyclization reaction is desirably performed with a solvent that facilitates liquid separation in the post-treatment step after the cyclization reaction. However, since the reaction solvent often used in the cyclization reaction is a halogenated hydrocarbon solvent (such as dichloromethane) and an amide solvent (such as DMF), which have a large environmental load, it is also required to find an alternative solvent from this point (Non-Patent Literature 5).

Therefore, from the viewpoint of cost, environmental load, ease of production, and industrially applicability of the production method, it is important to find a reaction solvent, a reagent, and a combination of amino acid residues that serves as a reaction point that enable suppression of formation of byproducts such as epimers and oligomers when activating the carboxyl group of the peptide having a side chain on the α-carbon of the C-terminal amino acid residue to perform the cyclization reaction and a solvent that facilitates liquid separation after the reaction.

[Citation List]

[Non-Patent Literature]

[Non-Patent Literature 1]
J. Peptide Res., 2005, 65, 153-166.
[Non-Patent Literature 2]
Org. Lett. 2013, 15, 1155-1157.
[Non-Patent Literature 3]
Org. Lett. 2012, 14, 612-615.
[Non-Patent Literature 4]
Chem. Rev. 1997, 6, 2243-2266.
[Non-Patent Literature 5]
Green Chem. Lett. Rev., 2021, 14, 153-164.

[Summary of Invention]

[Technical Problem]

The present invention has been made in view of such a situation, and an object of the present invention is to provide, in a synthesis of a cyclic peptide compound from a peptide compound that comprises a C-terminal amino acid residue having a side chain on the α-carbon and has an N-substituted amino acid residue on either one or both of the N-terminal amino acid residue and the C-terminal amino acid residue, a method for efficiently producing a cyclic peptide compound of interest by suppressing epimerization and reducing formation of oligomers, from the viewpoints of cost, environmental load, and ease of preparation. Specifically, an object of the present invention is to provide a method of cyclizing a peptide compound having a predetermined N-terminal and C-terminal amino acid residues, which enables suppressing epimerization and reducing formation of oligomers.

[Solution to Problem]

The present inventors have intensively studied to solve the above problem and, as a result, have found that, in a synthesis of a cyclic peptide compound from a peptide compound that comprises a C-terminal amino acid residue having a side chain on the α-carbon and has an N-substituted amino acid residue on at least one or both of the N-terminal amino acid residue and the C-terminal amino acid residue, epimerization can be suppressed and formation of oligomers can be reduced, thereby a cyclic peptide compound of interest can be efficiently produced.

In one non-limiting specific aspect, the present invention encompasses the following.

[1] A method for producing a cyclic peptide compound, or a salt thereof, or a solvate thereof, the method comprising:

a step of linking an N-terminal amino acid residue of a peptide compound with a C-terminal amino acid residue of the peptide compound in a solvent, wherein
at least one of the N-terminal amino acid residue and the C-terminal amino acid residue is a cyclic amino acid residue, and
the solvent comprises one or more selected from the group consisting of acetonitrile, dimethyl carbonate, 2-MeTHF, and anisole.

[2] A method for producing a cyclic peptide compound, or a salt thereof, or a solvate thereof, the method comprising:

a step of linking an N-terminal amino acid residue of a peptide compound with a C-terminal amino acid residue of the peptide compound in a solvent, wherein
the N-terminal amino acid residue and the C-terminal amino acid residue of the peptide compound are one selected from the following a) to e):

a) the N-terminal amino acid residue is an N-substituted phenylalanine residue or an N-substituted phenylalanine derivative residue, and the C-terminal amino acid residue is a cyclic amino acid residue;
b) the N-terminal amino acid residue is an α,α-di-substituted amino acid residue, and the C-terminal amino acid residue is a cyclic amino acid residue;
c) the N-terminal amino acid residue is a cyclic amino acid residue, and the C-terminal amino acid residue is a

homophenylalanine residue or a homophenylalanine derivative residue;

d) the N-terminal amino acid residue is a cyclic amino acid residue, and the C-terminal amino acid residue is an N-substituted Ala residue; and

e) the N-terminal amino acid residue is an N-substituted Gly residue, and the C-terminal amino acid residue is an N-substituted phenylalanine residue or an N-substituted phenylalanine derivative residue.

[3] The method according to [2], wherein the solvent comprises one or more selected from the group consisting of acetonitrile, dimethyl carbonate, 2-MeTHF, and anisole.

[4] The method according to [1] or [2], wherein the solvent is one selected from the group consisting of acetonitrile, dimethyl carbonate, 2-MeTHF, and anisole.

[5] The method according to [1] or [2], wherein the solvent is one selected from the group consisting of acetonitrile and dimethyl carbonate.

[6] The method according to [1] or [2], wherein the solvent is acetonitrile.

[7] The method according to [1] or [2], wherein the solvent is dimethyl carbonate.

[8] The method according to any of [1] to [7], wherein the linking the N-terminal amino acid residue of the peptide compound with the C-terminal amino acid residue of the peptide compound is linking an amino group of the N-terminal amino acid residue with a carboxyl group of the C-terminal amino acid residue.

[9] The method according to any of [1] to [7], wherein the linking the N-terminal amino acid residue of the peptide compound with the C-terminal amino acid residue of the peptide compound is linking an amino group of the N-terminal amino acid residue with a carboxyl group of the C-terminal amino acid residue by an amide bond.

[10] The method according to any of [1] to [9], wherein the linking step is performed in the presence of a condensation reagent.

[11] The method according to [10], wherein the condensation reagent is one selected from the group consisting of HATU, COMU, DMT-MM, PyOxim, PyBOP, and PyClop.

[12] The method according to [10], wherein the condensation reagent is one selected from the group consisting of HATU and COMU.

[13] The method according to [10], wherein the condensation reagent is HATU.

[14] The method according to [10], wherein the condensation reagent is COMU.

[15] The method according to [10], wherein the condensation reagent is HATU, and the solvent is dimethyl carbonate.

[16] The method according to [10], wherein the condensation reagent is HATU, and the solvent is acetonitrile.

[17] The method according to [10], wherein the condensation reagent is COMU, and the solvent is dimethyl carbonate.

[18] The method according to [10], wherein the condensation reagent is COMU, and the solvent is acetonitrile.

[19] The method according to any of [1] to [18], wherein the linking step is performed in the presence of a base.

[20] The method according to [19], wherein the base is an organic base.

[21] The method according to [19], wherein the base is an organic base comprising a tertiary amine.

[22] The method according to [19], wherein the base is one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP).

[23] The method according to [19], wherein the base is N,N-diisopropylethylamine (DIPEA).

[24] The method according to [19], wherein the condensation reagent is HATU, the solvent is dimethyl carbonate, and the base is N,N-diisopropylethylamine (DIPEA).

[25] The method according to [19], wherein the condensation reagent is HATU, the solvent is acetonitrile, and the base is N,N-diisopropylethylamine (DIPEA).

[26] The method according to [19], wherein the condensation reagent is COMU, the solvent is dimethyl carbonate, and the base is N,N-diisopropylethylamine (DIPEA).

[27] The method according to [19], wherein the condensation reagent is COMU, the solvent is acetonitrile, and the base is N,N-diisopropylethylamine (DIPEA).

[28] The method according to any of [1] to [27], wherein the C-terminal amino acid residue contained in the peptide compound is an amino acid residue having a side chain on an $\alpha$-carbon of a carboxyl group.

[29] The method according to any of [1] to [27], wherein the peptide compound comprises an amino acid residue of formula (1) at any one of amino acid residues other than the N-terminal amino acid residue and the C-terminal amino acid residue:

[Formula 1]

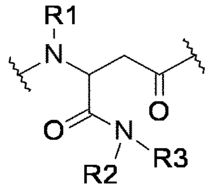

(1)

wherein

$R^1$ is hydrogen or $C_1$-$C_6$ alkyl, and
$R^2$ and $R^3$ are each independently hydrogen or $C_1$-$C_6$ alkyl, or $R^2$ and $R^3$ together with a nitrogen atom to which they are attached form a 4- to 7-membered saturated heterocycle.

[30] The method according to [29], wherein $R^1$ is $C_1$-$C_6$ alkyl.
[31] The method according to [29], wherein $R^1$ is methyl.
[32] The method according to any of [29] to [31], wherein $R^2$ and $R^3$ are each independently $C_1$-$C_6$ alkyl.
[33] The method according to any of [29] to [31], wherein $R^2$ and $R^3$ each are methyl.
[34] The method according to [29], wherein $R^1$ is $C_1$-$C_6$ alkyl, and $R^2$ and $R^3$ are each independently $C_1$-$C_6$ alkyl, or $R^2$ and $R^3$ together with a nitrogen atom to which they are attached form a 4- to 7-membered saturated heterocycle.
[35] The method according to [29], wherein $R^1$ is methyl, and $R^2$ and $R^3$ each are methyl.
[36] The method according to any of [1] to [35], wherein the N-terminal amino acid residue of the peptide compound is a cyclic amino acid residue, or the C-terminal amino acid residue of the peptide compound is a cyclic amino acid residue.
[37] The method according to any of [1] to [35], wherein the N-terminal amino acid residue of the peptide compound is a cyclic amino acid residue and the C-terminal amino acid residue is a non-natural amino acid residue, or the N-terminal amino acid residue of the peptide compound is a non-natural amino acid residue and the C-terminal amino acid residue is a cyclic amino acid residue.
[38] The method according to any of [1] to [35], wherein the N-terminal amino acid residue of the peptide compound is a cyclic amino acid residue and the C-terminal amino acid residue is a homophenylalanine residue, a homophenylalanine derivative residue, or an N-substituted Ala residue, or the N-terminal amino acid residue of the peptide compound is an N-substituted phenylalanine residue, an N-substituted phenylalanine derivative residue, or an $\alpha,\alpha$-disubstituted amino acid residue and the C-terminal amino acid residue is a cyclic amino acid residue.
[38-1] The method according to any of [1] to [35], wherein the N-terminal amino acid residue of the peptide compound is a cyclic amino acid residue and the C-terminal amino acid residue is a homophenylalanine derivative residue or an N-substituted Ala residue, or the N-terminal amino acid residue of the peptide compound is an N-substituted phenylalanine derivative residue or an $\alpha,\alpha$-disubstituted amino acid residue and the C-terminal amino acid residue is a cyclic amino acid residue.
[39] The method according to any of [1] to [35], wherein the N-terminal amino acid residue of the peptide compound is an N-substituted phenylalanine residue or an N-substituted phenylalanine derivative residue, and the C-terminal amino acid residue is a cyclic amino acid residue.
[39-1] The method according to any of [1] to [35], wherein the N-terminal amino acid residue of the peptide compound is an N-substituted phenylalanine derivative residue, and the C-terminal amino acid residue is a cyclic amino acid residue.
[40] The method according to any of [1] to [35], wherein the N-terminal amino acid residue of the peptide compound is an $\alpha,\alpha$-disubstituted amino acid residue, and the C-terminal amino acid residue is a cyclic amino acid residue.
[41] The method according to any of [1] to [35], wherein the N-terminal amino acid residue of the peptide compound is a cyclic amino acid residue, and the C-terminal amino acid residue is a homophenylalanine residue or a homophenylalanine derivative residue.
[41-1] The method according to any of [1] to [35], wherein the N-terminal amino acid residue of the peptide compound is a cyclic amino acid residue, and the C-terminal amino acid residue is a homophenylalanine derivative residue.
[42] The method according to any of [1] to [35], wherein the N-terminal amino acid residue of the peptide compound is a cyclic amino acid residue, and the C-terminal amino acid residue is an N-substituted Ala residue.
[43] The method according to any of [2] to [35], wherein the N-terminal amino acid residue of the peptide compound is an N-substituted Gly residue, and the C-terminal amino acid residue is an N-substituted phenylalanine residue or an N-substituted phenylalanine derivative residue.
[43-1] The method according to any of [2] to [35], wherein the N-terminal amino acid residue of the peptide compound

is an N-substituted Gly residue, and the C-terminal amino acid residue is an N-substituted phenylalanine derivative residue.

[44] The method according to any of [1] to [42], wherein the cyclic amino acid residue is one selected from a Pro residue, a proline derivative residue, and an Aze(2) residue.

[45] The method according to any of [1] to [42], wherein the cyclic amino acid residue is one selected from a Pro residue and an Aze(2) residue.

[46] The method according to any of [1] to [42], wherein the cyclic amino acid residue is a Pro residue.

[47] The method according to any of [1] to [42], wherein the cyclic amino acid residue is an Aze(2) residue.

[48] The method according to any of [2] to [39] and [43], wherein the N-substituted phenylalanine derivative residue is an N-alkylphenylalanine derivative residue.

[49] The method according to [48], wherein the N-alkylphenylalanine derivative residue is an EtPhe derivative residue.

[50] The method according to [49], wherein the EtPhe derivative residue is an EtPhe(4-Me) residue.

[51] The method according to any of [2] to [38] and [41], wherein the homophenylalanine derivative residue is a Hph(3,5-diF-4-$CF_3$) residue.

[52] The method according to any of [2] to [38] and [40], wherein the $\alpha,\alpha$-disubstituted amino acid residue is a cLeu residue.

[53] The method according to any of [2] to [38] and [42], wherein the N-substituted Ala residue is an N-alkyl Ala residue.

[54] The method according to [53], wherein the N-alkyl Ala residue is a MeAla residue.

[55] The method according to any of [2] to [37] and [43], wherein the N-substituted Gly residue is an N-alkyl Gly residue.

[56] The method according to [55], wherein the N-alkyl Gly residue is a MeGly residue.

[57] The method according to any of [2] to [37], wherein the N-terminal amino acid residue and the C-terminal amino acid residue contained in the peptide compound are one selected from the following a') to e'):

a') the N-terminal amino acid residue is an EtPhe(4-Me) residue, and the C-terminal amino acid residue is an Aze(2) residue;

b') the N-terminal amino acid residue is a cLeu residue, and the C-terminal amino acid residue is a Pro residue;

c') the N-terminal amino acid residue is a Pro residue, and the C-terminal amino acid residue is a Hph(3,5-diF-4-$CF_3$) residue;

d') the N-terminal amino acid residue is a MeGly residue, and the C-terminal amino acid residue is an EtPhe(4-Me) residue; and

e') the N-terminal amino acid residue is an Aze(2) residue, and the C-terminal amino acid residue is a MeAla residue.

[58] The method according to any of [1] to [57], wherein the number of amino acid residues of the cyclic peptide compound is from 9 to 15.

[59] The method according to any of [1] to [57], wherein the number of amino acid residues of the cyclic peptide compound is 11.

[60] The method according to any of [1] to [59], wherein the peptide compound is a linear peptide compound.

[61] The method according to [60], wherein the number of amino acid residues of the linear peptide compound is from 9 to 15.

[62] The method according to [60], wherein the number of amino acid residues of the linear peptide compound is 11.

[63] The method according to any of [1] to [57], wherein the peptide compound is a linear peptide compound selected from the group consisting of:

[Formula 2]

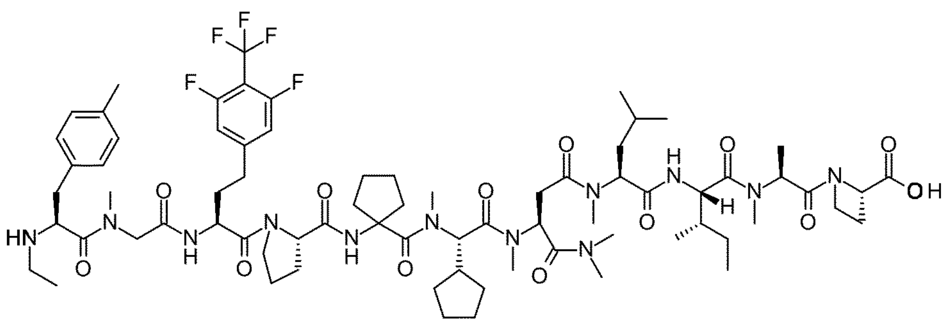

,

[Formula 3]

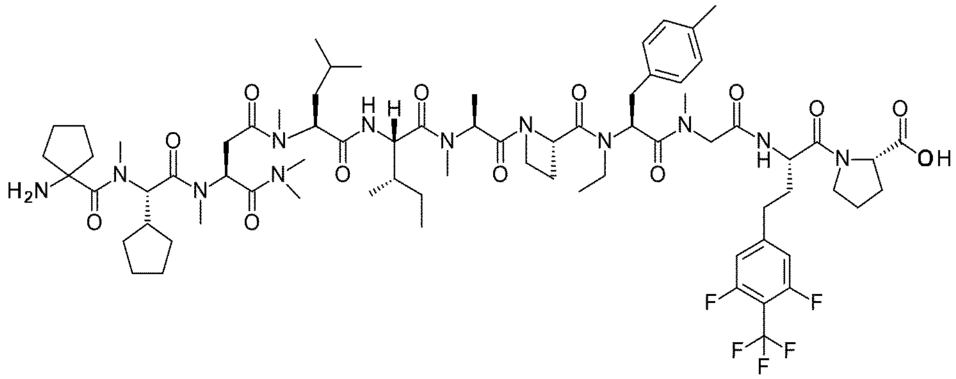

,

[Formula 4]

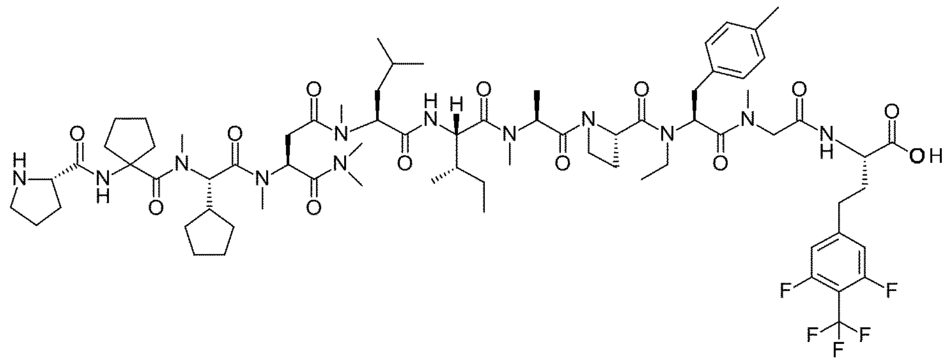

,

[Formula 5]

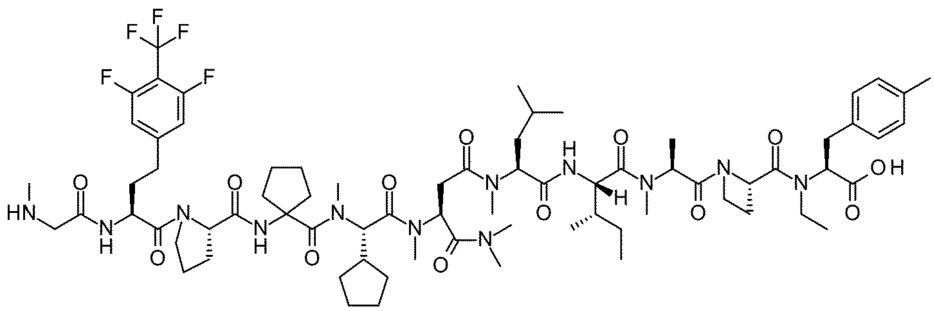

,

and

[Formula 6]

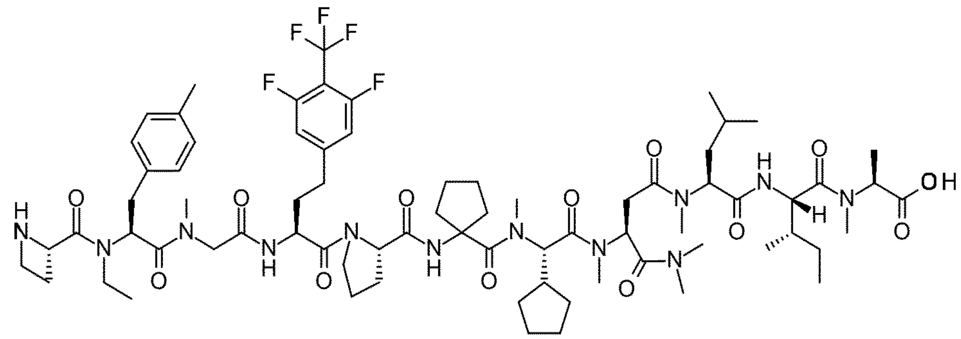

, or a salt thereof, or a solvate thereof.

[64] The method according to any of [1] to [63], wherein the cyclic peptide compound, or a salt thereof, or a solvate thereof is a solvate of the cyclic peptide compound.

[65] The method according to [64], wherein the solvate of the cyclic peptide compound is a hydrate of the cyclic peptide compound.

[66] The method according to any of [1] to [65], wherein the cyclic peptide compound is represented by the following formula (2):

[Formula 7]

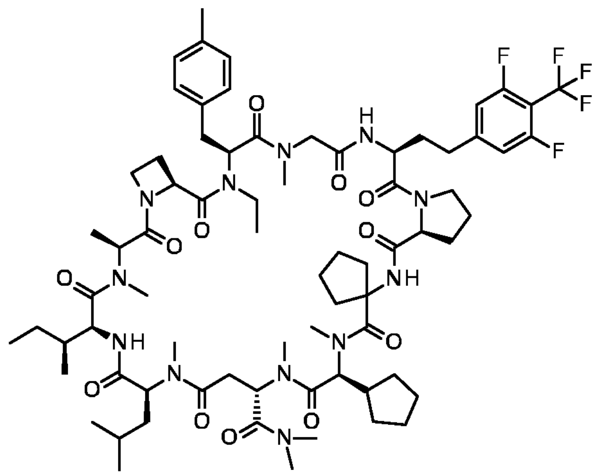

(2)

[66-1] The method according to any of [1] to [66], wherein column chromatography is not used for isolation and/or purification of the cyclic peptide compound.

[66-2] The method according to any of [1] to [66-1], further comprising a step of isolating and/or purifying the cyclic peptide compound by crystallization to obtain a crystal of the cyclic peptide compound.

[66-3] The method according to [66-2], wherein the crystal of the cyclic peptide compound is a non-solvate crystal or a solvate crystal of the cyclic peptide compound represented by the following formula (2):

[Formula 8]

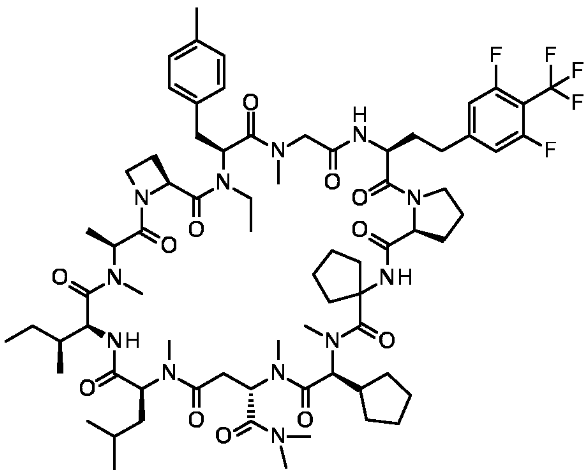

(2)

[66-4] The method according to [66-3], wherein the crystal of the cyclic peptide compound is a solvate crystal.
[66-5] The method according to [66-4], wherein the solvate crystal of the cyclic peptide compound is a hydrate crystal.

[67] The method according to any of [1] to [66], further comprising a step of providing the peptide compound.
[68] The method according to any of [1] to [67], wherein the linking step is performed by a liquid phase method.
[69] The method according to any of [1] to [68], wherein in the linking step, the peptide compound and the base are mixed in a mixed solution obtained by mixing the solvent and the condensation reagent.
[70] The method according to any of [1] to [69], wherein a content of the total byproducts formed in the linking step is less than 20%, less than 15%, less than 10%, less than 5%, or less than 3%, as determined by UVarea value at 220 nm using HPLC analysis, based on the total amount of products.
[71] The method according to any of [1] to [69], wherein a content of each of byproducts formed in the linking step is less than 15%, less than 10%, less than 5%, less than 3%, less than 1%, or an undetectable amount, as determined by UVarea value at 220 nm using HPLC analysis, based on the total amount of products.
[72] The method according to any of [1] to [69], wherein a content of each of byproducts formed in the linking step is less than 15%, less than 10%, less than 5%, less than 3%, less than 1%, or an undetectable amount, as determined by UVarea value at 220 nm using HPLC analysis, based on the total amount of products, and the byproducts are an epimer, a dimer, and a trimer.
[73] The method according to any of [1] to [69], wherein byproducts formed in the linking step comprise an epimer of the cyclic peptide compound, and a content of the epimer is less than 10%, less than 7.5%, less than 5%, less than 2.5%, or less than 1%, as determined by UVarea value at 220 nm using HPLC analysis, based on the total amount of products.
[74] The method according to any of [1] to [69], wherein byproducts formed in the linking step comprise a dimer, and a content of the dimer is less than 15%, less than 10%, less than 5%, less than 2.5%, or less than 1%, as determined by UVarea value at 220 nm using HPLC analysis, based on the total amount of products.
[75] The method according to any of [1] to [69], wherein byproducts formed in the linking step comprise a trimer, and a content of the trimer is less than 5%, less than 2.5%, less than 1%, or an undetectable amount, as determined by UVarea value at 220 nm using HPLC analysis, based on the total amount of products.
[76] The method according to any of [1] to [69], wherein byproducts formed in the linking step comprise a dimer and a trimer, and the total content of the dimer and the trimer is less than 15%, less than 10%, less than 5%, less than 2.5%, or less than 1%, as determined by UVarea value at 220 nm using HPLC analysis, based on the total amount of products.
[77] A method for producing a cyclic peptide compound represented by the following formula (2):

[Formula 10]

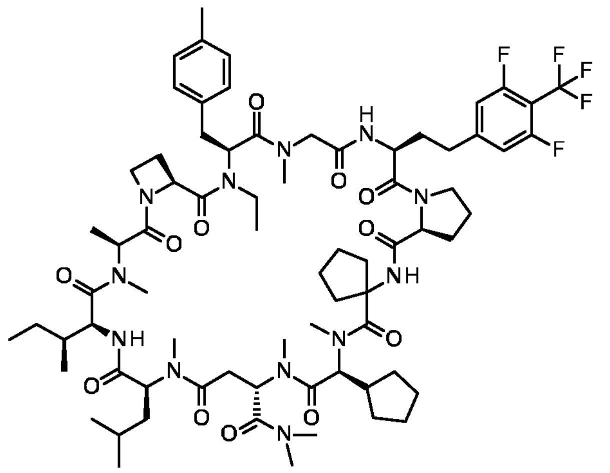

(2)

or a salt thereof, or a solvate thereof, the method comprising steps of:

(1) providing a linear peptide compound represented by the following formula:

[Formula 9]

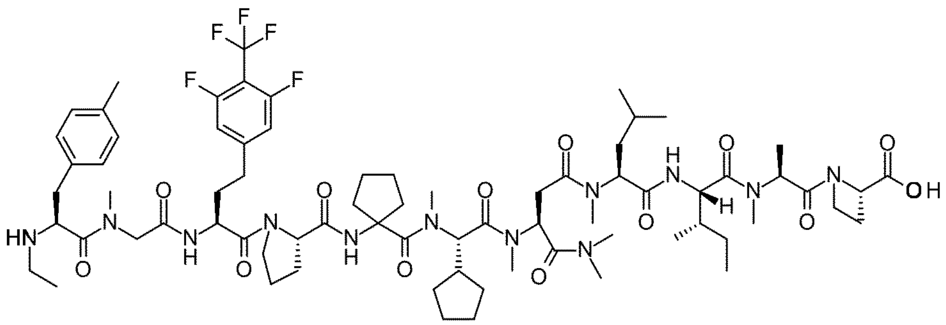

or a salt thereof, or a solvate thereof; and
(2) linking an N-terminal amino acid residue of the linear peptide compound, or a salt thereof, or a solvate thereof with a C-terminal amino acid residue of the linear peptide compound, or a salt thereof, or a solvate thereof.

[77-1] The method according to [77], wherein column chromatography is not used for isolation and/or purification of the cyclic peptide compound.
[77-2] The method according to any of [77] to [77-1], further comprising a step of isolating and/or purifying the cyclic peptide compound by crystallization to obtain a crystal of the cyclic peptide compound.
[77-3] The method according to [77-2], wherein the crystal of the cyclic peptide compound is a non-solvate crystal or a solvate crystal of the cyclic peptide compound represented by the following formula (2):

[Formula 11]

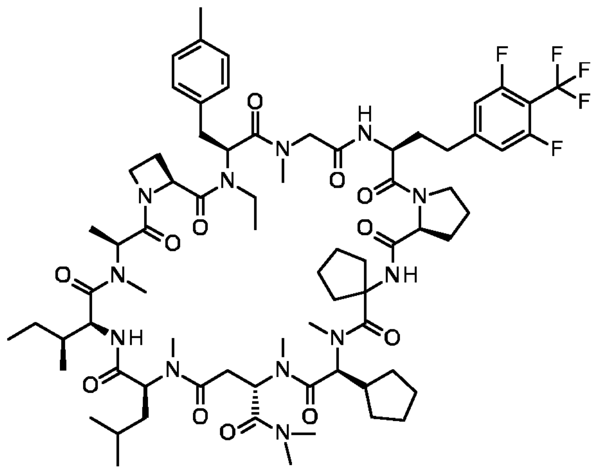

(2)

[77-4] The method according to [77-3], wherein the crystal of the cyclic peptide compound is a solvate crystal.

[77-5] The method according to [77-4], wherein the solvate crystal of the cyclic peptide compound is a hydrate crystal.

[77-6] The method according to [77], wherein the linking step is performed by a liquid phase method.

[78] The method according to [77], wherein the step (2) is performed in the presence of a solvent.

[79] The method according to [78], wherein the solvent comprises one or more selected from the group consisting of acetonitrile, dimethyl carbonate, and 2-MeTHF.

[80] The method according to [78], wherein the solvent is one selected from the group consisting of acetonitrile, dimethyl carbonate, and 2-MeTHF.

[81] The method according to [78], wherein the solvent is one selected from the group consisting of acetonitrile and dimethyl carbonate.

[82] The method according to any of [77] to [81], wherein the step (2) is performed in the presence of a condensation reagent.

[83] The method according to [82], wherein the condensation reagent is one selected from the group consisting of COMU, HATU, PyBOP, PyOxim, PyClop, DMT-MM, DEPBT, FDPP, T3P, and BEP-BF4.

[84] The method according to [82], wherein the condensation reagent is one selected from the group consisting of COMU and HATU.

[85] The method according to [82], wherein the condensation reagent is one selected from the group consisting of COMU and HATU, and the solvent comprises one or more selected from the group consisting of acetonitrile, dimethyl carbonate, and 2-MeTHF.

[86] The method according to [82], wherein the condensation reagent is one selected from the group consisting of COMU and HATU, and the solvent is one selected from the group consisting of acetonitrile and dimethyl carbonate.

[87] The method according to any of [77] to [86], wherein the linking step is performed in the presence of a base.

[88] The method according to [87], wherein the base is an organic base.

[89] The method according to [87], wherein the base is an organic base comprising a tertiary amine.

[90] The method according to [87], wherein the base is one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP).

[91] The method according to [87], wherein the base is N,N-diisopropylethylamine (DIPEA).

[92] The method according to [87], wherein the condensation reagent is one selected from the group consisting of COMU, HATU, PyBOP, PyOxim, PyClop, DMT-MM, DEPBT, FDPP, T3P, and BEP-BF4, the solvent comprises one or more selected from the group consisting of acetonitrile, dimethyl carbonate, and 2-MeTHF, and the base is N,N-diisopropylethylamine (DIPEA).

[93] The method according to [87], wherein the condensation reagent is one selected from the group consisting of COMU and HATU, the solvent comprises one or more selected from the group consisting of acetonitrile, dimethyl carbonate, and 2-MeTHF, and the base is N,N-diisopropylethylamine (DIPEA).

[94] The method according to [87], wherein the condensation reagent is one selected from the group consisting of COMU and HATU, the solvent is one selected from the group consisting of acetonitrile and dimethyl carbonate, and the base is N,N-diisopropylethylamine (DIPEA).
[95] The method according to any of [77] to [94], wherein in the step (2), the linear peptide compound, or a salt thereof, or a solvate thereof, and the base are mixed in a mixed solution obtained by mixing the solvent and the condensation reagent.
[96] The method according to any of [77] to [95], wherein a content of the total byproducts formed in the step is less than 10%, less than 7.5%, less than 5%, less than 3%, or less than 1%, as determined by UVarea value at 220 nm using HPLC analysis, based on the total amount of products.
[97] The method according to any of [77] to [95], wherein a content of each of byproducts formed in the step is less than 10%, less than 7.5%, less than 5%, less than 3%, less than 1%, or an undetectable amount, as determined by UVarea value at 220 nm using HPLC analysis, based on the total amount of products.
[98] The method according to any of [77] to [95], wherein a content of each of byproducts formed in the step is less than 10%, less than 7.5%, less than 5%, less than 3%, less than 1%, or an undetectable amount, as determined by UVarea value at 220 nm using HPLC analysis, based on the total amount of products, and the byproducts are an epimer, a dimer, and a trimer.
[99] The method according to any of [77] to [95], wherein byproducts formed in the step comprise an epimer of the cyclic peptide compound, and a content of the epimer is less than 3%, less than 1%, or an undetectable value, as determined by UVarea value at 220 nm using HPLC analysis, based on the total amount of products.
[100] The method according to any of [77] to [95], wherein byproducts formed in the step comprise a dimer, and a content of the dimer is less than 5%, less than 3%, or less than 1%, as determined by UVarea value at 220 nm using HPLC analysis, based on the total amount of products.
[101] The method according to any of [77] to [95], wherein byproducts formed in the step comprise a trimer, and a content of the trimer is less than 3%, less than 1%, or an undetectable amount, as determined by UVarea value at 220 nm using HPLC analysis, based on the total amount of products.
[102] The method according to any of [77] to [95], wherein byproducts formed in the step comprise a dimer and a trimer, and the total content of the dimer and the trimer is less than 5%, less than 3%, or less than 1%, as determined by UVarea value at 220 nm using HPLC analysis, based on the total amount of products.
[103] A method for producing a cyclic peptide compound represented by the following formula (2):

[Formula 13]

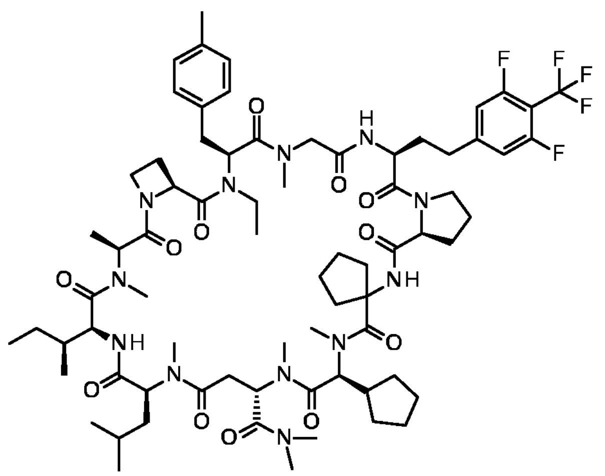

(2)

or a salt thereof, or a solvate thereof, the method comprising steps of:

(1) providing a linear peptide compound represented by the following formula:

[Formula 12]

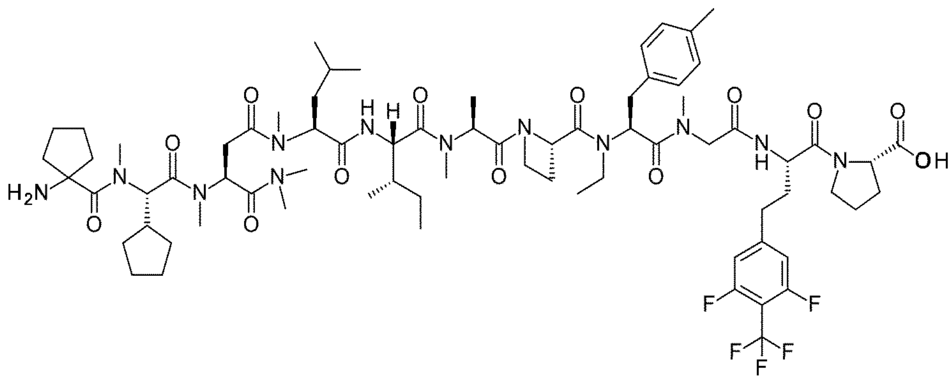

or a salt thereof, or a solvate thereof; and
(2) linking an N-terminal amino acid residue of the linear peptide compound, or a salt thereof, or a solvate thereof with a C-terminal amino acid residue of the linear peptide compound, or a salt thereof, or a solvate thereof.

[103-1] The method according to [103], wherein column chromatography is not used for isolation and/or purification of the cyclic peptide compound.
[103-2] The method according to any of [103] to [103-1], further comprising a step of isolating and/or purifying the cyclic peptide compound by crystallization to obtain a crystal of the cyclic peptide compound.
[103-3] The method according to [103-2], wherein the crystal of the cyclic peptide compound is a non-solvate crystal or a solvate crystal of the cyclic peptide compound represented by the following formula (2):

[Formula 14]

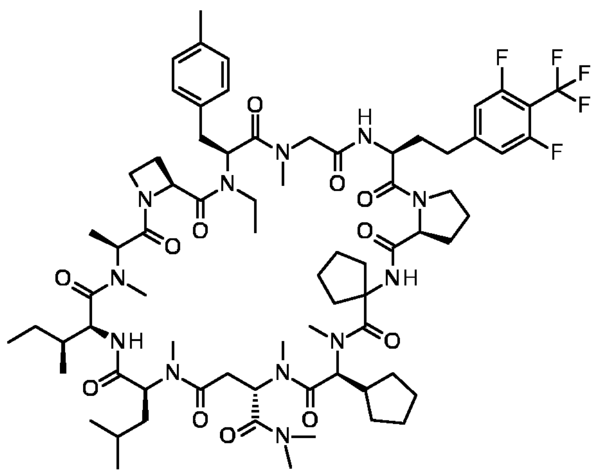

(2)

[103-4] The method according to [103-3], wherein the crystal of the cyclic peptide compound is a solvate crystal.
[103-5] The method according to [103-4], wherein the solvate crystal of the cyclic peptide compound is a hydrate crystal.
[103-6] The method according to [103], wherein the linking step is performed by a liquid phase method.

[104] The method according to [103], wherein the step (2) is performed in the presence of a solvent.
[105] The method according to [104], wherein the solvent comprises one or more selected from the group consisting of acetonitrile, dimethyl carbonate, 2-MeTHF, and anisole.
[106] The method according to [104], wherein the solvent is one selected from the group consisting of acetonitrile, dimethyl carbonate, 2-MeTHF, and anisole.
[107] The method according to [104], wherein the solvent is dimethyl carbonate.
[108] The method according to any of [103] to [107], wherein the step (2) is performed in the presence of a condensation reagent.

[109] The method according to [108], wherein the condensation reagent is one selected from the group consisting of COMU, HATU, PyBOP, PyOxim, PyClop, and DMT-MM.

[110] The method according to [108], wherein the condensation reagent is one selected from the group consisting of COMU and HATU.

[111] The method according to [108], wherein the condensation reagent is COMU.

[112] The method according to [108], wherein the condensation reagent is one selected from the group consisting of COMU, HATU, PyBOP, PyOxim, PyClop, and DMT-MM, and the solvent comprises one or more selected from the group consisting of acetonitrile, dimethyl carbonate, 2-MeTHF, and anisole.

[113] The method according to [108], wherein the condensation reagent is COMU, and the solvent is one selected from the group consisting of acetonitrile and dimethyl carbonate.

[114] The method according to [108], wherein the condensation reagent is COMU, and the solvent is dimethyl carbonate.

[115] The method according to any of [103] to [114], wherein the linking step is performed in the presence of a base.

[116] The method according to [115], wherein the base is an organic base.

[117] The method according to [115], wherein the base is an organic base comprising a tertiary amine.

[118] The method according to [115], wherein the base is one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP).

[119] The method according to [115], wherein the base is N,N-diisopropylethylamine (DIPEA).

[120] The method according to [115], wherein the condensation reagent is COMU, the solvent is one selected from the group consisting of acetonitrile and dimethyl carbonate, and the base is N,N-diisopropylethylamine (DIPEA).

[121] The method according to [115], wherein the condensation reagent is COMU, the solvent is dimethyl carbonate, and the base is N,N-diisopropylethylamine (DIPEA).

[122] The method according to any of [103] to [121], wherein in the step (2), the linear peptide compound, or a salt thereof, or a solvate thereof, and the base are mixed in a mixed solution obtained by mixing the solvent and the condensation reagent.

[123] The method according to any of [103] to [122], wherein a content of the total byproducts formed in the step is less than 20%, less than 15%, less than 10%, less than 5%, or less than 3%, as determined by UVarea value at 220 nm using HPLC analysis, based on the total amount of products.

[124] The method according to any of [103] to [122], wherein a content of each of byproducts formed in the step is less than 15%, less than 10%, less than 7.5%, less than 5%, less than 2.5%, or less than 1%, as determined by UVarea value at 220 nm using HPLC analysis, based on the total amount of products.

[125] The method according to any of [103] to [122], wherein a content of each of byproducts formed in the step is less than 15%, less than 10%, less than 7.5%, less than 5%, less than 2.5%, or less than 1%, as determined by UVarea value at 220 nm using HPLC analysis, based on the total amount of products, and the byproducts are an epimer, a dimer, and a trimer.

[126] The method according to any of [103] to [122], wherein byproducts formed in the step comprise an epimer of the cyclic peptide compound, and a content of the epimer is less than 3%, less than 1%, or an undetectable value, as determined by UVarea value at 220 nm using HPLC analysis, based on the total amount of products.

[127] The method according to any of [103] to [122], wherein byproducts formed in the step comprise a dimer, and a content of the dimer is less than 15%, less than 10%, or less than 5%, as determined by UVarea value at 220 nm using HPLC analysis, based on the total amount of products.

[128] The method according to any of [103] to [122], wherein byproducts formed in the step comprise a trimer, and a content of the trimer is less than 5%, less than 3%, or less than 1%, as determined by UVarea value at 220 nm using HPLC analysis, based on the total amount of products.

[129] The method according to any of [103] to [122], wherein byproducts formed in the step comprise a dimer and a trimer, and the total content of the dimer and the trimer is less than 20%, less than 15%, less than 10%, less than 5%, or less than 3%, as determined by UVarea value at 220 nm using HPLC analysis, based on the total amount of products.

[130] A method for producing a cyclic peptide compound represented by the following formula (2):

[Formula 16]

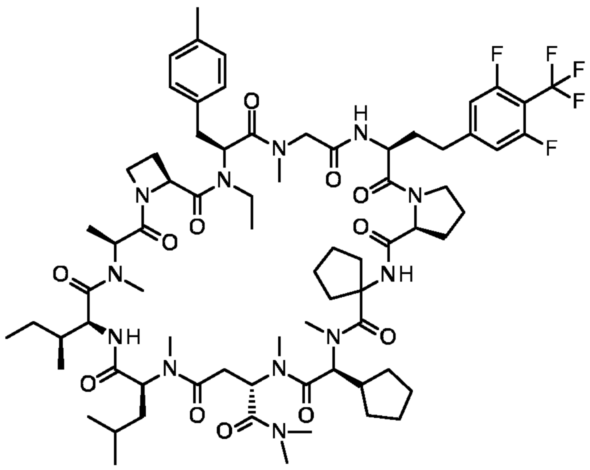

(2)

or a salt thereof, or a solvate thereof, the method comprising steps of:

(1) providing a linear peptide compound represented by the following formula:

[Formula 15]

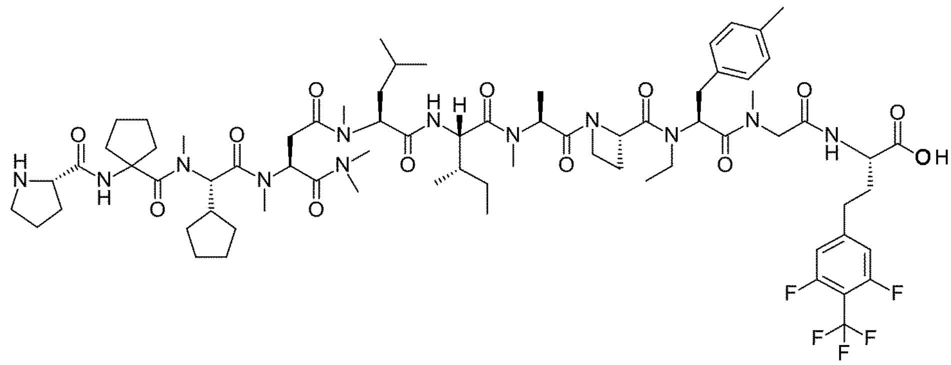

or a salt thereof, or a solvate thereof; and

(2) linking an N-terminal amino acid residue of the linear peptide compound, or a salt thereof, or a solvate thereof with a C-terminal amino acid residue of the linear peptide compound, or a salt thereof, or a solvate thereof.

[130-1] The method according to [130], wherein column chromatography is not used for isolation and/or purification of the cyclic peptide compound.

[130-2] The method according to any of [130] to [130-1], further comprising a step of isolating and/or purifying the cyclic peptide compound by crystallization to obtain a crystal of the cyclic peptide compound.

[130-3] The method according to [130-2], wherein the crystal of the cyclic peptide compound is a non-solvate crystal or a solvate crystal of the cyclic peptide compound represented by the following formula (2):

[Formula 17]

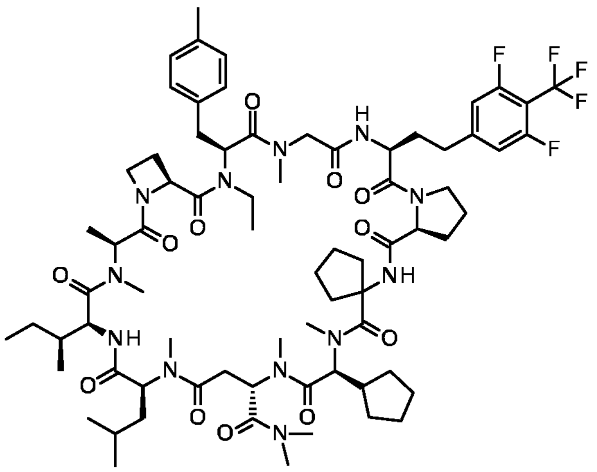

(2)

[130-4] The method according to [130-3], wherein the crystal of the cyclic peptide compound is a solvate crystal.
[130-5] The method according to [130-4], wherein the solvate crystal of the cyclic peptide compound is a hydrate crystal.
[130-6] The method according to [130], wherein the linking step is performed by a liquid phase method.

[131] The method according to [130], wherein the step (2) is performed in the presence of a solvent.
[132] The method according to [131], wherein the solvent comprises one or more selected from the group consisting of acetonitrile, dimethyl carbonate, 2-MeTHF, and anisole.
[133] The method according to [131], wherein the solvent is one selected from the group consisting of acetonitrile, dimethyl carbonate, 2-MeTHF, and anisole.
[134] The method according to [131], wherein the solvent is dimethyl carbonate.
[135] The method according to any of [130] to [134], wherein the step (2) is performed in the presence of a condensation reagent.
[136] The method according to [135], wherein the condensation reagent is one selected from the group consisting of COMU, HATU, PyBOP, PyOxim, PyClop, and DMT-MM.
[137] The method according to [135], wherein the condensation reagent is one selected from the group consisting of COMU and HATU.
[138] The method according to [135], wherein the condensation reagent is COMU.
[139] The method according to [135], wherein the condensation reagent is one selected from the group consisting of COMU, HATU, PyBOP, PyOxim, PyClop, and DMT-MM, and the solvent comprises one or more selected from the group consisting of acetonitrile, dimethyl carbonate, 2-MeTHF, and anisole.
[140] The method according to [135], wherein the condensation reagent is one selected from the group consisting of COMU and HATU, and the solvent is one selected from the group consisting of acetonitrile and dimethyl carbonate.
[141] The method according to [135], wherein the condensation reagent is COMU, and the solvent is dimethyl carbonate.
[142] The method according to any of [130] to [141], wherein the linking step is performed in the presence of a base.
[143] The method according to [142], wherein the base is an organic base.
[144] The method according to [142], wherein the base is an organic base comprising a tertiary amine.
[145] The method according to [142], wherein the base is one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP).
[146] The method according to [142], wherein the base is N,N-diisopropylethylamine (DIPEA).
[147] The method according to [142], wherein the condensation reagent is one selected from the group consisting of COMU and HATU, the solvent is one selected from the group consisting of acetonitrile and dimethyl carbonate, and the base is N,N-diisopropylethylamine (DIPEA).
[148] The method according to [142], wherein the condensation reagent is COMU, the solvent is dimethyl carbonate, and the base is N,N-diisopropylethylamine (DIPEA).

[149] The method according to any of [130] to [148], wherein in the step (2), the linear peptide compound, or a salt thereof, or a solvate thereof, and the base are mixed in a mixed solution obtained by mixing the solvent and the condensation reagent.
[150] The method according to any of [130] to [149], wherein a content of the total byproducts formed in the step is less than 10%, less than 7.5%, less than 5%, or less than 2.5%, as determined by UVarea value at 220 nm using HPLC analysis, based on the total amount of products.
[151] The method according to any of [130] to [149], wherein a content of each of byproducts formed in the step is less than 10%, less than 7.5%, less than 5%, less than 3%, less than 1%, or an undetectable amount, as determined by UVarea value at 220 nm using HPLC analysis, based on the total amount of products.
[152] The method according to any of [130] to [149], wherein a content of each of byproducts formed in the step is less than 10%, less than 7.5%, less than 5%, less than 3%, less than 1%, or an undetectable amount, as determined by UVarea value at 220 nm using HPLC analysis, based on the total amount of products, and the byproducts are an epimer, a dimer, and a trimer.
[153] The method according to any of [130] to [149], wherein byproducts formed in the step comprise an epimer of the cyclic peptide compound, and a content of the epimer is less than 7.5%, less than 5%, less than 3%, or less than 1%, as determined by UVarea value at 220 nm using HPLC analysis, based on the total amount of products.
[154] The method according to any of [130] to [149], wherein byproducts formed in the step comprise a dimer, and a content of the dimer is less than 10%, less than 7.5%, less than 5%, less than 2.5%, or less than 1%, as determined by UVarea value at 220 nm using HPLC analysis, based on the total amount of products.
[155] The method according to any of [130] to [149], wherein byproducts formed in the step comprise a trimer, and a content of the trimer is less than 3%, less than 1%, or an undetectable amount, as determined by UVarea value at 220 nm using HPLC analysis, based on the total amount of products.
[156] The method according to any of [130] to [149], wherein byproducts formed in the step comprise a dimer and a trimer, and the total content of the dimer and the trimer is less than 10%, less than 7.5%, less than 5%, less than 2.5%, or less than 1%, as determined by UVarea value at 220 nm using HPLC analysis, based on the total amount of products.
[157] A method for producing a cyclic peptide compound represented by the following formula (2):

[Formula 19]

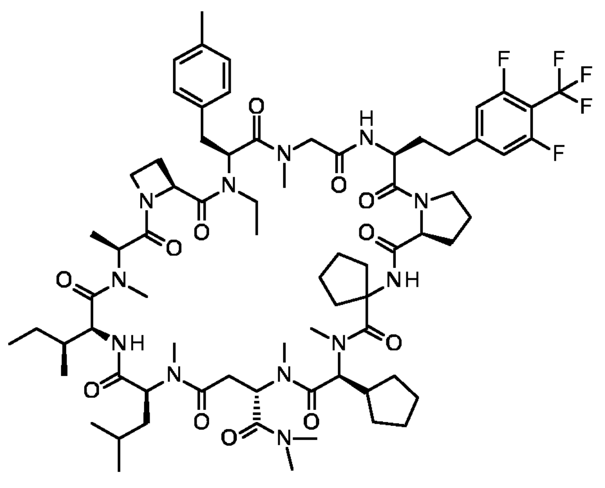

(2)

or a salt thereof, or a solvate thereof, the method comprising steps of:

(1) providing a linear peptide compound represented by the following formula:

[Formula 18]

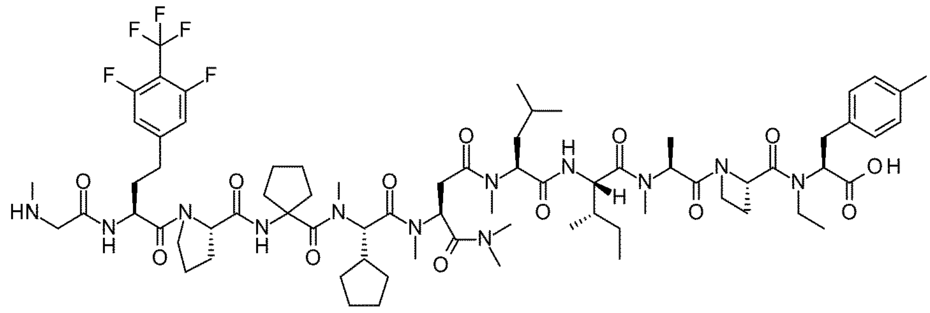

or a salt thereof, or a solvate thereof; and

(2) linking an N-terminal amino acid residue of the linear peptide compound, or a salt thereof, or a solvate thereof with a C-terminal amino acid residue of the linear peptide compound, or a salt thereof, or a solvate thereof.

[157-1] The method according to [157], wherein column chromatography is not used for isolation and/or purification of the cyclic peptide compound.

[157-2] The method according to any of [157] to [157-1], further comprising a step of isolating and/or purifying the cyclic peptide compound by crystallization to obtain a crystal of the cyclic peptide compound.

[157-3] The method according to [157-2], wherein the crystal of the cyclic peptide compound is a non-solvate crystal or a solvate crystal of the cyclic peptide compound represented by the following formula (2):

[Formula 20]

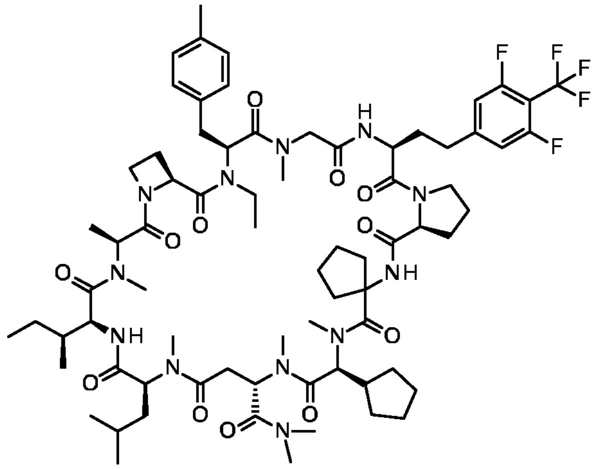

(2)

[157-4] The method according to [157-3], wherein the crystal of the cyclic peptide compound is a solvate crystal.

[157-5] The method according to [157-4], wherein the solvate crystal of the cyclic peptide compound is a hydrate crystal.

[157-6] The method according to [157], wherein the linking step is performed by a liquid phase method.

[158] The method according to [157], wherein the step (2) is performed in the presence of a solvent.

[159] The method according to [158], wherein the solvent comprises one or two selected from acetonitrile and dimethyl carbonate.

[160] The method according to [158], wherein the solvent is dimethyl carbonate.

[161] The method according to any of [157] to [160], wherein the step (2) is performed in the presence of a condensation reagent.

[162] The method according to [161], wherein the condensation reagent is one selected from the group consisting of COMU, HATU, PyBOP, PyOxim, PyClop, DMT-MM, and DEPBT.

[163] The method according to [161], wherein the condensation reagent is one selected from the group consisting of COMU and HATU.

[164] The method according to [161], wherein the condensation reagent is HATU.

[165] The method according to [161], wherein the condensation reagent is one selected from the group consisting of COMU, HATU, PyBOP, PyOxim, PyClop, DMT-MM, and DEPBT, and the solvent comprises one or two selected from the group consisting of acetonitrile and dimethyl carbonate.
[165-1] The method according to [161], wherein the condensation reagent is one selected from the group consisting of COMU and HATU, and the solvent is one selected from the group consisting of acetonitrile and dimethyl carbonate.
[166] The method according to [161], wherein the condensation reagent is one selected from the group consisting of COMU and HATU, and the solvent is dimethyl carbonate.
[167] The method according to [161], wherein the condensation reagent is HATU, and the solvent is dimethyl carbonate.
[168] The method according to any of [157] to [167], wherein the linking step is performed in the presence of a base.
[169] The method according to [168], wherein the base is an organic base.
[170] The method according to [168], wherein the base is an organic base comprising a tertiary amine.
[171] The method according to [168], wherein the base is one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP).
[172] The method according to [168], wherein the base is N,N-diisopropylethylamine (DIPEA).
[172-1] The method according to [168], wherein the condensation reagent is one selected from the group consisting of COMU and HATU, the solvent is one selected from the group consisting of acetonitrile and dimethyl carbonate, and the base is N,N-diisopropylethylamine (DIPEA).
[173] The method according to [168], wherein the condensation reagent is one selected from the group consisting of COMU and HATU, the solvent is dimethyl carbonate, and the base is N,N-diisopropylethylamine (DIPEA).
[174] The method according to [168], wherein the condensation reagent is HATU, the solvent is dimethyl carbonate, and the base is N,N-diisopropylethylamine (DIPEA).
[175] The method according to any of [157] to [174], wherein in the step (2), the linear peptide compound, or a salt thereof, or a solvate thereof, and the base are mixed in a mixed solution obtained by mixing the solvent and the condensation reagent.
[176] The method according to any of [157] to [175], wherein a content of the total byproducts formed in the step is less than 10%, less than 7.5%, less than 5%, or less than 2.5%, as determined by UVarea value at 220 nm using HPLC analysis, based on the total amount of products.
[177] The method according to any of [157] to [175], wherein a content of each of byproducts formed in the step is less than 10%, less than 7.5%, less than 5%, less than 3%, less than 1%, or an undetectable amount, as determined by UVarea value at 220 nm using HPLC analysis, based on the total amount of products.
[178] The method according to any of [157] to [175], wherein a content of each of byproducts formed in the step is less than 10%, less than 7.5%, less than 5%, less than 3%, less than 1%, or an undetectable amount, as determined by UVarea value at 220 nm using HPLC analysis, based on the total amount of products, and the byproducts are an epimer, a dimer, and a trimer.
[179] The method according to any of [157] to [175], wherein byproducts formed in the step comprise an epimer of the cyclic peptide compound, and a content of the epimer is less than 7.5%, less than 5%, less than 3%, or less than 1%, as determined by UVarea value at 220 nm using HPLC analysis, based on the total amount of products.
[180] The method according to any of [157] to [175], wherein byproducts formed in the step comprise a dimer, and a content of the dimer is less than 10%, less than 7.5%, less than 5%, less than 2.5%, or less than 1%, as determined by UVarea value at 220 nm using HPLC analysis, based on the total amount of products.
[181] The method according to any of [157] to [175], wherein byproducts formed in the step comprise a trimer, and a content of the trimer is less than 3%, less than 1%, or an undetectable amount, as determined by UVarea value at 220 nm using HPLC analysis, based on the total amount of products.
[182] The method according to any of [157] to [175], wherein byproducts formed in the step comprise a dimer and a trimer, and the total content of the dimer and the trimer is less than 10%, less than 7.5%, less than 5%, less than 2.5%, or less than 1%, as determined by UVarea value at 220 nm using HPLC analysis, based on the total amount of products.
[183] A method for producing a cyclic peptide compound represented by the following formula (2):

[Formula 22]

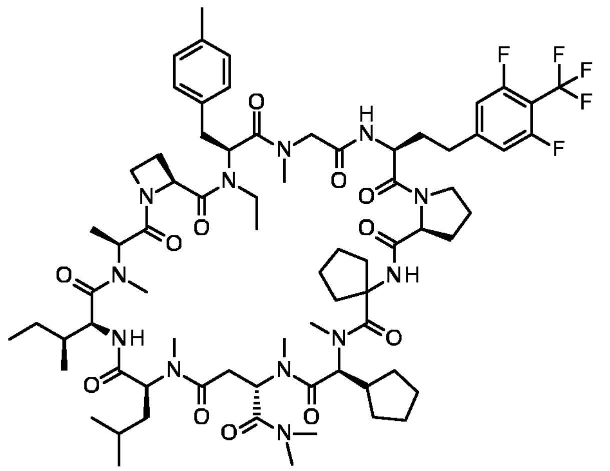

(2)

or a salt thereof, or a solvate thereof, the method comprising steps of:

(1) providing a linear peptide compound represented by the following formula:

[Formula 21]

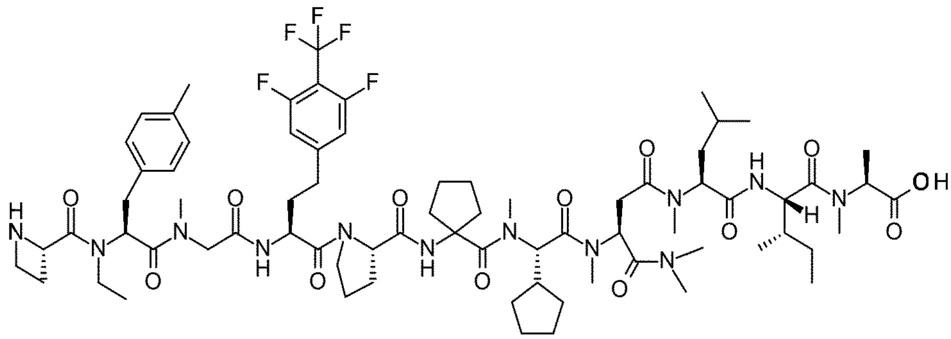

or a salt thereof, or a solvate thereof; and
(2) linking an N-terminal amino acid residue of the linear peptide compound, or a salt thereof, or a solvate thereof with a C-terminal amino acid residue of the linear peptide compound, or a salt thereof, or a solvate thereof.

[183-1] The method according to [182], wherein column chromatography is not used for isolation and/or purification of the cyclic peptide compound.
[183-2] The method according to any of [183] to [183-1], further comprising a step of isolating and/or purifying the cyclic peptide compound by crystallization to obtain a crystal of the cyclic peptide compound.
[183-3] The method according to [183-2], wherein the crystal of the cyclic peptide compound is a non-solvate crystal or a solvate crystal of the cyclic peptide compound represented by the following formula (2):

[Formula 23]

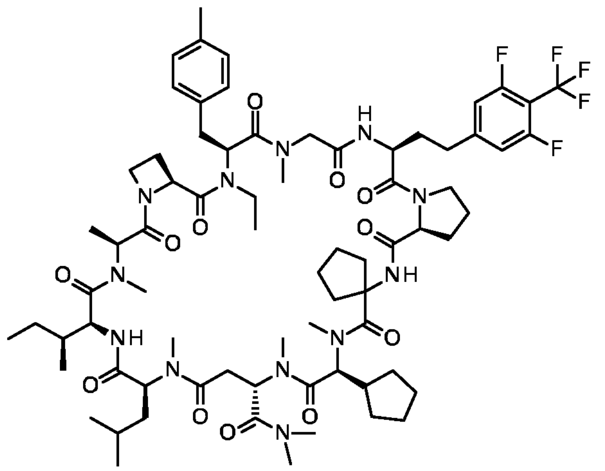

(2)

[183-4] The method according to [183-3], wherein the crystal of the cyclic peptide compound is a solvate crystal.
[183-5] The method according to [183-4], wherein the solvate crystal of the cyclic peptide compound is a hydrate crystal.
[183-6] The method according to [183], wherein the linking step is performed by a liquid phase method.

[184] The method according to [183], wherein the step (2) is performed in the presence of a solvent.
[185] The method according to [184], wherein the solvent comprises one or two selected from the group consisting of acetonitrile and dimethyl carbonate.
[186] The method according to [184], wherein the solvent is acetonitrile.
[187] The method according to any of [183] to [186], wherein the step (2) is performed in the presence of a condensation reagent.
[188] The method according to [187], wherein the condensation reagent is one selected from the group consisting of COMU, HATU, PyBOP, PyOxim, PyClop, and DMT-MM.
[189] The method according to [187], wherein the condensation reagent is one selected from the group consisting of COMU, HATU, and PyOxim.
[190] The method according to [187], wherein the condensation reagent is COMU.
[191] The method according to [187], wherein the condensation reagent is one selected from the group consisting of COMU, HATU, PyBOP, PyOxim, PyClop, and DMT-MM, and the solvent comprises one or two selected from the group consisting of acetonitrile and dimethyl carbonate.
[192] The method according to [187], wherein the condensation reagent is one selected from the group consisting of COMU, HATU, and PyOxim, and the solvent is one selected from the group consisting of acetonitrile and dimethyl carbonate.
[193] The method according to [187], wherein the condensation reagent is COMU, and the solvent is one selected from the group consisting of acetonitrile and dimethyl carbonate.

[193-1] The method according to [187], wherein the condensation reagent is COMU, and the solvent is acetonitrile.
[193-2] The method according to [187], wherein the condensation reagent is HATU, and the solvent is dimethyl carbonate.

[194] The method according to [187], wherein the condensation reagent is PyOxim, and the solvent is dimethyl carbonate.
[195] The method according to any of [183] to [194], wherein the linking step is performed in the presence of a base.
[196] The method according to [195], wherein the base is an organic base.
[197] The method according to [195], wherein the base is an organic base comprising a tertiary amine.
[198] The method according to [195], wherein the base is one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP).
[199] The method according to [195], wherein the base is N,N-diisopropylethylamine (DIPEA).
[200] The method according to [195], wherein the condensation reagent is one selected from the group consisting of COMU, HATU, and PyOxim, the solvent is one selected from the group consisting of acetonitrile and dimethyl carbonate, and the base is N,N-diisopropylethylamine (DIPEA).
[201] The method according to [195], wherein the condensation reagent is COMU, the solvent is one selected from the group consisting of acetonitrile and dimethyl carbonate, and the base is N,N-diisopropylethylamine (DIPEA).

[201-1] The method according to [195], wherein the condensation reagent is COMU, the solvent is acetonitrile, and the base is N,N-diisopropylethylamine (DIPEA).
[201-2] The method according to [195], wherein the condensation reagent is HATU, the solvent is dimethyl carbonate, and the base is N,N-diisopropylethylamine (DIPEA).

[202] The method according to [195], wherein the condensation reagent is PyOxim, the solvent is dimethyl carbonate, and the base is N,N-diisopropylethylamine (DIPEA).
[203] The method according to any of [183] to [202], wherein in the step (2), the linear peptide compound, or a salt thereof, or a solvate thereof, and the base are mixed in a mixed solution obtained by mixing the solvent and the condensation reagent.
[204] The method according to any of [183] to [203], wherein a content of the total byproducts formed in the step is less than 15%, less than 10%, less than 7.5%, or less than 5%, as determined by UVarea value at 220 nm using HPLC analysis, based on the total amount of products.
[205] The method according to any of [183] to [203], wherein a content of each of byproducts formed in the step is less than 10%, less than 7.5%, less than 5%, less than 3%, less than 1%, or an undetectable amount, as determined by UVarea value at 220 nm using HPLC analysis, based on the total amount of products.
[206] The method according to any of [183] to [203], wherein a content of each of byproducts formed in the step is less than 10%, less than 7.5%, less than 5%, less than 3%, less than 1%, or an undetectable amount, as determined by UVarea value at 220 nm using HPLC analysis, based on the total amount of products, and the byproducts are an epimer, a dimer, and a trimer.
[207] The method according to any of [183] to [203], wherein byproducts formed in the step comprise an epimer of the cyclic peptide compound, and a content of the epimer is less than 5%, less than 3%, or less than 1%, as determined by UVarea value at 220 nm using HPLC analysis, based on the total amount of products.
[208] The method according to any of [183] to [203], wherein byproducts formed in the step comprise a dimer, and a content of the dimer is less than 10%, less than 7.5%, or less than 5%, as determined by UVarea value at 220 nm by HPLC analysis, based on the total amount of products.
[209] The method according to any of [183] to [203], wherein byproducts formed in the step comprise a trimer, and a content of the trimer is less than 3%, less than 1%, or an undetectable amount, as determined by UVarea value at 220 nm by HPLC analysis, based on the total amount of products.
[210] The method according to any of [183] to [203], wherein byproducts formed in the step comprise a dimer and a trimer, and the total content of the dimer and the trimer is less than 10%, less than 7.5%, or less than 5%, as determined by UVarea value at 220 nm by HPLC analysis, based on the total amount of products.
[211] A method of suppressing epimerization of a cyclic peptide compound in production of the cyclic peptide compound, or a salt thereof, or a solvate thereof, the method comprising

a step of linking an N-terminal amino acid residue of a peptide compound with a C-terminal amino acid residue of the peptide compound in a solvent, wherein
at least one of the N-terminal amino acid residue and the C-terminal amino acid residue is a cyclic amino acid residue, and
the solvent comprises one or more selected from the group consisting of acetonitrile, dimethyl carbonate, 2-MeTHF, and anisole.

[212] A method of suppressing epimerization of a cyclic peptide compound in production of the cyclic peptide compound, or a salt thereof, or a solvate thereof, the method comprising

a step of linking an N-terminal amino acid residue of a peptide compound with a C-terminal amino acid residue of the peptide compound in a solvent, wherein
the N-terminal amino acid residue and the C-terminal amino acid residue of the peptide compound are any of the following a) to e):

a) the N-terminal amino acid residue is an N-substituted phenylalanine residue or an N-substituted phenylalanine derivative residue, and the C-terminal amino acid residue is a cyclic amino acid residue;
b) the N-terminal amino acid residue is an $\alpha,\alpha$-di-substituted amino acid residue, and the C-terminal amino acid residue is a cyclic amino acid residue;
c) the N-terminal amino acid residue is a cyclic amino acid residue, and the C-terminal amino acid residue is a homophenylalanine residue or a homophenylalanine derivative residue;
d) the N-terminal amino acid residue is a cyclic amino acid residue, and the C-terminal amino acid residue is an N-substituted Ala residue; and
e) the N-terminal amino acid residue is an N-substituted Gly residue, and the C-terminal amino acid residue is an N-substituted phenylalanine residue or an N-substituted phenylalanine derivative residue.

[213] A method of suppressing epimerization of a cyclic peptide compound in production of the cyclic peptide compound, or a salt thereof, or a solvate thereof, the method comprising the method according to any of [1] to [210].
[214] The method according to any of [211] to [213], wherein a content of epimer formed in the step is less than 20%, less than 15%, less than 10%, less than 5%, or less than 3%, as determined by UVarea value at 220 nm using HPLC analysis, based on the total amount of products.
[215] The method according to any of [211] to [213], wherein a content of epimer formed in the step is less than 15%, less than 10%, less than 5%, less than 3%, less than 1%, or an undetectable amount, as determined by UVarea value at 220 nm using HPLC analysis, based on the total amount of products.
[216] A method of suppressing formation of an oligomer of a cyclic peptide compound in production of the cyclic peptide compound, or a salt thereof, or a solvate thereof, the method comprising

a step of linking an N-terminal amino acid residue of a peptide compound with a C-terminal amino acid residue of the peptide compound in a solvent, wherein
at least one of the N-terminal amino acid residue and the C-terminal amino acid residue is a cyclic amino acid residue, and
the solvent comprises one or more selected from the group consisting of acetonitrile, dimethyl carbonate, 2-MeTHF, and anisole.

[217] A method of suppressing formation of an oligomer of a cyclic peptide compound in production of the cyclic peptide compound, or a salt thereof, or a solvate thereof, the method comprising

a step of linking an N-terminal amino acid residue of a peptide compound with a C-terminal amino acid residue of the peptide compound in a solvent, wherein
the N-terminal amino acid residue and the C-terminal amino acid residue of the peptide compound are any of the following a) to e):

a) the N-terminal amino acid residue is an N-substituted phenylalanine residue or an N-substituted phenylalanine derivative residue, and the C-terminal amino acid residue is a cyclic amino acid residue;
b) the N-terminal amino acid residue is an $\alpha,\alpha$-di-substituted amino acid residue, and the C-terminal amino acid residue is a cyclic amino acid residue;
c) the N-terminal amino acid residue is a cyclic amino acid residue, and the C-terminal amino acid residue is a homophenylalanine residue or a homophenylalanine derivative residue;
d) the N-terminal amino acid residue is a cyclic amino acid residue, and the C-terminal amino acid residue is an N-substituted Ala residue; and
e) the N-terminal amino acid residue is an N-substituted Gly residue, and the C-terminal amino acid residue is an N-substituted phenylalanine residue or an N-substituted phenylalanine derivative residue.

[218] A method of suppressing formation of an oligomer of a cyclic peptide compound in production of the cyclic peptide compound, or a salt thereof, or a solvate thereof, the method comprising the method according to any of [1] to [210].
[219] The method according to any of [216] to [218], wherein byproducts formed in the step comprise a dimer, and a content of the dimer is less than 15%, less than 10%, less than 5%, less than 2.5%, or less than 1%, as determined by UVarea value at 220 nm using HPLC analysis, based on the total amount of products.
[220] The method according to any of [216] to [218], wherein byproducts formed in the step comprise a trimer, and a content of the trimer is less than 5%, less than 2.5%, less than 1%, or an undetectable amount, as determined by UVarea value at 220 nm using HPLC analysis, based on the total amount of products.
[221] The method according to any of [216] to [218], wherein byproducts formed in the step comprise a dimer and a

trimer, and the total content of the dimer and the trimer is less than 15%, less than 10%, less than 5%, less than 2.5%, or less than 1%, as determined by UVarea value at 220 nm using HPLC analysis, based on the total amount of products.

In the above numberings, the number cited in the dependent item includes the branch number of the number, unless otherwise mentioned. For example, the [66] cited in the dependent item shows that not only [66] but also its branch number [66-1] and the like are included. The same applies to other numberings.

[Advantageous Effects of Invention]

According to the present invention, a cyclic peptide compound, or a salt thereof, or a solvate thereof can be efficiently produced while epimerization of amino acid residues and formation of oligomers are suppressed, even when the cyclic peptide compound has an amino acid sequence containing a plurality of non-natural amino acid residues. The production method of the present invention enables reducing costs in production of peptide compounds and also reducing the environmental loads, thereby the production method of the present invention is particularly useful for the synthesis of peptides on a large scale.

[Description of Embodiments]

Abbreviations

The abbreviations used herein are listed below.

2-MeTHF: 2-methyltetrahydrofuran
EtOAc: ethyl acetate
Alloc: allyloxycarbonyl
BEP: 2-bromo-1-ethylpyridinium tetrafluoroborate
BHT: 2,6-di-tert-butyl-4-methylphenol
Boc: t-butoxycarbonyl
Cbz: benzyloxycarbonyl
COMU: (1-cyano-2-ethoxy-2-oxoethylideneaminoxy)dimethylamino-morpholino-carbenium hexafluorophosphate
CPME: cyclopentyl methyl ether
CSA: 10-camphorsulfonic acid
DEPBT: diethyl 3,4-dihydro-4-oxo-1,2,3-benzotriazin-3-yl phosphate
DIPEA: N,N-diisopropylethylamine
DMA: dimethylacetamide
DMAP: 4-dimethylaminopyridine
DMF: N,N-dimethylformamide
DMSO: dimethylsulfoxide
DMT-MM: 4-(4,6-dimethoxy-1,3,5-triazin-2-yl)-4-methylmorpholinium chloride
EDCI: 1-ethyl-3-(3-dimethylaminopropyl)carbodiimide
FDPP: pentafluorophenyl diphenyl phosphinate
Fmoc: 9-fluorenylmethyloxycarbonyl
HATU: O-(7-azabenzotriazol-1-yl)-N,N,N',N'-tetramethyluronium hexafluorophosphate
HMDS: 1,1,1,3,3,3-hexamethyldisilazane
HOAt: 1-hydroxy-7-azabenzotriazole
HOBt: 1-hydroxybenzotriazole
IPAc: isopropyl acetate
MeCN: acetonitrile
MTBE: methyl tert-butyl ether
2-MeTHF: 2-methyltetrahydrofuran
MTHP: 4-methyltetrahydropyran
NMM: 4-methylmorpholine
NMP: N-methylpyrrolidone
PyBOP: 1H-benzotriazole-1-yloxytripyrrolidinophosphonium hexafluorophosphate
PyClop: chlorotripyrrolidino phosphonium hexafluorophosphate
PyOxim: (ethylcyano(hydroxyimino)acetat-O2)-tri-(1-pyrrolidinyl)-phosphonium hexafluorophosphate
T3P: propylphosphonic anhydride

TBAF: tetrabutylammonium fluoride
Teoc: 2-(trimethylsilyl)ethoxycarbonyl
TFA: trifluoroacetic acid
THF: tetrahydrofuran
TMSOTf: trimethylsilyl trifluoromethanesulfonate
Troc: 2,2,2-trichloroethoxycarbonyl

Definition of functional groups and the like (All terms and phrases herein are used as commonly understood in the art. Examples are given below, but they are not limited thereto.)

Examples of the "halogen atom" as used herein include F, Cl, Br, and I.

The term "alkyl" as used herein is a monovalent group derived from an aliphatic hydrocarbon by removing any one hydrogen atom, and has a subset of hydrocarbyl or hydrocarbon group structures that do not contain a hetero atom (which is an atom other than carbon and hydrogen atoms) or an unsaturated carbon-carbon bond, and contain hydrogen and carbon atoms in the backbone. The alkyl includes not only a linear form but a branched form. Specifically, the alkyl is an alkyl having 1 to 20 carbon atoms ($C_1$-$C_{20}$), preferably $C_1$-$C_{10}$ alkyl, more preferably $C_1$-$C_6$ alkyl. Hereinafter, "$C_p$-$C_q$" means that it has p to q carbon atoms. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The term "alkenyl" as used herein is a monovalent group having at least one double bond (two adjacent $SP^2$ carbon atoms). Depending on the conformation of the double bond and a substituent (if present), the geometric morphology of the double bond can assume entgegen (E) or zusammen (Z) and cis or trans conformations. The alkenyl includes not only a linear form but a branched form. The alkenyl is preferably $C_2$-$C_{10}$ alkenyl, more preferably $C_2$-$C_6$ alkenyl. Specific examples thereof include vinyl, allyl, 1-propenyl, 2-propenyl, 1-butenyl, 2-butenyl (which includes cis and trans), 3-butenyl, pentenyl, 3-methyl-2-butenyl, and hexenyl.

The term "alkynyl" as used herein is a monovalent group having at least one triple bond (two adjacent SP carbon atoms). The alkynyl includes not only a linear form but a branched form. The alkynyl is preferably $C_2$-$C_{10}$ alkynyl, more preferably $C_2$-$C_6$ alkynyl. Specific examples thereof include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

The term "cycloalkyl" as used herein means a saturated or partially saturated cyclic monovalent aliphatic hydrocarbon group and includes a monocyclic ring, a bicyclo ring, and a spiro ring. The cycloalkyl is preferably $C_3$-$C_8$ cycloalkyl. Specific examples thereof include cyclopropyl, cyclobutyl, cyclopentyl, cyclohexyl, cycloheptyl, cyclooctyl, bicyclo[2.2.1]heptyl, and spiro[3.3]heptyl.

The term "aryl" as used herein means a monovalent aromatic hydrocarbon ring and is preferably $C_6$-$C_{10}$ aryl. Specific examples of the aryl include phenyl and naphthyl (e.g., 1-naphthyl and 2-naphthyl).

The term "heterocyclyl" as used herein means a nonaromatic cyclic monovalent group containing a carbon atom as well as 1 to 5 heteroatoms. The heterocyclyl may have a double and/or triple bond in the ring. A carbon atom in the ring may form carbonyl through oxidation, and the ring may be a monocyclic ring or a condensed ring. The number of atoms constituting the ring is preferably 4 to 10 (4- to 10-membered heterocyclyl), more preferably 4 to 7 (4- to 7-membered heterocyclyl). Specific examples of the heterocyclyl include azetidinyl, oxiranyl, oxetanyl, azetidinyl, dihydrofuryl, tetrahydrofuryl, dihydropyranyl, tetrahydropyranyl, tetrahydropyridyl, tetrahydropyrimidyl, morpholinyl, thiomorpholinyl, pyrrolidinyl, piperidinyl, piperazinyl, pyrazolidinyl, imidazolinyl, imidazolidinyl, oxazolidinyl, isoxazolidinyl, thiazolidinyl, isothiazolidinyl, 1,2-thiazinane, thiadiazolidinyl, azetidinyl, oxazolidone, benzodioxanyl, benzoxazolyl, dioxolanyl, dioxanyl, tetrahydropyrrolo[1,2-c]imidazole, thietanyl, 3,6-diazabicyclo[3.1.1]heptanyl, 2,5-diazabicyclo[2.2.1]heptanyl, 3-oxa-8-azabicyclo[3.2.1]octanyl, sultam, and 2-oxaspiro[3.3]heptyl.

The term "heteroaryl" as used herein means an aromatic cyclic monovalent group containing a carbon atom as well as 1 to 5 heteroatoms. The ring may be a monocyclic ring or a condensed ring with another ring and may be partially saturated. The number of atoms forming the ring is preferably 5 to 10 (5- to 10-membered heteroaryl), more preferably 5 to 7 (5-to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

The term "alkoxy" as used herein means an oxy group bonded to the "alkyl" defined above and is preferably $C_1$-$C_6$ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The term "alkenyloxy" as used herein means an oxy group bonded to the "alkenyl" defined above and is preferably $C_2$-$C_6$ alkenyloxy. Specific examples of the alkenyloxy include vinyloxy, allyloxy, 1-propenyloxy, 2-propenyloxy, 1-butenyloxy, 2-butenyloxy (which includes cis and trans), 3-butenyloxy, pentenyloxy, and hexenyloxy.

The term "cycloalkoxy" as used herein means an oxy group bonded to the "cycloalkyl" defined above and is preferably $C_3$-$C_8$ cycloalkoxy. Specific examples of the cycloalkoxy include cyclopropoxy, cyclobutoxy, and cyclopentyloxy.

The term "aryloxy" as used herein means an oxy group bonded to the "aryl" defined above and is preferably $C_6$-$C_{10}$ aryloxy. Specific examples of the aryloxy include phenoxy, 1-naphthyloxy, and 2-naphthyloxy.

The term "amino" as used herein means -$NH_2$ in the narrow sense and means -NRR' in the broad sense. In this context, R and R' are each independently selected from hydrogen, alkyl, alkenyl, alkynyl, cycloalkyl, heterocyclyl, aryl, and heteroaryl, or R and R' form a ring together with a nitrogen atom to which they are attached. Preferred examples of the amino include - $NH_2$, mono-$C_1$-$C_6$ alkylamino, di-$C_1$-$C_6$ alkylamino, and 4- to 8-membered cyclic amino.

The term "monoalkylamino" as used herein means a group of the "amino" defined above in which R is hydrogen, and R' is the "alkyl" defined above, and is preferably mono-$C_1$-$C_6$ alkylamino. Specific examples of the monoalkylamino include methylamino, ethylamino, n-propylamino, i-propylamino, n-butylamino, s-butylamino, and t-butylamino.

The term "dialkylamino" as used herein means a group of the "amino" defined above in which R and R' are each independently the "alkyl" defined above, and is preferably di-$C_1$-$C_6$ alkylamino. Specific examples of the dialkylamino include dimethylamino and diethylamino.

The term "cyclic amino" as used herein means a group of the "amino" defined above in which R and R' form a ring together with the nitrogen atom bonded thereto, and is preferably 4-to 8-membered cyclic amino. Specific examples of the cyclic amino include 1-azetidyl, 1-pyrrolidyl, 1-piperidyl, 1-piperazyl, 4-morpholinyl, 3-oxazolidyl, 1,1-dioxidothiomorpholinyl-4-yl, and 3-oxa-8-azabicyclo[3.2.1]octan-8-yl.

The "protected amino" as used herein means an amino group protected with an optional protecting group. Specific examples of the protected amino include amino protected with a protecting group such as Boc, Fmoc, Cbz, Troc, Alloc, Teoc, or trifluoroacetyl.

The term "aminocarbonyl" as used herein means a carbonyl group bonded to the "amino" defined above and is preferably -$CONH_2$, mono-$C_1$-$C_6$ alkylaminocarbonyl, di-$C_1$-$C_6$ alkylaminocarbonyl, or 4- to 8-membered cyclic aminocarbonyl. Specific examples of the aminocarbonyl include -$CONH_2$, dimethylaminocarbonyl, 1-azetidinylcarbonyl, 1-pyrrolidinylcarbonyl, 1-piperidinylcarbonyl, 1-piperazinylcarbonyl, 4-morpholinylcarbonyl, 3-oxazolidinylcarbonyl, 1,1-dioxidothiomorpholinyl-4-ylcarbonyl, and 3-oxa-8-azabicyclo[3.2.1]octane-8-ylcarbonyl.

The term "alkenyloxycarbonyl" as used herein means a carbonyl group bonded to the "alkenyloxy" defined above and is preferably $C_2$-$C_6$ alkenyloxycarbonyl. Specific examples of the alkenyloxycarbonyl include vinyloxycarbonyl, allyloxycarbonyl, 1-propenyloxycarbonyl, 2-propenyloxycarbonyl, 1-butenyloxycarbonyl, 2-butenyloxycarbonyl (which includes cis and trans), 3-butenyloxycarbonyl, pentenyloxycarbonyl, and hexenyloxycarbonyl.

The term "alkylsulfonyl" as used herein means a sulfonyl group bonded to the "alkyl" defined above and is preferably $C_1$-$C_6$ alkylsulfonyl. Specific examples of the alkylsulfonyl include methylsulfonyl.

The term "hydroxyalkyl" as used herein means a group in which one or more hydrogen of the "alkyl" defined above are replaced with a hydroxy group, and is preferably $C_1$-$C_6$ hydroxyalkyl. Specific examples of the hydroxyalkyl include hydroxymethyl, 1-hydroxyethyl, 2-hydroxyethyl, 2-hydroxy-2-methylpropyl, and 5-hydroxypentyl.

The term "haloalkyl" as used herein means a group in which one or more hydrogen of the "alkyl" defined above are replaced with halogen, and is preferably $C_1$-$C_6$ haloalkyl, more preferably $C_1$-$C_6$ fluoroalkyl. Specific examples of the haloalkyl include difluoromethyl, trifluoromethyl, 2,2-difluoroethyl, 2,2,2-trifluoroethyl, 3,3-difluoropropyl, 4,4-difluorobutyl, and 5,5-difluoropentyl.

The term "cyanoalkyl" as used herein means a group in which one or more hydrogen of the "alkyl" defined above are replaced with cyano, and is preferably $C_1$-$C_6$ cyanoalkyl. Specific examples of the cyanoalkyl include cyanomethyl and 2-cyanoethyl.

The term "aminoalkyl" as used herein means a group in which one or more hydrogen of the "alkyl" defined above are replaced with the "amino" defined above, and is preferably $C_1$-$C_6$ aminoalkyl. Specific examples of the aminoalkyl include 1-pyridylmethyl, 2-(1-piperidyl)ethyl, 3-(1-piperidyl)propyl, and 4-aminobutyl.

The term "carboxyalkyl" as used herein means a group in which one or more hydrogen of the "alkyl" defined above are replaced with carboxy, and is preferably $C_2$-$C_6$ carboxyalkyl. Specific examples of the carboxyalkyl include carboxymethyl.

The term "alkenyloxycarbonylalkyl" as used herein means a group in which one or more hydrogen of the "alkyl" defined above are replaced with the "alkenyloxycarbonyl" defined above, and is preferably $C_2$-$C_6$ alkenyloxycarbonyl-$C_1$-$C_6$ alkyl, more preferably $C_2$-$C_6$ alkenyloxycarbonyl-$C_1$-$C_2$ alkyl. Specific examples of the alkenyloxycarbonylalkyl include allyloxycarbonylmethyl and 2-(allyloxycarbonyl)ethyl.

The term "alkoxyalkyl" as used herein means a group in which one or more hydrogen of the "alkyl" defined above

are replaced with the "alkoxy" defined above, and is preferably $C_1$-$C_6$ alkoxy-$C_1$-$C_6$ alkyl, more preferably $C_1$-$C_6$ alkoxy-$C_1$-$C_2$ alkyl. Specific examples of the alkoxyalkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

The term "cycloalkylalkyl" as used herein means a group in which one or more hydrogen of the "alkyl" defined above are replaced with the "cycloalkyl" defined above, and is preferably $C_3$-$C_8$ cycloalkyl-$C_1$-$C_6$ alkyl, more preferably $C_3$-$C_6$ cycloalkyl-$C_1$-$C_2$ alkyl. Specific examples of the cycloalkylalkyl include cyclopropylmethyl, cyclobutylmethyl, cyclopentylmethyl, and cyclohexylmethyl.

The term "cycloalkoxyalkyl " as used herein means a group in which one or more hydrogen of the "alkyl" defined above are replaced with the "cycloalkoxy" defined above, and is preferably $C_3$-$C_8$ cycloalkoxy-$C_1$-$C_6$ alkyl, more preferably $C_3$-$C_6$ cycloalkoxy-$C_1$-$C_2$ alkyl. Specific examples of the cycloalkoxyalkyl include cyclopropoxymethyl and cyclobutoxymethyl.

The term "heterocyclylalkyl" as used herein means a group in which one or more hydrogen of the "alkyl" defined above are replaced with the "heterocyclyl" defined above, and is preferably 4- to 7-membered heterocyclyl-$C_1$-$C_6$ alkyl, more preferably 4- to 7-membered heterocyclyl-$C_1$-$C_2$ alkyl. Specific examples of the heterocyclylalkyl include 2-(tetrahydro-2H-pyran-4-yl)ethyl and 2-(azetidin-3-yl)ethyl.

The term "alkylsulfonylalkyl" as used herein means a group in which one or more hydrogen of the "alkyl" defined above are replaced with the "alkylsulfonyl" defined above, and is preferably $C_1$-$C_6$ alkylsulfonyl-$C_1$-$C_6$ alkyl, more preferably $C_1$-$C_6$ alkylsulfonyl-$C_1$-$C_2$ alkyl. Specific examples of the alkylsulfonylalkyl include methylsulfonylmethyl and 2-(methylsulfonyl)ethyl.

The term "aminocarbonylalkyl" as used herein means a group in which one or more hydrogen of the "alkyl" defined above are replaced with the "aminocarbonyl" defined above, and is preferably aminocarbonyl-$C_1$-$C_6$ alkyl, more preferably aminocarbonyl-$C_1$-$C_4$ alkyl. Specific examples of the aminocarbonylalkyl include methylaminocarbonylmethyl, dimethylaminocarbonylmethyl, t-butylaminocarbonylmethyl, 1-azetidinylcarbonylmethyl, 1-pyrrolidinylcarbonylmethyl, 1-piperidinylcarbonylmethyl, 4-morpholinylcarbonylmethyl, 2-(methylaminocarbonyl)ethyl, 2-(dimethylaminocarbonyl)ethyl, 2-(1-azetidinylcarbonyl)ethyl, 2-(1-pyrrolidinylcarbonyl)ethyl, 2-(4-morpholinylcarbonyl)ethyl, 3-(dimethylaminocarbonyl)propyl, and 4-(dimethylaminocarbonyl)butyl.

The term "aryloxyalkyl" as used herein means a group in which one or more hydrogen of the "alkyl" defined above are replaced with the "aryloxy" defined above, and is preferably $C_6$-$C_{10}$ aryloxy-$C_1$-$C_6$ alkyl, more preferably $C_6$-$C_{10}$ aryloxy-$C_1$-$C_2$ alkyl. Specific examples of the aryloxyalkyl include phenoxymethyl and 2-phenoxyethyl.

The term "aralkyl (arylalkyl)" as used herein means a group in which at least one hydrogen atom of the "alkyl" defined above is replaced with the "aryl" defined above, and is preferably $C_7$-$C_{14}$ aralkyl, more preferably $C_7$-$C_{10}$ aralkyl. Specific examples of the aralkyl include benzyl, phenethyl, and 3-phenylpropyl.

The term "aralkoxy" as used herein means an oxy group bonded to the "aralkyl" defined above and is preferably $C_7$-$C_{14}$ aralkoxy, more preferably $C_7$-$C_{10}$ aralkoxy. Specific examples of the aralkoxy include benzyloxy, phenethyloxy, and 3-phenylpropoxy.

The term "aralkoxyalkyl" as used herein means a group in which one or more hydrogen of the "alkyl" defined above are replaced with the "aralkoxy" defined above, and is preferably $C_7$-$C_{14}$ alkoxy-$C_1$-$C_6$ alkyl, more preferably $C_7$-$C_{14}$ alkoxy-$C_1$-$C_2$ alkyl. Specific examples of the aralkoxyalkyl include benzyloxymethyl and 1-(benzyloxy)ethyl.

The term "heteroarylalkyl" as used herein means a group in which at least one hydrogen atom of the "alkyl" defined above is replaced with the "heteroaryl" defined above, and is preferably 5- to 10-membered heteroaryl-$C_1$-$C_6$ alkyl, more preferably 5- to 10-membered heteroaryl-$C_1$-$C_2$ alkyl. Specific examples of the heteroarylalkyl include 3-thienylmethyl, 4-thiazolylmethyl, 2-pyridylmethyl, 3-pyridylmethyl, 4-pyridylmethyl, 2-(2-pyridyl)ethyl, 2-(3-pyridyl)ethyl, 2-(4-pyridyl)ethyl, 2-(6-quinolyl)ethyl, 2-(7-quinolyl)ethyl, 2-(6-indolyl)ethyl, 2-(5-indolyl)ethyl, and 2-(5-benzofuranyl)ethyl.

The term "heteroarylalkoxy" as used herein means an oxy group bonded to the "heteroarylalkyl" defined above and is preferably 5- to 10-membered heteroaryl-$C_1$-$C_6$ alkoxy, more preferably 5- to 10-membered heteroaryl-$C_1$-$C_2$ alkoxy. Specific examples of the heteroarylalkoxy include 3-thienylmethoxy and 3-pyridylmethoxy.

The term "heteroarylalkoxyalkyl" as used herein means a group in which one or more hydrogen of the "alkyl" defined above are replaced with the "heteroarylalkoxy" defined above, and is preferably 5- to 10-membered heteroaryl-$C_1$-$C_6$ alkoxy-$C_1$-$C_6$ alkyl, more preferably 5- to 10-membered heteroaryl-$C_1$-$C_2$ alkoxy-$C_1$-$C_2$ alkyl. Specific examples of the heteroarylalkoxyalkyl include 3-pyridylmethoxymethyl.

The term "heterocycloalkylidenealkyl" as used herein means a group in which one or more hydrogen of the "alkyl" defined above are replaced with the "heterocycloalkylidene" defined above, and is preferably 4- to 7-membered heterocycloalkylidene-$C_1$-$C_6$ alkyl, more preferably 4- to 7-membered heterocycloalkylidene-$C_1$-$C_2$ alkyl. Specific examples of the heterarylalkoxyalkyl include tetrahydro-4H-pyran-4-ylidenemethyl and azetidin-3-ylidenemethyl.

The term "alkoxyalkenyl" as used herein means a group in which one or more hydrogen of the "alkenyl" defined above are replaced with the "alkoxy" defined above, and is preferably $C_1$-$C_6$ alkoxy $C_2$-$C_6$ alkenyl. Specific examples of the alkoxyalkenyl include (E)-4-methoxybut-2-en-1-yl.

The term "aminocarbonylalkenyl" as used herein means a group in which one or more hydrogen of the "alkenyl" defined above are replaced with the "aminocarbonyl" defined above, and is preferably aminocarbonyl-$C_2$-$C_6$ alkenyl. Specific examples of the aminocarbonylalkenyl include (E)-3-(dimethylaminocarbonyl)-prop-2-en-1-yl.

The term "haloalkoxy" as used herein means a group in which one or more hydrogen of the "alkoxy" defined above are replaced with halogen, and is preferably $C_1$-$C_6$ haloalkoxy. Specific examples of the haloalkoxy include difluoromethoxy, trifluoromethoxy, 2,2-difluoroethoxy, and 2,2,2-trifluoroethoxy.

The term "alkylene" as used herein means a divalent group derived from the "alkyl" described above by further removing one arbitrary hydrogen atom, and is preferably $C_4$-$C_8$ alkylene. Specific examples of the alkylene include $-CH_2-$, $-(CH_2)_2-$, $-(CH_2)_3-$, $-CH(CH_3)CH_2-$, $-C(CH_3)_2-$, $-(CH_2)_4-$, $-CH(CH_3)CH_2CH_2-$, $-C(CH_3)_2CH_2-$, $-CH_2CH(CH_3)CH_2-$, $-CH_2C(CH_3)_2-$, $-CH_2CH_2CH(CH_3)-$, $-(CH_2)_5-$, $-(CH_2)_6-$, $-(CH_2)_7-$, and $-(CH_2)_8-$.

The term "alicyclic ring" as used herein means a nonaromatic hydrocarbon ring. The alicyclic ring may have an unsaturated bond in the ring and may be a polycyclic ring having two or more rings. A carbon atom constituting the ring may form carbonyl through oxidation. The alicyclic ring is preferably a 3- to 8-membered alicyclic ring. Specific examples thereof include a cyclopropane ring, a cyclobutane ring, a cyclopentane ring, a cyclohexane ring, a cycloheptane ring, a cyclooctane ring, and a bicyclo[2.2.1]heptane ring.

The term "saturated heterocyclic ring" as used herein means a nonaromatic heterocyclic ring containing 1 to 5 heteroatoms in addition to a carbon atom, without containing a double bond and/or a triple bond in the ring. The saturated heterocycle may be a monocyclic ring or may form a condensed ring with another ring, for example, an aromatic ring such as a benzene ring. The saturated heterocyclic ring is preferably a 4- to 7-membered saturated heterocyclic ring. Specific examples include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, and an indoline ring.

The term "optionally substituted" as used herein means that a group may be substituted by any substituent.

The term "optionally protected" as used herein means that a group may be protected by any protecting group.

The term "one or more" as used herein means the number of 1 or 2 or larger. When the term "one or more" is used in the context associated with a substituent of a group, the term means a number from 1 to the maximum number of substituents acceptable by the group. Specific examples of the term "one or more" include 1, 2, 3, 4, 5, 6, 7, 8, 9, 10, and/or larger numbers.

The term "peptide compound (or "peptide")" as used herein means a compound in which two or more amino acids are linked by an amide bond. Peptide compounds having an ester bond in part of the main chain, such as depsipeptide, are also included in the term peptide compound (or peptide) herein. The number of amino acid residues in the peptide compound is not particularly limited, but preferably 5 to 30 residues, more preferably 8 to 15 residues, and further preferably 9 to 13 residues. The peptide compound synthesized in the present invention contains preferably at least three N-substituted amino acids, more preferably at least five N-substituted amino acids, in one peptide. These N-substituted amino acids may be present continuously or discontinuously in the peptide compound. The peptide compound according to the present invention may be linear or cyclic, and is preferably a cyclic peptide compound. The number of residues contained in the peptide compound is preferably 5 to 30 residues, more preferably 8 to 15 residues, and further preferably 9 to 13 residues. The peptide compound preferably contains at least three N-substituted amino acids, more preferably at least five N-substituted amino acids in one peptide compound. These N-substituted amino acids may be present continuously or discontinuously in the peptide compound. The peptide compound according to the present invention may be linear or cyclic, and is preferably a cyclic peptide compound.

The "linear peptide compound" as used herein means a peptide compound in which amino acids are linearly linked by an amide bond. Peptide compounds having an ester bond in part of the main chain and in which amino acids are linearly linked, such as depsipeptide, are also included in the linear peptide compound herein. The linear peptide compound has an amino group that may be protected with a protecting group at the N-terminal amino acid residue and a carboxyl group that may be protected with a protecting group at the C-terminal amino acid residue.

The term "cyclic peptide compound" as used herein refers to a peptide compound having a cyclic structure composed of 4 or more amino acid residues. The cyclic structure of the cyclic peptide compound may contain a bond other than an amide bond, for example, an ester bond, an ether bond, a thioether bond, or a carbon-carbon bond. The cyclic peptide compound may have an amino acid or chain peptide structure that is not contained in the cyclic structure, in addition to the cyclic structure. It may also have a structure other than amino acids and chain peptide structures. Among these, a covalent bond such as an amide bond, a thioether bond, or a carbon-carbon bond is preferred, and an amide bond is particularly preferred. The position of the carboxyl and amino groups of the cyclic peptide compound may be on the main chain or the side chain.

The term "cyclization" of a peptide compound means a formation of a cyclic portion containing 4 or more amino acid residues. The number of amino acids contained in the cyclic portion of the cyclic peptide compound is not particularly

limited herein, but examples thereof include 4-20 residues, 5-18 residues, 6-17 residues, 7-16 residues, 9-15 residues, 10-15 residues, and 11-14 residues. Conversion of a linear peptide compound to a cyclic peptide compound can be performed by the methods described in Comprehensive Organic Transformations, A Guide to Functional Group Preparations, 3rd Edition (by R. C. Larock) or March's Advanced Organic Chemistry: Reactions, Mechanisms, and Structure, 7th Edition (by M. B. Smith, J. March) or the like or by performing a bond formation reaction in the molecule. A functional group conversion reaction may also be performed after the bond formation reaction. Examples of the bond formation reaction include a C(O)-N bond formed from a carboxylic acid and an amine, a C-O-C bond, a C(O)-O bond, and a C(S)-O bond via an oxygen atom, a C(O)-S bond, a C(S)-S bond, a C-S-S-C bond, a C-S-C bond, a C-S(O)-C bond, and a C-S(O2)-C bond via a sulfur atom, and a C-N-C bond, a C=N-C bond, a N-C(O)-N bond, a N-C(S)N bond, and a C(S)-N bond via a nitrogen atom. Further examples thereof include a C-C bond formation reaction catalyzed by a transition metal, such as the Suzuki reaction, the Heck reaction, and the Sonogashira reaction. Examples of the functional group conversion reaction that is further performed after the bond formation reaction include an oxidation reaction or a reduction reaction. Specific examples thereof include a reaction in which a sulfur atom is oxidized and converted to a sulfoxide group or a sulfone group. Other examples thereof include a reduction reaction in which, among carbon-carbon bonds, a triple bond or a double bond is reduced and converted to a double bond or a single bond. Two amino acids may be bonded at the main chain of the amino acid to form a closed ring structure by a peptide bond, or a covalent bond may be formed between two amino acids via, for example, a bond between side chains or between a side chain and the main chain of the two amino acids.

The term "amino acid" as used herein includes a natural amino acid and a non-natural amino acid. The term "amino acid" as used herein may mean an amino acid residue. The term "natural amino acid" as used herein refers to Gly, Ala, Ser, Thr, Val, Leu, Ile, Phe, Tyr, Trp, His, Glu, Asp, Gln, Asn, Cys, Met, Lys, Arg, and Pro. Examples of the non-natural amino acid include, but are not particularly limited to, a β-amino acid, a D-type amino acid, an N-substituted amino acid (excluding Pro), an α,α-disubstituted amino acid, an amino acid having a side chain different from that of natural amino acids, and a hydroxycarboxylic acid. The amino acid as used herein accepts an arbitrary conformation. There are no specific restrictions on the selection of a side chain of the amino acid , and the side chain is freely selected from, in addition to a hydrogen atom, for example, an alkyl group, an alkenyl group, an alkynyl group, an aryl group, a heteroaryl group, an aralkyl group, a heteroarylalkyl group, a cycloalkyl group, a spiro-bonded cycloalkyl group. Each of the side chains may have a substituent. The substituent is not restricted either and, for example, may be one or two or more freely selected independently from any substituents including a halogen atom, an O atom, an S atom, an N atom, a B atom, a Si atom, or a P atom. That is, examples of the side chain include an alkyl group, an alkoxy group, an alkoxyalkyl group, an alkenyl group, an alkynyl group, an aryl group, a heteroaryl group, an aralkyl group and a cycloalkyl group which may be substituted, or oxo, aminocarbonyl, and a halogen atom.

As used herein, the "amino acid residue" constituting a peptide compound is sometimes referred to simply as an "amino acid".

The term "N-terminal amino acid residue" as used herein, means an amino acid residue located at the N-terminus of a peptide. The term "C-terminal amino acid residue" as used herein, means an amino acid residue located at the C-terminus of a peptide.

The "non-natural amino acid residue" as used herein means an amino acid residue having a structure different from that of natural amino acid residues. Examples of the non-natural amino acid include a β-amino acid residue, a D-type amino acid residue, an N-substituted amino acid residue (excluding Pro), an α,α-disubstituted amino acid residue, an amino acid residue having a side chain different from that of natural amino acids. The "N-substituted non-natural amino acid residue" as used herein means an amino acid residue in which the hydrogen on the nitrogen of the amino group of the main chain of a non-natural amino acid residue is substituted with another atom or functional group. An amino acid residue in which hydrogen on the nitrogen of the amino group in the main chain of a natural amino acid is substituted with another atom or functional group (excluding Pro), and an amino acid residue that has a structure different from that of natural amino acids in the side chain and in which hydrogen on the nitrogen of the amino group of the main chain is substituted with another atom or functional group fall under the N-substituted non-natural amino acid residue. Examples of the N-substituted non-natural amino acid residue include an N-methylglycine (MeGly) residue, an N-methylalanine (MeAla) residue, and an N-methylhomophenylalanine (MeHph) residue.

The term "cyclic amino acid residue" as used herein means an amino acid residue having a cyclic structure in which the nitrogen atom of the amino group of the amino acid residue and any atom of the side chain together forms a ring. Examples of the cyclic amino acid residue include a Pro residue (formula (i)), a proline derivative residue (formula (ii)), and an Aze(2) residue (formula (iii)):

[Formula 24]

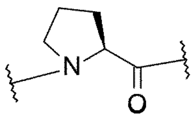 formula (i) 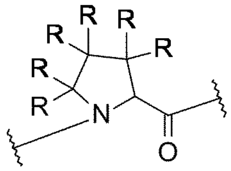 formula (ii) 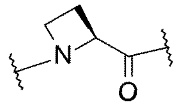 (formula (iii)

**In** the formula (ii), Rs each independently represent an arbitrary substituent and may be, for example, alkyl, hydroxy, alkoxy, amino, alkylamino, or the like.

The "N-substituted phenylalanine residue" as used herein means a phenylalanine residue in which hydrogen on the nitrogen of the amino group of a phenylalanine residue is substituted with another atom or functional group. The "N-substituted phenylalanine derivative residue" as used herein means an amino acid residue in which hydrogen on the nitrogen of the amino group of a phenylalanine residue is substituted with another atom or functional group, and hydrogen on the phenyl group of the phenylalanine residue is substituted with another atom or substituent. Examples of such a substituent include alkyl, halogen, alkyl substituted with halogen, hydroxy, alkoxy, amino, and alkylamino. Examples of the N-substituted phenylalanine residue include an N-alkylphenylalanine residue in which the substituent on the nitrogen of the amino group of the N-substituted phenylalanine residue is alkyl. Examples of the N-alkylphenylalanine residues include an EtPhe residue (formula (v)). Examples of the N-substituted phenylalanine derivative residue include an N-alkylphenylalanine derivative residue in which the substituent on the nitrogen of the amino group of the N-substituted phenylalanine derivative residue is alkyl. Examples of the N-alkylphenylalanine derivative residue include an EtPhe derivative residue, and examples of the EtPhe derivative residue include an EtPhe (4-Me) residue (formula (vi)).

[Formula 25]

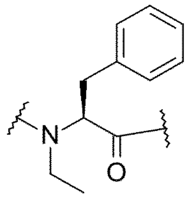 (formula (v)) 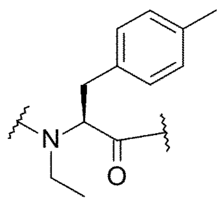 (formula (vi))

The term "α,α-di-substituted amino acid residue" as used herein means an amino acid residue in which two hydrogens on α-carbon are both substituted with other atoms and/or functional groups other than hydrogen. Examples of the α,α-disubstituted amino acid residue include an α,α-dialkylamino acid residue such as an α,α-dimethylamino acid residue and a cLeu residue (formula (vii)) in which two groups present at position α are linked to form an alicyclic ring.

[Formula 26]

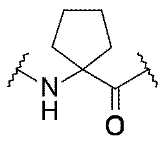 (formula (vii))

The term "homophenylalanine residue" as used herein means an amino acid residue in which the amino acid is homophenylalanine, and the term "homophenylalanine derivative residue" means an amino acid residue in which hydrogen on the phenyl group of a homophenylalanine residue is substituted with another atom or substituent. Examples of such a substituent include alkyl, halogen, alkyl substituted with halogen, hydroxy, alkoxy, amino, and alkylamino. Examples of the homophenylalanine derivative residue include a Hph(3,5-diF-4-$CF_3$) residue (formula (viii)).

[Formula 27]

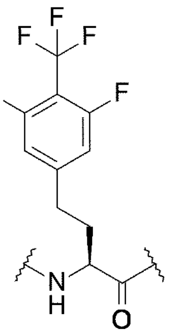

(formula (viii))

The "N-substituted Ala residue" as used herein means an alanine residue in which hydrogen on the nitrogen of the amino group of the alanine residue is substituted with another atom or functional group. Examples of the N-substituted Ala residue include an N-alkylAla residue (formula (ix)) in which the substituent on the nitrogen of the amino group of the N-substituted alanine residue is alkyl. Examples of the N-alkylAla residue preferably include $C_1$-$C_{10}$ alkylAla residue, more preferably a $C_1$-$C_6$ alkylAla residue such as a MeAla residue (formula (x)) or an EtAla residue (formula (xi)).

[Formula 28]

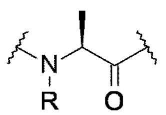

R = alkyl (formula (ix))

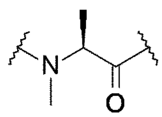

(formula (x))

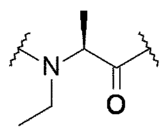

(formula (xi))

The "N-substituted Gly residue" as used herein means a glycine residue in which hydrogen on the nitrogen of the amino group of the glycine residue is substituted with another atom or functional group. Examples of the N-substituted Gly residue include an N-alkylGly residue (formula (xii)) in which the substituent on the nitrogen of the amino group of the N-substituted glycine residue is alkyl. Examples of the N-alkylGly residue preferably include $C_1$-$C_{10}$ alkylGly residue, more preferably a $C_1$-$C_6$ alkylGly residue such as a MeGly residue (formula (xiii)) or an EtGly residue (formula (xiv)).

[Formula 29]

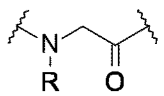

R = alkyl (formula (xii))

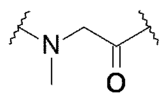

(formula (xiii))

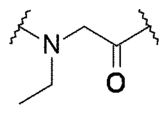

(formula (xiv))

The "side chain of amino acid" as used herein, in the case of an α-amino acid, means an atomic group bonded to a carbon (α-carbon) to which an amino group and a carboxyl group are bonded. For example, the methyl group of Ala is a side chain of the amino acid. In the case of a β-amino acid, an atomic group attached to an α-carbon and/or a β-carbon can be a side chain of the amino acid, and in the case of a γ-amino acid, an atomic group attached to an α-carbon, a β-carbon, and/or a γ-carbon can be a side chain of the amino acid. Note that the α-carbon in an α-amino acid, a β-amino acid, and a γ-amino acid refers to the first (position α) carbon adjacent to the carboxyl group in the main chain of the amino acid. In addition, the carbon next to the α-carbon (second from the carboxyl group (at position β)) is called β-carbon, and the carbon next to the β-carbon (third (position y) from the carboxyl group) is called γ-carbon.

The term "main chain of an amino acid" as used herein means a branched portion formed by an amino group, α-carbon, and a carboxyl group in the case of an α-amino acid, a branched portion formed by an amino group, β-carbon, α-carbon and a carboxyl group in the case of a β-amino acid, and a branched portion formed by an amino group, γ-carbon, β-carbon, α-carbon and a carboxyl group in the case of a γ-amino acid.

The amino group in the main chain of the amino acid may be unsubstituted ($-NH_2$) or substituted (i.e., -NHR). R

represents alkyl, alkenyl, alkynyl, aryl, heteroaryl, aralkyl or cycloalkyl optionally having a substituent, or a carbon chain bonded to the N atom and a carbon atom at position $\alpha$ may form a ring as in proline. Such an amino acid in which an amino group on the main chain is substituted is sometimes referred to herein as "N-substituted amino acid" or "N-substituted amino acid residue". Examples of the "N-substituted amino acid" or "N-substituted amino acid residue" as used herein include preferably, but are not limited to, N-alkylamino acid, N-$C_1$-$C_6$ alkylamino acid, N-$C_1$-$C_4$ alkylamino acid, N-methylamino acid, N-ethylamino acid, N-$C_7$-$C_{14}$ aralkylamino acid, N-benzylamino acid, N-phenethylamino acid, proline, and Aze(2).

As used herein, the term "number of amino acids (amino acid number)" or "number of amino acid residues (amino acid residue number)" refers to the number of amino acid residues (amino acid units) constituting a peptide compound, and means the number of amino acid units generated upon cleavage of amide bonds, ester bonds, and bonds of cyclized portions that link the amino acids.

The "amino acid" as used herein constituting a peptide compound includes all isotopes corresponding to each. The isotope of the "amino acid" is a form in which at least one atom is replaced with an atom having the same atomic number (proton number) and a different mass number (total number of protons and neutrons) at an abundance ratio different from the natural abundance ratio. Examples of the isotope included in the "amino acid" forming a peptide compound herein include a hydrogen atom, a carbon atom, a nitrogen atom, an oxygen atom, a phosphorus atom, a sulfur atom, a fluorine atom, a chlorine atom, and the like, and they include $^{2}H$, $^{3}H$; $^{13}C$, $^{14}C$; $^{15}N$; $^{17}O$, $^{18}O$; $^{31}P$, $^{32}P$; $^{35}S$; $^{18}F$; $^{36}Cl$; and the like, respectively. For the compounds as used herein, all the compounds containing any proportions of radioactive or nonradioactive isotopic elements are encompassed within the scope of the present invention.

Examples of the substituent containing a halogen atom herein include an alkyl group, a cycloalkyl group, an alkenyl group, an alkynyl group, an aryl group, a heteroaryl group, and an aralkyl group having halogen as a substituent and more specifically include fluoroalkyl, difluoroalkyl, and trifluoroalkyl.

Examples of the substituent containing an O atom include hydroxy (-OH), oxy (-OR), carbonyl (-C=O-R), carboxy ($-CO_2H$), oxycarbonyl (-C=O-OR), carbonyloxy (-O-C=O-R), thiocarbonyl (-C=O-SR), carbonylthio (-S-C=O-R), aminocarbonyl (-C=O-NHR), carbonylamino (-NH-C=O-R), oxycarbonylamino (-NH-C=O-OR), sulfonylamino ($-NH-SO_2-R$), aminosulfonyl ($-SO_2-NHR$), sulfamoylamino ($-NH-SO_2-NHR$), thiocarboxyl (-C(=O)-SH) and carboxylcarbonyl ($-C(=O)-CO_2H$) groups.

Examples of the oxy (-OR) include alkoxy, cycloalkoxy, alkenyloxy, alkynyloxy, aryloxy, heteroaryloxy, and aralkyloxy. The alkoxy is preferably $C_1$-$C_4$ alkoxy or $C_1$-$C_2$ alkoxy, particularly preferably methoxy or ethoxy.

Examples of the carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of the oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of the carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of the thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of the carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of the aminocarbonyl (-C=O-NHR) include alkylaminocarbonyl (e.g. $C_1$-$C_6$ or $C_1$-$C_4$ alkylaminocarbonyl, particularly, ethylaminocarbonyl and methylaminocarbonyl), cycloalkylaminocarbonyl, alkenylaminocarbonyl, alkynylaminocarbonyl, arylaminocarbonyl, heteroarylaminocarbonyl, and aralkylaminocarbonyl. Examples thereof additionally include groups in which the H atom bonded to the N atom in -C=O-NHR is further replaced with alkyl, cycloalkyl, alkenyl, alkynyl, aryl, heteroaryl, or aralkyl.

Examples of the carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Additional examples thereof include groups in which the H atom bonded to the N atom in -NH-C=O-R is further substituted with alkyl, cycloalkyl, alkenyl, alkynyl, aryl, heteroaryl, or aralkyl.

Examples of the oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Additional examples thereof include groups in which the H atom bonded to the N atom in -NH-C=O-OR is further substituted with alkyl, cycloalkyl, alkenyl, alkynyl, aryl, heteroaryl, or aralkyl.

Examples of sulfonylamino ($-NH-SO_2-R$) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples thereof include groups in which the H atom bonded to the N atom in $-NH-SO_2-R$ is further substituted with alkyl, cycloalkyl, alkenyl, alkynyl, aryl, heteroaryl, or aralkyl.

Examples of aminosulfonyl ($-SO_2-NHR$) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Additional examples thereof include groups in which the H atom bonded to the N atom in -$SO_2$-NHR is further substituted with alkyl, cycloalkyl, alkenyl, alkynyl, aryl, heteroaryl, or aralkyl.

Examples of sulfamoylamino (-NH-$SO_2$-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. The two H atoms bonded to the N atoms in -NH-$SO_2$-NHR may be substituted by substituents each independently selected from the group consisting of alkyl, cycloalkyl, alkenyl, alkynyl, aryl, heteroaryl, and aralkyl, and these two substituents may form a ring.

Examples of the substituent containing an S atom include groups such as thiol (-SH), thio (-S-R), sulfinyl (-S=O-R), sulfonyl (-$SO_2$-R), and sulfo (-$SO_3$H).

Examples of thio (-S-R) that can be selected include alkylthio, cycloalkylthio, alkenylthio, alkynylthio, arylthio, heteroarylthio, and aralkylthio.

Examples of the sulfonyl (-$SO_2$-R) include alkylsulfonyl, cycloalkylsulfonyl, alkenylsulfonyl, alkynylsulfonyl, arylsulfonyl, heteroarylsulfonyl, and aralkylsulfonyl.

Examples of the substituent containing a N atom include groups such as azide (-$N_3$; also referred to as an "azide group"), cyano (-CN), primary amino (-$NH_2$), secondary amino (-NH-R; also referred to as mono-substituted amino), tertiary amino (-NR(R'); also referred to as di-substituted amino), amidino (-C(=NH)-$NH_2$), substituted amidino (-C(=NR)-NR'R"), guanidino (-NH-C(=NH)-$NH_2$), substituted guanidino (-NR-C(=NR‴)-NR'R"), aminocarbonylamino (-NR-CO-NR'R"), pyridyl, piperidino, morpholino, and azetidinyl.

Examples of the secondary amino (-NH-R: mono-substituted amino) include alkylamino, cycloalkylamino, alkenylamino, alkynylamino, arylamino, heteroarylamino, and aralkylamino.

Examples of the tertiary amino (-NR(R'): di-substituted amino) include an amino group having arbitrary two substituents each independently selected from alkyl, cycloalkyl, alkenyl, alkynyl, aryl, heteroaryl, and aralkyl, for example, alkyl(aralkyl)amino. These arbitrary two substituents may form a ring. Specific examples thereof include dialkylamino, particularly, $C_1$-$C_6$ dialkylamino, $C_1$-$C_4$ dialkylamino, dimethylamino, and diethylamino. The term "$C_p$-$C_q$ dialkylamino group" as used herein refers to a group in which an amino group is substituted by two $C_p$-$C_q$ alkyl groups. The $C_p$-$C_q$ alkyl groups may be the same or different.

Examples of the substituted amidino (-C(=NR)-NR'R") include groups in which three substituents R, R', and R" on the N atoms are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR‴)-NR'R") include groups in which R, R', R", and R‴ are each independently selected from alkyl, cycloalkyl, alkenyl, alkynyl, aryl, heteroaryl, and aralkyl, and groups in which these substituents form a ring.

Examples of the aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

The compound according to the present invention may be a salt thereof, preferably a chemically or pharmaceutically acceptable salt. The compound according to the present invention, or a salt thereof may be a solvate thereof, preferably a chemically or pharmaceutically acceptable solvate. Examples of the salt of the compound according to the present invention include: hydrochloride hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salt, and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing the compound into contact with an acid or a base which can be used for production of a medicament. In the present invention, the solvate of the compound refers to a molecular group formed by the compound with a solvent, and is not particularly limited as long as it is a solvate formed by a solvent which is permitted to be taken concomitantly with administration of a drug. When the solvent is water, the solvate is called a hydrate. Examples of the solvate of the compound according to the present invention is preferably a hydrate, and specific examples of the hydrate include mono- to decahydrate, preferably mono- to pentahydrate, more preferably mono- to trihydrate. The solvate of the compound according to the present invention includes not only solvates with a single solvent such as water, alcohol (e.g. methanol, ethanol, 1-propanol or 2-propanol), or dimethylformamide, but also solvates with a plurality of solvents.

When the compound according to the present invention is obtained as a free form, the compound can be conventionally transformed into a state of a hydrate thereof or a solvate thereof. When the compound according to the present invention is obtained as a free form, the compound can be conventionally transformed into a state of a salt, a hydrate thereof or a solvate thereof which may be formed by the compound. Examples thereof include hydrates and ethanolates of the compound represented by the formula (2) or a salt thereof. Specific examples thereof include, but are not limited to, hemihydrates, monohydrates, dihydrates, trihydrates, tetrahydrates, pentahydrates, hexahydrates, heptahydrates, octahydrates, nonahydrates, decahydrates, or monoethanolates of the compound represented by the formula

(2); hemihydrates, monohydrates, dihydrates, trihydrates, tetrahydrates, pentahydrates, hexahydrates, heptahydrates, octahydrates, nonahydrates, decahydrates, or monoethanolates of sodium salts of the compound represented by the formula (2); or hydrates or ethanolates of hydrochlorides of the compound represented by the formula (2). The hydrate or solvate may be produced in a crystalline form or non-crystalline form. In the case of the crystalline form, the hydrate or solvate may have crystalline polymorphs. As for the method for producing the hydrate or solvate, the hydrate or solvate can be obtained by a conventional method, for example, by adding a solvent such as ethanol and/or water to the compound represented by the formula (2) or the peptide compound described herein, followed by stirring, cooling, concentrating, and/or drying.

[Formula 30]

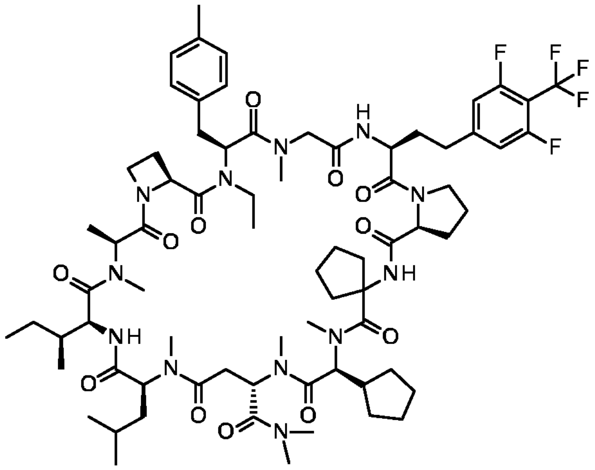

(2)

When the compound according to the present invention is obtained as a salt, a hydrate, or a solvate of the compound, the compound can be conventionally transformed into a free form thereof.

The meaning of the term "and/or" as used herein includes any combination in which "and" and "or" are appropriately combined. Specifically, for example, the term "A, B and/or C" includes the following seven variations: (i) A, (ii) B, (iii) C, (iv) A and B, (v) A and C, (vi) B and C, and (vii) A, B and C.

The term "epimer of a cyclic peptide compound" as used herein means a compound in which a configuration of a side chain attached to α-carbon of an amino acid residue constituting a cyclic peptide compound is sterically inverted (epimer). The "epimer of a cyclic peptide compound" includes a cyclic peptide compound in which the α-carbon of the C-terminal amino acid residue of a linear peptide compound is sterically inverted when the linear peptide compound is cyclized to produce the cyclic peptide compound. The epimer of a cyclic peptide compound in the total products including the cyclic peptide compound produced by the method of the present invention can be determined, for example, by UVarea value at 210 nm or 220 nm by HPLC analysis.

The term "epimerization" as used herein refers to inverting the steric configuration on at least one asymmetric carbon in a plurality of asymmetric carbons present in a molecule.

The term "oligomer " as used herein means a compound in which two or more peptide compounds, which are starting materials of a cyclic peptide compound, are bound together. In particular, a compound formed by bonding two peptide compounds is referred to as a "dimer", and a compound formed by bonding three peptide compounds is referred to as a "trimer". The dimer may be a compound in which peptide compounds are linearly bound to each other, or a cyclic dimer peptide compound (also referred to as a "cyclic dimer") in which peptide compounds are linearly bound and then further cyclized. The trimer may be a compound in which peptide compounds are linearly bound to each other, or a cyclic trimer peptide compound (also referred to as a "cyclic trimer") in which peptide compounds are linearly bound and then further cyclized. The oligomer may be a compound in which peptide compounds are linearly bound to each other, or a cyclic oligomer peptide compound in which peptide compounds are linearly bound and then further cyclized. The oligomer (preferably dimer, trimer, more preferably cyclic dimer, cyclic trimer) in the total products including the cyclic peptide compound produced by the method of the present invention can be determined, for example, by UVarea value at 210 nm or 220 nm by HPLC analysis.

Method for producing cyclic peptide compound

In an aspect, the present invention relates to a method for producing a cyclic peptide compound, or a salt thereof, or a solvate thereof, the method comprising a step of linking an N-terminal amino acid residue of a peptide compound with a

C-terminal amino acid residue of the peptide compound in a solvent, wherein at least one of the N-terminal amino acid residue and the C-terminal amino acid residue is a cyclic amino acid residue, and the solvent comprises one or more selected from the group consisting of acetonitrile, dimethyl carbonate, 2-MeTHF, and anisole. Hereinafter, this aspect is also referred to as "aspect 1".

In aspect 1, it is preferable that the N-terminal amino acid residue of the peptide compound is a cyclic amino acid residue, or the C-terminal amino acid residue of the peptide compound is a cyclic amino acid residue.

In aspect 1, it is preferable that the N-terminal amino acid residue of the peptide compound is a cyclic amino acid residue and the C-terminal amino acid residue is a non-natural amino acid residue, or the N-terminal amino acid residue of the peptide compound is a non-natural amino acid residue and the C-terminal amino acid residue is a cyclic amino acid residue.

In aspect 1, it is preferable that the N-terminal amino acid residue of the peptide compound is a cyclic amino acid residue and the C-terminal amino acid residue is a homophenylalanine residue, a homophenylalanine derivative residue, or an N-substituted Ala residue, or the N-terminal amino acid residue of the peptide compound is an N-substituted phenylalanine residue, an N-substituted phenylalanine derivative residue, or an $\alpha,\alpha$-disubstituted amino acid residue and the C-terminal amino acid residue is a cyclic amino acid residue.

In aspect 1, it is more preferable that the N-terminal amino acid residue of the peptide compound is an N-substituted phenylalanine derivative residue, and the C-terminal amino acid residue is a cyclic amino acid residue.

In aspect 1, it is more preferable that the N-terminal amino acid residue of the peptide compound is an $\alpha,\alpha$-disubstituted amino acid residue, and the C-terminal amino acid residue is a cyclic amino acid residue.

In aspect 1, it is more preferable that the N-terminal amino acid residue of the peptide compound is a cyclic amino acid residue, and the C-terminal amino acid residue is a homophenylalanine derivative residue.

In aspect 1, more preferably, the N-terminal amino acid residue of the peptide compound is a cyclic amino acid residue, and the C-terminal amino acid residue is an N-substituted Ala residue.

In an aspect, the present invention relates to a method for producing a cyclic peptide compound, or a salt thereof, or a solvate thereof, the method comprising a step of linking an N-terminal amino acid residue of a peptide compound with a C-terminal amino acid residue of the peptide compound in a solvent, wherein the N-terminal amino acid residue and the C-terminal amino acid residue of the peptide compound are one selected from the following a) to e):

a) the N-terminal amino acid residue is an N-substituted phenylalanine residue or an N-substituted phenylalanine derivative residue, and the C-terminal amino acid residue is a cyclic amino acid residue;
b) the N-terminal amino acid residue is an $\alpha,\alpha$-di-substituted amino acid residue, and the C-terminal amino acid residue is a cyclic amino acid residue;
c) the N-terminal amino acid residue is a cyclic amino acid residue, and the C-terminal amino acid residue is a homophenylalanine residue or a homophenylalanine derivative residue;
d) the N-terminal amino acid residue is a cyclic amino acid residue, and the C-terminal amino acid residue is an N-substituted Ala residue; and
e) the N-terminal amino acid residue is an N-substituted Gly residue, and the C-terminal amino acid residue is an N-substituted phenylalanine residue or an N-substituted phenylalanine derivative residue. Hereinafter, this aspect is also referred to as "aspect 2".

In an aspect, the cyclic amino acid residue is one selected from a Pro residue, a proline derivative residue, and an Aze(2) residue, preferably one selected from a Pro residue and an Aze(2) residue, more preferably a Pro residue or an Aze(2) residue.

In an aspect, the N-substituted phenylalanine derivative residue is an N-alkylphenylalanine derivative residue, preferably an EtPhe derivative residue, more preferably an EtPhe(4-Me) residue.

In an aspect, the homophenylalanine derivative residue is an Hph(3,5-diF-4-$CF_3$) residue.

In an aspect, the $\alpha,\alpha$-di-substituted amino acid residue is a cLeu residue.

In an aspect, the N-substituted Ala residue is an N-alkylAla residue, preferably a MeAla residue.

In an aspect, the N-substituted Gly residue is an N-alkylGly residue, preferably a MeGly residue.

In aspect 2, the N-terminal amino acid residue and the C-terminal amino acid residue preferably contained in the peptide compound are one selected from the following a') to e'):

a') the N-terminal amino acid residue is an EtPhe(4-Me) residue, and the C-terminal amino acid residue is an Aze(2) residue;
b') the N-terminal amino acid residue is a cLeu residue, and the C-terminal amino acid residue is a Pro residue;
c') the N-terminal amino acid residue is a Pro residue, and the C-terminal amino acid residue is a Hph(3,5-diF-4-$CF_3$) residue;
d') the N-terminal amino acid residue is a MeGly residue, and the C-terminal amino acid residue is an EtPhe(4-Me)

residue; and

e') the N-terminal amino acid residue is an Aze(2) residue, and the C-terminal amino acid residue is a MeAla residue.

In aspect 2, the solvent preferably comprises one or more selected from the group consisting of acetonitrile, dimethyl carbonate, 2-MeTHF, and anisole.

In an aspect, the solvent is one selected from the group consisting of acetonitrile, dimethyl carbonate, 2-MeTHF, and anisole, preferably one selected from the group consisting of acetonitrile and dimethyl carbonate, and more preferably acetonitrile or dimethyl carbonate.

In an aspect, the peptide compound of the present invention may be a linear peptide compound. In another aspect, the peptide compound of the present invention may be a cyclic peptide compound. In an aspect, the linear or cyclic peptide compound may comprise a cyclic structure as its moiety structure. Specific examples of the cyclic structure include those in which the side chain of one amino acid residue is linked to a side chain of another amino acid residue, those in which the N-substituent of one amino acid residue is linked to the side chain of another amino acid residue, and those in which the N-substituent of one amino acid residue is linked to the N-substituent of another amino acid residue. The two amino acid residues involved in the linking for the cyclic structure may be adjacent, or any number of amino acid residues, for example, 1, 2, 3, 4, 5, 6, 7, 8, 9, 10, 11, 12, 13, 14, 15, 16, 17, 18, or 19 amino acid residues may be present therebetween. Examples of the size of the ring formed by the cyclic structure include, but are not intended to be particularly limited to, a 4-membered ring, a 5-membered ring, a 6-membered ring, a 7-membered ring, an 8-membered ring, a 9-membered ring, a 10-membered ring, an 11-membered ring, a 12-membered ring, a 13-membered ring, a 14-membered ring, a 15-membered ring, a 16-membered ring, a 17-membered ring, an 18-membered ring, a 19-membered ring, a 20-membered ring, a 21-membered ring, a 22-membered ring, a 23-membered ring, a 24-membered ring, a 25-membered ring, a 26-membered ring, a 27-membered ring, a 28-membered ring, a 29-membered ring, a 30-membered ring, a 31-membered ring, a 32-membered ring, a 33-membered ring, a 34-membered ring, and a 35-membered ring. When a cyclic structure is present in the peptide compound, the number of cyclic structures is not limited, but it is preferable that one, two, three, four, or five cyclic structures be present.

**In** an aspect, the linking an N-terminal amino acid residue of a peptide compound with a C-terminal amino acid residue of the peptide compound is linking an amino group of the N-terminal amino acid residue with a carboxyl group of the C-terminal amino acid residue. In an aspect, an amino group of the N-terminal amino acid residue and a carboxyl group of the C-terminal amino acid residue of the peptide compound are linked by an amide bond.

When an N-terminal amino acid residue and a C-terminal amino acid residue of a peptide compound are linked by an amide bond, a cyclic peptide compound, or a salt thereof, or a solvate thereof can be produced by condensing an amino group of the N-terminal amino acid residue with a carboxyl group of the C-terminal amino acid residue. The amide bond may be formed between an amino group in the main chain of the N-terminal amino acid residue and a carboxyl group in the main chain of the C-terminal amino acid residue, may be formed between an amino group in the main chain of the N-terminal amino acid residue and a carboxyl group in the side chain of the C-terminal amino acid residue, may be formed between an amino group in the side chain of the N-terminal amino acid residue and a carboxyl group in the main chain of the C-terminal amino acid residue, and may be formed between an amino group in the side chain of the N-terminal amino acid residue and a carboxyl group in the side chain of the C-terminal amino acid residue. At the time of the condensation, the carboxyl group may be activated in the system using a condensation reagent, or the carboxyl group may be converted to an active ester in advance. The term "condensation of an amino group and a carboxyl group" as used herein refers to a condensation in which an amino group and a carboxyl group are linked by an amide bond.

In an aspect, the linking step can be performed by stirring the reaction mixture in a solvent in the presence or absence of a condensation reagent, in the presence or absence of a base, at a temperature between -20°C and near the boiling point of the solvent, preferably between -20°C and 100°C, preferably between -5°C and 60°C, for 10 minutes to 48 hours. When a condensation reagent is used in the linking step, the condensation reagent or a solution containing the condensation reagent may be added to a solution containing the peptide compound and a base, or a solution containing starting materials and optionally a base may be added to a solution containing the condensation reagent. When a condensation reagent is not used in the linking step, the carboxyl group may be converted to an active ester in advance.

The condensation reagent, the base, and the use amounts thereof when linking the amino group with the carboxyl group by an amide bond is not particularly limited as long as they can form the amide bond, but preferably a condensation reagent, a base and use amounts commonly used in peptide synthesis (see, for example, Peptide Coupling Reagents, More than a Letter Soup (Chem. Rev. 2011, 111, 6557-6602.)).

Specific examples of the condensation reagent include N,N'-dicyclohexylcarbodiimide (DCC), N,N'-diisopropylcarbodiimide (DIC), 1-ethyl-3-(3-dimethylaminopropyl)carbodiimide hydrochloride (EDCI HCl), 1-hydroxy-1H-benzotriazole (HOBt), 1-hydroxy-7-azabenzotriazole (HOAt), 2-cyano-2-(hydroxyimino)ethyl acetate (oxyma), 3,4-dihydro-3-hydroxy-4-oxo-1,2,3-benzotriazine (HOOBt or HODhbt), N-hydroxy-5-norbornene-2,3-dicarboximide (HONB), 2,3,4,5,6-pentafluorophenol (HOPfp), N-hydroxysuccinimide (HOSu), 6-chloro-1-hydroxy-1H-benzotriazole (Cl-HOBt), O-(1H-benzotriazol-1-yl)-N,N,N',N'-tetramethyluronium hexafluorophosphate (HBTU), O-(7-aza-1H-benzotriazol-1-yl)-N,N,N',N'-tetramethyluronium hexafluorophosphate (HATU), N-[1-(cyano-2-ethoxy-2-oxoethylideneaminooxy)dimethylamino(morpholino)]uronium hexafluorophosphate (COMU), O-[(ethoxycarbonyl)cyanomethyleneamino]-N,N,N',N'-tetramethyluronium hexafluorophosphate (HOTU), O-(1H-benzotriazol-1-yl)-N,N,N',N'-tetramethyluronium tetrafluoroborate (TBTU), O-(7-azabenzotriazol-1-yl)-N,N,N',N'-tetramethyluronium tetrafluoroborate (TATU), [ethylcyano(hydroxyimino)acetato-$O^2$]tri-1-pyrrolidinylphosphonium hexafluorophosphate (PyOxim), 2-bromo-1-ethylpyridinium tetrafluoroborate (BEP), 1H-benzotriazole-1-yloxytri(pyrrolidino)phosphonium hexafluorophosphate (PyBOP), 1H-benzotriazole-1-yloxy-tris (dimethylamino)phosphonium hexafluorophosphate (BOP), bromotri(pyrrolidino) phosphonium hexafluorophosphate (PyBroP), chlorotri(pyrrolidino)phosphonium hexafluorophosphate (PyCloP), (7-azabenzotriazol-1-yloxy)tripyrrolidino phosphonium hexafluorophosphate (PyAOP), bromotris(dimethylamino)phosphonium hexafluorophosphate (Brop), 3-(diethoxyphosphoryloxy)-1,2,3-benzotriazin-4(3H)-one (DEPBT), N,N,N',N'-tetramethyl-O-(N-succinimidyl)uronium tetrafluoroborate (TSTU), N,N,N',N'-tetramethyl-O-(N-succinimidyl)uronium hexafluorophosphate (HSTU), O-(3,4-dihydro-4-oxo-1,2,3-benzotriazin-3-yl)-N,N,N',N'-tetramethyluronium tetrafluoroborate (TDBTU), tetramethylthiuronium S-(1-oxide-2-pyridyl)-N,N,N',N'-tetrafluoroborate (TOTT), O-(2-oxo-1(2H)pyridyl)-N,N,N',N'-tetramethyluronium tetrafluoroborate (TPTU), N,N' -carbonyl diimidazole (CDI), 1,1'-carbonyl-di-(1,2,4-triazole) (CDT), 4-(4,6-dimethoxy-1,3,5-triazin-2-yl)-4-methylmorpholinium chloride (DMT-MM), and propylphosphonic anhydride (T3P). Among these, the condensation reagent of the present invention is preferably HATU, COMU, DMT-MM, PyOxim, PyBOP, or PyClop, more preferably HATU or COMU, from the viewpoint of increasing the conversion rate of the cyclization reaction and suppressing byproducts. In addition, the combination of the solvent and the condensation reagent is preferably HATU and dimethyl carbonate or acetonitrile, or COMU and dimethyl carbonate or acetonitrile because it allows further suppression of epimerization and formation of dimer and trimer byproducts.

As the base, organic bases are suitably used, and particularly, organic bases containing tertiary amines are preferred. Specific examples of the base include N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP). Among them, N,N-diisopropylethylamine (DIPEA) is preferred as the base of the present invention, from the viewpoint of increasing the conversion rate of cyclization reaction and suppressing byproducts. In addition, the combination of the solvent, the condensation reagent, and the base is preferably HATU, dimethyl carbonate, and N,N-diisopropylethylamine (DIPEA), HATU, acetonitrile, and N,N-diisopropylethylamine (DIPEA), COMU, dimethyl carbonate, and N,N-diisopropylethylamine (DIPEA), or COMU, acetonitrile, and N,N-diisopropylethylamine (DIPEA), because epimerization and formation of dimer and trimer byproducts can be further suppressed.

In an aspect, the cyclic peptide compound produced by the method of the present invention may comprise 8 to 20, preferably 9 to 15 amino acid residues, and at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, or at least 19 of the amino acid residues may be non-natural amino acid residues. In an aspect, the proportion of the non-natural amino acids contained in a cyclic peptide compound produced by the method of the present invention is, for example, 30% or more, 40% or more, 50% or more, 60% or more, 70% or more, or 80% or more with respect to the total number of amino acids contained in the peptide compound.

The non-natural amino acid residue contained in the cyclic peptide compound may be an N-substituted non-natural amino acid residue or an N-non-substituted non-natural amino acid residue. An amino acid residue in which the amino group in the main chain of the natural amino acid is substituted with an atom or functional group other than hydrogen, or an amino acid residue that has a different structure from natural amino acids in the side chain, and in which the amino group in the main chain is substituted with an atom or functional group other than hydrogen, fall under the N-substituted non-natural amino acid residue. An amino acid residue in which the amino group in the main chain is not substituted but which has a different structure from natural amino acids in the side chain falls under the N-non-substituted non-natural amino acid residue.

In an aspect, a cyclic peptide compound produced by the method of the present invention can comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, or at least 19 N-substituted amino acid residues. In an aspect, the proportion of the N-substituted amino acid residues contained in a cyclic peptide compound produced by the method of the present invention is, for example, 30% or more, 40% or more, 50% or more, 60% or more, 70% or more, or 80% or more with respect to the total number of amino acids contained in the peptide compound. The N-substituted amino acid residue may be an N-substituted non-natural amino acid residue.

In an aspect, a cyclic peptide compound produced by the method of the present invention can comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, or at least 19 N-non-substituted non-natural amino acid residues. In an aspect, the proportion of the N-non-substituted non-natural amino acid residues contained in a cyclic

peptide compound produced by the method of the present invention is, for example, 30% or more, 40% or more, 50% or more, 60% or more, 70% or more, or 80% or more with respect to the total number of amino acids contained in the peptide compound.

In an aspect, a cyclic peptide compound produced by the method of the present invention can comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, or at least 19 α,α-di-substituted amino acid residues. In an aspect, the proportion of the α,α-di-substituted amino acid residues contained in a cyclic peptide compound produced by the method of the present invention is, for example, 5% or more, 10% or more, 20% or more, 30% or more, 40% or more, 50% or more, 60% or more, 70% or more, or 80% or more with respect to the total number of amino acids contained in the peptide compound.

The cyclic peptide compound produced by the method of the present invention preferably comprises 9 to 15, and more preferably comprises 11 amino acid residues. In the cyclic peptide compound, one or more, two or more, three or more, four or more, five or more, or six or more of the amino acid residues may be N-substituted amino acid residues, and one or more, or two or more of the amino acid residues may be N-non-substituted non-natural amino acid residues. The method of the present invention is particularly useful for the large-scale production of a cyclic peptide compound containing a large number of such non-natural amino acid residues.

In an aspect, the C-terminal amino acid residue contained in the peptide compound is an amino acid residue having a side chain on an α-carbon of a carboxyl group.

In an aspect, the peptide compound comprises an amino acid residue of formula (1) at any one of amino acid residues other than the N-terminal amino acid residue and the C-terminal amino acid residue of the peptide compound:

[Formula 31]

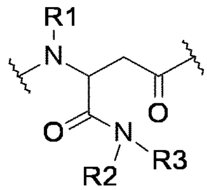

(1)

In the formula, $R^1$ is hydrogen or $C_1$-$C_6$ alkyl; and $R^2$ and $R^3$ are each independently hydrogen or $C_1$-$C_6$ alkyl, or $R^2$ and $R^3$ together with a nitrogen atom to which they are attached form a 4- to 7-membered saturated heterocycle.

In amino acid residues of the formula (1), $R^1$ is preferably $C_1$-$C_6$ alkyl, more preferably methyl.

In amino acid residues of the formula (1), $R^2$ and $R^3$ are, each independently, preferably $C_1$-$C_6$ alkyl, more preferably methyl.

In another aspect of amino acid residues of the formula (1), $R^1$ is $C_1$-$C_6$ alkyl; and $R^2$ and $R^3$ are each independently $C_1$-$C_6$ alkyl, or $R^2$ and $R^3$ together with a nitrogen atom to which they are attached form a 4- to 7-membered saturated heterocycle.

In another aspect of amino acid residues of the formula (1), $R^1$ is preferably methyl; and $R^2$ and $R^3$ each are methyl.

In an aspect, the peptide compound is a linear peptide compound. The linear peptide compound preferably comprises 9 to 15, more preferably 11 amino acid residues.

In an aspect, the peptide compound is a linear peptide compound selected from the group consisting of:

[Formula 32]

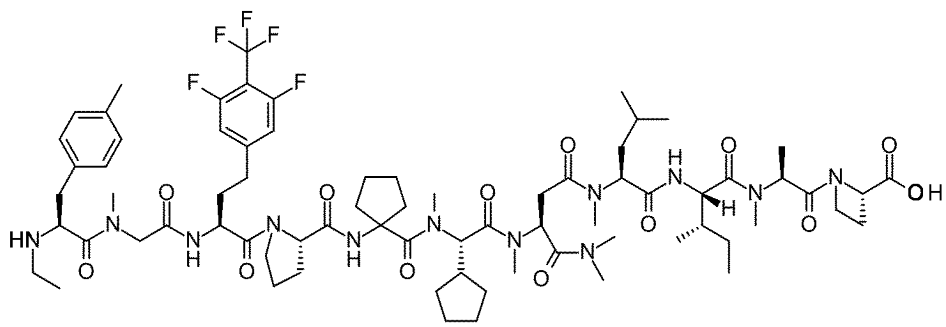

,

[Formula 33]

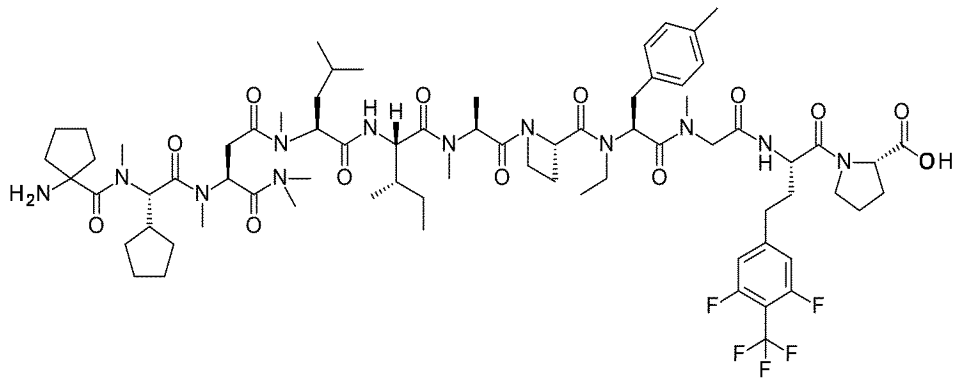

,

[Formula 34]

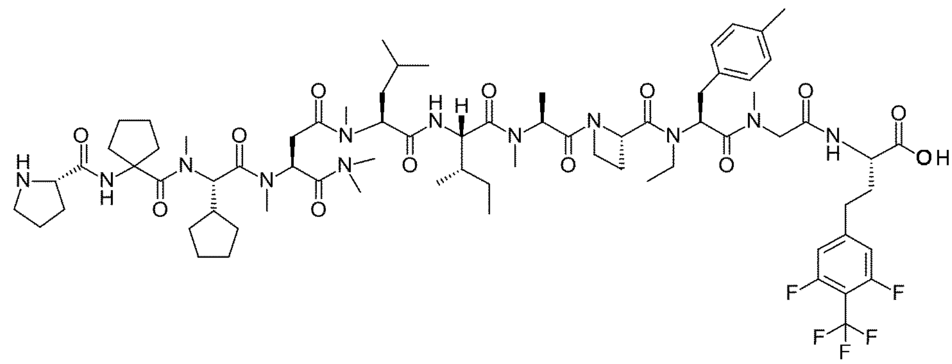

,

[Formula 35]

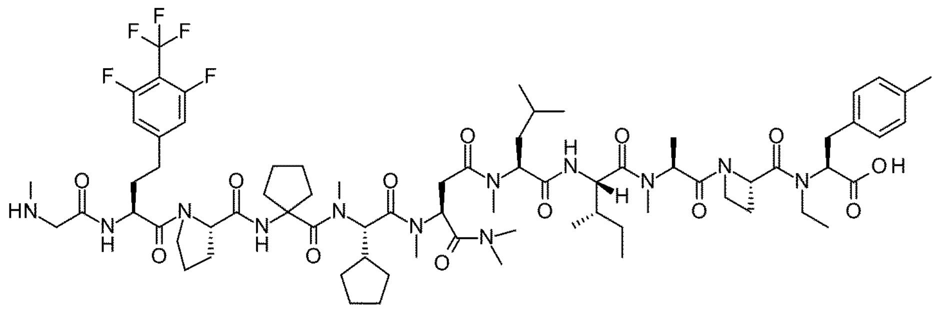

,

and

[Formula 36]

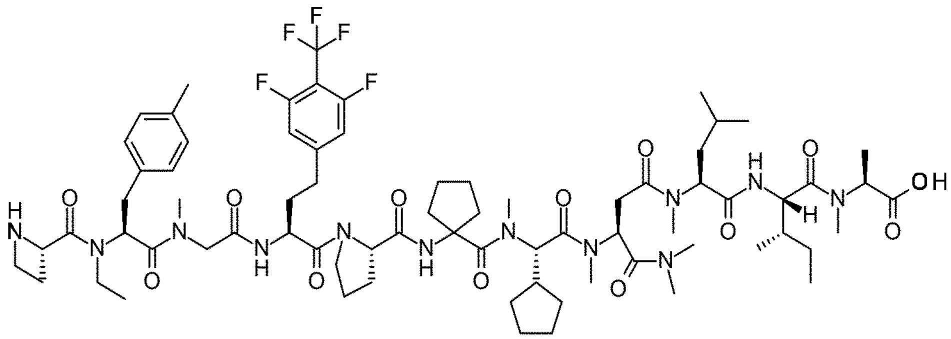

or a salt thereof, or a solvate thereof.

In an aspect, the cyclic peptide compound produced by the method of the present invention is preferably a solvate, more preferably a hydrate, a DMSO-hydrate, an acetone-hydrate, or a DMSO-solvate, further preferably a hydrate.

In an aspect, the cyclic peptide compound produced by the method of the present invention is a cyclic peptide compound represented by the following formula (2):

[Formula 37]

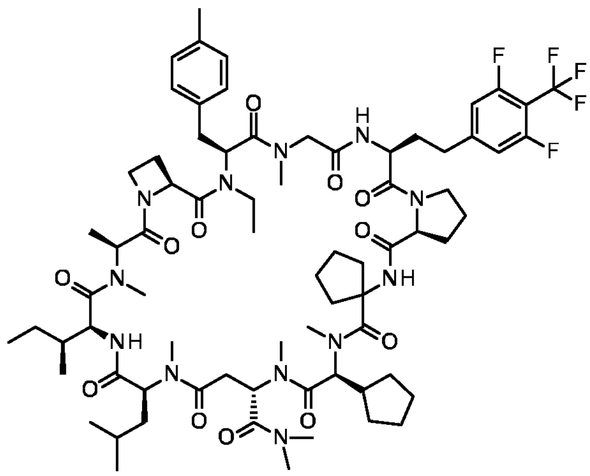

(2)

or a salt thereof, or a solvate thereof. As described in International Publication No. WO2021/090855, the cyclic peptide compound represented by the above formula is useful as a KRAS inhibitor and can be used for a variety of KRAS-related diseases, e.g., KRAS-related cancers.

In an aspect, it is preferable that column chromatography is not used in the isolation and/or purification of the cyclic peptide compound, or a salt thereof, or a solvate thereof produced by the method of the present invention.

The cyclic peptide compound, or a salt thereof, or a solvate thereof produced by the method of the present invention can be isolated and/or purified by, for example, crystallization, instead of using column chromatography.

Specifically, for example, the reaction solution after the condensation reaction may be subjected to a separate operation, the organic layer be concentrated and/or filtered as necessary, then a solvent suitable for crystallization be added to the obtained residue, optionally a seed crystal is added, and stirred as necessary to obtain a crystal of the cyclic peptide compound, or a salt thereof, or a solvate thereof. The solvent added during crystallization is not particularly limited as long as it is a solvent with which the cyclic peptide compound can be crystallized, but preferably a solvent with which an operation to reduce the solubility of the cyclic peptide compound can be performed for a solution in which the cyclic peptide compound is dissolved. For example, when a peptide compound can be crystallized by reducing the solubility of the cyclic peptide compound by addition of a poor solvent or the cooling of a solution, a solvent capable of such operation can be used. In addition, when a crystal of the cyclic peptide compound can be obtained by maintaining a crude crystal of the cyclic peptide compound in a suspension state for any period of time, a solvent capable of such manipulation can be used for

crystallization. Specific examples of the solvent added during crystallization include acetone, water, DMSO, acetonitrile, ethanol, and a mixed solvent thereof.

In an aspect, the crystal of the cyclic peptide compound, or a salt thereof, or a solvate thereof produced by the method of the present invention can be a non-solvate crystal, a solvate crystal, a crystal of a salt, or a solvate crystal of a salt of the above compound of formula (2), as described below. In an aspect, the non-solvate crystal (solvate-free crystal) may refer to a crystal other than solvate crystals or hydrate crystals. The crystal of the cyclic peptide compound represented by the formula (2), or a salt thereof, or a solvate thereof, is preferably a solvate crystal, more preferably a hydrate crystal.

In an aspect, the method of the present invention further comprises a step of providing a peptide compound. The peptide compound can be produced, for example, by repeating step 1 and step 2 below, and optionally step 1 and step 2 multiple times (preferably 2 to 20 times), and/or step 1 and step 3 below, and optionally step 1 and step 3 multiple times (preferably 2 to 20 times), and finally step 1 and step 4:

(Step 1) linking/condensing the C-protecting amino acid or C-protecting peptide with the N-protecting amino acid or N-protecting peptide;
(Step 2) removing/deprotecting the N-protecting group after step 1;
(Step 3) removing/deprotecting the C-protecting group after step 1;
(Step 4) removing/deprotecting the C-protecting group, followed by the N-protecting group, after step 1.

The term "C-protected amino acid" as used herein means a natural or non-natural amino acid in which a carboxyl group is protected, and the term "C-protected peptide" means a peptide in which a carboxyl group of a C-terminal amino acid residue is protected. The peptide may be composed of only natural amino acid residues, only non-natural amino acid residues, or any combination of natural and non-natural amino acid residues.

Any protecting group known in the art can be used as the protecting group for the carboxy group of "C-protected amino acid" and "C-protected peptide". The solubility of the C-protected amino acid and C-protecting peptide is at least 1% (w/v) or more, further preferably 5% (w/v) or more, in a solvent used in the reaction. Specific examples of such a protecting group for the carboxy group include a methyl group, an ethyl group, a t-Bu group, a trityl group, and a cumyl group, and among these, a t-Bu group is preferred.

The term "N-protected amino acid" as used herein means a natural or non-natural amino acid in which an amino group is protected, and the term "N-protected peptide" means a peptide in which an amino group of an amino acid residue at the N-terminus is protected. The peptide may be composed of only natural amino acid residues, only non-natural amino acid residues, or any combination of natural and non-natural amino acid residues.

Any protecting group known in the art can be used as the protecting group for the amino group of "N-protected amino acid" and "N-protected peptide". Specific examples of such a protecting group for the amino group include Cbz, p-nitrobenzyloxycarbonyl, 2-naphthylmethyloxycarbonyl, diphenylmethyloxycarbonyl, 9-anthrylmethyloxycarbonyl, Teoc, Boc, trifluoroacetyl, or Alloc, and among these, Cbz, Teoc, or trifluoroacetyl is preferred.

As is known in the art, protecting groups for each of the N-protected and C-protected amino acids and/or N-protected and C-protected peptides are typically selected according to chemical reaction conditions and can be determined by conventional methods known in the art. For example, when a solvent that is not miscible with water (e.g., a lipophilic solvent) is used, a hydrophilic protecting group may reduce the solubility of the protected compound in the organic solvent, thus such a hydrophilic protecting group may not be a suitable protecting group. Thus, when using a solvent that is not miscible with water as described herein, the lipophilic protecting group can be a preferred protecting group because, for example, it can maintain the solubility of a peptide compound in a solvent that is not miscible with water.

The selection of such a protecting group can be performed by methods known in the art or described herein, such as the methods described in "Greene's Protective Groups in Organic Synthesis, Fifth Edition, 2014". As a non-limiting embodiment, examples of the N-protecting group that may be used in the method of the present invention include a Cbz group. When an amino acid has a sterically disturbing functional group such as a spiro-cycloalkyl group at position $\alpha$ in the amino acid residue, preferred examples include trifluoroacetyl.

In an aspect, the linking step in the production method of the present invention is performed by a liquid phase method.

In an aspect, the linking step in the production method of the present invention is performed by mixing a peptide compound and optionally a base in a mixed solution obtained by mixing a solvent and a condensation reagent. The operation may be herein referred to as "inverse dropwise addition". Inverse dropwise addition of the peptide compound and the base over a long period of time, e.g., several hours to several days, preferably 1 to 24 hours, more preferably 1 to 10 hours, can suppress formation of dimer and trimer byproducts without using a large amount of solvent for dilution.

The cyclic peptide compounds produced by the method of the present invention are of high purity with a low content of byproducts (e.g., epimers, dimers, and trimers) as described below.

In an aspect, a content of the total byproducts formed in the linking step in the production method of the present invention is less than 20%, less than 15%, less than 10%, less than 5%, or less than 3%, as determined by UVarea value at

220 nm using HPLC analysis, based on the total amount of products.

In an aspect, a content of each of byproducts formed in the linking step in the production method of the present invention is less than 15%, less than 10%, less than 5%, less than 3%, less than 1%, or an undetectable amount, as determined by UVarea value at 220 nm using HPLC analysis, based on the total amount of products, and the byproducts are an epimer, a dimer, and a trimer.

In an aspect, byproducts formed in the linking step in the production method of the present invention comprise an epimer of the cyclic peptide compound, and a content of the epimer is less than 10%, less than 7.5%, less than 5%, less than 2.5%, or less than 1%, as determined by UVarea value at 220 nm using HPLC analysis, based on the total amount of products.

In an aspect, byproducts formed in the linking step in the production method of the present invention comprise a dimer, and a content of the dimer is less than 15%, less than 10%, less than 5%, less than 2.5%, or less than 1%, as determined by UVarea value at 220 nm using HPLC analysis, based on the total amount of products.

In an aspect, byproducts formed in the linking step in the production method of the present invention comprise a trimer, and a content of the trimer is less than 5%, less than 2.5%, less than 1%, or an undetectable amount, as determined by UVarea value at 220 nm using HPLC analysis, based on the total amount of products.

In an aspect, byproducts formed in the linking step in the production method of the present invention comprise a dimer and a trimer, and the total content of the dimer and the trimer is less than 15%, less than 10%, less than 5%, less than 2.5%, or less than 1%, as determined by UVarea value at 220 nm using HPLC analysis, based on the total amount of products.

In an aspect, the present invention relates to a method for producing a cyclic peptide compound represented by the following formula (2):

[Formula 39]

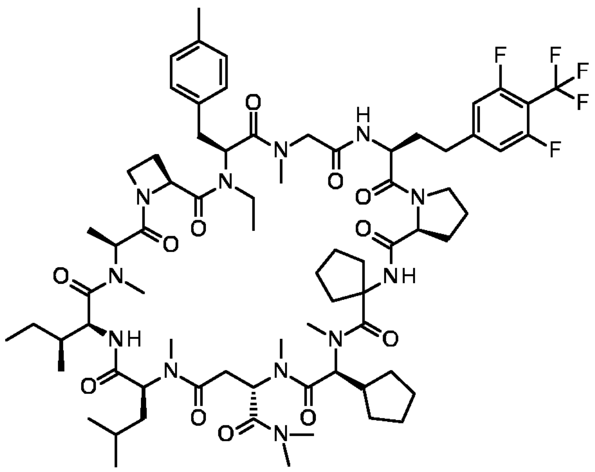

(2)

or a salt thereof, or a solvate thereof, the method comprising steps of:

(1) providing a linear peptide compound represented by the following formula:

[Formula 38]

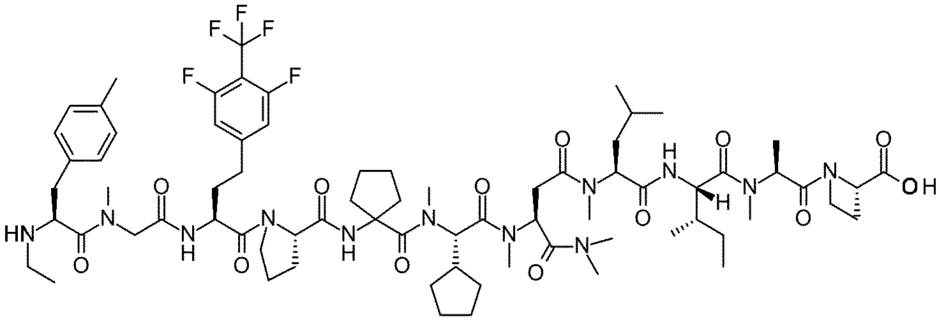

or a salt thereof, or a solvate thereof (compound B13); and

(2) linking an N-terminal amino acid residue of the linear peptide compound, or a salt thereof, or a solvate thereof with a C-terminal amino acid residue of the linear peptide compound, or a salt thereof, or a solvate thereof. Hereinafter, this aspect is also referred to as "aspect 3".

In aspect 3, the step (1) can provide the linear peptide compound, or a salt thereof, or a solvate thereof (compound B13), according to, for example, the methods described in Examples B-1 to B-13 below.

In aspect 3, the step (2) is preferably performed in the presence of a solvent. The solvent used in the step (2) preferably comprises one or more selected from the group consisting of acetonitrile, dimethyl carbonate, and 2-MeTHF, and more preferably is one selected from the group consisting of acetonitrile, dimethyl carbonate, and 2-MeTHF, further preferably one selected from the group consisting of acetonitrile and dimethyl carbonate.

In aspect 3, the step (2) is preferably performed in the presence of a condensation reagent. The condensation reagent used in the step (2) is preferably one selected from the group consisting of COMU, HATU, PyBOP, PyOxim, PyClop, DMT-MM, DEPBT, FDPP, T3P, and BEP-BF4, more preferably one selected from the group consisting of COMU and HATU.

In aspect 3, a combination of the condensation reagent and the solvent is preferably a combination of one selected from the group consisting of COMU and HATU, and one or more selected from the group consisting of acetonitrile, dimethyl carbonate, and 2-MeTHF, more preferably a combination of one selected from the group consisting of COMU and HATU, and one selected from the group consisting of acetonitrile and dimethyl carbonate.

In aspect 3, the step (2) is preferably performed in the presence of a base. The base used in the step (2) is preferably an organic base, more preferably an organic base comprising a tertiary amine, further preferably one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP), and particularly preferably N,N-diisopropylethylamine (DIPEA).

In aspect 3, a combination of the condensation reagent and the solvent and the base is preferably a combination of one selected from the group consisting of COMU, HATU, PyBOP, PyOxim, PyClop, DMT-MM, DEPBT, FDPP, T3P, and BEP-BF4; one or more selected from the group consisting of acetonitrile, dimethyl carbonate, and 2-MeTHF; and N,N-diisopropylethylamine (DIPEA), more preferably a combination of one selected from the group consisting of COMU and HATU; one or more selected from the group consisting of acetonitrile, dimethyl carbonate, and 2-MeTHF; and N,N-diisopropylethylamine (DIPEA), and further preferably a combination of one selected from the group consisting of COMU and HATU; one selected from the group consisting of acetonitrile and dimethyl carbonate; and N,N-diisopropylethylamine (DIPEA).

In aspect 3, the step (2) is preferably performed by mixing the peptide compound and the base in a mixed solution obtained by mixing the solvent and the condensation reagent. This can suppress formation of dimer and trimer byproducts without using a large amount of solvent for dilution.

In aspect 3, a content of the total byproducts formed is preferably less than 10%, less than 7.5%, less than 5%, less than 3%, or less than 1%, as determined by UVarea value at 220 nm using HPLC analysis, based on the total amount of products.

In aspect 3, a content of each of byproducts formed is preferably less than 10%, less than 7.5%, less than 5%, less than 3%, less than 1%, or an undetectable amount, as determined by UVarea value at 220 nm using HPLC analysis, based on the total amount of products.

In aspect 3, a content of each of byproducts formed is preferably less than 10%, less than 7.5%, less than 5%, less than 3%, less than 1%, or an undetectable amount, as determined by UVarea value at 220 nm using HPLC analysis, based on the total amount of products, and the byproducts are an epimer, a dimer, and a trimer.

In aspect 3, byproducts formed preferably comprise an epimer of the cyclic peptide compound, and a content of the epimer is less than 3%, less than 1%, or an undetectable value, as determined by UVarea value at 220 nm using HPLC analysis, based on the total amount of products.

In aspect 3, byproducts formed preferably comprise a dimer, and a content of the dimer is less than 5%, less than 3%, or less than 1%, as determined by UVarea value at 220 nm using HPLC analysis, based on the total amount of products.

In aspect 3, byproducts formed preferably comprise a trimer, and a content of the trimer is less than 3%, less than 1%, or an undetectable amount, as determined by UVarea value at 220 nm using HPLC analysis, based on the total amount of products.

In aspect 3, byproducts formed preferably comprise a dimer and a trimer, and the total content of the dimer and the trimer is less than 5%, less than 3%, or less than 1%, as determined by UVarea value at 220 nm using HPLC analysis, based on the total amount of products.

In an aspect, the present invention relates to a method for producing a cyclic peptide compound represented by the following formula (2):

[Formula 41]

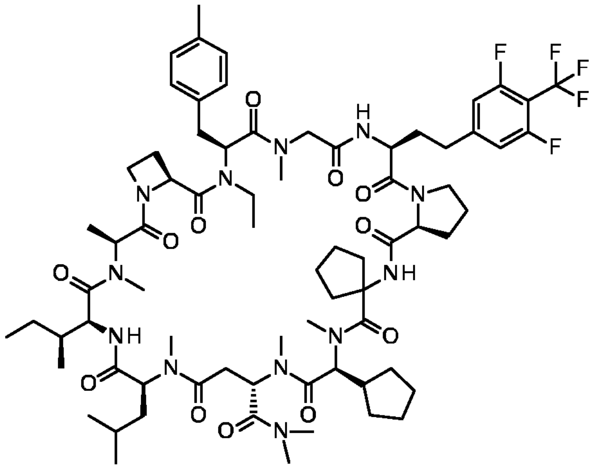

(2)

or a salt thereof, or a solvate thereof, the method comprising steps of:

(1) providing a linear peptide compound represented by the following formula:

[Formula 40]

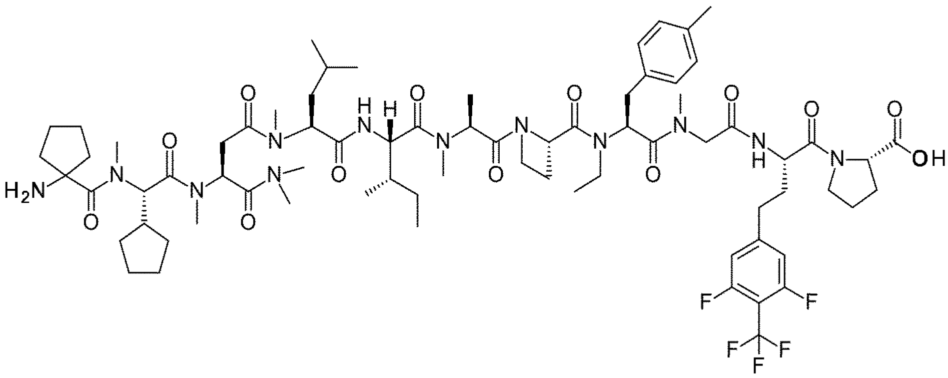

or a salt thereof, or a solvate thereof (compound D21); and
(2) linking an N-terminal amino acid residue of the linear peptide compound, or a salt thereof, or a solvate thereof with a C-terminal amino acid residue of the linear peptide compound, or a salt thereof, or a solvate thereof. Hereinafter, this aspect is also referred to as "aspect 4".

In aspect 4, the step (1) can provide the linear peptide compound, or a salt thereof, or a solvate thereof (compound D21), for example, according to the methods described in Examples D-1 to D-21 below.

In aspect 4, the step (2) is preferably performed in the presence of a solvent. The solvent used in the step (2) preferably comprises one or more selected from the group consisting of acetonitrile, dimethyl carbonate, 2-MeTHF, and anisole, and more preferably is one selected from the group consisting of acetonitrile, dimethyl carbonate, 2-MeTHF, and anisole, and further preferably is dimethyl carbonate.

In aspect 4, the step (2) is preferably performed in the presence of a condensation reagent. The condensation reagent used in the step (2) is preferably one selected from the group consisting of COMU, HATU, PyBOP, PyOxim, PyClop, and DMT-MM, more preferably one selected from the group consisting of COMU and HATU, and further preferably is COMU.

In aspect 4, a combination of the condensation reagent and the solvent is preferably a combination of one selected from the group consisting of COMU, HATU, PyBOP, PyOxim, PyClop, and DMT-MM, and one or more selected

from the group consisting of acetonitrile, dimethyl carbonate, 2-MeTHF, and anisole, more preferably a combination of COMU, and one selected from the group consisting of acetonitrile and dimethyl carbonate, and further preferably a combination of COMU and dimethyl carbonate.

In aspect 4, the step (2) is preferably performed in the presence of a base. The base used in the step (2) is preferably an organic base, more preferably an organic base comprising a tertiary amine, further preferably one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP), and particularly preferably N,N-diisopropylethylamine (DIPEA).

In aspect 4, a combination of the condensation reagent and the solvent and the base is preferably a combination of COMU, one selected from the group consisting of acetonitrile and dimethyl carbonate, and N,N-diisopropylethylamine (DIPEA), and more preferably a combination of COMU, dimethyl carbonate, and N,N-diisopropylethylamine (DIPEA).

In aspect 4, the step (2) is preferably performed by mixing the peptide compound and the base in a mixed solution obtained by mixing the solvent and the condensation reagent. This can suppress formation of dimer and trimer byproducts without using a large amount of solvent for dilution.

In aspect 4, a content of the total byproducts formed is preferably less than 20%, less than 15%, less than 10%, less than 5%, or less than 3%, as determined by UVarea value at 220 nm using HPLC analysis, based on the total amount of products.

In aspect 4, a content of each of byproducts formed is preferably less than 15%, less than 10%, less than 7.5%, less than 5%, less than 2.5%, or less than 1%, as determined by UVarea value at 220 nm using HPLC analysis, based on the total amount of products.

In aspect 4, a content of each of byproducts formed is preferably less than 15%, less than 10%, less than 7.5%, less than 5%, less than 2.5%, or less than 1%, as determined by UVarea value at 220 nm using HPLC analysis, based on the total amount of products, and the byproducts are an epimer, a dimer, and a trimer.

In aspect 4, byproducts formed preferably comprise an epimer of the cyclic peptide compound, and a content of the epimer is less than 3%, less than 1%, or an undetectable value, as determined by UVarea value at 220 nm using HPLC analysis, based on the total amount of products.

In aspect 4, byproducts formed preferably comprise a dimer, and a content of the dimer is less than 15%, less than 10%, or less than 5%, as determined by UVarea value at 220 nm using HPLC analysis, based on the total amount of products.

In aspect 4, byproducts formed preferably comprise a trimer, and a content of the trimer is less than 5%, less than 3%, or less than 1%, as determined by UVarea value at 220 nm using HPLC analysis, based on the total amount of products.

In aspect 4, byproducts formed preferably comprise a dimer and a trimer, and the total content of the dimer and the trimer is less than 20%, less than 15%, less than 10%, less than 5%, or less than 3%, as determined by UVarea value at 220 nm by HPLC analysis, based on the total amount of products.

In an aspect, the present invention relates to a method for producing a cyclic peptide compound represented by the following formula (2):

[Formula 43]

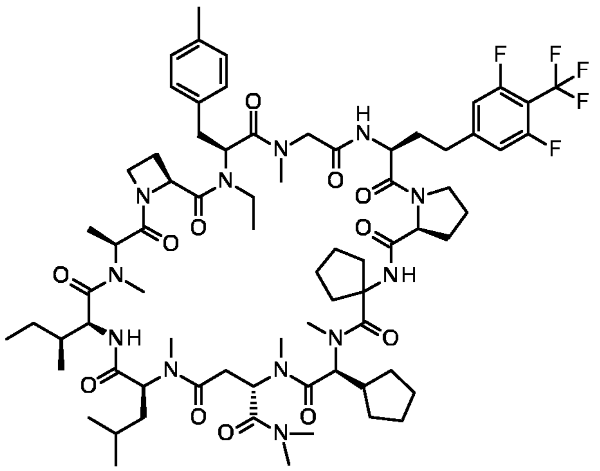

(2)

or a salt thereof, or a solvate thereof, the method comprising steps of:

(1) providing a linear peptide compound represented by the following formula:

[Formula 42]

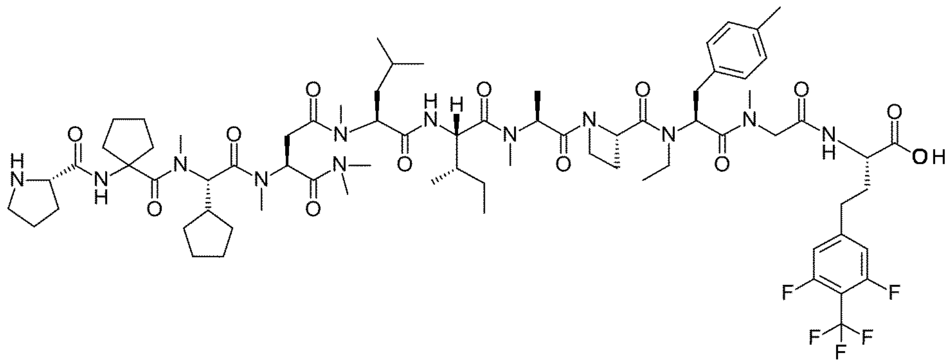

or a salt thereof, or a solvate thereof (compound E9); and

(2) linking an N-terminal amino acid residue of the linear peptide compound, or a salt thereof, or a solvate thereof with a C-terminal amino acid residue of the linear peptide compound, or a salt thereof, or a solvate thereof. Hereinafter, this aspect is also referred to as "aspect 5".

In aspect 5, the step (1) can provide the linear peptide compound, or a salt thereof, or a solvate thereof (compound E9), for example, according to the methods described in Examples E-1 to E-9 below.

In aspect 5, the step (2) is preferably performed in the presence of a solvent. The solvent used in the step (2) preferably comprises one or more selected from the group consisting of acetonitrile, dimethyl carbonate, 2-MeTHF, and anisole, and more preferably is one selected from the group consisting of acetonitrile, dimethyl carbonate, 2-MeTHF, and anisole, and further preferably is dimethyl carbonate.

In aspect 5, the step (2) is preferably performed in the presence of a condensation reagent. The condensation reagent used in the step (2) is preferably one selected from the group consisting of COMU, HATU, PyBOP, PyOxim, PyClop, and DMT-MM, more preferably one selected from the group consisting of COMU and HATU, and further preferably is COMU.

In aspect 5, a combination of the condensation reagent and the solvent is preferably a combination of one selected from the group consisting of COMU, HATU, PyBOP, PyOxim, PyClop, and DMT-MM, and one or more selected from the group consisting of acetonitrile, dimethyl carbonate, 2-MeTHF, and anisole, more preferably a combination of one selected from the group consisting of COMU and HATU, and one selected from the group consisting of acetonitrile and dimethyl carbonate, and further preferably a combination of COMU and dimethyl carbonate.

In aspect 5, the step (2) is preferably performed in the presence of a base. The base used in the step (2) is

preferably an organic base, more preferably an organic base comprising a tertiary amine, further preferably one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP), and particularly preferably N,N-diisopropylethylamine (DIPEA).

In aspect 5, a combination of the condensation reagent and the solvent and the base is preferably a combination of one selected from the group consisting of COMU and HATU; one selected from the group consisting of acetonitrile and dimethyl carbonate; and N,N-diisopropylethylamine (DIPEA), and more preferably a combination of COMU, dimethyl carbonate, and N,N-diisopropylethylamine (DIPEA).

In aspect 5, the step (2) is preferably performed by mixing the peptide compound and the base in a mixed solution obtained by mixing the solvent and the condensation reagent. This can suppress formation of dimer and trimer byproducts without using a large amount of solvent for dilution.

In aspect 5, a content of the total byproducts formed is preferably less than 10%, less than 7.5%, less than 5%, or less than 2.5%, as determined by UVarea value at 220 nm using HPLC analysis, based on the total amount of products.

In aspect 5, a content of each of byproducts formed is preferably less than 10%, less than 7.5%, less than 5%, less than 3%, less than 1%, or an undetectable amount, as determined by UVarea value at 220 nm using HPLC analysis, based on the total amount of products.

In aspect 5, a content of each of byproducts formed is preferably less than 10%, less than 7.5%, less than 5%, less than 3%, less than 1%, or an undetectable amount, as determined by UVarea value at 220 nm using HPLC analysis, based on the total amount of products, and the byproducts are an epimer, a dimer, and a trimer.

In aspect 5, byproducts formed preferably comprise an epimer of the cyclic peptide compound, and a content of the epimer is less than 7.5%, less than 5%, less than 3%, or less than 1%, as determined by UVarea value at 220 nm using HPLC analysis, based on the total amount of products.

In aspect 5, byproducts formed preferably comprise a dimer, and a content of the dimer is less than 10%, less than 7.5%, less than 5%, less than 2.5%, or less than 1%, as determined by UVarea value at 220 nm using HPLC analysis, based on the total amount of products.

In aspect 5, byproducts formed preferably comprise a trimer, and a content of the trimer is less than 3%, less than 1%, or an undetectable amount, as determined by UVarea value at 220 nm using HPLC analysis, based on the total amount of products.

In aspect 5, byproducts formed preferably comprise a dimer and a trimer, and the total content of the dimer and the trimer is less than 10%, less than 7.5%, less than 5%, less than 2.5%, or less than 1%, as determined by UVarea value at 220 nm using HPLC analysis, based on the total amount of products.

In an aspect, the present invention relates to a method for producing a cyclic peptide compound represented by the following formula (2):

[Formula 45]

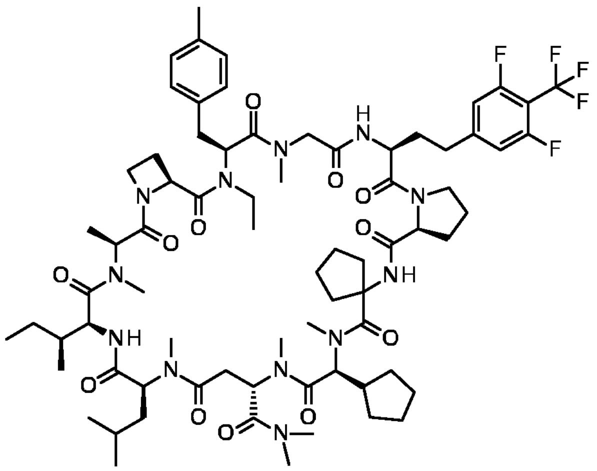

(2)

or a salt thereof, or a solvate thereof, the method comprising steps of:

(1) providing a linear peptide compound represented by the following formula:

[Formula 44]

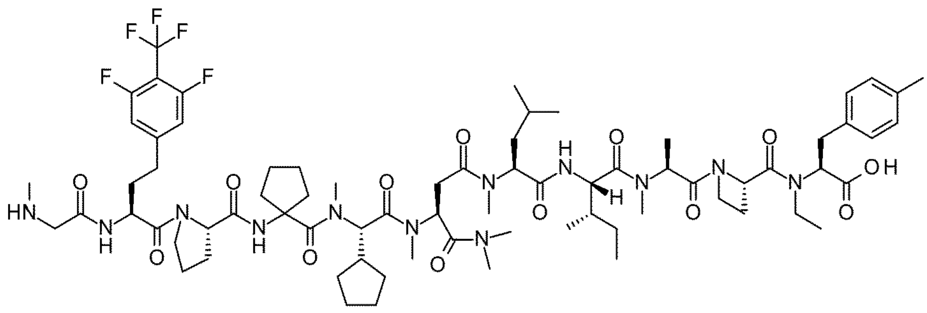

or a salt thereof, or a solvate thereof (compound C15); and

(2) linking an N-terminal amino acid residue of the linear peptide compound, or a salt thereof, or a solvate thereof with a C-terminal amino acid residue of the linear peptide compound, or a salt thereof, or a solvate thereof. Hereinafter, this aspect is also referred to as "aspect 6".

In aspect 6, the step (1) can provide the linear peptide compound, or a salt thereof, or a solvate thereof (compound C15), for example, according to the methods described in Examples C-1 to C-14 below.

In aspect 6, the step (2) is preferably performed in the presence of a solvent. The solvent used in the step (2) preferably comprises one or two selected from acetonitrile and dimethyl carbonate, and more preferably is dimethyl carbonate.

In aspect 6, the step (2) is preferably performed in the presence of a condensation reagent. The condensation reagent used in the step (2) is preferably one selected from the group consisting of COMU, HATU, PyBOP, PyOxim, PyClop, DMT-MM, and DEPBT, more preferably one selected from the group consisting of COMU and HATU, and further preferably is HATU.

In aspect 6, a combination of the condensation reagent and the solvent is preferably a combination of one selected from the group consisting of COMU, HATU, PyBOP, PyOxim, PyClop, DMT-MM, and DEPBT, and one or two selected from the group consisting of acetonitrile and dimethyl carbonate, more preferably a combination of one selected from the group consisting of COMU and HATU, and one selected from the group consisting of acetonitrile and dimethyl carbonate, further preferably a combination of one selected from the group consisting of COMU and HATU, and dimethyl carbonate, and particularly preferably a combination of HATU and dimethyl carbonate.

In aspect 6, the step (2) is preferably performed in the presence of a base. The base used in the step (2) is preferably an organic base, more preferably an organic base comprising a tertiary amine, further preferably one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP), and particularly preferably N,N-diisopropylethylamine (DIPEA).

In aspect 6, a combination of the condensation reagent and the solvent and the base is preferably a combination of one selected from the group consisting of COMU and HATU, one selected from the group consisting of acetonitrile and dimethyl carbonate, and N,N-diisopropylethylamine (DIPEA), more preferably a combination of one selected from the group consisting of COMU and HATU, dimethyl carbonate, and N,N-diisopropylethylamine (DIPEA), and further preferably a combination of HATU, dimethyl carbonate, and N,N-diisopropylethylamine (DIPEA).

In aspect 6, the step (2) is preferably performed by mixing the peptide compound and the base in a mixed solution obtained by mixing the solvent and the condensation reagent. This can suppress formation of dimer and trimer byproducts without using a large amount of solvent for dilution.

In aspect 6, a content of the total byproducts formed is preferably less than 10%, less than 7.5%, less than 5%, or less than 2.5%, as determined by UVarea value at 220 nm using HPLC analysis.

In aspect 6, a content of each of byproducts formed is preferably less than 10%, less than 7.5%, less than 5%, less than 3%, less than 1%, or an undetectable amount, as determined by UVarea value at 220 nm using HPLC analysis, based on the total amount of products.

In aspect 6, a content of each of byproducts formed is preferably less than 10%, less than 7.5%, less than 5%, less than 3%, less than 1%, or an undetectable amount, as determined by UVarea value at 220 nm using HPLC analysis, based on the total amount of products, and the byproducts are an epimer, a dimer, and a trimer.

In aspect 6, byproducts formed preferably comprise an epimer of the cyclic peptide compound, and a content of

the epimer is less than 7.5%, less than 5%, less than 3%, or less than 1%, as determined by UVarea value at 220 nm using HPLC analysis, based on the total amount of products.

In aspect 6, byproducts formed preferably comprise a dimer, and a content of the dimer is less than 10%, less than 7.5%, less than 5%, less than 2.5%, or less than 1%, as determined by UVarea value at 220 nm using HPLC analysis, based on the total amount of products.

In aspect 6, byproducts formed preferably comprise a trimer, and a content of the trimer is less than 3%, less than 1%, or an undetectable amount, as determined by UVarea value at 220 nm using HPLC analysis, based on the total amount of products.

In aspect 6, byproducts formed preferably comprise a dimer and a trimer, and the total content of the dimer and the trimer is less than 10%, less than 7.5%, less than 5%, less than 2.5%, or less than 1%, as determined by UVarea value at 220 nm using HPLC analysis, based on the total amount of products.

In an aspect, the present invention relates to a method for producing a cyclic peptide compound represented by the following formula (2):

[Formula 47]

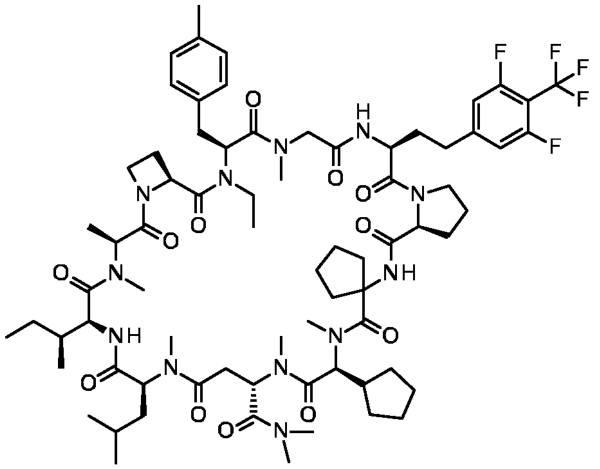

(2)

or a salt thereof, or a solvate thereof, the method comprising steps of:

(1) providing a linear peptide compound represented by the following formula:

[Formula 46]

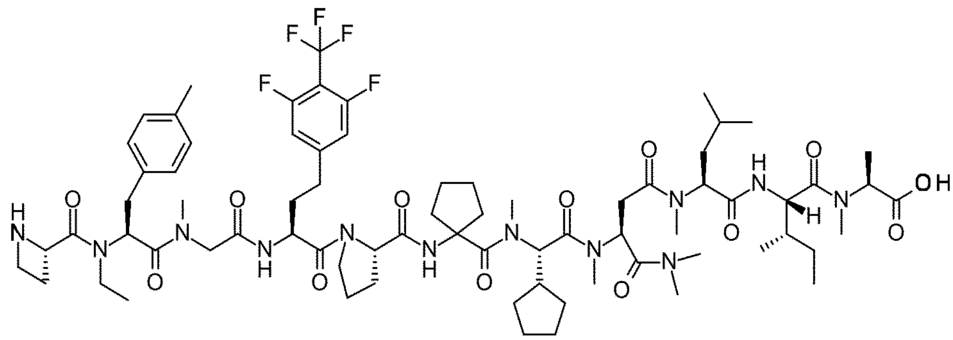

or a salt thereof, or a solvate thereof (compound A36); and

(2) linking an N-terminal amino acid residue of the linear peptide compound, or a salt thereof, or a solvate thereof with a C-terminal amino acid residue of the linear peptide compound, or a salt thereof, or a solvate thereof. Hereinafter, this aspect is also referred to as "aspect 7".

In aspect 7, the step (1) can provide the linear peptide compound, or a salt thereof, or a solvate thereof (compound A36), for example, according to the methods described in Examples A-1 to A-25 below.

In aspect 7, the step (2) is preferably performed in the presence of a solvent. The solvent used in the step (2)

preferably comprises one or two selected from the group consisting of acetonitrile and dimethyl carbonate, and more preferably is acetonitrile.

In aspect 7, the step (2) is preferably performed in the presence of a condensation reagent. The condensation reagent used in the step (2) is preferably one selected from the group consisting of COMU, HATU, PyBOP, PyOxim, PyClop, and DMT-MM, more preferably one selected from the group consisting of COMU, HATU, and PyOxim, and further preferably is COMU.

In aspect 7, a combination of the condensation reagent and the solvent is preferably a combination of one selected from the group consisting of COMU, HATU, PyBOP, PyOxim, PyClop, and DMT-MM, and one or two selected from the group consisting of acetonitrile and dimethyl carbonate, more preferably a combination of one selected from the group consisting of COMU, HATU, and PyOxim, and one selected from the group consisting of acetonitrile and dimethyl carbonate, further preferably a combination of COMU, and one selected from the group consisting of acetonitrile and dimethyl carbonate, and particularly preferably a combination of COMU and acetonitrile. In another embodiment of aspect 7, a combination of the condensation reagent and the solvent is preferably a combination of HATU and dimethyl carbonate, and a combination of PyOxim and dimethyl carbonate.

In aspect 7, the step (2) is preferably performed in the presence of a base. The base used in the step (2) is preferably an organic base, more preferably an organic base comprising a tertiary amine, further preferably one selected from the group consisting of N,N-diisopropylethylamine (DIPEA), 1,8-diazabicyclo[5.4.0]-7-undecene (DBU), 2,3,6,7-tetrahydro-1H,5H-9-azabenzo[ij]quinolizine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-methylmorpholine, N-ethylmorpholine, and p-dimethylaminopyridine (DMAP), and particularly preferably N,N-diisopropylethylamine (DIPEA).

In aspect 7, a combination of the condensation reagent and the solvent and the base is preferably a combination of one selected from the group consisting of COMU, HATU, and PyOxim, one selected from the group consisting of acetonitrile and dimethyl carbonate, and N,N-diisopropylethylamine (DIPEA), more preferably a combination of COMU, one selected from the group consisting of acetonitrile and dimethyl carbonate, and N,N-diisopropylethylamine (DIPEA), and further preferably a combination of COMU, acetonitrile, and N,N-diisopropylethylamine (DIPEA). In another embodiment of aspect 7, a combination of the condensation reagent and the solvent and the base is preferably a combination of HATU, dimethyl carbonate, and N,N-diisopropylethylamine (DIPEA), and a combination of PyOxim, dimethyl carbonate, and N,N-diisopropylethylamine (DIPEA).

In aspect 7, the step (2) is preferably performed by mixing the peptide compound and the base in a mixed solution obtained by mixing the solvent and the condensation reagent. This can suppress formation of dimer and trimer byproducts without using a large amount of solvent for dilution.

In aspect 7, a content of the total byproducts formed is preferably less than 15%, less than 10%, less than 7.5%, or less than 5%, as determined by UVarea value at 220 nm using HPLC analysis.

In aspect 7, a content of each of byproducts formed is preferably less than 10%, less than 7.5%, less than 5%, less than 3%, less than 1%, or an undetectable amount, as determined by UVarea value at 220 nm using HPLC analysis, based on the total amount of products.

In aspect 7, a content of each of byproducts formed is preferably less than 10%, less than 7.5%, less than 5%, less than 3%, less than 1%, or an undetectable amount, as determined by UVarea value at 220 nm using HPLC analysis, based on the total amount of products, and the byproducts are an epimer, a dimer, and a trimer.

In aspect 7, byproducts formed preferably comprise an epimer of the cyclic peptide compound, and a content of the epimer is less than 5%, less than 3%, or less than 1%, as determined by UVarea value at 220 nm using HPLC analysis, based on the total amount of products.

In aspect 7, byproducts formed preferably comprise a dimer, and a content of the dimer is less than 10%, less than 7.5%, or less than 5%, as determined by UVarea value at 220 nm using HPLC analysis, based on the total amount of products.

In aspect 7, byproducts formed preferably comprise a trimer, and a content of the trimer is less than 3%, less than 1%, or an undetectable amount, as determined by UVarea value at 220 nm using HPLC analysis, based on the total amount of products.

In aspect 7, byproducts formed preferably comprise a dimer and a trimer, and the total content of the dimer and the trimer is less than 10%, less than 7.5%, or less than 5%, as determined by UVarea value at 220 nm using HPLC analysis, based on the total amount of products.

<u>Method of suppressing epimerization of cyclic peptide compound</u>

In an aspect, the present invention relates to a method of suppressing epimerization of a cyclic peptide compound in production of the cyclic peptide compound, or a salt thereof, or a solvate thereof, the method comprising a step of linking

an N-terminal amino acid residue of a peptide compound with a C-terminal amino acid residue of the peptide compound in a solvent, wherein at least one of the N-terminal amino acid residue and the C-terminal amino acid residue is a cyclic amino acid residue, and the solvent comprises one or more selected from the group consisting of acetonitrile, dimethyl carbonate, 2-MeTHF, and anisole. Hereinafter, this aspect is also referred to as "aspect 8".

In an aspect, the present invention relates to a method of suppressing epimerization of a cyclic peptide compound in production of the cyclic peptide compound, or a salt thereof, or a solvate thereof, the method comprising a step of linking an N-terminal amino acid residue of a peptide compound with a C-terminal amino acid residue of the peptide compound in a solvent, wherein the N-terminal amino acid residue and the C-terminal amino acid residue of the peptide compound are one selected from the following a) to e):

a) the N-terminal amino acid residue is an N-substituted phenylalanine residue or an N-substituted phenylalanine derivative residue, and the C-terminal amino acid residue is a cyclic amino acid residue;
b) the N-terminal amino acid residue is an $\alpha,\alpha$-di-substituted amino acid residue, and the C-terminal amino acid residue is a cyclic amino acid residue;
c) the N-terminal amino acid residue is a cyclic amino acid residue, and the C-terminal amino acid residue is a homophenylalanine residue or a homophenylalanine derivative residue;
d) the N-terminal amino acid residue is a cyclic amino acid residue, and the C-terminal amino acid residue is an N-substituted Ala residue; and
e) the N-terminal amino acid residue is an N-substituted Gly residue, and the C-terminal amino acid residue is an N-substituted phenylalanine residue or an N-substituted phenylalanine derivative residue. Hereinafter, this aspect is also referred to as "aspect 9".

In aspects 8 and 9, the step of linking an N-terminal amino acid residue of a peptide compound with a C-terminal amino acid residue of the peptide compound is as described in "Method for producing cyclic peptide compound" above.

In aspects 8 and 9, a content of epimer formed is preferably less than 20%, less than 15%, less than 10%, less than 5%, or less than 3%, as determined by UVarea value at 220 nm using HPLC analysis, based on the total amount of products.

In aspects 8 and 9, a content of epimer formed is preferably less than 15%, less than 10%, less than 5%, less than 3%, less than 1%, or an undetectable amount, as determined by UVarea value at 220 nm using HPLC analysis, based on the total amount of products.

Method of suppressing formation of oligomer of cyclic peptide compound

In an aspect, the present invention relates to a method of suppressing formation of an oligomer of a cyclic peptide compound in production of the cyclic peptide compound, or a salt thereof, or a solvate thereof, the method comprising a step of linking an N-terminal amino acid residue of a peptide compound with a C-terminal amino acid residue of the peptide compound in a solvent, wherein at least one of the N-terminal amino acid residue and the C-terminal amino acid residue is a cyclic amino acid residue, and the solvent comprises one or more selected from the group consisting of acetonitrile, dimethyl carbonate, 2-MeTHF, and anisole. Hereinafter, this aspect is also referred to as "aspect 10".

In an aspect, the present invention relates to a method of suppressing formation of an oligomer of a cyclic peptide compound in production of the cyclic peptide compound, or a salt thereof, or a solvate thereof, the method comprising a step of linking an N-terminal amino acid residue of a peptide compound with a C-terminal amino acid residue of the peptide compound in a solvent, wherein the N-terminal amino acid residue and the C-terminal amino acid residue of the peptide compound are one selected from the following a) to e):

a) the N-terminal amino acid residue is an N-substituted phenylalanine residue or an N-substituted phenylalanine derivative residue, and the C-terminal amino acid residue is a cyclic amino acid residue;
b) the N-terminal amino acid residue is an $\alpha,\alpha$-di-substituted amino acid residue, and the C-terminal amino acid residue is a cyclic amino acid residue;
c) the N-terminal amino acid residue is a cyclic amino acid residue, and the C-terminal amino acid residue is a homophenylalanine residue or a homophenylalanine derivative residue;
d) the N-terminal amino acid residue is a cyclic amino acid residue, and the C-terminal amino acid residue is an N-substituted Ala residue; and
e) the N-terminal amino acid residue is an N-substituted Gly residue, and the C-terminal amino acid residue is an N-substituted phenylalanine residue or an N-substituted phenylalanine derivative residue. Hereinafter, this aspect is also referred to as "aspect 11".

In aspects 10 and 11, the step of linking an N-terminal amino acid residue of a peptide compound with a C-terminal amino acid residue of the peptide compound is as described in "Method for producing cyclic peptide compound" above.

In aspects 10 and 11, byproducts formed preferably comprise a dimer, and the content of the dimer is less than 15%, less than 10%, less than 5%, less than 2.5%, or less than 1%, as determined by UVarea value at 220 nm using HPLC analysis, based on the total amount of products.

In aspects 10 and 11, byproducts formed preferably comprise a trimer, and the content of the trimer is less than 5%, less than 2.5%, less than 1%, or an undetectable amount, as determined by UVarea value at 220 nm using HPLC analysis, based on the total amount of products.

In aspects 10 and 11, byproducts formed preferably comprise a dimer and a trimer, and the total content of the dimer and the trimer is less than 15%, less than 10%, less than 5%, less than 2.5%, or less than 1%, as determined by UVarea value at 220 nm using HPLC analysis, based on the total amount of products.

[Examples]

The contents of the present invention will be further described by the following Examples, but the present invention is not limited to their contents. Unless otherwise specified, starting substances, starting raw materials, solvents, and reagents were obtained from commercial suppliers, or synthesized using known methods.

The analysis conditions by HPLC are shown below.

HPLC analysis conditions method 1

Apparatus: Waters ACQUITY UPLC H-Class
Column: CAPCELL CORE ADME (OSAKA SODA), 2.1 mm ID × 50 mm, 2.7 μm
Mobile phase: 0.05% TFA/water (A), 0.05% TFA/MeCN (B)
Elution method: B): 5% (0 min) → 100% (5 min) → 5% (5.1 min) → 5% (7 min)
Flow rate: 0.5 mL/min
Column temperature: 35°C
Detection wavelength: 210 nm (PDA)

HPLC analysis conditions method 2

Apparatus: Waters ACQUITY UPLC H-Class
Column: CAPCELL CORE ADME (OSAKA SODA), 2.1 mm ID × 50 mm, 2.7 μm
Mobile phase: 0.05% TFA/water (A), 0.05% TFA/MeCN (B)
Elution method: B): 5% (0 min) → 100% (7 min) → 5% (7.1 min) → 5% (9 min)
Flow rate: 0.5 mL/min
Column temperature: 35°C
Detection wavelength: 210 nm (PDA)

HPLC analysis conditions method 3

Apparatus: Waters ACQUITY UPLC H-Class
Column: ACQUITY UPLC CSH C18 (Waters), 2.1 mm ID×150 mm, 1.7 μm
Mobile phase: 0.05% TFA/water (A), 0.05% TFA/MeCN (B)
Elution method: B) 20%(0 min)→100%(24 min)→100%(29 min)→20%(29.1 min)→20%(34 min)
Flow rate: 0.3 mL/min
Column temperature: 50°C
Detection wavelength: 220 nm (PDA)

HPLC analysis conditions method 4

Apparatus: Waters ACQUITY UPLC H-Class
Column: Ascentis Express 90A C18 (Sigma-Aldrich), 2.1 mm ID × 50 mm, 2.7 μm

Mobile phase: 0.05% TFA/water (A), 0.05% TFA/MeCN (B)
Elution method: B): 5% (0 min) → 100% (5 min) → 5% (5.1 min) → 5% (7 min)
Flow rate: 0.5 mL/min
Column temperature: 35°C
Detection wavelength: 210 nm (PDA)

HPLC analysis conditions method 5

Apparatus: Waters ACQUITY UPLC H-Class
Column: CAPCELL CORE ADME (OSAKA SODA), 2.1 mm ID × 50 mm, 2.7 μm
Mobile phase: 0.05% TFA/water (A), 0.05% TFA/MeCN (B)
Elution method: B): 5% (0 min) → 100% (10 min) → 5% (10.1 min) → 5% (12 min)
Flow rate: 0.5 mL/min
Column temperature: 35°C
Detection wavelength: 210 nm (PDA)

HPLC analysis conditions method 6

Apparatus: Waters ACQUITY UPLC H-Class
Column: ACQUITY UPLC CSH C18 (Waters), 2.1 mm ID × 100 mm, 1.7 μm
Mobile phase: 0.05% TFA/water (A), 0.05% TFA/MeCN (B)
Elution method: B) 20% (0 min) → 100% (10 min) → 100% (13.5 min) → 20% (13.6 min) → 20% (18.0 min)
Flow rate: 0.3 mL/min
Column temperature: 50°C
Detection wavelength: 210 nm (PDA)

HPLC analysis conditions method 7

Apparatus: Waters ACQUITY UPLC H-Class
Column: Ascentis Express 90A C18 (Sigma-Aldrich), 2.1 mm ID × 50 mm, 2.7 μm
Mobile phase: 0.05% TFA/water (A), 0.05% TFA/MeCN (B)
Elution method: B): 5% (0 min) → 100% (6 min) → 5% (6.1 min) → 5% (8 min)
Flow rate: 0.5 mL/min
Column temperature: 35°C
Detection wavelength: 210 nm (PDA)

HPLC analysis conditions method 8

Apparatus: Waters ACQUITY UPLC H-Class
Column: ACQUITY UPLC CSH C18 (Waters), 2.1 mm ID × 150 mm, 1.7 μm
Mobile phase: 0.05% TFA/water (A), 0.05% TFA/MeCN (B)
Elution method: B) 20% (0 min)→100% (84 min) → 100% (89 min) → 20% (89.1 min) → 20% (94 min)
Flow rate: 0.3 mL/min
Column temperature: 50°C
Detection wavelength: 210 nm (PDA)

HPLC analysis conditions method 9

Apparatus: Waters ACQUITY UPLC H-Class
Column: CHIRALPAK IC-3 (DAICEL) 4.6 mm ID × 150 mm, 3 μm
Mobile phase: 0.05% TFA/water (A), 0.05% TFA/MeCN (B)
Elution method: B): 5% (0 min) → 100% (20 min) → 5% (20.1 min) → 5% (25 min)
Flow rate: 1.0 mL/min
Column temperature: 30°C
Detection wavelength: 210 nm (PDA)

[Table 1]

| Example No. (Compound No.) | HPLC analysis conditions | Retention time (min) | Example No. (Compound No.) | HPLC analysis conditions | Retention time (min) |
|---|---|---|---|---|---|
| Example A-1 (Compound A2) | Method 1 | 3.769 | Example B-36 (Compound B25) | Method 1 | 4.422 |
| Example A-3 (Compound A5) | Method 1 | 4.161 | Example B-37 (Compound B26) | Method 1 | 3.459 |
| Example A-4 (Compound A7) | Method 1 | 5.195 | Example B-38 (Compound B27) | Method 6 | 9.019 |
| Example A-5 (Compound A8) | Method 1 | 5.180 | Example B-39 (Compound B28) | Method 6 | 6.063 |
| Example A-6 (Compound A9) | Method 1 | 2.915 | Example B-40 (Compound B11) | Method 6 | 11.367 |
| Example A-7 (Compound A11) | Method 1 | 3.510 | Example B-42 (Compound B29) | Method 6 | 10.254 |
| Example A-8 (Compound A12) | Method 1 | 1.560 | Example B-43 (Compound B30) | None | None |
| Example A-9 (Compound A14) | Method 1 | 4.356 | Example C-1 (Compound C1) | Method 1 | 4.663 |
| Example A-10 (Compound A15) | Method 1 | 2.297 | Example C-2 (Compound C2) | Method 1 | 2.714 |
| Example A-11 (Compound A17) | Method 1 | 6.166 | Example C-3 (Compound C3) | Method 1 | 4.443 |
| Example A-12 (Compound A18) | Method 1 | 2.725 | Example C-4 (Compound C4) | Method 1 | 2.918 |
| Example A-13 (Compound A20) | Method 1 | 4.189 | Example C-5 (Compound C5) | Method 1 | 4.445 |
| Example A-14 (Compound A21) | Method 1 | 2.846 | Example C-6 (Compound C6) | Method 1 | 2.892 |
| Example A-15 (Compound A23) | Method 1 | 4.978 | Example C-7 (Compound C7) | Method 1 | 4.701 |
| Example A-16 (Compound A24) | Method 1 | 4.220 | Example C-8 (Compound C8) | Method 1 | 5.566 |
| Example A-17 (Compound A25) | Method 3 | 21.235 | Example C-9 (Compound C9) | Method 1 | 3.582 |
| Example A-18 (Compound A28) | Method 4 | 4.500 | Example C-10 (Compound C10) | Method 3 | 22.077 |
| Example A-19 (Compound A29) | Method 4 | 2.389 | Example C-11 (Compound C11) | Method 2 | 4.628 |

(continued)

| Example No. (Compound No.) | HPLC analysis conditions | Retention time (min) | Example No. (Compound No.) | HPLC analysis conditions | Retention time (min) |
|---|---|---|---|---|---|
| Example A-20 (Compound A31) | Method 4 | 4.065 | Example C-12 (Compound C13) | Method 3 | 21.380 |
| Example A-22 (Compound A33) | Method 2 | 4.256 | Example C-13 (Compound C14) | Method 3 | 18.926 |
| Example A-23 (Compound A34) | Method 3 | 21.670 | Example C-14 (Compound C15) | Method 3 | 13.418 |
| Example A-24 (Compound A35) | Method 3 | 19.027 | Example D-1 (Compound D1) | Method 1 | 3.695 |
| Example A-25 (Compound A36) | Method 3 | 13.323 | Example D-2 (Compound D2) | Method 1 | 1.974 |
| Example A-29 (Compound A37) | Method 4 | 2.937 | Example D-10 (Compound D3) | Method 4 | 2.538 |
| Example B-1 (Compound B1) | Method 1 | 3.519 | Example D-11 (Compound D4) | Method 4 | 4.004 |
| Example B-3 (Compound B3) | Method 1 | 3.543 | Example D-12 (Compound D5) | Method 4 | 2.510 |
| Example B-4 (Compound B4) | Method 1 | 1.670 | Example D-13 (Compound D6) | Method 4 | 4.235 |
| Example B-5 (Compound B5) | Method 1 | 3.893 | Example D-15 (Compound D7) | Method 7 | 5.964 |
| Example B-6 (Compound B6) | Method 1 | 4.937 | Example D-16 (Compound D8) | Method 4 | 3.057 |
| Example B-7 (Compound B7) | Method 1 | 2.747 | Example D-17 (Compound D9) | Method 1 | 5.452 |
| Example B-8 (Compound B8) | Method 3 | 20.037 | Example D-18 (Compound D10) | Method 1 | 4.564 |
| Example B-10 (Compound B10) | Method 2 | 4.054 | Example 0-19 (Compound D11) | Method 3 | 21.550 |
| Example B-11 (Compound B11) | Method 3 | 21.617 | Example D-20 (Compound D12) | Method 3 | 19.111 |
| Example B-12 (Compound B12) | Method 3 | 19.638 | Example D-21 (Compound D13) | Method 3 | 12.952 |
| Example B-13 (Compound B13) | Method 3 | 12.613 | Example E-2 (Compound E2) | Method 1 | 4.469 |
| Example B-22 (Compound B16) | Method 1 | 4.613 | Example E-3 (Compound E3) | Method 1 | 2.956 |
| Example B-23 (Compound B17) | Method 1 | 3.686 | Example E-4 (Compound E4) | Method 1 | 3.427 |
| Example B-25 (Compound B18) | Method 1 | 4.374 | Example E-5 (Compound E5) | Method 1 | 5.361 |
| Example B-26 (Compound B20) | Method 1 | 4.481 | Example E-6 (Compound E6) | Method 1 | 4.510 |
| Example B-27 (Compound B21) | Method 1 | 3.107 | Example E-7 (Compound E7) | Method 3 | 21.328 |

(continued)

| Example No. (Compound No.) | HPLC analysis conditions | Retention time (min) | Example No. (Compound No.) | HPLC analysis conditions | Retention time (min) |
|---|---|---|---|---|---|
| Example B-30 (Compound B22) | None | None | Example E-8 (Compound E8) | Method 3 | 19.054 |
| Example B-31 (Compound B23) | Method 1 | 4.249 | Example E-9 (Compound E9) | Method 3 | 13.427 |

The analysis conditions by LCMS are shown below.

LCMS analysis conditions method 1

Apparatus: Waters ACQUITY UPLC H-Class + ACQUITY QDA
Column: CAPCELL CORE ADME (OSAKA SODA), 2.1 mm ID × 50 mm, 2.7 μm
Mobile phase: 0.05% TFA/water (A), 0.05% TFA/MeCN (B)
Elution method: B): 5% (0 min) → 100% (5 min) → 5% (5.1 min) → 5% (7 min)
Flow rate: 0.5 mL/min
Column temperature: 35°C
Detection wavelength: 210 nm (PDA)

LCMS analysis conditions method 2

Apparatus: Waters ACQUITY UPLC H-Class + ACQUITY QDA
Column: Ascentis Express 90A C18 (Sigma-Aldrich), 2.1 mm ID × 50 mm, 2.7 μm
Mobile phase: 0.05% TFA/water (A), 0.05% TFA/MeCN (B)
Elution method: B) 5% (0 min) → 100% (6 min) → 5% (6.1 min) → 5% (8 min)
Flow rate: 0.5 mL/min
Column temperature: 35°C
Detection wavelength: 210 nm (PDA)

LCMS analysis conditions method 3

Apparatus: Waters SQD2
Column: ACQUITY UPLC CSH C18 (Waters), 2.1 mm ID×150 mm, 1.7 μm
Mobile phase: 0.05% TFA/water (A), 0.05% TFA/MeCN (B)
Elution method: B) 20% (0 min) → 100% (24 min) → 100% (29 min) → 20% (29.1 min) → 20% (34 min)
Flow rate: 0.3 mL/min
Column temperature: 50°C
Detection wavelength: 220 nm (PDA)

LCMS analysis conditions method 4

Apparatus: Waters ACQUITY UPLC H-Class + ACQUITY QDA
Column: Ascentis Express 90A C18 (Sigma-Aldrich), 2.1 mm ID × 50 mm, 2.7 μm
Mobile phase: 0.05% TFA/water (A), 0.05% TFA/MeCN (B)
Elution method: B): 5% (0 min) → 100% (5 min) → 5% (5.1 min) → 5% (7 min)
Flow rate: 0.5 mL/min
Column temperature: 35°C

Detection wavelength: 210 nm (PDA)

LCMS analysis conditions method 5

Apparatus: Waters ACQUITY UPLC H-Class + ACQUITY QDA
Column: CAPCELL CORE ADME (OSAKA SODA), 2.1 mm ID × 50 mm, 2.7 μm
Mobile phase: 0.05% TFA/water (A), 0.05% TFA/MeCN (B)
Elution method: B) 5% (0 min) → 100% (10 min) → 5% (10.1 min) → 5% (12 min)
Flow rate: 0.5 mL/min
Column temperature: 35°C
Detection wavelength: 210 nm (PDA)

LCMS analysis conditions method 6

Apparatus: Waters ACQUITY UPLC H-Class + SQ Detector 2
Column: ACQUITY UPLC CSH C18 (Waters), 2.1 mm ID × 100 mm, 1.7 μm
Mobile phase: 0.05% TFA/water (A), 0.05% TFA/MeCN (B)
Elution method: B) 20% (0 min) → 100% (10 min) → 100% (13.5 min) → 20% (13.6 min) → 20% (18.0 min)
Flow rate: 0.3 mL/min
Column temperature: 50°C
Detection wavelength: 210 nm (PDA)

[Table 2]

| Example No. (Compound No.) | HPLC analysis conditions | Retention time (min) | MS Found (m/z) | MS polarity | Example No. (Compound No.) | HPLC analysis conditions | Retention time (min) | MS Found (m/z) | MS polarity |
|---|---|---|---|---|---|---|---|---|---|
| Example A-1 (Compound A2) | Method 1 | 3.845 | 316 | $[M+Na]^+$ | Example B-22 (Compound B16) | Method 1 | 4.642 | 449 | $[M+H]^+$ |
| Example A-2 (Compound A5) | Method 2 | 3.708 | 407 | $[M+H]^+$ | Example B-23 (Compound B17) | Method 1 | 3.635 | 393 | $[M+H]^+$ |
| Example A-5 (Compound A8) | Method 1 | 4.936 | 544 | $[M+H]^+$ | Example B-25 (Compound B18) | Method 1 | 4.374 | 639 | $[M+H]^+$ |
| Example A-6 (Compound A9) | Method 1 | 2.637 | 400 | $[M+H]^+$ | Example B-26 (Compound B20) | Method 4 | 4.241 | 593 | $[M+Na]^+$ |
| Example A-7 (Compound A11) | Method 1 | 3.496 | 387 | $[M+Na]^+$ | Example B-27 (Compound B21) | Method 4 | 2.958 | 437 | $[M+H]^+$ |
| Example A-8 (Compound A12) | Method 1 | 1.532 | 231 | $[M+H]^+$ | Example B-30 (Compound B22) | Method 4 | 4.741 | 832 | $[M+H]^+$ |
| Example A-9 (Compound A14) | Method 1 | 4.329 | 526 | $[M+Na]^+$ | Example B-31 (Compound B23) | Method 1 | 4.237 | 775 | $[M+H]^+$ |

(continued)

| Example No. (Compound No.) | HPLC analysis conditions | Retention time (min) | MS Found (m/z) | MS polarity | Example No. (Compound No.) | HPLC analysis conditions | Retention time (min) | MS Found (m/z) | MS polarity |
|---|---|---|---|---|---|---|---|---|---|
| Example A-10 (Compound A15) | Method 1 | 2.229 | 370 | $[M+H]^+$ | Example B-36 (Compound B25) | Method 5 | 6.914 | 637 | $[M+Na]^+$ |
| Example A-11 (Compound A17) | Method 1 | 6.181 | 599 | $[M+Na]^+$ | Example B-37 (Compound B26) | Method 1 | 3.307 | 581 | $[M+Na]^+$ |
| Example A-12 (Compound A18) | Method 1 | 2.749 | 503 | $[M+Na]^+$ | Example B-38 (Compound B27) | Method 6 | 9.79 | 1046 | $[M+H]^+$ |
| Example A-13 (Compound A20) | Method 1 | 4.206 | 735 | $[M+Na]^+$ | Example B-39 (Compound B28) | Method 6 | 5.50 | 890 | $[M+H]^+$ |
| Example A-14 (Compound A21) | Method 1 | 2.839 | 578 | $[M+H]^+$ | Example B-40 (Compound B11) | Method 6 | 10.66 | 1668 | $[M+Na]^+$ |
| Example A-15 (Compound A23) | Method 1 | 4.827 | 1000 | $[M+Na]^+$ | Example B-42 (Compound B29) | Method 6 | 11.00 | 1261 | $[M+Na]^+$ |
| Example A-16 (Compound A24) | Method 1 | 4.133 | 943 | $[M+Na]^+$ | Example B-43 (Compound B30) | Method 4 | 4.320 | 1182 | $[M+H]^+$ |
| Example A-17 (Compound A25) | Method 3 | 21.82 | 1325 | $[M+Na]^+$ | Example C-3 (Compound C3) | Method 1 | 4.339 | 481 | $[M+H]^+$ |
| Example A-18 (Compound A28) | Method 4 | 4.507 | 469 | $[M+H]^+$ | Example C-5 (Compound C5) | Method 1 | 4.374 | 566 | $[M+H]^+$ |
| Example A-19 (Compound A29) | Method 4 | 2.411 | 335 | $[M+H]^+$ | Example C-7 (Compound C7) | Method 2 | 4.226 | 679 | $[M+H]^+$ |
| Example A-20 (Compound A31) | Method 4 | 4.083 | 574 | $[M+Na]^+$ | Example C-8 (Compound C8) | Method 1 | 5.337 | 816 | $[M+H]^+$ |
| Example A-21 (Compound A32) | Method 2 | 3.045 | 496 | $[M+H]^+$ | Example C-9 (Compound C9) | Method 1 | 3.249 | 672 | $[M+H]^+$ |
| Example A-23 (Compound A34) | Method 3 | 22.30 | 1668 | $[M+Na]^+$ | Example C-10 (Compound C10) | Method 3 | 22.67 | 1575 | $[M+H]^+$ |
| Example A-25 (Compound A36) | Method 3 | 13.76 | 1456 | $[M+H]^+$ | Example C-12 (Compound C13) | Method 3 | 22.04 | 1668 | $[M+Na]^+$ |
| Example B-1 (Compound B1) | Method 1 | 3.620 | 314 | $[M+Na]^+$ | Example C-14 (Compound C15) | Method 3 | 13.93 | 1456 | $[M+H]^+$ |

(continued)

| Example No. (Compound No.) | HPLC analysis conditions | Retention time (min) | MS Found (m/z) | MS polarity | Example No. (Compound No.) | HPLC analysis conditions | Retention time (min) | MS Found (m/z) | MS polarity |
|---|---|---|---|---|---|---|---|---|---|
| Example B-3 (Compound B3) | Method 1 | 3.519 | 377 | $[M+H]^+$ | Example D-1 (Compound D1) | Method 1 | 3.815 | 328 | $[M+Na]^+$ |
| Example B-5 (Compound B5) | Method 2 | 3.477 | 490 | $[M+H]^+$ | Example D-17 (Compound D9) | Method 3 | 21.57 | 1306 | $[M+Na]^+$ |
| Example B-6 (Compound B6) | Method 1 | 4.739 | 627 | $[M+H]^+$ | Example D-21 (Compound D13) | Method 3 | 13.34 | 1456 | $[M+H]^+$ |
| Example B-7 (Compound B7) | Method 1 | 2.488 | 483 | $[M+H]^+$ | Example E-2 (Compound E2) | Method 1 | 4.563 | 474 | $[M+H]^+$ |
| Example B-8 (Compound B8) | Method 3 | 20.69 | 1408 | $[M+Na]^+$ | Example E-3 (Compound E3) | Method 1 | 2.841 | 340 | $[M+H]^+$ |
| Example B-9 (Compound B9) | Method 2 | 3.347 | 413 | $[M+H]^+$ | Example E-5 (Compound E5) | Method 3 | 21.14 | 1403 | $[M+Na]^+$ |
| Example B-11 (Compound B11) | Method 3 | 22.21 | 1668 | $[M+Na]^+$ | Example E-9 (Compound E9) | Method 3 | 13.85 | 1456 | $[M+H]^+$ |
| Example B-13 (Compound B13) | Method 3 | 13.10 | 1456 | $[M+H]^+$ | | | | | |

The $^1$H-NMR spectrum was measured using a nuclear magnetic resonance device ECX500II (manufactured by JEOL Ltd.), the chemical shift of $Me_4Si$ used as the internal standard substance was set at 0 ppm, and a deuterium lock signal from a sample solvent was referred. As the sample solvent, commercially available deuterated solvents were used, depending on the purpose of measurement. The integral value of signals was calculated on the basis of a ratio of the signal area intensity of each signal.

The measuring method by qNMR was performed by dissolving a residue containing a target compound and an internal standard substance in DMSO-$d_6$ and subjecting to the following analysis conditions. The yield was calculated by the following expression using the value of the content of target material in the residue calculated by qNMR, and the value of the purity of target material in the residue calculated by HPLC.

[Expression 1]

$$\text{Yield (\%)} = \frac{\text{Weight of residue (mg)} \times \text{Content (\%)} \times \text{Purity (\%)}}{\text{Theoretical yield (mg)}} \times 100$$

Measuring apparatus: JNM-ECZ500R

Internal standard substance: 1,3,5-trimethoxybenzene or 3,5-bis(trifluoromethyl)benzoic acid Measurement conditions ($^1$H-NMR): DMSO-$d_6$, 24.3°C, pulse angle 90°, digital resolution 0.25 Hz, relaxation time 60 seconds, no spin, cumulative number 8 times

Measurement conditions ($^{19}$F-NMR): DMSO-$d_6$, 24.3°C, pulse angle 90°, digital resolution 0.22 Hz, relaxation time 60 seconds, no spin, cumulative number 8 times

The measuring method by HPLC and LCMS was performed by preparing a mixed solution containing a target

compound as a sample according to any of the following methods and subjecting it to the analysis conditions described above.

Sample preparation method 1: a mixed solution containing a target compound was diluted with acetonitrile.
Sample preparation method 2: a mixed solution containing a target compound was diluted with a mixed solution of acetonitrile and propylamine in a ratio of 9 : 1.
Sample preparation method 3: a mixed solution containing a target compound was diluted with methanol.
Sample preparation method 4: a mixed solution containing a target compound was diluted with a mixed solution of methanol and water in a ratio of 4 : 1.
Sample preparation method 5: a mixed solution containing a target compound was diluted with a mixed solution of acetonitrile and propylamine in a ratio of 10 : 1.

The reaction conversion rate was calculated by any of the following formulas using the area value of starting material and the area value of target material, or the area value of starting material, the area value of propylamide derivative of starting material and the area value of target material, or the area value of starting material before reaction and the area value of starting material after reaction calculated by HPLC analysis.

Calculation expression 1: reaction conversion rate (%) = area value of target material/(area value of starting material + area value of target material) $\times$ 100

Calculation expression 2: reaction conversion rate (%) = 100 - (area value of starting material after reaction/area value of starting material before reaction $\times$ 100)

Calculation expression 3: reaction conversion rate (%) = area value of target material/(area value of starting material + area value of propylamide derivative of starting material + area value of target material) $\times$ 100

Example A-1

Synthesis of compound A2: tert-butyl (2S)-2-[benzyloxycarbonyl(methyl)amino]propanoate

[Formula 48]

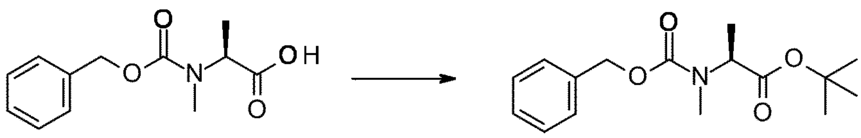

A1 A2

Compound A1 (5.00 g) was added to a reaction vessel. Next, dichloromethane (10 mL) and cyclohexane (40 mL) were added to the reaction vessel at room temperature. The inside of the reaction vessel was replaced with hydrogen and tert-butyl 2,2,2-trichloroacetimidate (7.55 mL) and boron trifluoride diethyl ether complex (267 μL) were added, and the mixture was then stirred at room temperature for 1 hour. The reaction mixture was sampled and prepared as a sample (sample preparation method 1), and it was confirmed by HPLC analysis that the reaction conversion rate was 91.7% (calculation expression 1 of reaction conversion rate). The resulting slurry was filtered and the solid residue was washed with cyclohexane. The resulting filtrate was washed with 10% aqueous citric acid solution (40 mL $\times$ 9), 5% aqueous sodium carbonate solution (40 mL $\times$ 3), and saturated aqueous NaCl solution (40 mL). The resulting organic layer was dehydrated with sodium sulfate and filtered to remove sodium sulfate. The resulting filtrate was concentrated under reduced pressure at the external temperature of 40°C to obtain a residue (5.05 g) containing compound A2.
LC retention time of compound A2: 3.769 minutes (HPLC analysis conditions: method 1) LCMS (ESI) retention time of compound A2: 3.845 minutes, m/z = 316.08 $[M+Na]^+$ (LCMS analysis conditions: method 1)

Example A-2

Synthesis of compound A3: tert-butyl (2S)-2-(methylamino)propanoate

[Formula 49]

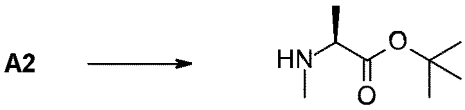

A3

The residue (4.03 g) containing compound A2 obtained in Example A-1 was added to a reaction vessel. Next, 2-MeTHF (42 mL) and 5% Pd/C (2.92 g, 50% aqueous content) were sequentially added at room temperature. The inside of the reaction vessel was replaced with hydrogen, and the mixture was stirred at room temperature for 2 hours. The inside of the reaction vessel was replaced again with hydrogen, and then the mixture was stirred at room temperature for an additional 1 hour. The reaction mixture was sampled and prepared as a sample (sample preparation method 1), and it was confirmed by HPLC analysis that the reaction conversion rate was 99.9% or more (calculation expression 2 of reaction conversion rate). The inside of the reaction vessel was replaced with nitrogen, then the reaction mixture was filtered under reduced pressure using filter paper and a filter. The reaction vessel and the filter paper and the filter were washed with 2-MeTHF (21 mL × 2). The resulting filtrate and the wash solutions were mixed and concentrated under reduced pressure at the external temperature of 40°C to obtain a residue (1.66 g) containing compound A3.

Example A-3

Synthesis of compound A5: tert-butyl (2S)-2-[[(2S,3S)-2-(benzyloxycarbonylamino)-3-methyl-pentanoyl]-methyl-amino]propanoate

[Formula 50]

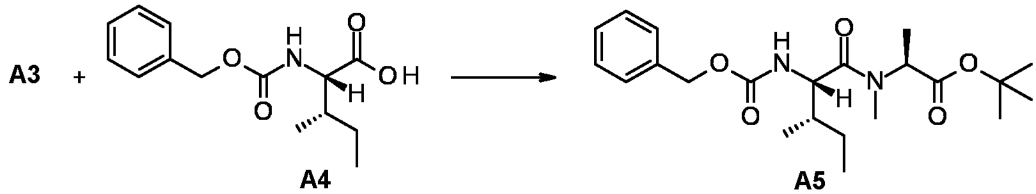

A4 A5

The residue (1.66 g) containing compound A3 obtained in Example A-2 and compound A4 (3.29 g) were added to a reaction vessel, and the reaction vessel was subjected to nitrogen replacement. Next, 2-MeTHF (16 mL), toluene (17 mL), MeCN (2.7 mL), and DIPEA (11.0 mL) were sequentially added at room temperature. HATU (5.96 g) was added with stirring, and then the mixture was stirred at room temperature for 2 hours. The reaction mixtures of 1 and 2 hours after the reaction were sampled and prepared as samples (sample preparation method 1), and it was confirmed by HPLC analysis that there was no difference in impurity profiles. N-methylimidazole (0.9 mL) was added to the reaction vessel, and 5% aqueous sodium carbonate solution (40 mL) was further added with stirring, and then the mixture was stirred for 1 hour. Next, 2.5% aqueous ammonia solution (40 mL) was added, and the mixture was stirred for 15 minutes. After the aqueous layer was discharged, the resulting organic layer was washed with 2.5% aqueous ammonia solution (60 mL), 5% aqueous sodium hydrogen sulfate monohydrate solution (60 mL × 2), and 3% aqueous dipotassium hydrogen phosphate solution (60 mL). The resulting organic layer was dehydrated with sodium sulfate and filtered to remove sodium sulfate. The resulting filtrate was concentrated under reduced pressure at the external temperature of 40°C to obtain a residue (3.88 g) containing compound A5.
LC retention time of compound A5: 4.161 minutes (HPLC analysis conditions: method 1) LCMS (ESI) retention time of compound A5: 3.708 minutes, m/z = 407.31 $[M+H]^+$ (LCMS analysis conditions: method 2, column of method 4 was used as LC column)

Example A-4

Synthesis of compound A7: (2,3,4,5,6-pentafluorophenyl) (2S)-4-methyl-2-[methyl(2-trimethylsilylethoxycarbonyl)amino]pentanoate

[Formula 51]

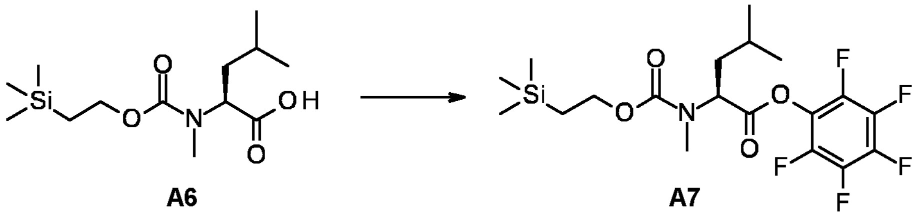

A6 A7

Compound A6 (15.8 g) and 2,3,4,5,6-pentafluorophenol (12.6 g) were added at room temperature to a reaction vessel, and the reaction vessel was subjected to nitrogen replacement. Next, isopropyl acetate (100 mL) and DMF (100 mL) were added. The external temperature of the reaction vessel was set to 0°C, and 1-ethyl-3-(3-dimethylaminopropyl)-carbodiimide (13.1 g) was added with stirring, and then the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). The external temperature of the reaction vessel was set to 0°C, and a 0.5 N aqueous hydrochloric acid solution (100 mL) was added with stirring, and then the mixture was stirred at room temperature for 30 minutes. After the aqueous layer was discharged, the resulting organic layer was washed with a 0.5 N aqueous hydrochloric acid solution (100 mL), and then DMF (10 mL) and 5% aqueous potassium carbonate solution (100 mL) were added for washing. Next, the organic layer was washed with 5% aqueous potassium carbonate solution (100 mL) and saturated aqueous NaCl solution (100 mL). The resulting organic layer was dehydrated with sodium sulfate and filtered to remove sodium sulfate. The resulting filtrate was concentrated under reduced pressure at the external temperature of 40°C to obtain a residue (23.9 g) containing compound A7.
LC retention time of compound A7: 5.195 minutes (HPLC analysis conditions: method 1)

Example A-5

Synthesis of compound A8: tert-butyl (2S)-2-[methyl-[(2S,3S)-3-methyl-2-[[(2S)-4-methyl-2-[methylf2-trimethylsilylethoxycarbonyl)amino]pentanoyl]amino]pentanoyl]amino]propanoate

[Formula 52]

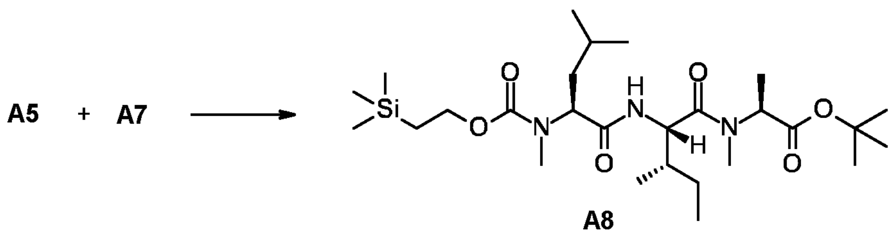

A8

The residue (2.88 g) containing compound A5 obtained in Example A-3 and isopropyl acetate (8.3 mL) were added to a reaction vessel. Next, the residue (3.88 g) containing compound A7 obtained in Example A-4, toluene (2.3 mL), acetone (23 mL), NMM (4.67 mL), and 5% Pd/C (1.51 g, 50% aqueous content) were sequentially added at room temperature. The inside of the reaction vessel was replaced with hydrogen, and the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, and then Pd/C was filtered under reduced pressure using filter paper and a filter. The reaction vessel, and the filter paper and the filter were washed with 2-MeTHF (50 mL). The resulting solution was concentrated under reduced pressure at the external temperature of 40°C. The resulting residue was dissolved in 2-MeTHF (40 mL) and 5% potassium carbonate (40 mL) and DMAP (873 mg) were

added with stirring, and then the mixture was stirred for 2.5 hours. Next, 5% aqueous potassium hydrogen sulfate solution (40 mL) was added, and the mixture was stirred for 30 minutes. After the aqueous layer was discharged, the resulting organic layer was washed with 5% aqueous potassium hydrogen sulfate solution (40 mL) and 5% aqueous potassium carbonate solution (40 mL × 2). The resulting organic layer was dehydrated with sodium sulfate and filtered to remove sodium sulfate. The resulting filtrate was concentrated under reduced pressure at the external temperature of 40°C to obtain a residue (3.58 g) containing compound A8.
LC retention time of compound A8: 5.180 minutes (HPLC analysis conditions: method 1) LCMS (ESI) retention time of compound A8: 4.936 minutes, m/z = 544.31 $[M+H]^+$ (LCMS analysis conditions: method 1)

Example A-6

Synthesis of compound A9: tert-butyl (2S)-2-[methyl-[(2S,3S)-3-methyl-2-[[(2S)-4-methyl-2-(methylamino)pentanoyl] amino]pentanoyl]amino]propanoate

[Formula 53]

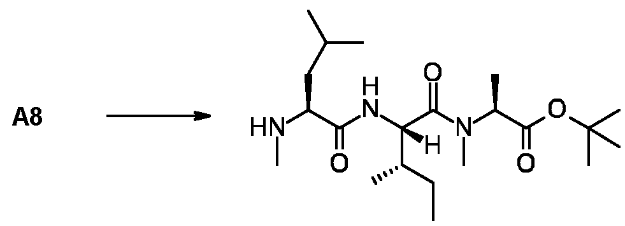

A9

The residue (2.56 g) containing compound A8 obtained in Example A-5, and 2-MeTHF (5.0 mL) were added at room temperature to a reaction vessel, and the reaction vessel was subjected to nitrogen replacement. Next, the external temperature of the reaction vessel was set to 50°C, and 1 M TBAF (11.8 mL) was added with stirring, and then the mixture was stirred at the external temperature of 50°C for 2 hours. The reaction mixture was sampled and prepared as a sample (sample preparation method 1), and it was confirmed by HPLC analysis that the reaction conversion rate was 99.9% or more (calculation expression 1 of reaction conversion rate). The external temperature setting was set to 0°C, and IPAc (10 mL) and 5% potassium carbonate (10 mL) were added with stirring, and then the mixture was stirred at room temperature for 15 minutes. After the aqueous layer was discharged, the resulting organic layer was washed with 5% potassium carbonate (10 mL × 2). The resulting organic layer was dehydrated with sodium sulfate and filtered to remove sodium sulfate. The resulting filtrate was concentrated under reduced pressure at the external temperature of 40°C to obtain a residue (1.54 g) containing compound A9.
LC retention time of compound A9: 2.915 minutes (HPLC analysis conditions: method 1) LCMS (ESI) retention time of compound A9: 2.637 minutes, m/z = 400.34 $[M+H]^+$ (LCMS analysis conditions: method 1)

Example A-7

Synthesis of compound A11: (tert-butyl (3S)-3-[benzyloxycarbonyl(methyl)amino]-4-(dimethylamino)-4-oxo-butanoate)

[Formula 54]

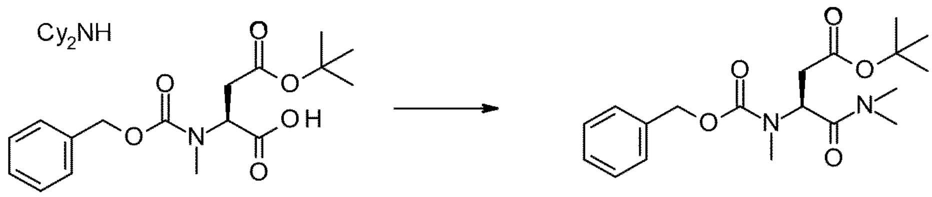

A10 A11

To a reaction vessel after nitrogen replacement, compound A10 (415 g) and 2-MeTHF (2.4 L) were sequentially added at room temperature. The external temperature of the reaction vessel was set to 10°C, and DIPEA (281 g) and a solution of dimethylamine-THF (2 M, THF solution, 560 mL) were sequentially added while the reaction mixture was stirred, and the mixture was stirred for 30 minutes. After T3P (50 w/w%, 2-MeTHF solution, 750 mL) was added, the external temperature of the reaction vessel was set to 25°C, and the mixture was stirred for 1 hour. The reaction mixture was sampled and prepared as a sample (sample preparation method 3), and it was confirmed by HPLC analysis that the reaction conversion rate was 100% (no starting materials were detected) (calculation expression 1 of reaction conversion rate). The external temperature of the reaction vessel was set to 10°C, and 10% aqueous citric acid monohydrate solution (2.5 L) was added to the reaction mixture. The external temperature of the reaction vessel was set to 25°C, and the mixture was stirred for 10 minutes. The stirring was then stopped, and the aqueous layer was discharged from the reaction vessel. The resulting organic layer was washed with 10% aqueous citric acid monohydrate solution (2.5 L) and 5% aqueous sodium carbonate solution (2.5 L × 2). The resultant was concentrated under reduced pressure at the external temperature of 40°C until the liquid volume reached about 500 mL to obtain a solution (500 g) containing compound A11.
LC retention time of compound A11: 3.510 minutes (HPLC analysis conditions: method 3)

Example A-8

Synthesis of compound A12: (tert-butyl (3S)-4-(dimethylamino)-3-(methylamino)-4-oxo-butanoate)

[Formula 55]

A11

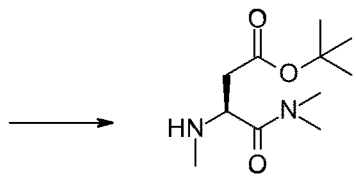

A12

To a reaction vessel after nitrogen replacement, 5% Pd/C (116 g, 50% aqueous content), the solution (485 g) containing compound A11 obtained in Example A-7, and 2-MeTHF (2.1 L) were sequentially added at room temperature. The external temperature of the reaction vessel was set to 25°C, and the reaction vessel was pressurized with hydrogen until the internal pressure of the reaction vessel reached 0.18 MPaG. After stirring for 1 hour and 30 minutes, the reaction mixture was sampled and prepared as a sample (sample preparation method 1), and it was confirmed by HPLC analysis that the reaction conversion rate was 100% (no starting materials were detected) (calculation expression 1 of reaction conversion rate). After replacing the inside of the reaction vessel with nitrogen, the reaction mixture was filtered under pressure. The inside of the reaction vessel and the filtering apparatus were washed with 2-MeTHF (1.2 L × 2), and the filtrate and the wash solution were collected into a storage vessel as a storage solution. The resultant was concentrated under reduced pressure at the external temperature of 40°C until the liquid volume reached about 300 mL to obtain a solution (291 g) containing compound A12.
LC retention time of compound A12: 1.56 minutes (HPLC analysis conditions: method 3)

Example A-9

Synthesis of compound A14: (tert-butyl (3S)-3-[[(25)-2-[benzyloxycarbonyl(methyl)amino]-2-cyclopentyl-acetyl]-methyl-amino]-4-(dimethylamino)-4-oxo-butanoate)

[Formula 56]

A12 +

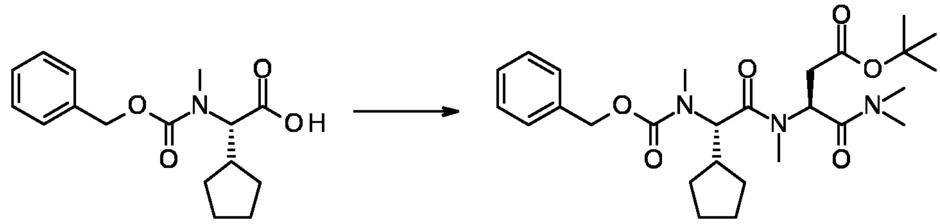

A13 A14

To a reaction vessel, the solution (291 g) containing A12 obtained in Example A-8, compound A13 (49 w/w% 2-MeTHF solution, 548 g), 2-MeTHF (445 mL), and acetonitrile (310 mL) were added at room temperature. The external temperature was cooled to 10°C, and DIPEA (439 g) and HATU (434 g) were sequentially added, and then the external temperature was raised to 25°C. After the reaction mixture was stirred at 25°C for 5 hours, the reaction mixture was sampled and prepared as a sample (sample preparation method 1), and it was confirmed by HPLC analysis that the reaction conversion rate was 98.6% (calculation expression 1 of reaction conversion rate). To the reaction vessel, CPME (402 mL), 5% aqueous potassium carbonate solution (309 mL), and N-methylimidazole (62.6 g) were sequentially added, and the mixture was stirred for 30 minutes. Then, 2.5% aqueous ammonia solution (1.2 L) was added, and the mixture was stirred for 10 minutes, and then the aqueous layer was discharged. The resulting organic layer was washed with 2.5% aqueous ammonia solution (1.5 L), 10% aqueous sodium hydrogen sulfate monohydrate solution (1.5 L × 2), and 5% aqueous potassium carbonate solution (1.5 L). The resulting organic layer was concentrated under reduced pressure with stirring at the external temperature of 40°C until the liquid volume reached about 700 mL to obtain a solution (649 g) containing compound A14.
LC retention time of compound A14: 4.356 minutes (HPLC analysis conditions: method 3)

Example A-10

Synthesis of compound A15: (tert-butyl (3S)-3-[[(2S)-2-cyclopentyl-2-(methylamino)acetyl]-methyl-amino]-4-(dimethylamino)-4-oxo-butanoate)

[Formula 57]

A14

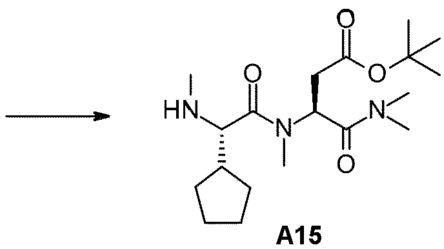

A15

To a reaction vessel after nitrogen replacement, 5% Pd/C (112 g, 50% aqueous content), the solution (642 g) containing compound A14 obtained in Example A-9, and 2-MeTHF (2.0 L) were sequentially added at room temperature. The external temperature of the reaction vessel was set to 25°C, and the reaction vessel was pressurized with hydrogen until the internal pressure of the reaction vessel reached 0.18 MPaG. The mixture was stirred for 2 hours, and no fluctuation in the internal pressure was confirmed. The reaction mixture was sampled and prepared as a sample (sample preparation method 1), and it was confirmed by HPLC analysis that the reaction conversion rate was 100% (no starting materials were detected) (calculation expression 1 of reaction conversion rate). After replacing the inside of the reaction vessel with nitrogen, the reaction mixture was filtered under pressure. The inside of the reaction vessel and the filtering apparatus were washed with 2-MeTHF (1.1 L × 2), and the filtrate and the wash solutions were collected as a storage solution. The resulting filtrate and the wash solutions were concentrated under reduced pressure with stirring at the external temperature of 40°C until the liquid volume of the reaction mixture reached about 550 mL to obtain a solution (597 g) containing compound A15.
LC retention time of compound A15: 2.297 minutes (HPLC analysis conditions: method 3)

Example A-11

Synthesis of compound A17: (tert-butyl (3S)-3-[[(2S)-2-cyclopentyl-2-[methyl-[1-[(2,2,2-trifluoroacetyl)amino]cyclopentanecarbonyl]amino]acetyl]-methyl-amino]-4-(dimethylamino)-4-oxo-butanoate)

[Formula 58]

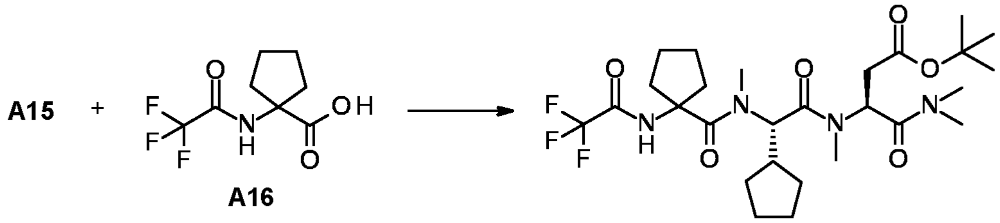

A15 + A16 → A17

To a reaction vessel after nitrogen replacement, compound A16 (333 g) and 2-MeTHF (1.8 L) were added at room temperature. The external temperature of the reaction vessel was set to 10°C, and DIPEA (477 g), the solution (585 g) containing compound A15 obtained in Example A-10, T3P (50 w/w%, 2-MeTHF solution, 1.2 L), and DMAP (180 g) were sequentially added. The external temperature of the reaction vessel was set to 50°C, and the mixture was stirred for 5 hours. The reaction mixture was sampled and prepared as a sample (sample preparation method 1), and it was confirmed by HPLC analysis that the reaction conversion rate was 99.4% (calculation expression 1 of reaction conversion rate). The external temperature of the reaction vessel was set to 10°C, and 5% aqueous sodium carbonate solution (2.2 L) was added. The external temperature of the reaction vessel was set to 25°C, and the mixture was stirred for 30 minutes, and then the stirring was stopped, and the aqueous layer was discharged from the reaction vessel. Then, 5% aqueous sodium hydrogen sulfate monohydrate solution (2.2 L) was added. After the mixture was stirred for 10 minutes, the stirring was stopped, and the aqueous layer was discharged from the reaction vessel. The resulting organic layer was washed with 5% aqueous sodium hydrogen sulfate monohydrate solution (2.2 L), and 5% aqueous sodium carbonate solution (2.2 L). The resultant was concentrated under reduced pressure at the external temperature of 40°C until the liquid volume reached about 850 mL. After 2-MeTHF (650 mL) was added, the mixture was concentrated again under reduced pressure at the external temperature of 40°C until the liquid volume reached about 850 mL to obtain a solution (721 g) containing compound A17.
LC retention time of compound A17: 6.166 minutes (HPLC analysis conditions: method 3)

Example A-12

Synthesis of compound A18: (tert-butyl (3S)-3-[[(2S)-2-[(1-aminocyclopentanecarbonyl)-methyl-amino]-2-cyclopentyl-acetyl]-methyl-amino]-4-(dimethylamino)-4-oxo-butanoate)

[Formula 59]

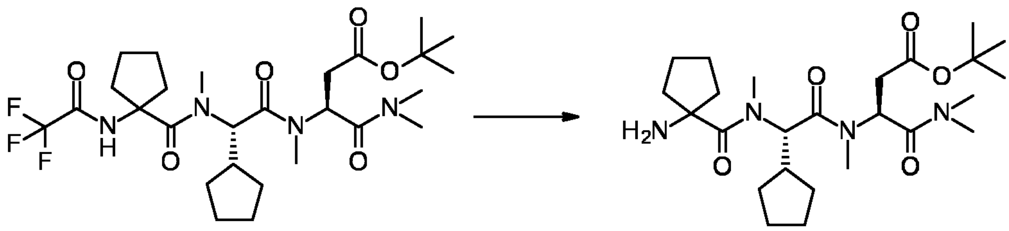

A17 → A18

To a reaction vessel after nitrogen replacement, a solution (3.42 kg) containing compound A17, 2-MeTHF (3.4 L), and methanol (450 mL) were sequentially added at room temperature. The external temperature of the reaction vessel was set to -20°C, and $LiBH_4$ (4 M, THF solution, 1.39 L) was added while stirring, and then the mixture was stirred for 1 hour. The

reaction mixture was sampled and prepared as a sample (sample preparation method 4), and it was confirmed by HPLC analysis that the reaction conversion rate was 100% (no starting materials were detected) (calculation expression 1 of reaction conversion rate). The external temperature of the reaction vessel was set to -10°C, and a 20% aqueous ammonium chloride solution (4.0 L) was added dropwise over 3 hours. The mixture was stirred at the external temperature of 25°C for 1 hour, then the stirring was stopped, and the aqueous layer was discharged from the reaction vessel. The external temperature of the reaction vessel was set to 10°C, and trifluoroacetic acid (158 g) was added. The external temperature of the reaction vessel was set to 25°C, and the mixture was stirred for 1 hour. The resulting reaction mixture and the wash solution obtained by washing the reaction vessel with 2-MeTHF (100 mL × 2) were combined and collected into a storage vessel. To another reaction vessel after nitrogen replacement, 2 M aqueous sodium hydroxide solution (4.0 L) was added at room temperature, and the external temperature of the reaction vessel was set to 10°C. The reaction mixture collected into the storage vessel was added thereto dropwise over 50 minutes, and then the external temperature of the reaction vessel was set to 25°C. After the mixture was stirred for 10 minutes, the stirring was stopped, and the aqueous layer was discharged from the reaction vessel. The resulting organic layer was washed with 2 M aqueous sodium hydroxide solution (4.0 L) and 10% aqueous dipotassium hydrogen phosphate solution (4.0 L). The resulting organic layer was concentrated under reduced pressure at the external temperature of 40°C until the liquid volume reached about 1.3 L. After 2-MeTHF (1.6 L) was added, the mixture was concentrated again under reduced pressure at the external temperature of 40°C until the liquid volume reached about 1.3 L. This concentration operation was repeated three times to obtain a solution (1.19 kg) containing compound A18. LC retention time of compound A18: 2.725 minutes (HPLC analysis conditions: method 3)

Example A13

Synthesis of compound A20: (benzyl (2S)-2-[[1-[[(1S)-2-[[(1S)-3-tert-butoxy-1-(dimethylcarbamoyl)-3-oxo-propyl]-methyl-amino]-1-cyclopentyl-2-oxo-ethyl]-methylcarbamoyl]cyclopentyl]carbamoyl]pyrrolidine-1-carboxylate)

[Formula 60]

A18 +

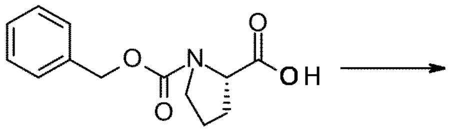

A19

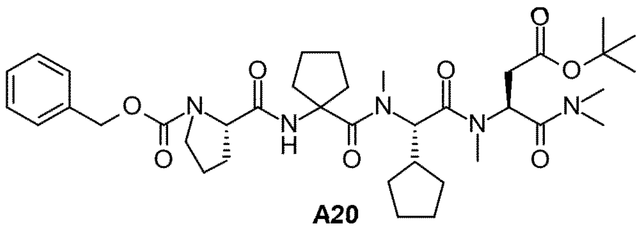

A20

To a reaction vessel after nitrogen replacement, the solution (585 g, 2-MeTHF solution) containing compound 18 obtained in Example A-12 and acetonitrile (1.6 L) were sequentially added at room temperature. The external temperature of the reaction vessel was set to 10°C, and compound A19 (220 g), DIPEA (264 g), 2-bromo-1-ethylpyridinium tetrafluoroborate (279 g) were sequentially added. The external temperature of the reaction vessel was set to 25°C, and the mixture was stirred for 1 hour. The reaction mixture was sampled and prepared as a sample (sample preparation method 1), and it was confirmed by HPLC analysis that the reaction conversion rate was 99.6% (calculation expression 1 of reaction conversion rate). The external temperature of the reaction vessel was set to 10°C, and CPME (3.3 L), 5% aqueous potassium carbonate solution (2.0 L), and N-methylimidazole (55.8 g) were sequentially added. The external temperature of the reaction vessel was set to 25°C, and the mixture was stirred for 30 minutes, and then the aqueous layer was discharged from the reaction vessel. The resulting organic layer was washed with 5% aqueous sodium hydrogen sulfate monohydrate solution (2.5 L × 2), and 5% aqueous sodium carbonate solution (2.0 L × 2). The resulting organic layer was concentrated under reduced pressure at the external temperature of 40°C until the liquid volume reached about 1 L. To the resulting residue, THF (1.6 L) was added to obtain a solution (2.09 kg) containing compound A20.
LC retention time of compound A20: 4.189 minutes (HPLC analysis conditions: method 3)

Example A-14

Synthesis of compound A21: (tert-butyl (3S)-3-[[(2S)-2-cyclopentyl-2-[methyl-[1-[[(2S)-pyrrolidine-2-carbonyl]amino]cyclopentanecarbonyl]amino]acetyl]-methyl-amino]-4-(dimethylamino)-4-oxo-butanoate)

[Formula 61]

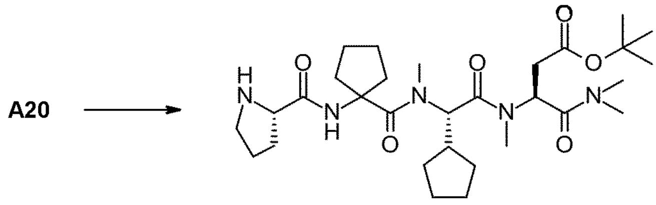

A21

To a reaction vessel after nitrogen replacement, 5% Pd/C (100 g, 50% aqueous content), the solution (2.09 kg) containing compound A20 obtained in Example A-13, and THF (0.5 L) were sequentially added at room temperature. The external temperature of the reaction vessel was set to 25°C, and the reaction vessel was pressurized with hydrogen until the internal pressure of the reaction vessel reached 0.18 MPaG. After 2 hours and 30 minutes, no fluctuation in the internal pressure was confirmed. The reaction vessel was then subjected to nitrogen replacement, and then pressurized to 0.18 MPaG with hydrogen, and the reaction mixture was stirred for an additional 2 hours. The reaction mixture was sampled and prepared as a sample (sample preparation method 1), and it was confirmed by HPLC analysis that the reaction conversion rate was 99.5% (calculation expression 1 of reaction conversion rate). After replacing the inside of the reaction vessel with nitrogen, the reaction mixture was filtered under pressure. The reaction vessel and the filtering apparatus were washed with 2-MeTHF (1.0 L × 2). The resulting filtrate and the wash solutions were concentrated under reduced pressure at the external temperature of 40°C until the liquid volume reached about 0.8 L to obtain a solution (530 g) containing compound A21.

LC retention time of compound A21: 2.846 minutes (HPLC analysis conditions: method 3)

Example A-15

Synthesis of compound A23: (tert-butyl (3S)-3-[[(2S)-2-[[1-[[(2S)-1-[(2S)-2-(benzyloxycarbonylamino)-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoate)

[Formula 62]

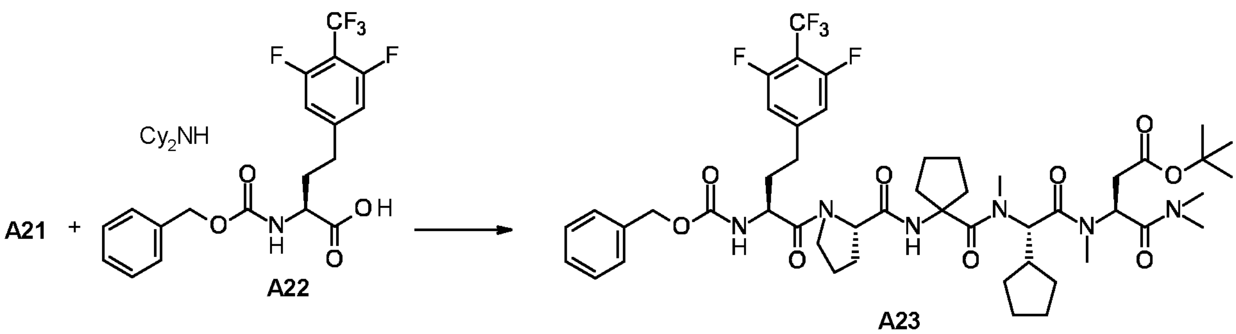

A22

A23

To a reaction vessel after nitrogen replacement, the solution (1.10 kg) containing compound A21 obtained in Example A-14, 2-MeTHF (1.4 L), and compound A22 (545 g) were sequentially added at room temperature. The external temperature of the reaction vessel was set to 10°C, and DIPEA (432 g) and T3P (50 w/w% 2-MeTHF solution, 1.16 kg) were added sequentially with stirring. The external temperature of the reaction vessel was set to 25°C, and the mixture was stirred for 1 hour. The reaction mixture was sampled and prepared as a sample (sample preparation method 1), and it was confirmed by HPLC analysis that the reaction conversion rate was 99.8% (calculation expression 1 of reaction conversion rate). The external temperature of the reaction vessel was set to 15°C, and 5% aqueous potassium carbonate solution (2.6 L) and N-methylimidazole (62.3 g) were added with stirring. The external temperature of the reaction vessel was set to 25°C, and the mixture was stirred for 30 minutes, and then the aqueous layer was discharged from the reaction vessel. After the resulting organic layer was washed with 10% aqueous sodium hydrogen sulfate monohydrate solution (2.6 L × 2), acetonitrile (1.0 L), MTBE (1.1 L), heptane (1.6 L), and 2.5% aqueous potassium carbonate solution (2.5 L) were sequentially added at the external temperature of 25°C. After the mixture was stirred for 10 minutes, the stirring was

stopped, and then the aqueous layer was discharged from the reaction vessel. To the resulting organic layer, acetonitrile (1.5 L), 2-MeTHF (0.44 L), and 2.5% aqueous potassium carbonate solution (3.8 L) were added, and the mixture was stirred for 10 minutes. After stirring was stopped, the aqueous layer was discharged from the reaction vessel. To the resulting organic layer, acetonitrile (1.5 L) and 2.5% aqueous potassium carbonate solution (3.8 L) were added, and the mixture was stirred for 10 minutes. After stirring was stopped, the aqueous layer was discharged from the reaction vessel. The resulting organic layer was concentrated under reduced pressure at the external temperature of 40°C until the liquid volume reached about 1.5 L. IPAc (1.5 L) was added to the resulting residue, and the operation of concentrating under reduced pressure was repeated twice until the liquid volume of the reaction mixture reached about 1.5 L to obtain a solution (1.55 kg) containing compound A23.
LC retention time of compound A23: 4.978 minutes (HPLC analysis conditions: method 3)

Example A-16

Synthesis of compound A24: ((3S)-3-[[(2S)-2-[[1-[[(2S)-1-[(2S)-2-(benzyloxycarbonylamino)-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoic acid)

[Formula 63]

A23

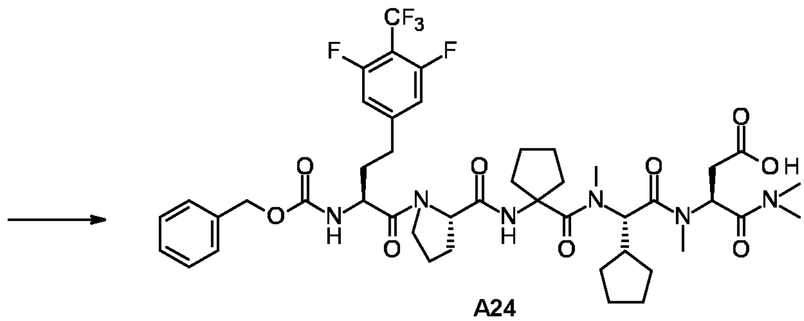

A24

To a reaction vessel after nitrogen replacement, the solution (1.31 kg) containing compound A23 obtained in Example A-15, isopropyl acetate (2.5 L), and hexamethyldisilazane (336 mL) were sequentially added at room temperature. The external temperature of the reaction vessel was set to 0°C and trimethylsilyl trifluoromethanesulfonate (232 mL) was added with stirring. The external temperature was set to 25°C, and the reaction mixture was stirred for 1 hour. The reaction mixture was sampled and prepared as a sample (sample preparation method 1), and it was confirmed by HPLC analysis that the reaction conversion rate was 99.9% (calculation expression 1 of reaction conversion rate). The external temperature of the reaction vessel was set to 0°C, and 2-MeTHF (3.2 L) and 5% aqueous disodium hydrogen phosphate solution (6.4 L) were sequentially added to the reaction vessel. The external temperature of the reaction vessel was set to 25°C, and the reaction mixture was stirred for 10 minutes. The stirring was then stopped, and the aqueous layer was discharged from the reaction vessel. The organic layer was then washed with 5% aqueous sodium dihydrogen phosphate solution (6.4 L) and 15% sodium chloride (6.4 L). To the resulting organic layer, DIPEA (366 g) was added with stirring, and then the mixture was concentrated under reduced pressure at the external temperature of 40°C until the liquid volume reached about 1.2 L to obtain a solution (1.27 kg) containing compound A24.
LC retention time of compound A24: 4.220 minutes (HPLC analysis conditions: method 3)

Example A-17

Synthesis of compound A25: tert-butyl (2S)-2-[[(2S,3S)-2-[[(2S)-2-[[(3S)-3-[[(2S)-2-[[1-[[(2S)-1-[(2S)-2-(benzyloxycarbonylamino)-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methylpentanoyl]amino]-3-methyl-pentanoyl]-methyl-amino]propanoate

[Formula 64]

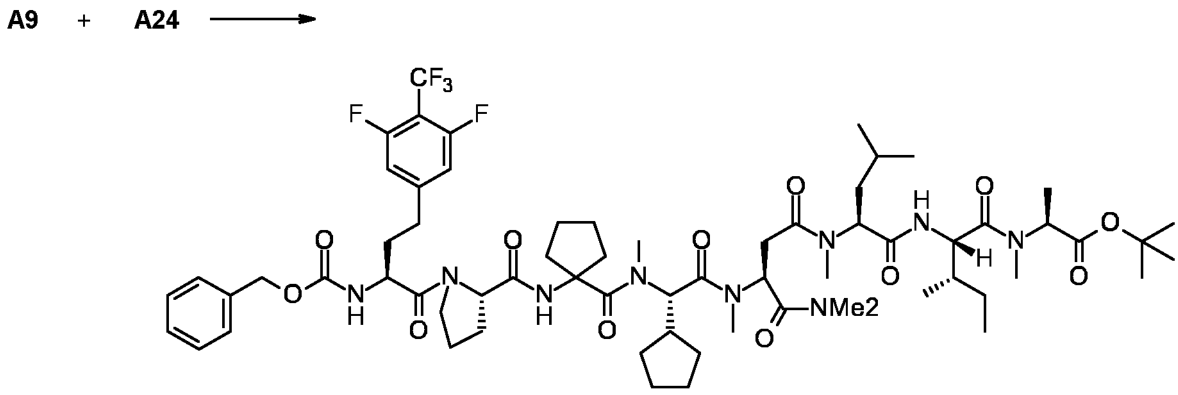


A25

The solution (4.02 g) containing compound A24 obtained in Example A-16 was added to a reaction vessel. The residue (0.797 g) containing compound A9 obtained in Example A-6, 2-MeTHF (36 mL), DMF (9.2 mL), and DIPEA (1.77 mL) were sequentially added at room temperature, and the reaction vessel was subjected to nitrogen replacement. Next, HATU (1.59 g) was added with stirring, and then the mixture was stirred at room temperature for 20 hours. Subsequently, the residue (0.04 g) containing compound A9 obtained in Example A-6 and HATU (0.37 g) were added, and the mixture was stirred for 1 hour. The reaction mixture was sampled and prepared as a sample (sample preparation method 1), and it was confirmed by HPLC analysis that the reaction conversion rate was 99.9% (calculation expression 1 of reaction conversion rate). To the reaction vessel, 2.5% aqueous ammonia solution (32 mL) was added, and the mixture was stirred for 30 minutes. After the aqueous layer was discharged, the resulting organic layer was washed with 10% aqueous sodium hydrogen sulfate monohydrate solution (28 mL × 3) and 5% aqueous potassium carbonate solution (28 mL × 2). The resulting organic layer was dehydrated with sodium sulfate and filtered to remove sodium sulfate. The resultant was concentrated under reduced pressure at the external temperature of 40°C, and the resulting residue was purified by silica gel column chromatography (eluent: ethyl acetate/heptane 70 : 30 to 100 : 0). The eluate containing the target material was concentrated under reduced pressure at the external temperature of 40°C to obtain a residue (2.08 g) containing compound A25.

LC retention time of compound A25: 21.235 minutes (HPLC analysis conditions: method 3)
LCMS(ESI) of compound A25: retention time: 21.82 minutes, m/z = 1325.478 [M+Na]$^+$ (LCMS analysis conditions: method 3)

Example A-18

Synthesis of compound A28: (tert-butyl 2-[[(2S)-2-[benzyloxycarbonyl(ethyl)amino]-3-(p-tolyl)propanoyl]-methyl-amino]acetate)

[Formula 65]

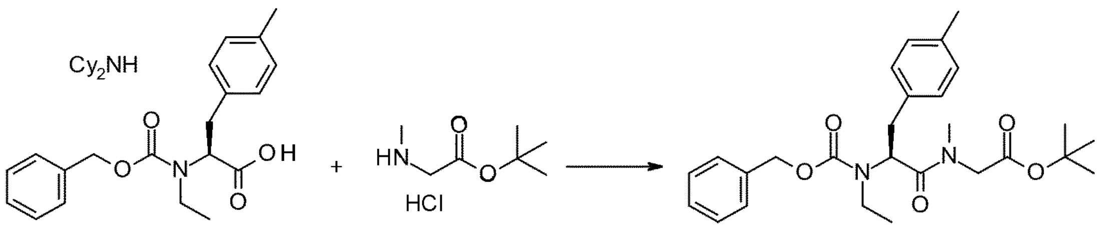


A27 A26 A28

To a reaction vessel after nitrogen replacement, compound A27 (350 g) and compound A26 (146 g) were added at room temperature, and then 2-MeTHF (2.0 L) was added, and the mixture was stirred. The external temperature of the reaction vessel was set to 10°C and DIPEA (630 mL) was added, and then T3P (50 w/w% 2-MeTHF solution, 1.02 kg) was

added dropwise. The external temperature of the reaction vessel was set to 25°C, and the mixture was stirred for 1 hour and 40 minutes. The reaction mixture was sampled and prepared as a sample (sample preparation method 2), and it was confirmed by HPLC analysis that the reaction conversion rate was 99.7% (calculation expression 3 of reaction conversion rate). 5% aqueous sodium carbonate solution (2.0 L) was added dropwise, and then water (0.50 L) was added. After the mixture was stirred for 15 minutes, the aqueous layer was discharged from the reaction vessel. The resulting organic layer was washed also at the external temperature of 25°C with 5% aqueous sodium hydrogen sulfate monohydrate solution (2.0 L × 2) and 5% aqueous sodium carbonate solution (2.0 L). The resulting organic layer was concentrated under reduced pressure at the external temperature of 40°C until the liquid volume reached about 600 mL to obtain a solution (595 g) containing compound A28.
LC retention time of compound A28: 4.500 minutes (HPLC analysis conditions: method 1)

Example A-19

Synthesis of compound A29: (tert-butyl 2-[[(2S)-2-(ethylamino)-3-(p-tolyl)propanoyl]-methyl-amino]acetate)

[Formula 66]

A28

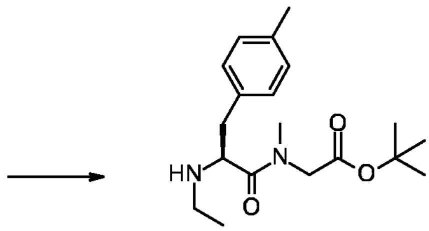

A29

To a reaction vessel after nitrogen replacement, the solution (589 g) containing compound A28 obtained in Example A-18 and 2-MeTHF (1.7 L) were added, and then 5% Pd/C (142 g, 50% aqueous content) was added. The external temperature of the reaction vessel was set to 25°C, and the reaction vessel was pressurized with hydrogen until the internal pressure of the reaction vessel reached 0.18 MPaG. After 1 hour, no fluctuation in the internal pressure was confirmed. The reaction vessel was then subjected to nitrogen replacement, and then further pressurized to 0.15 MPaG with hydrogen, and the reaction mixture was stirred for 5 hours. The reaction mixture was sampled and prepared as a sample (sample preparation method 1), and it was confirmed by HPLC analysis that the reaction conversion rate was 99.8% (calculation expression 1 of reaction conversion rate). After replacing the inside of the reaction vessel with nitrogen, the reaction mixture was filtered under pressure. The reaction vessel and the filtering apparatus were washed with 2-MeTHF (1.0 L), and the filtrate and the wash solution were collected into a storage vessel as a storage solution. The resulting filtrate and the wash solution were concentrated under reduced pressure at the external temperature of 40°C until the liquid volume reached about 500 mL to obtain a solution (527 g) containing compound A29.
LC retention time of compound A29: 2.389 minutes (HPLC analysis conditions: method 1)

Example A-20

Synthesis of compound A31: (benzyl (2S)-2-[[(1S)-2-[(2-tert-butoxy-2-oxo-ethyl)-methyl-amino]-2-oxo-1-(p-tolylmethyl)ethyl]-ethyl-carbamoyl]azetidine-1-carboxylate)

[Formula 67]

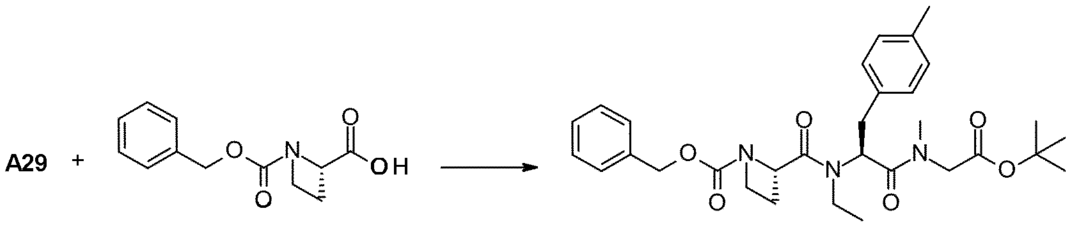

A30

A31

To a reaction vessel after nitrogen replacement, the solution (522 g) containing compound A29 obtained in Example A-19, 2-MeTHF (1.4 L), and compound A30 (234 g) were sequentially added at room temperature. The external temperature of the reaction vessel was set to 10°C, DIPEA (570 mL) was added with stirring, and then T3P (50 w/w% 2-MeTHF solution, 1.2 L) was added dropwise. The external temperature of the reaction vessel was set to 25°C, and the reaction mixture was stirred for 2 hours and 30 minutes. The reaction mixture was sampled and prepared as a sample (sample preparation method 1), and it was confirmed by HPLC analysis that the reaction conversion rate was 100% (no starting materials were detected) (calculation expression 1 of reaction conversion rate). The external temperature of the reaction vessel was set to 10°C, and 5% aqueous sodium carbonate solution (1.4 L) was added to the reaction mixture with stirring. The external temperature of the reaction vessel was set to 25°C, and the mixture was stirred for 10 minutes, and then the stirring was stopped, and the aqueous layer was discharged from the reaction vessel. The resulting organic layer was washed with 5% aqueous sodium hydrogen sulfate monohydrate solution (1.4 L × 2), and 5% aqueous sodium carbonate solution (1.4 L). The resulting organic layer was concentrated under reduced pressure until the liquid volume reached about 650 mL to obtain a solution (655 g) containing compound A31. A portion of the solution containing the resulting compound A31 (5.03 g) was purified by silica gel column chromatography (eluent: heptane/ethyl acetate 90 : 10 to 50 : 50). The eluate containing the target material was concentrated under reduced pressure at the external temperature of 40°C to obtain a residue (2.88 g) containing compound A31.
LC retention time of compound A31: 4.065 minutes (HPLC analysis conditions: method 1)

Example A-21

Synthesis of compound A32: 2-[[(2S)-2-[[(2S)-2-[benzyloxycarbonyl(methyl)amino]butanoyl]-ethyl-amino]-3-(p-tolyl)propanoyl]-methyl-amino]acetic acid

[Formula 68]

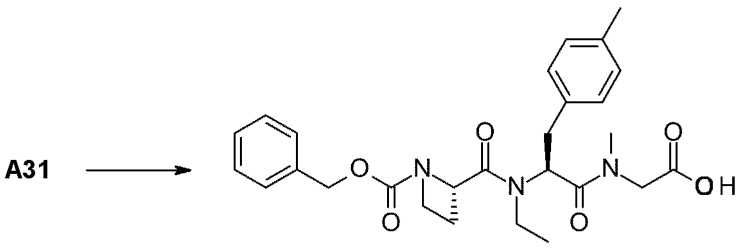

A32

The residue (2.57 g) containing compound A31 obtained in Example A-20 was added to a reaction vessel. 2-MeTHF (24 mL) and HMDS (6.85 mL) were sequentially added at room temperature, and the reaction vessel was subjected to nitrogen replacement. Next, the external temperature of the reaction vessel was set to 0°C, and TMSOTf (5.06 mL) was added with stirring, and then the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). The external temperature was set to 0°C, and 5% aqueous sodium hydrogen sulfate monohydrate solution (20 mL) was added to the reaction vessel, and the mixture was stirred at room temperature for 20 minutes. After the aqueous layer was discharged, the resulting organic layer was washed with 5% aqueous sodium hydrogen sulfate monohydrate solution (20 mL × 3). Next, 5% aqueous dipotassium hydrogen phosphate solution (20 mL × 2) was added to the resulting organic

layer to phase transfer the product to the aqueous layer. After the organic layer was discharged, isopropyl acetate (50 mL) and 5% aqueous sodium hydrogen sulfate monohydrate solution were added to the resulting aqueous layer until pH reached 1-3 to phase transfer the product to the organic layer. The extraction with isopropyl acetate (50 mL) was repeated twice, and all the resulting organic layers were dehydrated with sodium sulfate and filtered to remove sodium sulfate. Then, the resulting filtrate was concentrated under reduced pressure at the external temperature of 40°C. The resulting residue was purified by silica gel column chromatography (eluent: dichloromethane/methanol 100 : 0 to 80 : 20). The eluate containing the target material was concentrated under reduced pressure at the external temperature of 40°C to obtain a residue (1.18 g) containing compound A32.
LCMS (ESI) retention time of compound A32: 3.045 minutes, m/z = 496.27 $[M+H]^+$ (LCMS analysis conditions: method 2, column of method 4 was used as LC column)

Example A-22

Synthesis of compound A33: tert-butyl (2S)-2-[[(2S,3S)-2-[[(2S)-2-[[(3S)-3-[[(2S)-2-[[1-[[(2S)-1-[(2S)-2-amino-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoate

[Formula 69]

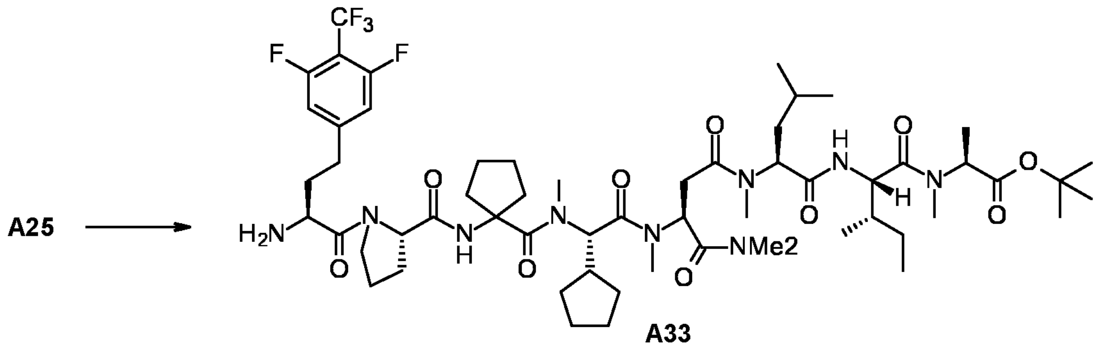

The compound A25 (1.87 g) obtained in Example A-17 was added to a reaction vessel. Next, THF (18 mL) and 5% Pd/C (296 mg, 50% aqueous content) were sequentially added at room temperature. The inside of the reaction vessel was replaced with hydrogen, and the mixture was stirred at room temperature. The inside of the reaction vessel was replaced again with hydrogen after 2 and 4 hours, 5% Pd/C (296 mg, 50% aqueous content) was added after 5 hours, and the mixture was stirred for an additional 3 hours. The reaction mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with THF (9.0 mL × 2). The filtrate and the wash solutions were combined and concentrated under reduced pressure at the external temperature of 40°C to obtain a residue (1.68 g) containing compound A33.
LC retention time of compound A33: 4.256 minutes (HPLC analysis conditions: method 2)

Example A-23

Synthesis of compound A34: benzyl (2S)-2-[[(1S)-2-[[2-[[(1S)-1-[(2S)-2-[[1-[[(1S)-2-[[(1S)-3-[[(1S)-1-[[(1S,2S)-1-[[(1S)-2-tert-butoxy-1-methyl-2-oxo-ethyl]-methyl-carbamoyl]-2-methylbutyl]carbamoyl]-3-methyl-butyl]-methyl-amino]-1-(dimethylcarbamoyl)-3-oxo-propyl]-methyl-amino]-1-cyclopentyl-2-oxo-ethyl]-methyl-carbamoyl]cyclopentyl]carbamoyl]pyrrolidine-1-carbonyl]-3-[3,5-difluoro-4-(trifluoromethyl)phenyl]propyl]amino-2-oxo-ethyl]-methyl-amino]-2-oxo-1-(p-tolylmethyl)ethyl]-ethyl-carbamoyl]azetidine-1-carboxylate

[Formula 70]

A32 + A33

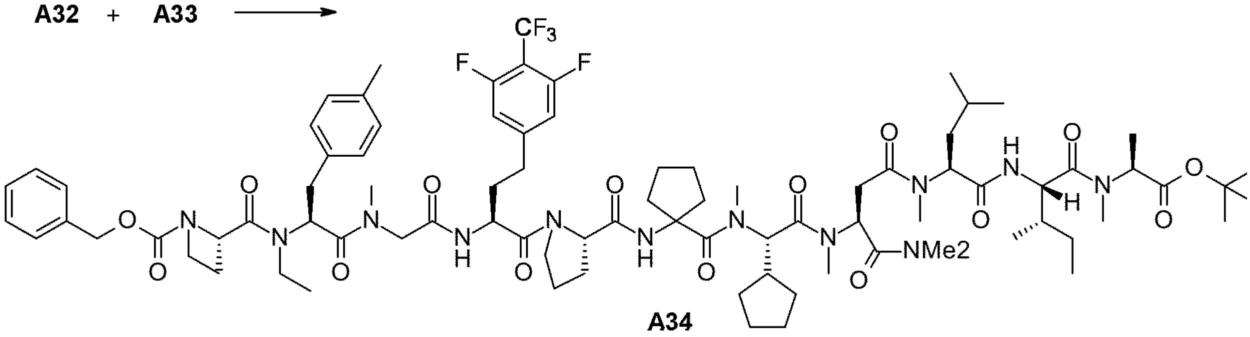

A34

The residue (1.68 g) containing compound A33 obtained in Example A-22 and the residue (0.856 g) containing compound A32 obtained in Example A-21 were added to a reaction vessel. 2-MeTHF (13 mL), DMF (4.2 mL), and DIPEA (1.41 mL) were sequentially added at room temperature, and the reaction vessel was subjected to nitrogen replacement. Next, HATU (1.26 g) was added with stirring, and then the mixture was stirred at room temperature for 3 hours. The reaction mixture was sampled and prepared as a sample (sample preparation method 1), and it was confirmed by HPLC analysis that the reaction conversion rate was 99.8% (calculation expression 1 of reaction conversion rate). To the reaction vessel, 2.5% aqueous ammonia solution (10 mL) was added, and the mixture was stirred for 15 minutes. After the aqueous layer was discharged, the resulting organic layer was washed with 10% aqueous sodium hydrogen sulfate monohydrate solution (10 mL × 3) and 5% aqueous potassium carbonate solution (10 mL × 2). The resulting organic layer was dehydrated with sodium sulfate and filtered to remove sodium sulfate. The resulting filtrate was concentrated under reduced pressure at the external temperature of 40°C, and the resulting residue was purified by silica gel column chromatography (eluent: ethyl acetate/methanol 100 : 0 to 90 : 10). The eluate containing the target material was concentrated under reduced pressure at the external temperature of 40°C to obtain a residue (1.96 g) containing compound 34.

LC retention time of compound A34: 21.670 minutes (HPLC analysis conditions: method 3)
LCMS (ESI) retention time of compound A34: 22.30 minutes, m/z = 1668.168 $[M+Na]^+$ (LCMS analysis conditions: method 3)

## Example A-24

Synthesis of compound A35: (2S)-2-[[(2S,3S)-2-[[(2S)-2-[[(3S)-3-[[(2S)-2-[[1-[[(2S)-1-[(2S)-2-[[2-[[(2S)-2-[[(2S)-1-benzyloxycarbonylazetidine-2-carbonyl]-ethyl-amino]-3-(p-tolyl)propanoyl]-methyl-amino]acetyl]amino]-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoic acid

[Formula 71]

A34

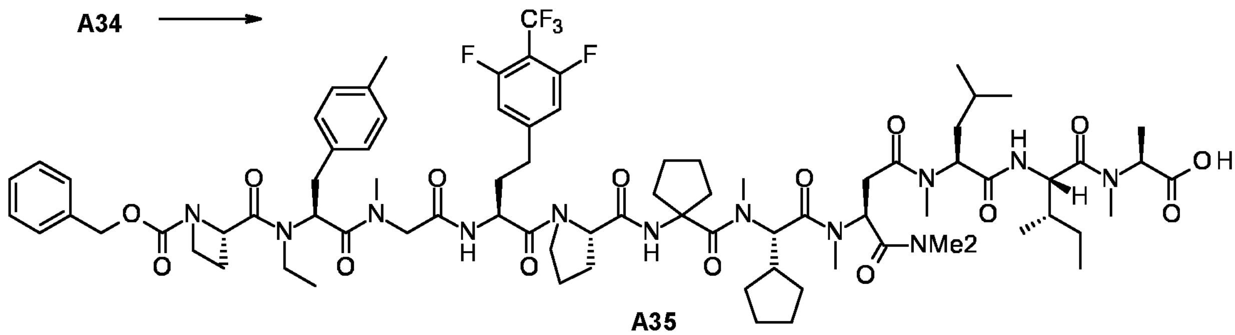

A35

The residue (1.62 g) containing compound A34 obtained in Example A-23 was added to a reaction vessel. 2-MeTHF (16 mL) and HMDS (1.03 mL) were sequentially added at room temperature, and the reaction vessel was subjected to nitrogen replacement. Next, the external temperature of the reaction vessel was set to 0°C, and TMSOTf (0.709 mL) was added with stirring, and then the mixture was stirred at room temperature for 3 hours. The reaction mixture was sampled and prepared as a sample (sample preparation method 1), and it was confirmed by HPLC analysis that the reaction conversion rate was 99.9% or more (calculation expression 1 of reaction conversion rate). The external temperature was set to 0°C, and 5% aqueous sodium hydrogen carbonate solution (15 mL) was added to the reaction vessel, and the mixture was stirred at room temperature for 30 minutes. The aqueous layer was discharged, and then the resulting organic layer was washed with 5% aqueous sodium dihydrogen phosphate solution (15 mL) and 10% aqueous NaCl solution (15 mL). The resulting organic layer was dehydrated with sodium sulfate and filtered to remove sodium sulfate. The resulting filtrate was concentrated under reduced pressure at the external temperature of 40°C to obtain a residue (1.66 g) containing compound A35.
LC retention time of compound A35: 19.027 minutes (HPLC analysis conditions: method 3)

Example A-25

Synthesis of compound A36: (2S)-2-[[(2S,3S)-2-[[(2S)-2-[[(3S)-3-[[(2S)-2-[[1-[[(2S)-1-[(2S)-2-[[2-[[(2S)-2-[[(2S)-azetidine-2-carbonyl]-ethyl-amino]-3-(p-tolyl)propanoyl]-methyl-amino]acetyl]amino]-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoic acid

[Formula 72]

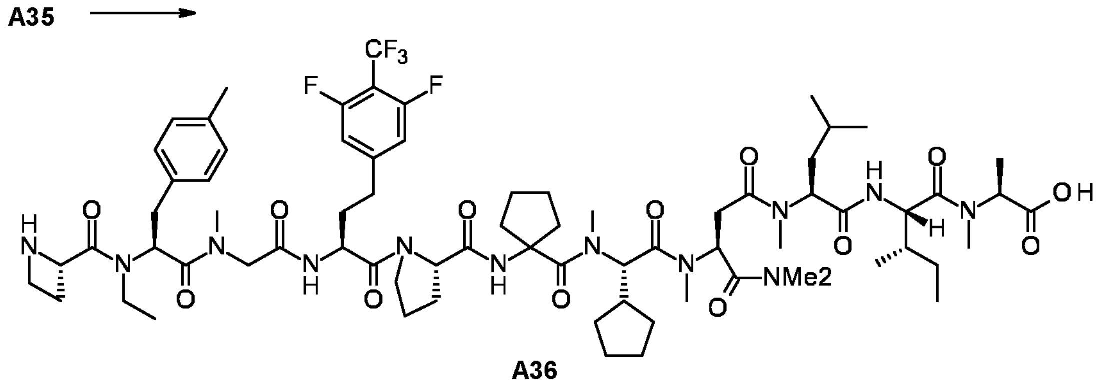

A36

The residue (1.66 g) containing compound A35 obtained in Example A-24 was added to a reaction vessel. Next, THF (11 mL) and 5% Pd/C (444 mg, 50% aqueous content) were sequentially added at room temperature. The inside of the reaction vessel was replaced with hydrogen, and the mixture was stirred at room temperature. After 2 hours, the inside of the reaction vessel was replaced again with hydrogen, and the mixture was stirred for 4 hours. The reaction mixture was sampled and prepared as a sample (sample preparation method 1), and it was confirmed by HPLC analysis that the reaction conversion rate was 99.8% (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with THF (6.0 mL × 2). The resulting filtrate and the wash solution were combined and concentrated under reduced pressure at the external temperature of 40°C. The resulting residue was purified by silica gel column chromatography (eluent: dichloromethane/methanol 100 : 0 to 80 : 20). The eluate containing the target material was concentrated under reduced pressure at the external temperature of 40°C to obtain a residue (1.02 g) containing compound A36.

LC retention time of compound A36: 13.323 minutes (HPLC analysis conditions: method 3)
LCMS (ESI) retention time of compound A36: 13.76 minutes, m/z = 1456.124 $[M+H]^+$ (LCMS analysis conditions: method 3)

Example B-1

Synthesis of compound B1: O2-tert-butyl O1-benzyl (2S)-azetidine-1,2-dicarboxylate

[Formula 73]

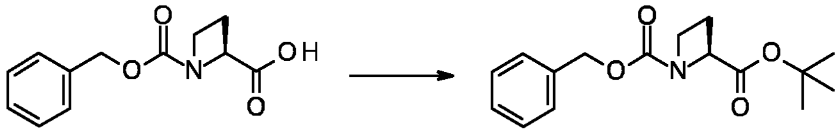

A30 B1

To a reaction vessel after nitrogen replacement, compound A30 (5.95 g), dichloromethane (12 mL), and cyclohexane (48 mL) were sequentially added at room temperature. After tert-butyl 2,2,2-trichloroacetimidate (9.06 mL) and boron trifluoride diethyl ether complex (320 μL) were added, the mixture was then stirred at room temperature for 1 hour. The reaction mixture was sampled and prepared as a sample (sample preparation method 1), and it was confirmed by HPLC analysis that the reaction conversion rate was 95.6% (calculation expression 1 of reaction conversion rate). The resulting slurry was filtered and the solid residue was washed with cyclohexane. The resulting filtrate was washed with 10% aqueous citric acid solution (40 mL × 9), 5% aqueous sodium carbonate solution (40 mL × 3), and 10% aqueous NaCl solution (40 mL). The resulting organic layer was dehydrated with sodium sulfate, and the resulting filtrate was filtered to remove sodium sulfate. The resultant was concentrated under reduced pressure at the external temperature of 40°C to obtain a residue (6.96 g) containing compound B1.

LC retention time of compound B1: 3.519 minutes (HPLC analysis conditions: method 1)
LCMS (ESI) retention time of compound B1: 3.620 minutes, m/z = 314.10 $[M+Na]^+$ (LCMS analysis conditions: method 1)

Example B-2

Synthesis of compound B2: tert-butyl (2S)-azetidine-2-carboxylate

[Formula 74]

B1 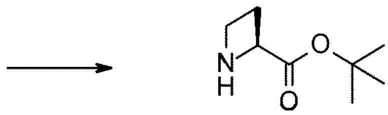

B2

The residue (6.00 g) containing compound B1 obtained in Example B-1 was added to a reaction vessel. Next, 2-MeTHF (62 mL) and 5% Pd/C (4.39 g, 50% aqueous content) were sequentially added at room temperature. The inside of the reaction vessel was replaced with hydrogen, and the mixture was stirred at room temperature for 2 hours. The inside of the reaction vessel was replaced again with hydrogen, and the mixture was stirred at room temperature for an additional 2 hours. The reaction mixture was sampled and prepared as a sample (sample preparation method 1), and it was confirmed by HPLC analysis that the reaction conversion rate was 99.9% or more (calculation expression 2 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF (30 mL × 2). The resulting solution was concentrated under reduced pressure at the external temperature of 40°C to obtain a residue (2.23 g) containing compound B2.

Example B-3

Synthesis of compound B3: tert-butyl (2S)-2-[methyl-[(2S)-2-[methyl-[(Z)-2-methylenepenta-3-enoxy]carbonyl-amino] propanoyl]amino]butanoate

[Formula 75]

B2 +

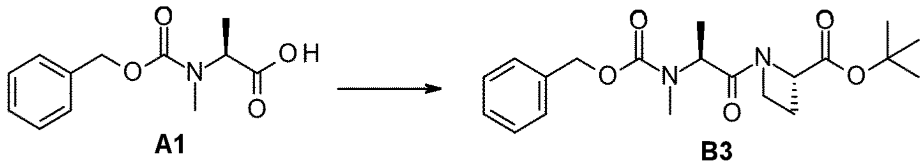

A1 B3

The residue (2.33 g) containing compound B2 obtained in Example B-2 and compound A1 (4.21 g) were added at room temperature to a reaction vessel, and the reaction vessel was subjected to nitrogen replacement. Next, 2-MeTHF (43 mL), DIPEA (11.2 mL), T3P (50 w/w% 2-MeTHF solution, 22.2 mL) were sequentially added, and then the mixture was stirred at room temperature for 2 hours. The reaction mixtures of 1 and 2 hours after the reaction were sampled and prepared as samples (sample preparation method 1), and it was confirmed by HPLC analysis that there was almost no difference in LC profiles. 5% aqueous sodium carbonate solution (50 mL) was added, and the mixture was stirred for 10 minutes. After the aqueous layer was discharged, the resulting organic layer was washed with 5% aqueous sodium carbonate solution (50 mL × 3), 5% aqueous sodium hydrogen sulfate monohydrate solution (50 mL × 2), and again 5% aqueous sodium carbonate solution (50 mL × 5). The resulting organic layer was dehydrated with sodium sulfate and filtered to remove sodium sulfate. The resulting filtrate was concentrated under reduced pressure at the external temperature of 40°C to obtain a residue (4.47 g) containing compound B3.

LC retention time of compound B3: 3.543 minutes (HPLC analysis conditions: method 1)
LCMS(ESI) of compound B3: retention time: 3.519 minutes, m/z = 377.23 $[M+H]^+$ (LCMS analysis conditions: method 1)

Example B-4

Synthesis of compound B4: tert-butyl (2S)-2-[methyl-[(2S)-2-(methylamino)propanoyl]amino]butanoate

[Formula 76]

B3

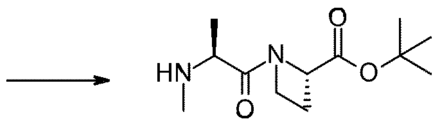

B4

The residue (3.98 g) containing compound B3 obtained in Example B-3 was added to a reaction vessel. Next, 2-MeTHF (32 mL) and 5% Pd/C (2.27 g, 50% aqueous content) were sequentially added at room temperature. The inside of the reaction vessel was replaced with hydrogen, and the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF (15 mL × 2). The resulting filtrate and the wash solution were combined and concentrated under reduced pressure at the external temperature of 40°C to obtain a residue (2.71 g) containing compound B4.
LC retention time of compound B4: 1.670 minutes (HPLC analysis conditions: method 1)

Example B-5

Synthesis of compound B5: tert-butyl (2S)-1-[(2S)-2-[[(2S,3S)-2-(benzyloxycarbonylamino)-3-methyl-pentanoyl]-methyl-amino]propanoyl]azetidine-2-carboxylate

[Formula 77]

B4 +

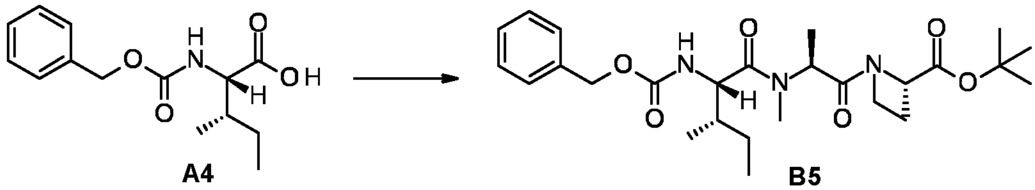

A4

B5

The residue (2.71 g) containing compound B4 obtained in Example B-4 and compound A4 (3.54 g) were added to a reaction vessel, and the reaction vessel was subjected to nitrogen replacement. Next, 2-MeTHF (17 mL), toluene (18 mL), MeCN (2.9 mL), and DIPEA (11.7 mL) were sequentially added at room temperature. HATU (6.35 g) was added with stirring, and then the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). N-methylimidazole (1.0 mL) was added to the reaction vessel, and 5% aqueous sodium carbonate solution (40 mL) was further added with stirring, and then the mixture was stirred for 1 hour. Next, 2.5% aqueous ammonia solution (40 mL) was added, and the mixture was stirred for 15 minutes. After the aqueous layer was discharged, the resulting organic layer was washed with 2.5% aqueous ammonia solution (60 mL), 5% aqueous sodium hydrogen sulfate monohydrate solution (60 mL × 2), and 3% aqueous dipotassium hydrogen phosphate solution (60 mL). The resulting organic layer was dehydrated with sodium sulfate and filtered to remove sodium sulfate. The obtained filtrate was concentrated under reduced pressure at the external temperature of 40°C to obtain a residue (5.62 g) containing compound B5.

LC retention time of compound B5: 3.893 minutes (HPLC analysis conditions: method 1)
LCMS (ESI) retention time of compound B5: 3.477 minutes, m/z = 490.31 $[M+H]^+$ (LCMS analysis conditions: method 2, column of method 4 was used as LC column)

Example B-6

Synthesis of compound B6: tert-butyl (2S)-1-[(2S)-2-[methyl-[(2S,3S)-3-methyl-2-[[(2S)-4-methyl-2-[methyl(2-trimethylsilylethoxycarbonyl)amino]pentanoyl]amino]pentanoyl]amino]propanoyl]azetidine-2-carboxylate

[Formula 78]

B5 + A7

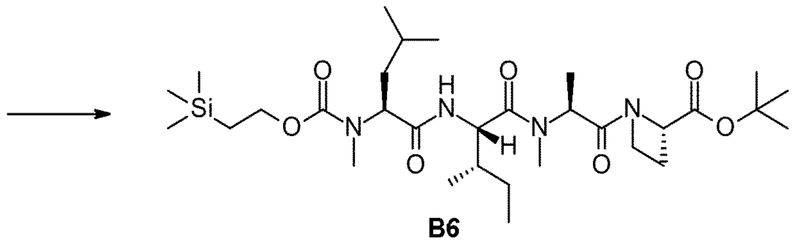

B6

The residue (4.60 g) containing compound B5 obtained in Example B-5 and isopropyl acetate (11 mL) were added to a reaction vessel. Next, the residue (5.13 g) containing compound A7 obtained in Example A-4, toluene (3.0 mL), acetone (31 mL), NMM (6.20 mL), and 5% Pd/C (2.00 g, 50% aqueous content) were sequentially added at room temperature. The inside of the reaction vessel was replaced with hydrogen, and the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, and then Pd/C was filtered under

reduced pressure using filter paper and a filter. The reaction vessel, and the filter paper and the filter were washed with 2-MeTHF (50 mL). The resulting solution was concentrated under reduced pressure at the external temperature of 40°C. The resulting residue was dissolved in MeTHF (40 mL) and 5% potassium carbonate (40 mL) and DMAP (1.15 g) were added with stirring, and then the mixture was stirred for 2.5 hours. Next, 5% aqueous potassium hydrogen sulfate solution (40 mL) was added, and the mixture was stirred for 30 minutes. After the aqueous layer was discharged, the resulting organic layer was washed with 5% aqueous potassium hydrogen sulfate solution (40 mL) and 5% aqueous potassium carbonate solution (40 mL × 2). The resulting organic layer was dehydrated with sodium sulfate and filtered to remove sodium sulfate. The resulting filtrate was concentrated under reduced pressure at the external temperature of 40°C to obtain a residue (5.05 g) containing compound B6.

LC retention time of compound B6: 4.937 minutes (HPLC analysis conditions: method 1)
LCMS (ESI) retention time of compound B6: 4.739 minutes, m/z = 627.37 $[M+H]^+$ (LCMS analysis conditions: method 1)

Example B-7

Synthesis of compound B7: tert-butyl (2S)-1-[(2S)-2-[methyl-[(2S,3S)-3-methyl-2-[[(2S)-4-methyl-2-(methylamino)pentanoyl]amino]pentanoyl]amino]propanoyl]azetidine-2-carboxylate

[Formula 79]

B6

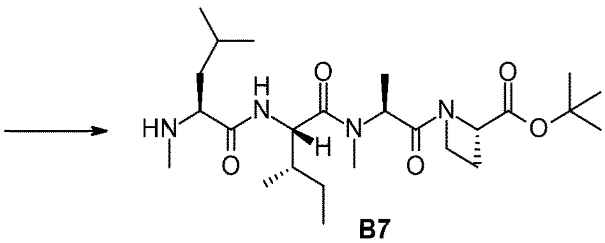

B7

The residue (4.05 g) containing compound B6 obtained in Example B-6, and 2-MeTHF (8.0 mL) were added at room temperature to a reaction vessel, and the reaction vessel was subjected to nitrogen replacement. Next, the external temperature of the reaction vessel was set to 50°C, and 1 M TBAF (16.1 mL) was added with stirring, and then the mixture was stirred at the external temperature of 50°C for 2 hours. The reaction mixture was sampled and prepared as a sample (sample preparation method 1), and it was confirmed by HPLC analysis that the reaction conversion rate was 99.9% or more (calculation expression 1 of reaction conversion rate). The external temperature setting was set to 0°C, and IPAc (20 mL) and 5% potassium carbonate (20 mL) were added with stirring, and then stirred at room temperature for 15 minutes. After the aqueous layer was discharged, the resulting organic layer was washed with 5% potassium carbonate (20 mL × 2). The resulting organic layer was dehydrated with sodium sulfate and filtered to remove sodium sulfate. The resulting filtrate was concentrated under reduced pressure at the external temperature of 40°C to obtain a residue (2.70 g) containing compound B7.

LC retention time of compound B7: 2.747 minutes (HPLC analysis conditions: method 1)
LCMS (ESI) retention time of compound B7: 2.488 minutes, m/z = 483.35 $[M+H]^+$ (LCMS analysis conditions: method 1)

Example B-8

Synthesis of compound B8: tert-butyl (2S)-1-[(2S)-2-[[(2S,3S)-2-[[(2S)-2-[[(3S)-3-[[(2S)-2-[[1-[[(2S)-1-[(2S)-2-(benzyloxycarbonylamino)-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methylpentanoyl]amino]-3-methyl-pentanoyl]-methyl-amino]propanoyl]azetidine-2-carboxylate

[Formula 80]

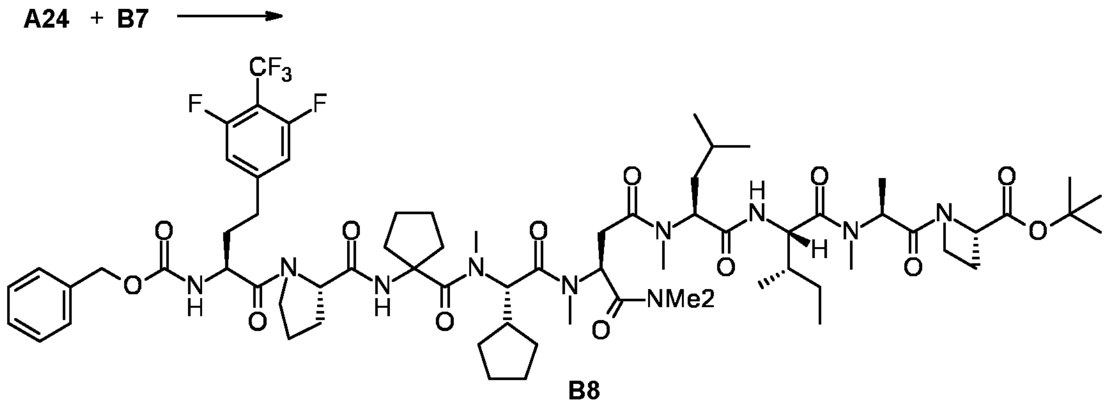


B8

The solution (4.00 g) containing compound A24 obtained in Example A-16 was added to a reaction vessel. The residue (0.962 g) containing compound B7 obtained in Example B-7, 2-MeTHF (36 mL), DMF (9.2 mL), and DIPEA (1.77 mL) were sequentially added at room temperature, and the reaction vessel was subjected to nitrogen replacement. Next, HATU (1.59 g) was added with stirring, and then the mixture was stirred at room temperature for 20 hours. Subsequently, the residue (0.154 g) containing compound B7 obtained in Example B-7 and HATU (1.20 g) were added, and the mixture was stirred for 3 hours. The reaction mixture was sampled and prepared as a sample (sample preparation method 1), and it was confirmed by HPLC analysis that the reaction conversion rate was 99.7% (calculation expression 1 of reaction conversion rate). To the reaction vessel, 2.5% aqueous ammonia solution (32 mL) was added, and the mixture was stirred for 30 minutes. After the aqueous layer was discharged, the resulting organic layer was washed with 10% aqueous sodium hydrogen sulfate monohydrate solution (28 mL × 3) and 5% aqueous potassium carbonate solution (28 mL × 2). The resulting organic layer was dehydrated with sodium sulfate and filtered to remove sodium sulfate. The resulting filtrate was concentrated under reduced pressure at the external temperature of 40°C, and the resulting residue was purified by silica gel column chromatography (eluent: ethyl acetate/heptane 80 : 20 to 100 : 0). The eluate containing the target material was concentrated under reduced pressure at the external temperature of 40°C to obtain a residue (1.75 g) containing compound B8.

LC retention time of compound B8: 20.037 minutes (HPLC analysis conditions: method 3)
LCMS (ESI) retention time of compound B8: 20.69 minutes, m/z = 1408.072 $[M+Na]^+$ (LCMS analysis conditions: method 3)

Example B-9

Synthesis of compound B9: 2-[[(2S)-2-[benzyloxycarbonyl(ethyl)amino]-3-(p-tolyl)propanoyl]-methyl-amino]acetic acid

[Formula 81]

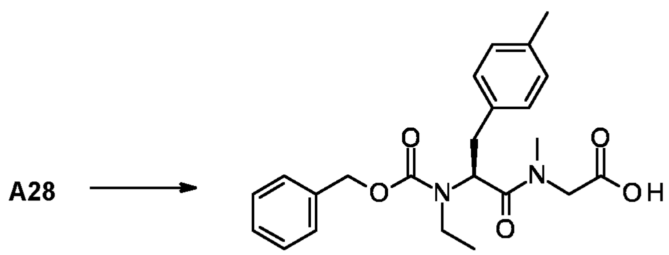


B9

The solution (5.01 g) containing compound A28 obtained in Example A-18 was purified by silica gel column chromatography (eluent: heptane/ethyl acetate 100 : 0 to 80 : 20). The resultant was concentrated under reduced pressure at the external temperature of 40°C, and the eluent was removed to obtain a residue (2.71 g) containing compound A28.

The residue (2.51 g) containing compound A28 was added to a reaction vessel. 2-MeTHF (28 mL) and HMDS (7.87 mL) were sequentially added at room temperature, and the reaction vessel was subjected to nitrogen replacement. Next, the external temperature of the reaction vessel was set to 0°C, and TMSOTf (5.82 mL) was added with stirring, and then the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). The external temperature was set to 0°C, and 5% aqueous sodium hydrogen sulfate monohydrate solution (20 mL) was added to the reaction vessel, and the mixture was stirred at room temperature for 20 minutes. After the aqueous layer was discharged, the resulting organic layer was washed with 5% aqueous sodium hydrogen sulfate monohydrate solution (20 mL × 3). Next, 5% aqueous dipotassium hydrogen phosphate solution (20 mL × 2) was added to the resulting organic layer to phase transfer the product to the aqueous layer. After the organic layer was discharged, isopropyl acetate (50 mL) and 5% aqueous sodium hydrogen sulfate monohydrate solution were added to the resulting aqueous layer until pH reached 1-3 to phase transfer the product to the organic layer. The extraction with isopropyl acetate (50 mL) was repeated twice, and all the resulting organic layers were dehydrated with sodium sulfate and filtered to remove sodium sulfate. The resulting filtrate was concentrated under reduced pressure at the external temperature of 40°C to obtain a residue (1.70 g) containing compound B9.
LCMS (ESI) retention time of compound B9: 3.347 minutes, m/z = 413.29 $[M+H]^+$ (LCMS analysis conditions: method 2, column of method 4 was used as LC column)

Example B-10

Synthesis of compound B10: tert-butyl (2S)-1-[(2S)-2-[[(2S,3S)-2-[[(2S)-2-[[(3S)-3-[[(2S)-2-[[1-[[(2S)-1-[(2S)-2-amino-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carboxylate

[Formula 82]

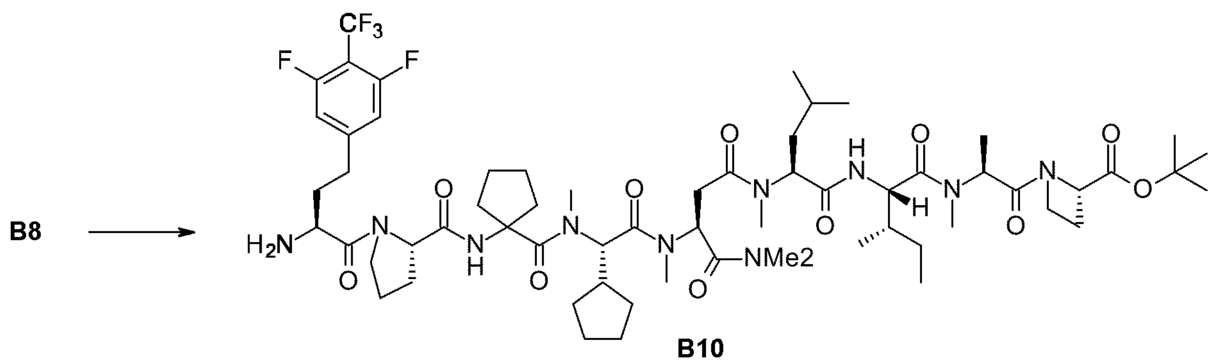

B10

The residue (1.46 g) containing compound B8 obtained in Example B-8 was added to a reaction vessel. Next, THF (15 mL) and 5% Pd/C (235 mg, 50% aqueous content) were sequentially added at room temperature. The inside of the reaction vessel was replaced with hydrogen, and the mixture was stirred at room temperature. The inside of the reaction vessel was replaced again with hydrogen after 2 and 4 hours, 5% Pd/C (223 mg, 50% aqueous content) was added after 5 hours, and the mixture was stirred for an additional 6 hours. The reaction mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with THF (7.0 mL × 2). The resulting solution was concentrated under reduced pressure at the external temperature of 40°C to obtain a residue (1.38 g) containing compound B10.
LC retention time of compound B10: 4.054 minutes (HPLC analysis conditions: method 2)

Example B-11

Synthesis of compound B11: tert-butyl (2S)-1-[(2S)-2-[[(2S,3S)-2-[[(2S)-2-[[(3S)-3-[[(2S)-2-[[1-[[(2S)-1-[(2S)-2-[[2-[[(2S)-2-[benzyloxycarbonylfethyl)amino]-3-(p-tolyl)propanoyl]-methyl-amino]acetyl]amino]-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carboxylate

[Formula 83]

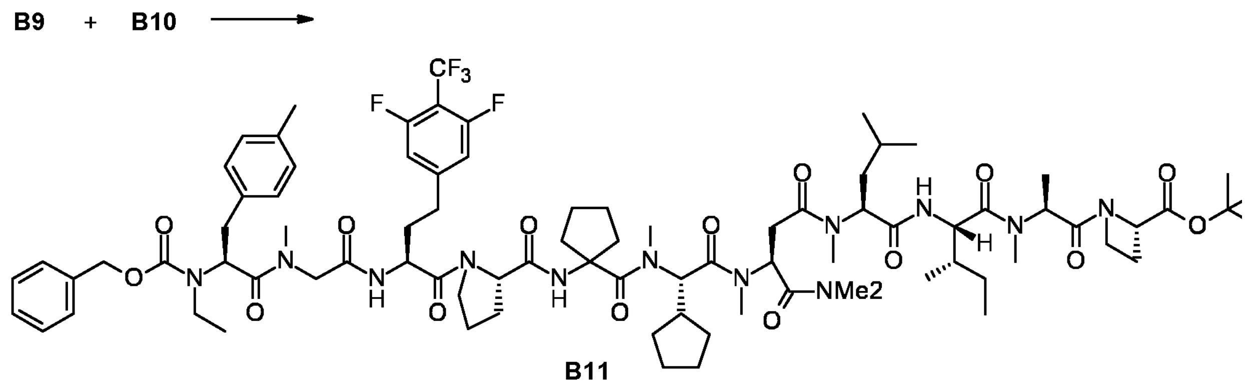

B11

The residue (1.38 g) containing compound B10 obtained in Example B-10 and the residue (0.581 g) containing compound B9 obtained in Example B-9 were added to a reaction vessel. 2-MeTHF (9.6 mL), DMF (3.2 mL), and DIPEA (1.08 mL) were sequentially added at room temperature, and the reaction vessel was subjected to nitrogen replacement. Next, HATU (0.971 g) was added with stirring, and then the mixture was stirred at room temperature for 3 hours. The reaction mixture was sampled and prepared as a sample (sample preparation method 1), and it was confirmed by HPLC analysis that the reaction conversion rate was 99.3% (calculation expression 1 of reaction conversion rate). To the reaction vessel, 2.5% aqueous ammonia solution (10 mL) was added, and the mixture was stirred for 15 minutes. After the aqueous layer was discharged, the resulting organic layer was washed with 10% aqueous sodium hydrogen sulfate monohydrate solution (10 mL × 3) and 5% aqueous potassium carbonate solution (10 mL × 2). The resulting organic layer was dehydrated with sodium sulfate and filtered to remove sodium sulfate. The resulting filtrate was concentrated under reduced pressure at the external temperature of 40°C, and the resulting residue was purified by silica gel column chromatography (eluent: ethyl acetate/methanol 100 : 0 to 90 : 10). The eluate containing the target material was concentrated under reduced pressure at the external temperature of 40°C to obtain a residue (1.32 g) containing compound B11.

LC retention time of compound B 11: 21.617 minutes (HPLC analysis conditions: method 3)
LCMS (ESI) retention time of compound B11: 22.21 minutes, m/z = 1668.111 $[M+Na]^+$ (LCMS analysis conditions: method 3)

Example B-12

Synthesis of compound B12: (2S)-1-[(2S)-2-[[(2S,3S)-2-[[(2S)-2-[[(3S)-3-[[(2S)-2-[[1-[[(2S)-1-[(2S)-2-[[2-[[(2S)-2-[benzyloxycarbonyl(ethyl)amino]-3-(p-tolyl)propanoyl]-methyl-amino]acetyl]amino]-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carboxylic acid

[Formula 84]

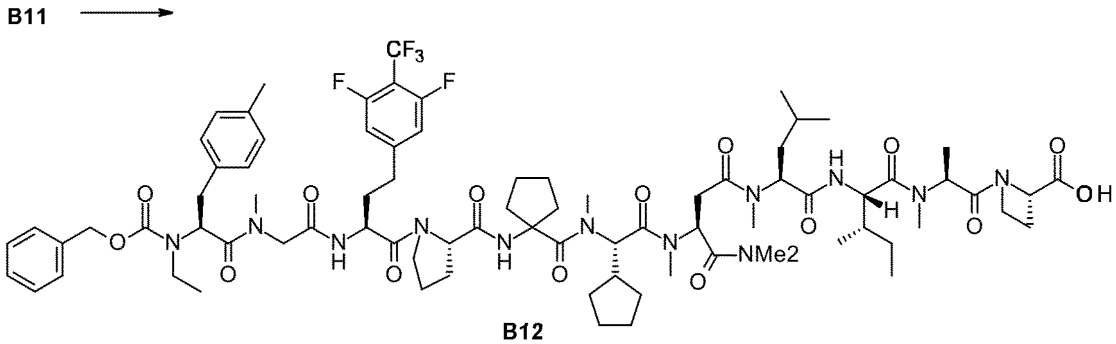

B12

The residue (1.08 g) containing compound B11 obtained in Example B-11 was added to a reaction vessel. 2-MeTHF (11 mL) and HMDS (0.688 mL) were sequentially added at room temperature, and the reaction vessel was subjected to nitrogen replacement. Next, the external temperature of the reaction vessel was set to 0°C, and TMSOTf (0.475 mL) was added with stirring, and then the mixture was stirred at room temperature for 3 hours. The reaction mixture was sampled and prepared as a sample (sample preparation method 1), and it was confirmed by HPLC analysis that the reaction conversion rate was 99.9% or more (calculation expression 1 of reaction conversion rate). The external temperature was set to 0°C, and 5% aqueous sodium hydrogen carbonate solution (15 mL) was added to the reaction vessel, and the mixture was stirred at room temperature for 30 minutes. The aqueous layer was discharged, and then the resulting organic layer was washed with 5% aqueous sodium dihydrogen phosphate solution (15 mL) and 10% aqueous NaCl solution (15 mL). The resulting organic layer was dehydrated with sodium sulfate and filtered to remove sodium sulfate. The resulting filtrate was concentrated under reduced pressure at the external temperature of 40°C, and the resulting residue was purified by silica gel column chromatography (eluent: dichloromethane/methanol 100 : 0 to 80 : 20). The eluate containing the target material was concentrated under reduced pressure at the external temperature of 40°C to obtain a residue (0.831 g) containing compound B12.
LC retention time of compound B12: 19.638 minutes (HPLC analysis conditions: method 3)

Example B-13

Synthesis of compound B13: (2S)-1-[(2S)-2-[[(2S,3S)-2-[[(2S)-2-[[(3S)-3-[[(2S)-2-cyclopentyl-2-[[1-[[(2S)-1-[(2S)-4-[3,5-difluoro-4-(trifluoromethyl)phenyl]-2-[[2-[(2S)-2-(ethylamino)-3-(p-tolyl)propanoyl]-methyl-amino]acetyl]amino]butanoyl]pyrrolidine-2-carbonyl]amino]cyclopentanecarbonyl]-methyl-amino]acetyl]-methyl-amino]-4-(dimethylamino)-4-oxo-butanoyl]-methyl-amino]-4-methyl-pentanoyl]amino]-3-methyl-pentanoyl]methyl-amino]propanoyl]azetidine-2-carboxylic acid

[Formula 85]

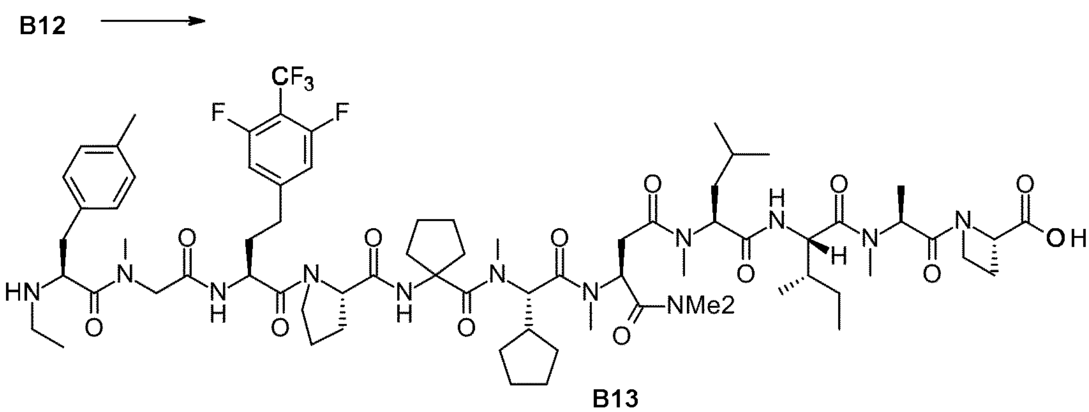

B13

The residue (0.831 g) containing compound B12 obtained in Example B-12 was added to a reaction vessel. Next, THF (5.7 mL) and 5% Pd/C (223 mg, 50% aqueous content) were sequentially added at room temperature. The inside of the reaction vessel was replaced with hydrogen, and the mixture was stirred at room temperature for 2 hours. The reaction

mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with THF (3.0 mL × 2). The resulting filtrate and the wash solution were combined and concentrated under reduced pressure at the external temperature of 40°C to obtain a residue (0.726 g) containing compound B13.

LC retention time of compound B13: 12.613 minutes (HPLC analysis conditions: method 3)
LCMS (ESI) retention time of compound B13: 13.10 minutes, m/z = 1456.124 $[M+H]^+$ (LCMS analysis conditions: method 3)

Example B-14

Synthesis of compound B1: (O1-benzyl O2-tert-butyl (2S)-azetidine-1,2-dicarboxylate)

[Formula 86]

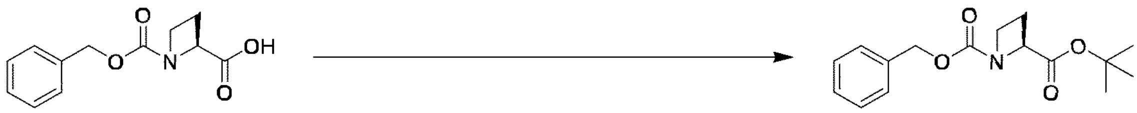

A30

B1

To a reaction vessel, compound A30 (6.02 g) and cyclohexane (48.0 mL), followed by dichloromethane (12.0 mL) were added at room temperature, and the mixture was stirred. After tert-butyl 2,2,2-trichloroacetimidate (9.14 mL) and boron trifluoride diethyl ether complex (0.32 mL) were added with stirring, the mixture was stirred at room temperature for 1 hour. The reaction mixture was sampled and prepared as a sample (sample preparation method 1), and it was confirmed by HPLC analysis that the reaction conversion rate was 99.0% or more (calculation expression 1 of reaction conversion rate). The reaction mixture was filtered using filter paper and a membrane filter. The resulting filtrate was washed with 10% aqueous citric acid solution (48.0 mL × 10) and 5% aqueous sodium carbonate solution (48.0 mL × 2). The resulting organic layer was concentrated under reduced pressure to obtain a residue (7.42 g) containing compound B1. The obtained residue was diluted with acetonitrile and subjected to LCMS analysis (method 1: retention time of compound B1; 3.567 minutes, m/z = 313.94 $[M+Na]^+$). The resulting residue and 3,5-bis(trifluoromethyl)benzoic acid were dissolved in DMSO-$d_6$ and subjected to qNMR analysis (yield: 96.3%).

Example B-15

Synthesis of compound B2: (tert-butyl (2S)-azetidine-2-carboxylate)

[Formula 87]

B1

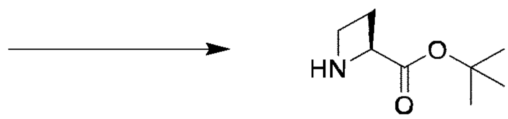

B2

To a reaction vessel, the residue (7.27 g, content: 96.2%) containing compound B1 obtained in Example B-14 and 2-MeTHF (72.5 mL), followed by 5% Pd/C (5.00 g, 50% aqueous content) were added at room temperature, and the mixture was stirred. Degassing with nitrogen gas was performed with stirring, then degassing with hydrogen gas was performed, and the mixture was stirred at room temperature for 3 hours. The reaction mixture was sampled and prepared as a sample (sample preparation method 1), and it was confirmed by HPLC analysis that the reaction conversion rate was 99.9% or more (calculation expression 2 of reaction conversion rate). The reaction mixture was suction-filtered using filter

paper and a membrane filter, and the residue was washed with 2-MeTHF (36.2 mL × 2). The resulting filtrate was concentrated under reduced pressure to obtain a residue (3.63 g) containing compound B2. The obtained residue and 1,3,5-trimethoxybenzene were dissolved in DMSO-$d_6$ and subjected to qNMR analysis (yield: 81.2%).

Example B-16

Synthesis of compound B3: (tert-butyl (2S)-1-[(2S)-2-[benzyloxycarbonyl(methyl)amino]propanoyl]azetidine-2-carboxylate)

[Formula 88]

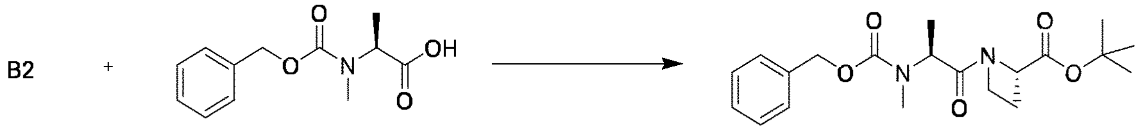

A1 B3

To a reaction vessel, the residue (3.39 g, content: 84.2%) containing compound B2 obtained in Example B-15 and compound A1 (3.91 g), followed by 2-MeTHF (49.9 mL) were added at room temperature, and the mixture was stirred. After NMM (4.89 mL) was added with stirring, T3P (1.6 M 2-MeTHF solution, 24.7 mL) was added dropwise, and the mixture was stirred at room temperature for 2 hours. The reaction mixture was sampled and prepared as a sample (sample preparation method 2), and it was confirmed by HPLC analysis that the reaction conversion rate was 99.9% or more (calculation expression 3 of reaction conversion rate). To the reaction mixture, 5% aqueous sodium carbonate solution (43.0 mL) was added with stirring. The mixture was stirred at room temperature for 10 minutes, and then the aqueous layer was discharged. The organic layer was washed with 5% aqueous sodium hydrogen sulfate monohydrate solution (43.0 mL × 2), and 5% aqueous sodium carbonate solution (43.0 mL × 1). The resulting organic layer was concentrated under reduced pressure to obtain a residue (6.40 g) containing compound B3. The obtained residue was diluted with acetonitrile and subjected to LCMS analysis (method 1: retention time of compound B3; 3.467 minutes, m/z = 377.11 $[M+H]^+$). The resulting residue and 3,5-bis(trifluoromethyl)benzoic acid were dissolved in DMSO-$d_6$ and subjected to qNMR analysis (yield: 96.5%). To the resulting residue, 2-MeTHF (12.0 mL) was added to obtain a solution (15.9 g, content: 37.0%) containing compound B3.

Example B-17

Synthesis of compound B4: (tert-butyl (2S)-1-[(2S)-2-(methylamino)propanoyl]azetidine-2-carboxylate)

[Formula 89]

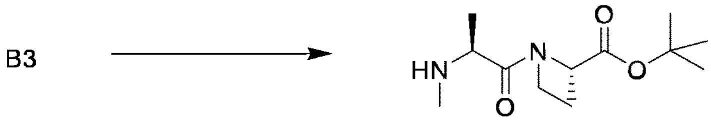

B4

To a reaction vessel, the solution (7.63 g, content: 37.0%) containing compound B3 obtained in Example B-16 and 2-MeTHF (17.1 mL), followed by 5% Pd/C (1.60 g, 50% aqueous content) were added at room temperature, and the mixture was stirred. Degassing with nitrogen gas was performed with stirring, then degassing with hydrogen gas was performed, and the mixture was stirred at room temperature for 3 hours. The reaction mixture was sampled and prepared as a sample (sample preparation method 1), and it was confirmed by HPLC analysis that the reaction conversion rate was 99.9% or more (calculation expression 1 of reaction conversion rate). The reaction mixture was suction-filtered using filter paper and a membrane filter, and the residue was washed with 2-MeTHF (11.3 mL × 2). The resulting filtrate was concentrated under reduced pressure to obtain a residue (1.97 g) containing compound B4. The obtained residue was diluted with acetonitrile and subjected to LCMS analysis (method 1: retention time of compound B4; 1.573 minutes, m/z = 243.09 $[M+H]^+$). The resulting residue and 1,3,5-trimethoxybenzene were dissolved in DMSO-$d_6$ and subjected to qNMR

analysis (yield: 99.4%).

Example B-18

Synthesis of compound B5: (tert-butyl (2S)-1-[(2S)-2-[[(2S,3S)-2-(benzyloxycarbonylamino)-3-methyl-pentanoyl]-methyl-amino]propanoyl]azetidine-2-carboxylate)

[Formula 90]

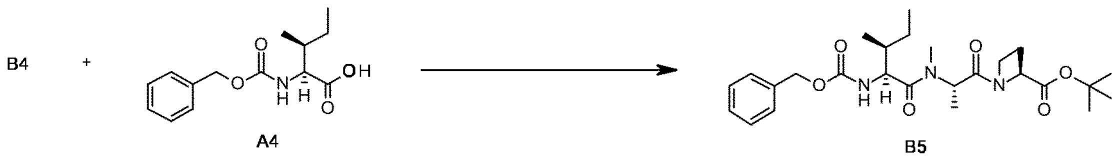

B4 + A4 B5

To a reaction vessel, the residue (1.86 g, content: 91.7%) containing compound B4 obtained in Example B-17, and compound A4 (2.25 g), followed by DIPEA (7.38 mL), 2-MeTHF (5.13 mL), toluene (5.13 mL), and acetonitrile (1.71 mL) were added at room temperature, and the mixture was stirred. HATU (4.03 g) was added with stirring, and the mixture was stirred at room temperature for 2 hours. The reaction mixture was sampled and prepared as a sample (sample preparation method 2), and it was confirmed by HPLC analysis that the reaction conversion rate was 99.9% or more (calculation expression 3 of reaction conversion rate). To the reaction mixture, N-methylimidazole (0.56 mL), then 5% aqueous sodium carbonate solution (10.3 mL) were added, and the mixture was stirred for 3 hours, then the aqueous layer was discharged. The organic layer was washed with 2.5% aqueous ammonia solution (13.7 mL × 2), 10% aqueous sodium hydrogen sulfate monohydrate solution (13.7 mL × 2), and 3% aqueous dipotassium hydrogen phosphate solution (13.7 mL×1). The resulting organic layer was concentrated under reduced pressure to obtain a residue (4.94 g) containing compound B5. The obtained residue was dissolved in 2-MeTHF (6.0 mL), toluene (6.0 mL), and acetonitrile (2.0 mL), and washed with 5% aqueous sodium chloride solution (12.0 mL). The resulting organic layer was concentrated under reduced pressure to obtain a residue (4.43 g) containing compound B5. The obtained residue was diluted with acetonitrile and subjected to LCMS analysis (method 4: retention time of compound B5; 3.636 minutes, m/z = 490.36 $[M+H]^+$). The resulting residue and 3,5-bis(trifluoromethyl)benzoic acid were dissolved in DMSO-$d_6$ and subjected to qNMR analysis (yield: 86.5%).

Example B-19

Synthesis of compound A7: ((2,3,4,5,6-pentafluorophenyl) (2S)-4-methyl-2-[methyl(2-trimethylsilylethoxycarbonyl) amino]pentanoate)

[Formula 91]

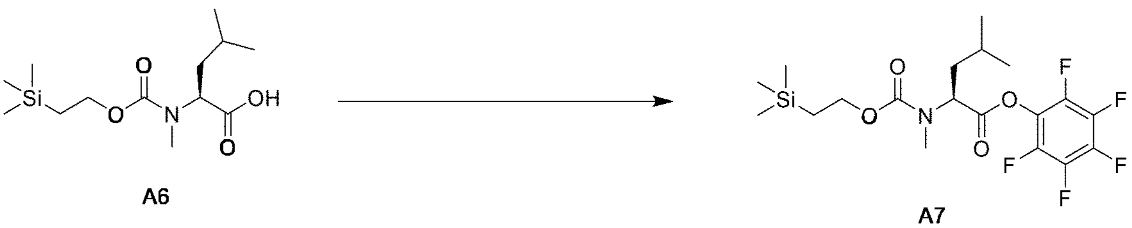

A6 A7

To a reaction vessel, compound A6 (10.0 g), isopropyl acetate (51.8 mL), and DMF (64.8 mL) were added at room temperature. A solution of pentafluorophenol (7.95 g) in isopropyl acetate (13.0 mL) was added. The reaction vessel was cooled in an ice bath and 1-(3-dimethylaminopropyl)-3-ethylcarbodiimide hydrochloride (8.28 g) was added, and the mixture was stirred for 30 minutes. Then, the reaction vessel was removed from the ice bath, and the mixture was stirred at room temperature for 2 hours. The reaction mixture was sampled and prepared as a sample (sample preparation method 1), and it was confirmed by HPLC analysis that the reaction conversion rate was 99.9% or more (calculation expression 2 of

reaction conversion rate). The reaction vessel was cooled in an ice bath and 0.5 M aqueous hydrochloric acid solution (64.8 mL) was added to the reaction mixture. The mixture was stirred for 10 minutes, and the aqueous layer was discharged. The organic layer was washed with 0.5 M aqueous hydrochloric acid solution (64.8 mL × 1), 5% aqueous potassium carbonate solution (64.8 mL × 2), and 10% aqueous sodium chloride solution (64.8 mL × 1). The resulting organic layer was concentrated under reduced pressure to obtain a residue (15.2 g) containing compound A7. The obtained residue was diluted with acetonitrile and subjected to LCMS analysis (method 4: retention time of compound A7; 5.365 minutes, m/z = 428.27 [M-$C_2H_4$ + H]$^+$). The resulting residue and 3,5-bis(trifluoromethyl)benzoic acid were dissolved in DMSO-$d_6$ and subjected to qNMR analysis (yield: 89.3%).

Example B-20

Synthesis of compound B6: (tert-butyl (2S)-1-[(2S)-2-[methyl-[(2S,3S)-3-methyl-2-[[(2S)-4-methyl-2-[methyl(2-trimethylsilylethoxycarbonyl)amino]pentanoyl]amino]pentanoyl]amino]propanoyl]azetidine-2-carboxylate)

[Formula 92]

B5

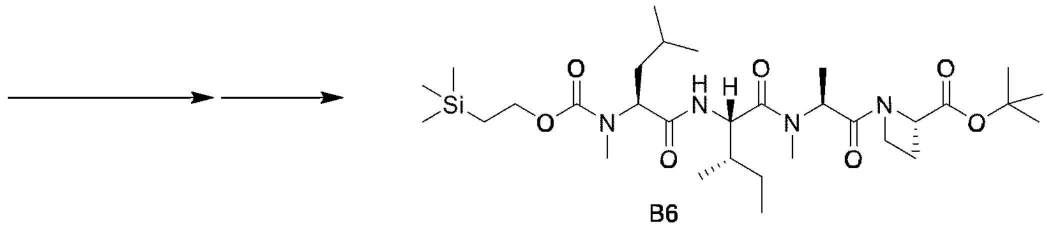

B6

To a reaction vessel, the residue (4.17 g, content: 67.5%) containing compound B5 obtained in Example B-18 and the residue (5.68 g, content: 92.5%) containing compound A7 obtained in Example B-19 followed by acetone (19.3 mL) and isopropyl acetate (6.76 mL), were added at room temperature, and the mixture was stirred. To the reaction mixture, NMM (3.82 mL) and 5% Pd/C (1.24 g, 50% aqueous content) were added with stirring. Degassing with nitrogen gas was performed with stirring, then degassing with hydrogen gas was performed, and the mixture was stirred at room temperature for 3 hours. The reaction mixture was sampled and prepared as a sample (sample preparation method 1), and it was confirmed by HPLC analysis that the reaction conversion rate was 99.9% or more (calculation expression 1 of reaction conversion rate). The reaction mixture was suction-filtered using filter paper and a membrane filter, and the residue was washed with acetone (9.64 mL × 2). The resulting filtrate was concentrated under reduced pressure, and the residue was dissolved in toluene (18.3 mL). 4-dimethylaminopyridine (0.708 g) and 5% aqueous potassium carbonate solution (18.3 mL) were added, and the mixture was stirred at room temperature for 2 hours. To the reaction mixture, 4-dimethylaminopyridine (0.351 g) was added, and the mixture was stirred for 2 hours. After standing still overnight, the reaction mixture was stirred at room temperature for 3 hours and the aqueous layer was discharged. The organic layer was washed with 5% aqueous potassium hydrogen sulfate solution (18.3 mL × 2) and 5% aqueous potassium carbonate solution (18.3 mL × 2). The resulting organic layer was concentrated under reduced pressure to obtain a residue (4.17 g) containing compound B6. The obtained residue was diluted with acetonitrile and subjected to LCMS analysis (method 1: retention time of compound B6; 4.793 minutes, m/z = 627.34 [M+H]$^+$). The resulting residue and 3,5-bis(trifluoromethyl)benzoic acid were dissolved in DMSO-$d_6$ and subjected to qNMR analysis (yield: 88.1%).

Example B-21

Synthesis of compound B7: (tert-butyl (2S)-1-[(2S)-2-[methyl-[(2S,3S)-3-methyl-2-[[(2S)-4-methyl-2-(methylamino)pentanoyl]amino]pentanoyl]amino]propanoyl]azetidine-2-carboxylate)

[Formula 93]

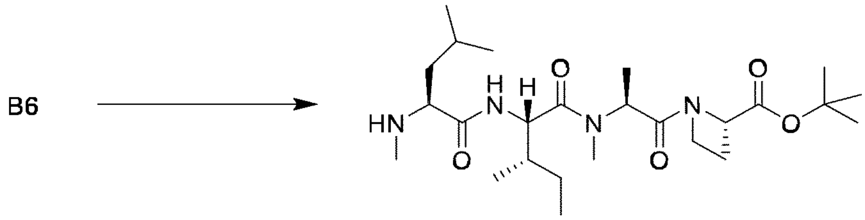

B7

To a reaction vessel, the residue (3.98 g, content: 76.5%) containing compound B6 and 2-MeTHF (9.06 mL) were added at room temperature. The reaction vessel was warmed up in an oil bath set at 47°C, and tetrabutylammonium fluoride (1 M THF solution, 12.1 mL) was added over 1 hour, and then the mixture was stirred for 2 hours. The reaction mixture was sampled and prepared as a sample (sample preparation method 1), and it was confirmed by HPLC analysis that the reaction conversion rate was 99.9% or more (calculation expression 1 of reaction conversion rate). The reaction vessel was removed from the oil bath, and isopropyl acetate (9.06 mL) and 5% aqueous potassium carbonate solution (9.06 mL) were added to the reaction mixture. The mixture was stirred at room temperature for 10 minutes, and the aqueous layer was discharged. The organic layer was washed with 5% aqueous potassium carbonate solution (9.06 mL × 2). The resulting organic layer was concentrated under reduced pressure to obtain a residue (3.08 g) containing compound B7. To the obtained residue, 2-MeTHF (9.1 mL) was added to obtain a solution (6.45 g, content: 31.2%) containing compound B7. The resulting solution was diluted with acetonitrile and subjected to LCMS analysis (method 1: retention time of compound B7; 2.552 minutes, m/z = 483.36 $[M+H]^+$). The resulting solution and 1,3,5-trimethoxybenzene were dissolved in DMSO-$d_6$ and subjected to qNMR analysis (yield: 85.4%).

Example B-22

Synthesis of compound B16: (tert-butyl (2S,3S)-2-[[(2S)-2-[benzyloxycarbonyl(methyl)amino]-4-methyl-pentanoyl]amino]-3-methyl-pentanoate)

[Formula 94]

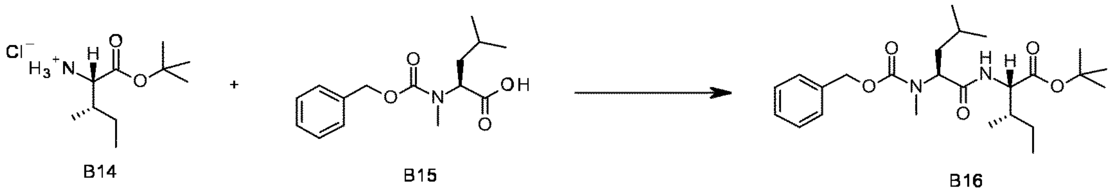

To a reaction vessel, compound N-((benzyloxy)carbonyl)-N-methyl-L-leucine B15 (4.51 g), and compound L-isoleucine tert-butyl hydrochloride B14 (4.34 g), then 2-MeTHF (48.7 mL) were added at room temperature, and the mixture was stirred. After DIPEA (15.2 mL) was added with stirring, T3P (1.6 M 2-MeTHF solution, 24.2 mL) was added dropwise while keeping the internal temperature to 25°C or lower, and the mixture was stirred at room temperature for 3 hours. The reaction mixture was sampled and prepared as a sample (sample preparation method 2), and it was confirmed by HPLC analysis that the reaction conversion rate was 99.7% or more (calculation expression 3 of reaction conversion rate). To the reaction mixture, 5% aqueous sodium carbonate solution (45.0 mL) was added with stirring while keeping the internal temperature to 25°C or lower. The mixture was stirred at room temperature for 10 minutes, and then the aqueous layer was discharged. The organic layer was washed with 5% aqueous sodium hydrogen sulfate monohydrate solution (45.0 mL × 2), and 5% aqueous sodium carbonate solution (45.0 mL × 1). The resulting organic layer was concentrated under reduced pressure to obtain a residue (7.74 g) containing compound B16. The obtained residue was diluted with acetonitrile and subjected to LCMS analysis (method 1: retention time of compound B16; 4.642 minutes, m/z = 449.24 $[M+H]^+$). The resulting residue and 3,5-bis(trifluoromethyl)benzoic acid were dissolved in DMSO-$d_6$ and subjected to qNMR analysis (yield: 99.9%).

Example B-23

Synthesis of compound B17: ((2S,3S)-2-[[(2S)-2-[benzyloxycarbonyl(methyl)amino]-4-methyl-pentanoyl]amino]-3-methyl-pentanoic acid)

[Formula 95]

B16

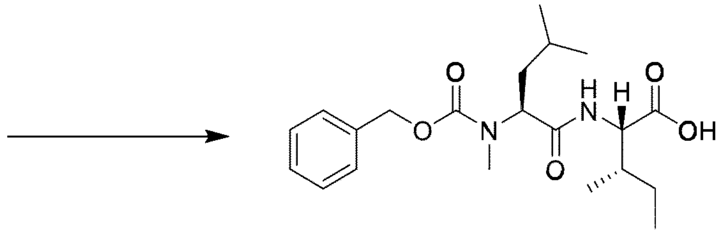

B17

To the residue (7.59 g, content: 93.3%) containing compound B16 obtained in Example B-22, isopropyl acetate (12.5 mL) was added to prepare a solution containing compound B16. To a reaction vessel, the solution (9.05 g) containing compound B16 prepared above and isopropyl acetate (28.6 mL) were added at room temperature, and then hexamethyldisilazane (4.14 mL) was added. The reaction vessel was cooled in an ice bath and trimethylsilyl trifluoromethanesulfonate (2.82 mL) was added with stirring. The reaction vessel was removed from the ice bath, and the mixture was stirred at room temperature for 4 hours. The reaction mixture was sampled and prepared as a sample (sample preparation method 1), and it was confirmed by HPLC analysis that the reaction conversion rate was 99.4% or more (calculation expression 1 of reaction conversion rate). The reaction vessel was cooled in an ice bath, and isopropyl acetate (9.5 mL) and 5% aqueous dipotassium hydrogen phosphate solution (24.5 mL) were added, and the resultant was mixed in a separatory funnel. To the separatory funnel, 0.5 M aqueous hydrochloric acid solution (20.0 mL) was added to wash the organic layer, and the aqueous layer was discharged. The organic layer was washed with 5% sodium chloride solution (35.0 mL). The resulting organic layer was concentrated under reduced pressure to obtain a residue (3.89 g) containing compound B17. The obtained residue was diluted with acetonitrile and subjected to LCMS analysis (method 1: retention time of compound B17; 3.635 minutes, m/z = 393.15 $[M+H]^+$). The resulting residue and 3,5-bis(trifluoromethyl)benzoic acid were dissolved in DMSO-$d_6$ and subjected to qNMR analysis (yield: 92.8%). To the resulting residue, 2-MeTHF (5.0 mL) was added to obtain a solution (8.06 g, content: 34.2%) containing compound B17.

Example B-24

Synthesis of compound B4: (tert-butyl (2S)-1-[(2S)-2-(methylamino)propanoyl]azetidine-2-carboxylate)

[Formula 96]

B3

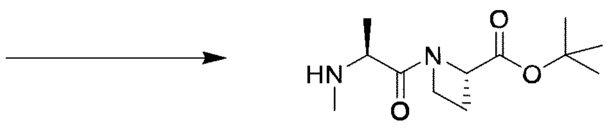

B4

To a reaction vessel, the solution (8.12 g) containing compound B3 obtained in Example B-16 and 2-MeTHF (18.1 mL) were added at room temperature, and then 5% Pd/C (1.70 g, 50% aqueous content) was added. Degassing with nitrogen gas was performed with stirring, then degassing with hydrogen gas was performed, and the mixture was stirred at room temperature for 2.5 hours. The reaction mixture was sampled and prepared as a sample (sample preparation method 1), and it was confirmed by HPLC analysis that the reaction conversion rate was 99.9% or more (calculation expression 1 of reaction conversion rate). The reaction mixture was suction-filtered using filter paper and a membrane filter, and the residue was washed with 2-MeTHF (12.1 mL × 2). The resulting filtrate was concentrated under reduced pressure to obtain a residue (2.13 g) containing compound B4. The obtained residue was diluted with acetonitrile and subjected to LCMS analysis (method 2: retention time of compound B4; 1.573 minutes, m/z = 243.09 $[M+H]^+$). The resulting residue and 1,3,5-trimethoxybenzene were dissolved in DMSO-$d_6$ and subjected to qNMR analysis (yield: 98.3%). To the resulting

residue, 2-MeTHF (3.5 mL) was added to obtain a solution (4.340 g, content: 40.8%) containing compound B4.

Example B-25

Synthesis of compound B18: (tert-butyl 2-[[(2S)-2-[[(2S,3S)-2-[[(2S)-2-[benzyloxycarbonyl(methyl)amino]-4-methyl-pentanoyl]amino]-3-methyl-pentanoyl]-methyl-amino]propanoyl]amino]acetate)

[Formula 97]

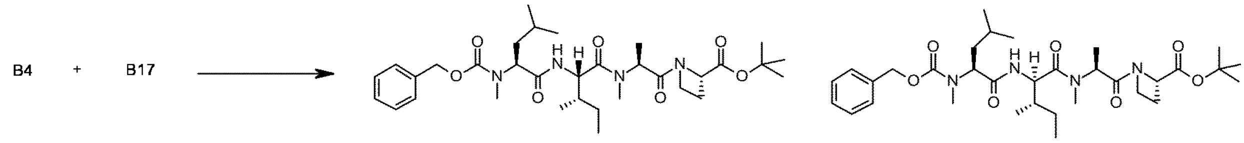


B18

BP1

To a reaction vessel, a solution (235 mg, content: 34.2%) containing compound B17, a solution (111 mg content: 40.8%) containing compound B4, DIPEA (67.9 μL) and acetonitrile (45 μL) were added. To the reaction vessel, HATU (78.6 mg) was added, and the mixture was stirred at room temperature for 7 hours. The reaction mixture was sampled and prepared as a sample (sample preparation method 2), and it was confirmed by HPLC analysis that the reaction conversion rate was 97.3% or more (calculation expression 4 of reaction conversion rate) (method 1: retention time of compound B18; 4.374 minutes, m/z = 639.37 $[M+Na]^+$). The ratio of compound B18 to BP1 was 40.2 : 59.8.

calculation expression 4 of reaction conversion rate: reaction conversion rate (%) = (area value of compound B18 + area value of compound BP1)/(area value of compound B4 + area value of compound B18 + area value of compound B18) × 100

Example B-26

Synthesis of compound B20: (2S)-1-[(2Sterttert-butyl ((S2SS)-2-(benzyloxycarbonylamino-(((benzyloxy)carbonyl)amino)-4-[-(-[-(3,5-difluoro-4-(trifluoromethyl)phenyl])])butanoyl]pyrrolidine-2-carboxylate tert-butyl)-L-prolinate

[Formula 98]

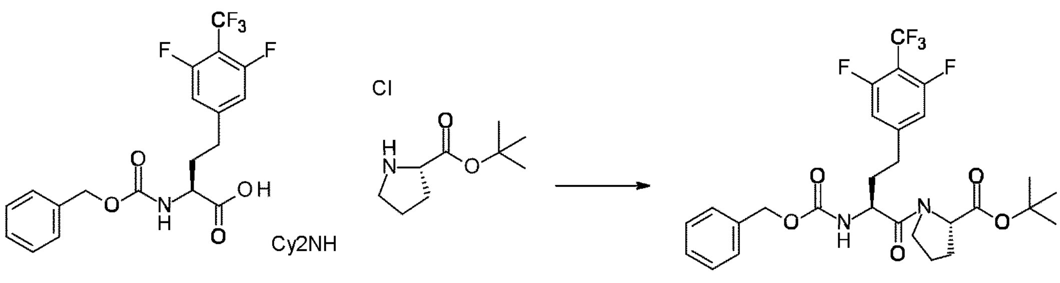

A22

B19

B20

Compound A22 (14.26 g (23.8 mmol)) and compound B19 (6.305 g (30.3 mmol)) were weighed into a reaction vessel and dissolved in 2-MeTHF (71.0 mL). DIPEA (22.5 mL) and 2-MeTHF (30 mL) were added with stirring at room temperature. The external temperature was cooled to 0°C, and T3P (1.6M in 2-MeTHF, 35.7 mL) was added. The external temperature was raised to room temperature, and the mixture was stirred for 2 hours. The reaction mixture was sampled and prepared as a sample (sample preparation method 2), and it was confirmed by HPLC analysis that the reaction conversion rate was 99.9% or more (calculation expression 3 of reaction conversion rate). The external temperature was cooled to 0°C, and 5% aqueous sodium carbonate solution (71.2 mL) was added to the reaction solution, and the mixture was stirred at room temperature for 10 minutes. The aqueous layer was discharged, and the organic layer was washed with 5% aqueous sodium hydrogen sulfate solution (71.2 mL × 2) and 5% aqueous sodium carbonate solution (71.2 mL). The resulting organic layer was concentrated under reduced pressure at the external temperature of 30°C, and 2-MeTHF (20.4

mL) was added to obtain a solution containing compound B20 (35.21 g, yield 82.2%).

LCMS (ESI): retention time: 4.244 minutes, m/z = 593.40 $[M+Na]^+$ (LCMS analysis conditions: method 4)
Yield: 82.2% (The obtained residue and 3,5-bis(trifluoromethyl)benzoic acid were dissolved in DMSO-$d_6$ and subjected to qNMR analysis.)

Example B-27

Synthesis of compound B21: (2S)-1-[(2Sterttert-butyl ((S2SS)-2-amino-4-[-(-[-(3,5-difluoro-4-(trifluoromethyl)phenyl])]) butanoyl]pyrrolidine-2-carboxylate tert-butyl)-L-prolinate

[Formula 99]

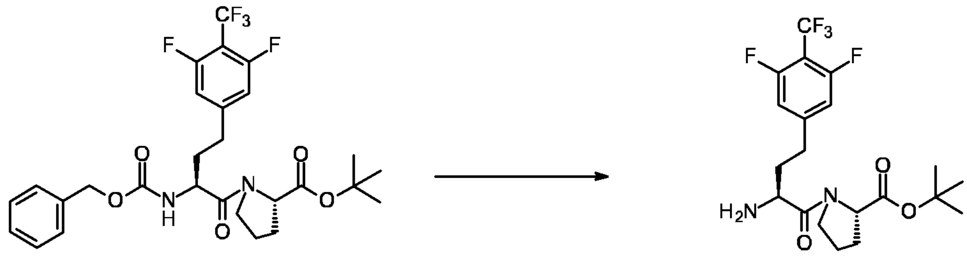

B20 B21

To a reaction vessel, the storage solution (14.96 g, 31.7wt%) containing compound B20 obtained in Example B-26 and 2-MeTHF (19.2 mL) were sequentially added at room temperature. To the reaction vessel, 5% Pd/C (1.752 g, 50% aqueous content) was added and then degassed 3 times with hydrogen gas, and the mixture was stirred for 3 hours. The reaction mixture was sampled and prepared as a sample (sample preparation method 1), and it was confirmed by HPLC analysis that the reaction conversion rate was 99.8% or more (calculation expression 1 of reaction conversion rate). The reaction mixture was filtered using filter paper and a membrane filter, and the residue was washed with 2-MeTHF (5.0 mL × 2). The resulting filtrate was concentrated under reduced pressure to obtain a residue containing compound B21 (6.02 g, yield 89.5%).

LCMS (ESI): retention time: 2.958 minutes, m/z = 437.28 $[M+H]^+$ (LCMS analysis conditions: method 4)
Yield: 89.5% (The obtained residue and 3,5-bis(trifluoromethyl)benzoic acid were dissolved in DMSO-$d_6$ and subjected to qNMR analysis.)

Example B-28

Synthesis of compound A28: (tert-butyl 2-[[(2S)-2-[benzyloxycarbonyl(ethyl)amino]-3-(p-tolyl)propanoyl]-methyl-amino]acetate)

[Formula 100]

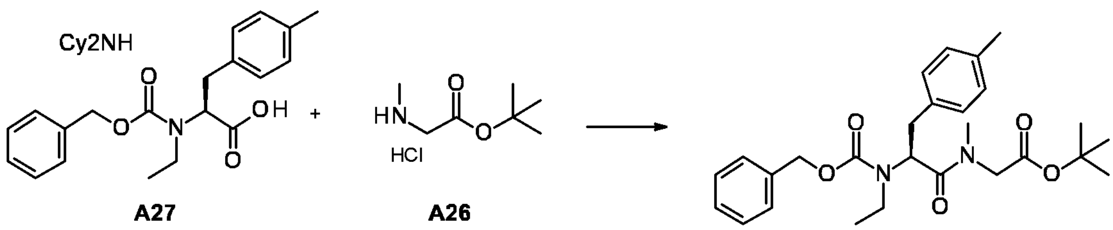


A27 A26 A28

To a reaction vessel after nitrogen replacement, compound A27 (72.0 kg) and compound A26 (30.0 kg) were

added at room temperature, and then 2-MeTHF (360 kg) was added, and the mixture was stirred. The external temperature of the reaction vessel was set to 10°C and DIPEA (96.2 kg) was added, and then T3P (50 w/w% 2-MeTHF solution, 212 kg) was added dropwise. The external temperature of the reaction vessel was set to 25°C, and the mixture was stirred for 3 hours. The reaction mixture was sampled and prepared as a sample (sample preparation method 2), and it was confirmed by HPLC analysis that the reaction conversion rate was 99.2% (calculation expression 3 of reaction conversion rate). The external temperature of the reaction vessel was set to 10°C, and 5% aqueous sodium carbonate solution (430 kg) was added dropwise, and then water (110 kg) was added. The external temperature of the reaction vessel was set to 25°C, and the mixture was stirred for 20 minutes, and then the aqueous layer was discharged from the reaction vessel. The resulting organic layer was washed also at the external temperature of 25°C with 5% aqueous sodium hydrogen sulfate monohydrate solution 3 times (420 kg, 420 kg, 420 kg, respectively), and 5% aqueous sodium carbonate solution (420 kg). The resulting organic layer was concentrated under reduced pressure with setting the external temperature to 55°C until the liquid volume reached about 400 L. The resulting concentrated solution and the wash solution obtained by washing the reaction vessel with 2-MeTHF (86 kg) were combined and transferred to another reactor. Then, the solution was concentrated under reduced pressure with setting the external temperature to 55°C until the liquid volume reached about 200 L to obtain a 2-MeTHF solution containing compound A28.

Example B-29

Synthesis of compound B9: (S)-N-(2-[[(2S)-2-[benzyloxycarbonyl(-(((-(((benzyloxy)carbonyl) (()(ethyl)amino]-)-]-)-3-(p-tolyl)propanoyl]-methyl-amino]acetic acid)-N-methylglycine

[Formula 101]

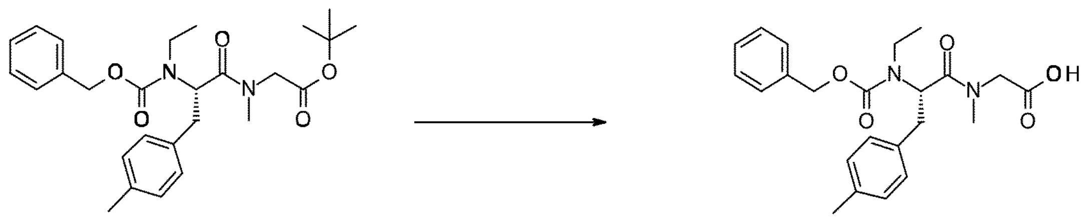

A28 B9

To a reaction vessel, compound A28 (29.92 g, 23.2wt %) was added and concentrated under reduced pressure. To the resultant residue, IPAc (69.4 mL) was added. HMDS (7.86 mL) was added while stirring the mixture at room temperature, and then the external temperature was cooled to 0°C, and TMSOTf (5.35 mL) was slowly added dropwise. The external temperature was raised to room temperature, and the mixture was stirred for 3 hours. The reaction mixture was sampled and prepared as a sample (sample preparation method 1), and it was confirmed by HPLC analysis that the reaction conversion rate was 99.9% or more (calculation expression 1 of reaction conversion rate). The external temperature was cooled to 0°C, and 5% aqueous dipotassium hydrogen phosphate solution (48.6 mL) and 0.5 M aqueous hydrochloric acid solution (35.0 mL) were added dropwise sequentially to the reaction solution, and the mixture was stirred at room temperature for 10 minutes. The aqueous layer was discharged, and the organic layer was washed with 5% sodium chloride solution (69.4 mL). The resulting organic layer was concentrated under reduced pressure at the external temperature of 30°C to obtain a solution containing compound B9 (17.78 g, yield 94.8%).

LCMS (ESI): retention time: 3.220 minutes, m/z = 413.35 $[M+H]^+$ (LCMS analysis conditions: method 4)
Yield: 94.8% (The obtained residue and 3,5-bis(trifluoromethyl)benzoic acid were dissolved in DMSO-$d_6$ and subjected to qNMR analysis.)

Example B-30

Synthesis of compound B22: acetic acid N-[(1S)-2-[[2-[[(1S)-3-[3,5-difluoro-4-(trifluoromethyl)phenyl]-1-(pyrrolidine-1-carbonyl) propyl]amino]-2-oxo-ethyl]-methyl-amino]-2-oxo-1-(p-tolylmethyl)ethyl]-N-ethyl-carbamate; tert-butylbenzyl

[Formula 102]

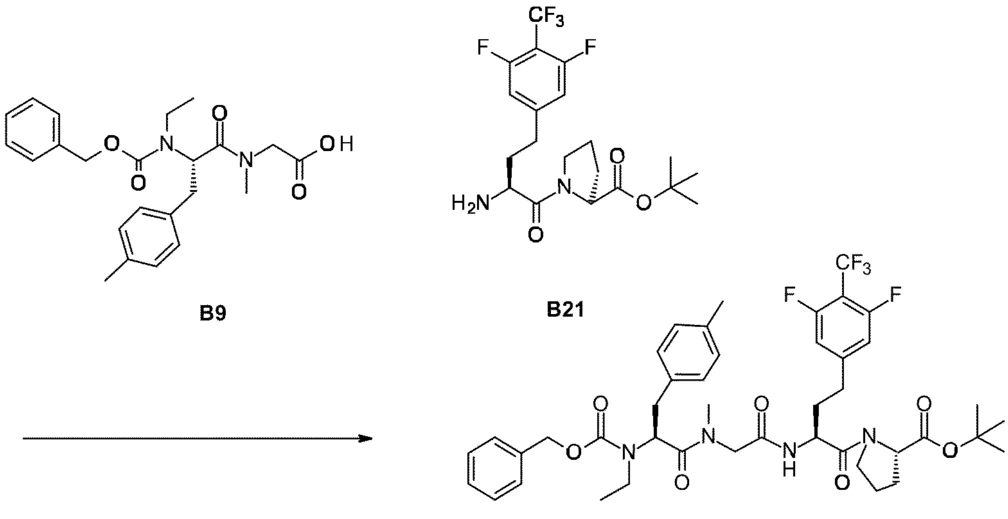

B9 B21

B22

Compound B9 (11.12 g, 33.1 wt%) and compound B21(6.80 g, 55.7 wt%) were weighed into a reaction vessel, and 2-MeTHF (18.3 mL) was added. DIPEA (5.86 mL) was added with stirring at room temperature. The external temperature was cooled to 0°C, and HATU (5.920 g) was added. The external temperature was raised to room temperature, and the mixture was stirred for 2 hours. The reaction mixture was sampled and prepared as a sample (sample preparation method 2), and it was confirmed by HPLC analysis that the reaction conversion rate was 99.9% or more (calculation expression 3 of reaction conversion rate). The external temperature was cooled to 0°C, and N-methylimidazole (0.67 mL) and 5% aqueous sodium carbonate solution (22.0 mL) were added to the reaction solution, and the mixture was stirred at room temperature for 10 minutes. After washing with 2-MeTHF (10.0 mL), the aqueous layer was discharged, and the organic layer was washed with 2.5% aqueous ammonia solution (22.0 mL), 5% sodium hydrogen sulfate solution (22.0 mL × 2), and 5% aqueous sodium carbonate solution (22.0 mL × 2). The resulting organic layer was concentrated and dried under reduced pressure at the external temperature of 30°C, and purified by silica gel chromatography (eluent: methanol/dichloromethane 5/95). The eluate containing the target material was concentrated under reduced pressure to obtain a residue containing compound B22 (4.88 g, yield 65.6%).

LCMS (ESI): retention time: 4.992 minutes, m/z = 831.66 $[M+H]^+$ (LCMS analysis conditions: method 4)
Yield: 90.4% (The obtained residue and 3,5-bis(trifluoromethyl)benzoic acid were dissolved in DMSO-$d_6$ and subjected to qNMR analysis.)

Example B-31

Synthesis of compound B23: ((2S)-1-[(2S)-2-[[2-[[(2S)-2-[benzyloxycarbonyl(ethyl)amino]-3-(p-tolyl)propanoyl]-methyl-amino]acetyl]amino]-4-[3,5-difluoro-4-(trifluoromethyl)phenyl]butanoyl]pyrrolidine-2-carboxylic acid)

[Formula 103]

B22

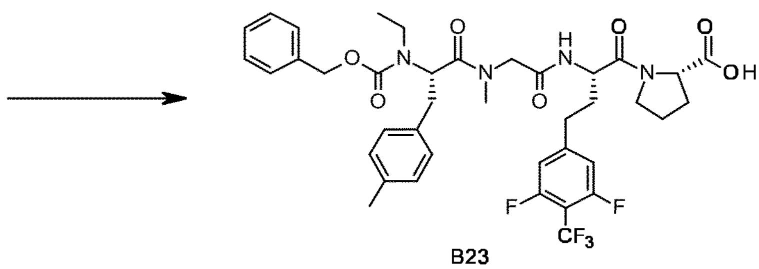

B23

To a reaction vessel, the residue (3.85 g, content: 93.7%) containing compound B22 obtained in Example B-29

and isopropyl acetate (18.1 mL) were added at room temperature, and then hexamethyldisilazane (2.31 mL) was added. The reaction vessel was cooled in an ice bath and trimethylsilyl trifluoromethanesulfonate (1.57 mL) was added with stirring. The reaction vessel was removed from the ice bath, and the mixture was stirred at room temperature for 2 hours. The reaction vessel was cooled in an ice bath and hexamethyldisilazane (2.31 mL) and trimethylsilyl trifluoromethanesulfonate (1.57 mL) were added with stirring. The reaction vessel was removed from the ice bath, and the mixture was stirred at room temperature for 2 hours. The reaction mixture was sampled and prepared as a sample (sample preparation method 1), and it was confirmed by HPLC analysis that the reaction conversion rate was 99.7% or more (calculation expression 1 of reaction conversion rate). The reaction vessel was cooled in an ice bath, and 2-MeTHF (18.1 mL) and 5% aqueous dipotassium hydrogen phosphate solution (18.1 mL) were added. The mixture was stirred for 10 minutes, and the aqueous layer was discharged. The organic layer was washed with 5% aqueous sodium dihydrogen phosphate solution (18.1 mL) and 5% aqueous sodium chloride solution (18.1 mL). The resulting organic layer was concentrated under reduced pressure to obtain a residue containing compound B23. To the obtained residue, 2-MeTHF (5.5 mL) was added to obtain a solution (8.069 g, content: 32.43%) containing compound B23. The resulting solution was diluted with acetonitrile and subjected to LCMS analysis (method 1: retention time of compound B23; 4.237 minutes, m/z = 775.48 $[M+H]^+$). The obtained white powder and 3,5-bis(trifluoromethyl)benzoic acid were dissolved in DMSO-$d_6$ and subjected to qNMR analysis (yield: 77.5%).

Example B-32

Synthesis of compound A11: (tert-butyl (3S)-3-[benzyloxycarbonyl(methyl)amino]-4-(dimethylamino)-4-oxo-butanoate)

[Formula 104]

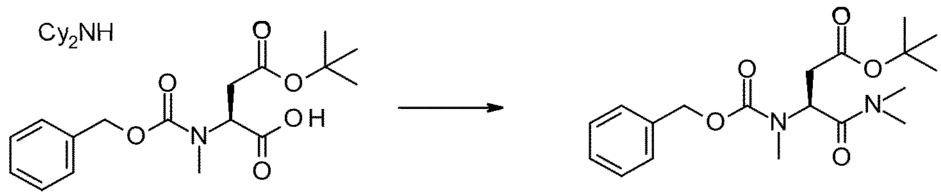

A10 A11

To a reaction vessel after nitrogen replacement, compound A10 (4.78 kg) and 2-MeTHF (24 kg) were sequentially added at room temperature. The external temperature of the reaction vessel was set to 10°C, and while the reaction mixture was stirred, DIPEA (3.22 kg) and a solution of dimethylamine-THF (2 M, THF solution, 5.49 kg) were sequentially added, and the mixture was stirred for 30 minutes. After T3P (50% w/w, 2-MeTHF solution, 8.64 kg) was added, the external temperature of the reaction vessel was set to 25°C, and the mixture was stirred for 6 hours. The reaction mixture was sampled and prepared as a sample (sample preparation method 3), and it was confirmed by HPLC analysis that the reaction conversion rate was 96.2% (calculation expression 1 of reaction conversion rate). The external temperature of the reaction vessel was set to 10°C, and 10% aqueous citric acid monohydrate solution (29 kg) was added to the reaction mixture. The external temperature of the reaction vessel was set to 25°C, and the mixture was stirred for 10 minutes, and then the stirring was stopped, and the aqueous layer was discharged from the reaction vessel. The resulting organic layer was washed with 10% aqueous citric acid monohydrate solution (29 kg × 2) and 5% aqueous sodium carbonate solution (29 kg × 3). To the resulting organic layer, 2-MeTHF (26.0 kg) was added, and the mixture was concentrated under reduced pressure at the external temperature of 60°C until the liquid volume reached about 7 L. The residue and the wash solution obtained by washing the reaction vessel with 2-MeTHF (6.8 kg × 2) were combined to obtain a solution (19.8 kg) containing compound A11.

Example B-33

Synthesis of compound A12: (tert-butyl (3S)-4-(dimethylamino)-3-(methylamino)-4-oxo-butanoate

[Formula 105]

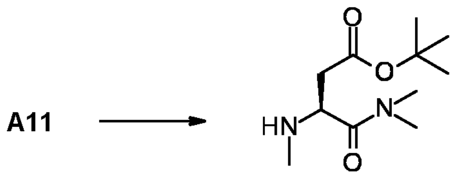

A12

To a reaction vessel after nitrogen replacement, 5% Pd/C (1.31 kg, 50% aqueous content), the solution (19.8 kg) containing compound A11 obtained in Example B-32, and 2-MeTHF (6.0 kg) were sequentially added at room temperature. The external temperature of the reaction vessel was set to 25°C, and the reaction vessel was pressurized with hydrogen until the internal pressure of the reaction vessel reached 0.18 MPaG. After stirring for 2 hours, no fluctuation in the internal pressure was confirmed. The reaction vessel was then pressurized to 0.18 M PaG with hydrogen, and the mixture was stirred for an additional 1.5 hours. The reaction mixture was sampled and prepared as a sample (sample preparation method 1), and it was confirmed by HPLC analysis that the reaction conversion rate was 100% (no starting materials were detected) (calculation expression 1 of reaction conversion rate). After replacing the inside of the reaction vessel with nitrogen, the reaction mixture was filtered under pressure. The inside of the reaction vessel and the filtering apparatus were washed with 2-MeTHF (11.3 kg × 3), and the filtrate and the wash solution were collected into a storage vessel as a storage solution. To the reaction vessel after nitrogen replacement, the storage solution and 2-MeTHF (0.40 kg) were added, and the mixture was concentrated under reduced pressure with stirring at the external temperature of 40°C until the liquid volume reached about 4 L. The residue and the wash solution obtained by washing the reaction vessel with 2-MeTHF (6.8 kg) were combined to obtain a solution (10.4 kg) containing compound A12.

Example B-34

Synthesis of compound A14: (tert-butyl (3S)-3-[[(2S)-2-[benzyloxycarbonyl(methyl)amino]-2-cyclopentyl-acetyl]-methyl-amino]-4-(dimethylamino)-4-oxo-butanoate)

[Formula 106]

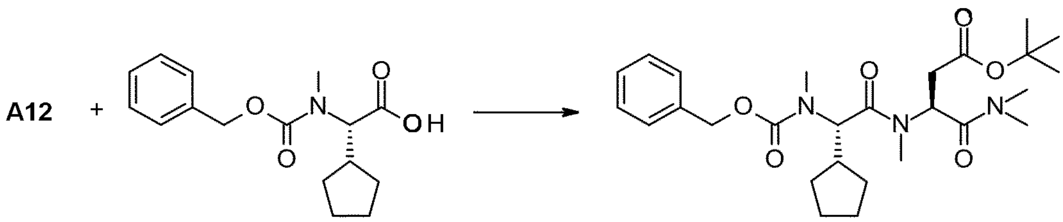

A13 A14

The solution (10.3 kg) containing compound A12 obtained in Example B-33 was added, and the mixture was concentrated under reduced pressure with stirring at the external temperature of 40°C until the liquid volume reached about 10 L. Compound A13 (61 w/w%, 2-MeTHF solution, 4.97 kg), 2-MeTHF (1.0 L), and acetonitrile (2.8 kg) were added at room temperature. The external temperature was cooled to 10°C, and DIPEA (4.93 kg) and HATU (4.95 kg) were sequentially added, and then the external temperature was raised to 25°C. After the reaction mixture was stirred at 25°C for 4 hours, the reaction mixture was sampled and prepared as a sample (sample preparation method 1), and it was confirmed by HPLC analysis that the reaction conversion rate was 99.3% (calculation expression 1 of reaction conversion rate). To the reaction vessel, CPME (4.0 kg), 5% aqueous potassium carbonate solution (3.5 kg), and N-methylimidazole (712 g) were sequentially added, and the mixture was stirred for 30 minutes. Then, 2.5% aqueous ammonia solution (14 kg) and 2-MeTHF (3.9 kg) were added, and the mixture was stirred for 10 minutes, and then the aqueous layer was discharged. The resulting organic layer was washed with 2.5% aqueous ammonia solution (18 kg), 10% aqueous sodium hydrogen sulfate monohydrate solution (18 kg × 3), and 5% aqueous potassium carbonate solution (18 kg). The resulting organic layer was concentrated under reduced pressure with stirring at the external temperature of 40°C until the liquid volume reached about 9 L. The residue and the wash solution obtained by washing the reaction vessel with 2-MeTHF (6.8 kg × 2) were combined and added to obtain a solution (21.6 kg) containing compound A14.

Example B-35

Synthesis of compound A15: (tert-butyl (3S)-3-[[(2S)-2-cyclopentyl-2-(methylamino)acetyl]-methyl-amino]-4-(dimethylamino)-4-oxo-butanoate)

[Formula 107]

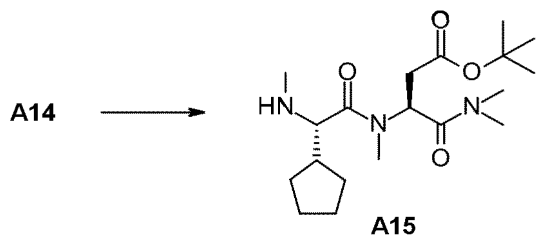

A15

To a reaction vessel after nitrogen replacement, 5% Pd/C (1.26 kg, 50% aqueous content), the solution (21.0 kg) containing compound A14 obtained in Example B-34, and 2-MeTHF (5.1 kg) were sequentially added at room temperature. The external temperature of the reaction vessel was set to 25°C, and pressurized with hydrogen until the internal pressure of the reaction vessel reached 0.18 MPaG. After stirring for 40 minutes, no fluctuation in the internal pressure was confirmed. The reaction vessel was then pressurized to 0.18 MPaG with hydrogen, and the reaction mixture was stirred for an additional 1 hour. The reaction mixture was sampled and prepared as a sample (sample preparation method 1), and it was confirmed by HPLC analysis that the reaction conversion rate was 100% (no starting materials were detected) (calculation expression 1 of reaction conversion rate). After replacing the inside of the reaction vessel with nitrogen, the reaction mixture was filtered under pressure. The inside of the reaction vessel and the filtering apparatus were washed with 2-MeTHF (11 kg × 4, 5.4 kg), and the filtrate and the wash solution were collected as a storage solution. The resulting filtrate and the wash solution were concentrated under reduced pressure at the external temperature of 60°C until the liquid volume of the reaction mixture reached about 6 L. The residue and the wash solution obtained by washing the reaction vessel with 2-MeTHF (6.8 kg) were combined to obtain a solution (11.8 kg) containing compound A15.

Example B-36

Synthesis of compound B25: tert-butyl (3S)-3-[[(2S)-2-[(1-(benzyloxycarbonylamino)cyclopentanecarbonyl)-methyl-amino]-2-cyclopentyl-acetyl]-methyl-amino]-4-(dimethylamino)-4-oxo-butanoate

[Formula 108]

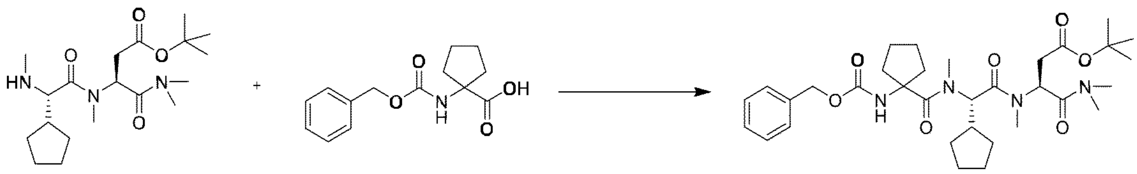

A15 B24 B25

To a reaction vessel, the solution (16.0 g, content: 32.5%) containing compound A15 obtained by the same preparation method as in Example B-35 was added at room temperature and concentrated under reduced pressure. The residue was dissolved in acetonitrile (10.0 mL), and the operation of concentrating under reduced pressure was repeated 3 times. To the reaction vessel, acetonitrile (53.9 mL), compound B24 (9.60 g) and DIPEA (12.7 mL) were added at room temperature, and the mixture was stirred. The external temperature of the reaction vessel was set to 55°C, and HATU (15.26 g) was added to the reaction mixture in two portions with stirring, and the mixture was stirred for 6 hours. The reaction mixture was sampled and prepared as a sample (sample preparation method 2), and it was confirmed by HPLC analysis that the reaction conversion rate was 99.6% or more (calculation expression 3 of reaction conversion rate). To the reaction mixture, N-methylimidazole (4.3 mL) was added, and the mixture was stirred for 5 minutes, and then water (27.0 mL) was added, and the mixture was stirred for 30 minutes. The external temperature of the reaction vessel was then decreased from 55°C to 25°C, and the reaction mixture was stirred overnight. The reaction mixture was suction-filtered using filter paper, and the residue was washed with a mixed solution of acetonitrile (18.0 mL) and water (9.0 mL). The

filtered crystals were dried in vacuo at 40°C for 3 hours to obtain a white powder (6.96 g) containing compound B25. The resulting white powder was diluted with acetonitrile and subjected to LCMS analysis (method 5: retention time of compound B25; 6.914 minutes, m/z = 637.30 $[M+Na]^+$). The resulting powder and 3,5-bis(trifluoromethyl)benzoic acid were dissolved in DMSO-$d_6$ and subjected to qNMR analysis (yield: 78.3%).

Example B-37

Synthesis of compound B26: (3S)-3-[[(2S)-2-[(1-benzyloxycarbonylamino)cyclopentanecarbonyl)-methyl-amino]-2-cyclopentyl-acetyl]-methyl-amino]-4-(dimethylamino)-4-oxo-butanoic acid)

[Formula 109]

B25

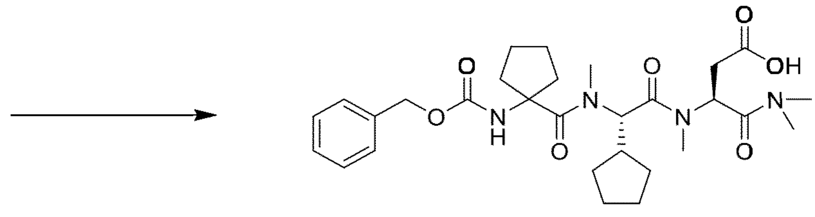

B26

To a reaction vessel, the white powder (2.50 g, content: 95.9%) containing compound B25 obtained in Example B-36 and dichloromethane (12.0 mL) were added at room temperature, and then hexamethyldisilazane (2.07 mL) was added. The reaction vessel was cooled in an ice bath and trimethylsilyl trifluoromethanesulfonate (1.41 mL) was added with stirring. The reaction vessel was removed from the ice bath, and the mixture was stirred at room temperature for 1 hour. The reaction mixture was sampled and prepared as a sample (sample preparation method 1), and it was confirmed by HPLC analysis that the reaction conversion rate was 99.9% or more (calculation expression 1 of reaction conversion rate). The reaction vessel was cooled in an ice bath, and dichloromethane (12.0 mL) and 5% aqueous dipotassium hydrogen phosphate solution (24.0 mL) were added. The mixture was stirred for 10 minutes, and the organic layer was discharged. To the aqueous layer, 2-MeTHF (72.0 mL) was added, and then the organic layer was washed with 0.5 M aqueous hydrochloric acid solution (12.0 mL) and 5% aqueous sodium chloride solution (24.0 mL). The resulting organic layer was concentrated under reduced pressure to obtain a white powder (1.98 g) containing compound B26. The obtained white powder was diluted with acetonitrile and subjected to LCMS analysis (method 1: retention time of compound B26; 3.307 minutes, m/z = 581.13 $[M+Na]^+$). The obtained white powder and 3,5-bis(trifluoromethyl)benzoic acid were dissolved in DMSO-$d_6$ and subjected to qNMR analysis (yield: 81.5%).

Example B-38

Synthesis of compound B27: (tert-butyl (2S)-1-[(25)-2-[[(2S,35)-2-[[(29)-2-[[(3S)-3-[[(2S)-2-[(1-(benzyloxycarbonylamino)cyclopentanecarbonyl)-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carboxylate)

[Formula 110]

B7 + B26

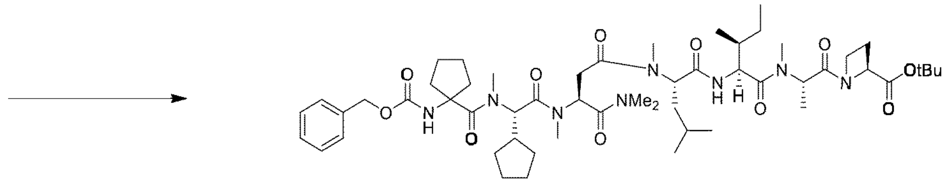

B27

To a reaction vessel, the solution (2.74 g, content: 31.08%) containing compound B7 obtained in Example B-21 and the white powder (1.21 g, content: 89.9%) containing compound B26 obtained in Example B-37, followed by 2-MeTHF

(2.13 mL) and DMF (2.13 mL) were added at room temperature, and the mixture was stirred. The external temperature of the reaction vessel was set to 25°C, and 2-MeTHF (125 μL), DIPEA (1.23 mL) and HATU (1.34 g) were added to the reaction mixture with stirring, and the mixture was stirred for 3 hours. The reaction mixture was sampled and prepared as a sample (sample preparation method 1), and it was confirmed by HPLC analysis that the reaction conversion rate was 97.5% or more (calculation expression 1 of reaction conversion rate). To the reaction mixture, N-methylimidazole (0.14 mL) and 5% aqueous sodium hydrogen carbonate solution (5.1 mL) were added, and the mixture was stirred for 2 hours. Then, 2-MeTHF (6.4 mL) and 2.5% aqueous ammonia solution (5.1 mL) were added to wash the organic layer, and the aqueous layer was discharged. The organic layer was washed with 2.5% aqueous ammonia solution (6.8 mL) and 5% aqueous sodium hydrogen sulfate monohydrate solution (6.8 mL × 2). Acetonitrile (2.1 mL) was added to the organic layer, and the organic layer was washed with 5% aqueous sodium hydrogen sulfate monohydrate solution (6.8 mL × 1) and 3% aqueous dipotassium hydrogen sulfate solution (6.8 mL × 1). The resulting organic layer was concentrated under reduced pressure to obtain a residue (2.60 g, content: 24.2%) containing compound B27. To the obtained residue, 2-MeTHF (4.50 mL) was added to obtain a solution (6.21 g) containing compound B27. The obtained solution was diluted with acetonitrile and subjected to LCMS analysis (method 6: retention time of compound B27; 9.79 minutes, m/z = 1046.33 $[M+Na]^+$). The resulting residue and 3,5-bis(trifluoromethyl)benzoic acid were dissolved in DMSO-$d_6$ and subjected to qNMR analysis (yield: 83.4%).

Example B-39

Compound B28: tert-butyl (2S)-1-[(2S)-2-[[(2S,3S)-2-[[(2S)-2-[[(3S)-3-[[(2S)-2-[(1-aminocyclopentanecarbonyl)-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carboxylate

[Formula 111]

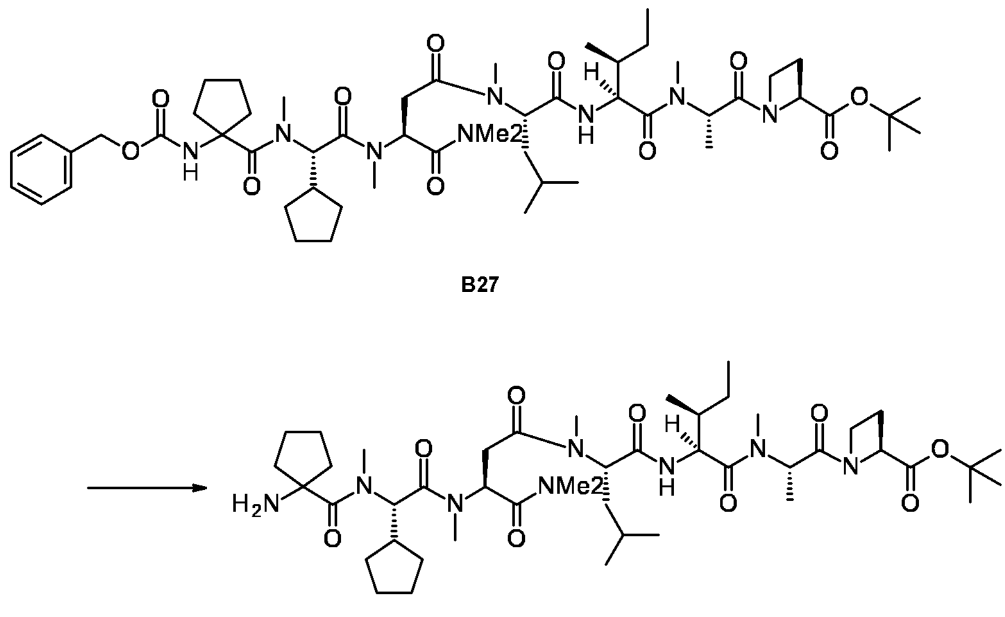

B27

B28

The compound B27 (4.20 g, content 24.18%) obtained in Example B-38 was added to a reaction vessel. Next, 2-MeTHF (4.4 mL) and 5% Pd/C (0.127 g, 50% aqueous content) were sequentially added at room temperature. After the inside of the reaction vessel was replaced with hydrogen, the mixture was stirred at room temperature for 2 hours under a hydrogen atmosphere at normal pressure, and then further stirred at room temperature for 5 hours at a hydrogen pressure of 0.20 MPa. The reaction mixture was sampled and prepared as a sample (sample preparation method 1), and it was confirmed by HPLC analysis (method 6) that the reaction conversion rate was 99.72% (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF. The resulting solution was concentrated under reduced pressure at the external temperature of 40°C. The

resulting residue (1.84 g) was analyzed by HPLC. Furthermore, the resulting residue and 3,5-bis(trifluoromethyl)benzoic acid were dissolved in DMSO-$d_6$ and subjected to qNMR analysis (content 50.01%, amount: 0.92 g, yield: quantitative).

LC retention time of compound B28: 6.063 minutes (HPLC analysis conditions: method 6)
LCMS(ESI) of compound B28: retention time: 5.50 minutes, m/z = 890 $[M+H]^+$ (LCMS analysis conditions: method 6)

Example B-40-1

Compound B11: tert-butyl (2S)-1-[(2S)-2-[[(2S,3S)-2-[[(2S)-2-[[(3S)-3-[[(2S)-2-[[1-[[(2S)-1-[(2S)-2-[[2-[[(2S)-2-[benzyloxycarbonyl(ethyl)aminol-3-(p-tolyl)propanoyl]-methyl-amino]acetyl]amino]-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carboxylate

[Formula 112]

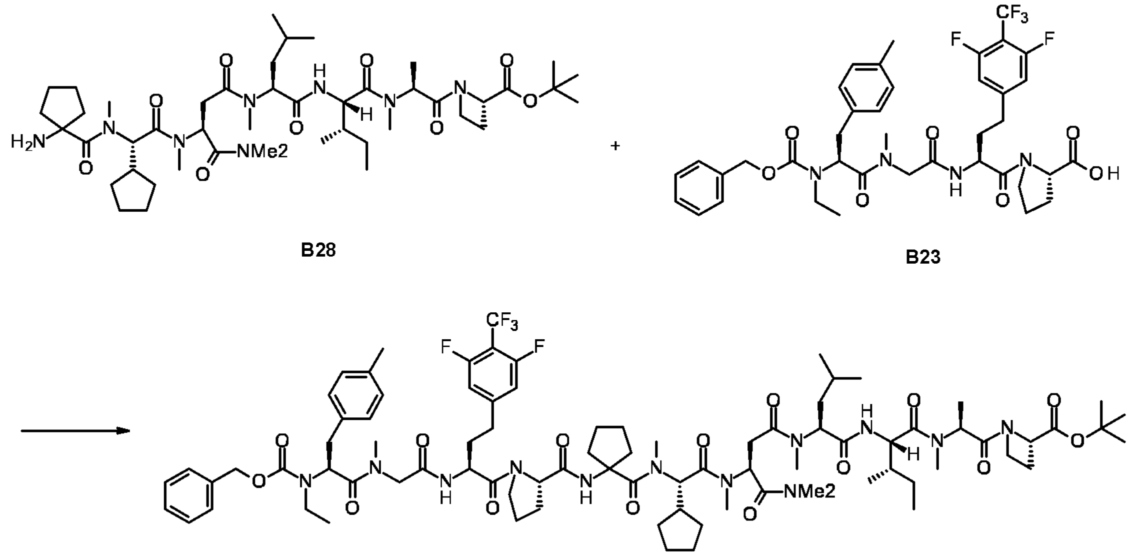

B28

B23

B11

The compound B28 (402.59 mg, content 50.01%) synthesized in Example B-39 and the compound B23 (596.20 mg, content 32.43%) synthesized in Example B-31 were added to a reaction vessel and concentrated under reduced pressure at the external temperature of 40°C. The reaction vessel was subjected to nitrogen replacement. Next, dimethyl carbonate (1 mL) and DIPEA (0.154 mL) were sequentially added. HATU (173.93 mg) was added with stirring at the external temperature of 0°C, and then the mixture was stirred at 25°C for 6 hours. To the reaction vessel, 2.5% aqueous ammonia solution (1.8 mL) was added, and the mixture was stirred for 30 minutes. Next, 2-MeTHF (2 mL) was added, and the mixture was stirred for 5 minutes, and then the aqueous layer was discharged. The resulting organic layer was washed with 10% aqueous sodium hydrogen sulfate monohydrate solution (1.8 mL × 3), and 5% aqueous potassium carbonate solution (1.8 mL × 2). The resulting organic layer was analyzed by HPLC. As a result of HPLC analysis using a standard preparation, the amount of the obtained compound B11 was 328.66 mg (yield: 88%).

LC retention time of compound B11: 11.367 minutes (HPLC analysis conditions: method 6)
Chiral LC retention time of compound B11: 61.330 minutes (HPLC analysis conditions: method 8)
LCMS (ESI) retention time of compound B11: 10.66 minutes, m/z = 1668.339 $[M+Na]^+$ (LCMS analysis conditions: method 6)

Fragment coupling of compound B28 and compound B23

The results of the synthesis of compound B11 using HATU, COMU or DMT-MM as the condensation reagent, and

dimethyl carbonate, 2-MeTHF or MeCN as the solvent are shown in Table 3. The experimental operation was in accordance with the case in which HATU was used as the condensation reagent, and dimethyl carbonate was used as the solvent (Example B-40-1). TM in the table is a target material (compound B11). The production amount of the target material (TM) and the epimer was shown as Area% ratio in the HPLC measurement (HPLC analysis condition: method 8). The yield was calculated by HPLC analysis using a standard preparation.

[Table 3]

| Solvent | Example No. | Condensation reagent | Reaction time (h) | Ratio of TM and epimer(%) | | Yield (%) |
|---|---|---|---|---|---|---|
| | | | | TM | epimer | |
| dimethyl carbonate | Example B-40-1 | HATU | 5 | 99.901 | 0.099 | 88 |
| | Example B-40-2 | COMU | 5 | 99.724 | 0.276 | 89 |
| | Example B-40-3 | DMT-MM | 5 | 99.621 | 0.379 | 88 |
| 2-MeTHF | Example B-40-4 | HATU | 5 | 99.857 | 0.143 | 82 |
| | Example B-40-5 | COMU | 5 | 99.883 | 0.117 | 83 |
| | Example B-40-6 | DMT-MM | 5 | 99.702 | 0.298 | 81 |
| MeCN | Example B-40-7 | HATU | 5 | 99.877 | 0.123 | 80 |
| | Example B-40-8 | COMU | 5 | 99.819 | 0.181 | 81 |
| | Example B-40-9 | DMT-MM | 5 | 99.657 | 0.343 | 84 |

Example B-41

Synthesis of compound A18: (tert-butyl (3S)-3-[[(28)-2-[(1-aminocyclopentanecarbonyl)-methyl-amino]-2-cyclopentyl-acetyl]-methyl-amino]-4-(dimethylamino)-4-oxo-butanoate)

[Formula 113]

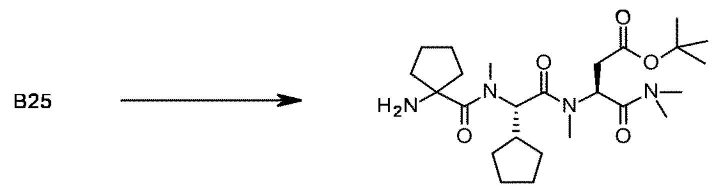

A18

To a reaction vessel, the white powder (2.70 g, content: 95.9%) containing compound B25 obtained in Example B-36 and THF (41.4 mL), followed by 5% Pd/C (0.899 g, 50% aqueous content) were added at room temperature, and the mixture was stirred. Degassing with nitrogen gas was performed with stirring, then degassing with hydrogen gas was performed, and the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). The reaction mixture was suction-filtered using filter paper and a membrane filter, and the residue was washed with THF (10.8 mL × 2). The resulting filtrate was concentrated under reduced pressure to obtain a residue (2.22 g) containing compound A18. To the obtained residue, 2-MeTHF (3.0 mL) was added to obtain a solution (4.71 g, content: 40.1%) containing compound A18. The obtained solution was diluted with acetonitrile and subjected to LCMS analysis (method 1: retention time of compound A18; 2.687 minutes, m/z = 503.28 $[M+Na]^+$). The obtained residue and 1,3,5-trimethoxybenzene were dissolved in DMSO-$d_6$ and subjected to qNMR analysis (yield: 93.4%).

Example B-42

Synthesis of compound B29: (tert-butyl (3S)-3-[[(2S)-2-[[1-[[(2S)-1-[(2S)-2-[[2-[[(2S)-2-[benzyloxycarbonyl(ethyl)amino]-3-(p-tolyl)propanoyl]-methyl-amino]acetyl]amino]-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoate)

[Formula 114]

A18 + B23

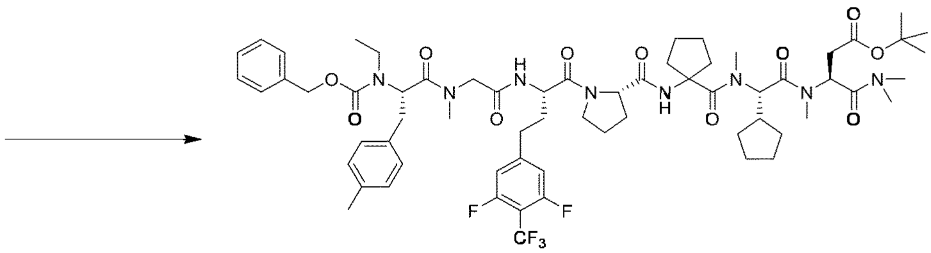

B29

To a reaction vessel, the solution (1.89 g, content: 40.1%) containing compound A18 obtained in Example B-41 and the solution (3.57 g, content: 32.4%) containing compound B23 obtained in Example B-31, followed by 2-MeTHF (3.02 mL) and acetonitrile (0.76 mL) were added at room temperature, and the mixture was stirred. The external temperature of the reaction vessel was set to 25°C, and DIPEA (1.10 mL) and HATU (1.20 g) were added to the reaction mixture with stirring, and the mixture was stirred for 3 hours. The reaction mixture was sampled and prepared as a sample (sample preparation method 2), and it was confirmed by HPLC analysis that the reaction conversion rate was 99.8% or more (calculation expression 3 of reaction conversion rate). To the reaction mixture, N-methylimidazole (0.12 mL) and 5% aqueous sodium carbonate solution (4.53 mL) were added, and the mixture was stirred for 2 hours. Then, 2.5% aqueous ammonia solution (4.53 mL) was added to wash the organic layer, and the aqueous layer was discharged. The organic layer was washed with 2.5% aqueous ammonia solution (6.04 mL × 2), 10% aqueous sodium hydrogen sulfate monohydrate solution (6.04 mL × 2), and 3% aqueous dipotassium hydrogen phosphate solution (6.04 mL × 1). The resulting organic layer was concentrated under reduced pressure to obtain a residue (2.68 g) containing compound B29. To the obtained residue, 2-MeTHF (4.50 mL) was added to obtain a solution (6.26 g, content: 25.3%) containing compound B29. The obtained solution was diluted with acetonitrile and subjected to LCMS analysis (method 6: retention time of compound B29; 11.00 minutes, m/z = 1260.53 $[M+Na]^+$). The obtained residue and 3,5-bis(trifluoromethyl)benzoic acid were dissolved in DMSO-$d_6$ and subjected to qNMR analysis (yield: 85.6%).

Example B-43

Synthesis of compound B30: (3S)-3-[[(2S)-2-[[1-[[(2S)-1-[(2S)-2-[[2-[[(2S)-2-[benzyloxycarbonyl(ethyl)amino]-3-(p-tolyl)propanoyl]-methyl-aminolacetyl]amino]-4-[3,5-difluoro-4-(trifluoromethyl)phenyl]butanoyl]pyrrolidine-2-carbonyl] amino]cyclopentanecarbonyl]-methyl-amino]-2-cyclopentyl-acetyl]-methyl-amino]-4-(dimethylamino)-4-oxo-butanoic acid

[Formula 115]

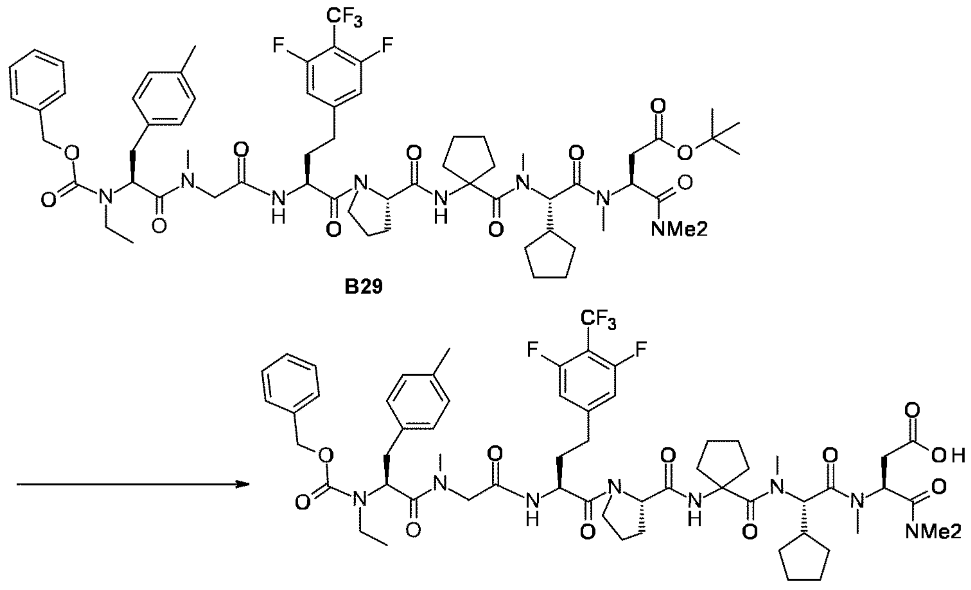

B29

B30

To a reaction vessel, the storage solution (3.293 g, 25.0 wt%) containing compound B30 obtained in Example B-42 was added and concentrated under reduced pressure, and IPAc (4.11 mL) was added to the resulting residue. HMDS (0.353 mL) was added while stirring the mixture at room temperature, and then the external temperature was cooled to 0°C, and TMSOTf (0.24 mL) was slowly added dropwise. The external temperature was raised to room temperature, and the mixture was stirred for 1 hour. The reaction mixture was sampled and prepared as a sample (sample preparation method 1), and it was confirmed by HPLC analysis that the reaction conversion rate was 99.9% or more (calculation expression 1 of reaction conversion rate). To the reaction solution, 5% aqueous dipotassium hydrogen phosphate solution (0.58 mL) was added dropwise, and the mixture was stirred for 10 minutes. The aqueous layer was discharged, and the organic layer was washed with 5% sodium chloride solution (0.82 mL). The resulting organic layer was concentrated and dried under reduced pressure at the external temperature of 30°C to obtain a residue containing compound B30 (1.298 g, yield 94.6%).

LCMS (ESI): retention time: 4.320 minutes, m/z = 1181.54 $[M+H]^+$ (LCMS analysis conditions: method 4)
Yield: 94.6% (The obtained residue and 3,5-bis(trifluoromethyl)benzoic acid were dissolved in DMSO-$d_6$ and subjected to qNMR analysis.)

Example B-44

Synthesis of compound B11: tert-butyl (2S)-1-[(2S)-2-[[(2S,3S)-2-[[(2S)-2-[[(3S)-3-[[(2S)-2-[[1-[[(2S)-1-[(2S)-2-[[2-[[(2S)-2-[benzyloxycarbonyl(ethyl)amino]-3-(p-tolyl)propanoyl]-methyl-amino]acetyl]amino]-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carboxylate

[Formula 116]

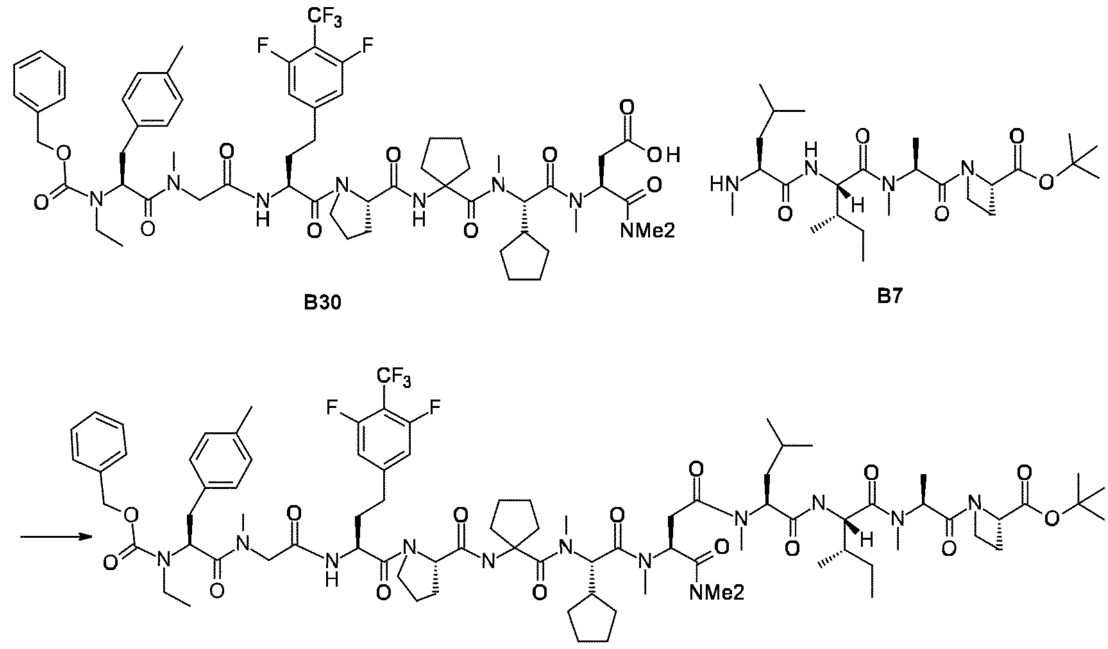

B30 B7

B11

A storage solution (276.1 mg, 75.2 wt%) containing compound B30 and compound B7 (316.8 mg, 30.0 wt%) were weighed into a reaction vessel, and 2-MeTHF (1.6 mL) was added. DIPEA (171 μL) was added with stirring at room temperature, followed by HATU (155.9 mg). After stirring at room temperature for 3 hours, the reaction mixture was sampled and prepared as a sample (sample preparation method 2), and it was confirmed by HPLC analysis that the reaction conversion rate was 99.6% (calculation expression 3 of reaction conversion rate). The external temperature was cooled to 0°C, and N-methylimidazole (14 μL (0.175 mmol)) and 5% aqueous sodium carbonate solution (2.0 mL) were added to the reaction solution, and the mixture was stirred at room temperature for 10 minutes. The aqueous layer was discharged, and the organic layer was washed with 2.5% aqueous ammonia solution (2.0 mL), 5% aqueous sodium hydrogen sulfate solution (2.0 mL × 2), and 5% aqueous sodium carbonate solution (2.0 mL × 2). The resulting organic layer was concentrated and dried under reduced pressure at the external temperature of 30°C and dissolved in 2-MeTHF to obtain a solution containing compound B11 (1.24 g, yield 96.1%).
Yield: 96.1% (The resulting residue and 3,5-bis(trifluoromethyl)benzoic acid were dissolved in DMSO-$d_6$ and subjected to qNMR analysis.)

<u>Example B-45</u>

<u>Compound B31: (2S)-1-[(2S)-2-[[(2S,3S)-2-(benzyloxycarbonylamino)-3-methyl-pentanoyl]-methyl-amino]propanoyl] azetidine-2-carboxylic acid</u>

[Formula 117]

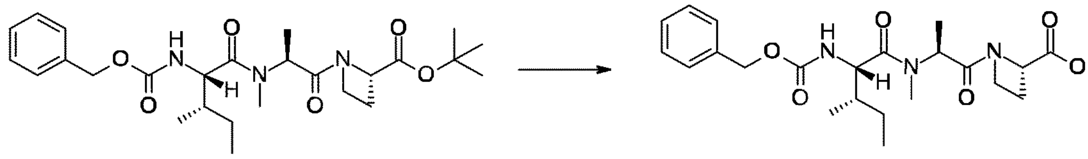

B5 B31

Compound B5 (879.48 mg, content 62.67%) was added to a reaction vessel. IPAc (5.5 mL) and HMDS(0.59 mL) were sequentially added at room temperature, and the reaction vessel was subjected to nitrogen replacement. Next, the external temperature of the reaction vessel was set to 0°C, and TMSOTf (0.415 mL) was added with stirring, and then the

mixture was stirred at room temperature for 3 hours. The external temperature was set to 0°C, and 2-MeTHF (5.5 mL) and 5% aqueous dipotassium hydrogen phosphate solution (5.5 mL) were added to the reaction vessel, and the mixture was stirred at room temperature for 8 minutes and the aqueous layer was discharged (aqueous layer-1). The organic layer was washed with 5% aqueous sodium dihydrogen phosphate solution (5.5 mL), and the aqueous layer was discharged (aqueous layer-2). The aqueous layer-1 and aqueous layer-2 were mixed and 1 N aqueous hydrochloric acid solution (1.1 mL) was added, and the mixture was extracted with EtOAc (5.5 mL × 2). To the aqueous layer, 1 N aqueous hydrochloric acid solution (1.1 mL) was further added, and the mixture was extracted with EtOAc (5.5 mL × 2). The resulting organic layers were mixed, concentrated under reduced pressure at the external temperature of 40°C, and azeotroped with MeCN (2.2 mL × 2). After azeotrope, the amount of the obtained compound B31 was 853.58 mg (content 53.84%, yield 94%).

LC retention time of compound B31: 2.869 minutes (HPLC analysis conditions: method 4)
LCMS(ESI) of compound B31: retention time: 2.619 minutes, m/z = 456 $[M+Na]^+$ (LCMS analysis conditions: method 4)

Example B-46-1

Compound D6: tert-butyl 2-[[(2S)-2-[[(2S)-1-[(2S)-2-[[(2S,3S)-2-(benzyloxycarbonylamino)-3-methyl-pentanoyl]-methyl-amino]propanoyl]azetidine-2-carbonyl]-ethyl-amino]-3-(p-tolyl)propanoyl]-methyl-amino]acetate

[Formula 118]

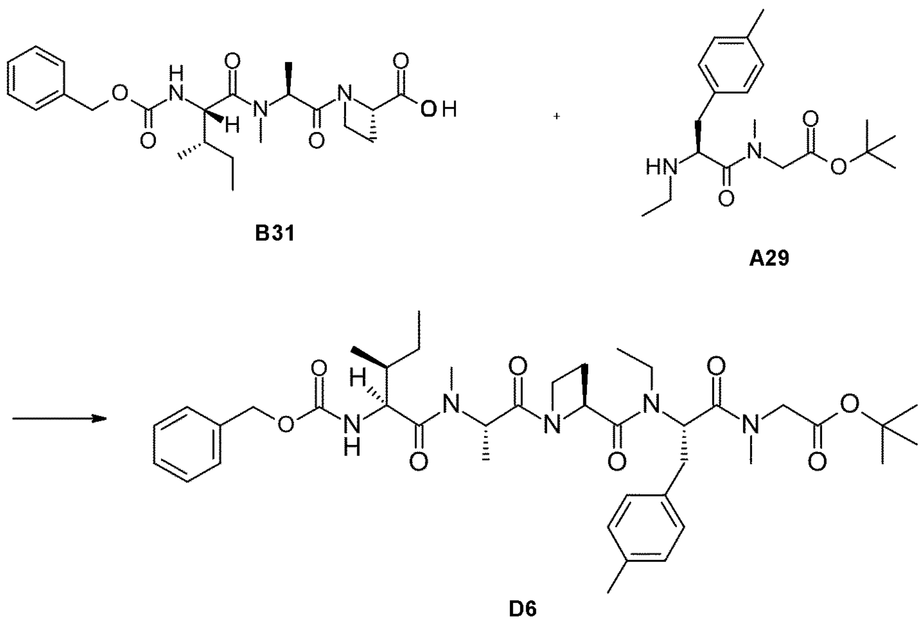

B31

A29

D6

Compound B31 (90.58 mg, content 53.84%) and compound A29 (53.42 mg, content 85.19%) were added to the reaction vessel, and the reaction vessel was subjected to nitrogen replacement. Next, MTHP(0.25 mL) and DIPEA(0.101 mL) were sequentially added at the external temperature of 25°C. PyOxim (183 mg) was added with stirring, and then the mixture was stirred at 25°C for 4 hours. 2-MeTHF (1 mL) and N-methylimidazole (9.1 μL) were added to the reaction vessel, and 5% aqueous sodium carbonate solution (0.5 mL) was further added with stirring, and then the mixture was stirred for 30 minutes. Next, 2.5% aqueous ammonia solution (0.5 mL) was added, and the mixture was stirred for 5 minutes. After the aqueous layer was discharged, the resulting organic layer was washed with 2.5% aqueous ammonia solution (0.5 mL), 10% aqueous sodium hydrogen sulfate monohydrate solution (0.5 mL × 2), and 3% aqueous dipotassium hydrogen phosphate solution (0.5 mL). The resulting organic layer was analyzed by HPLC. As a result of HPLC analysis using a standard preparation, the amount of the obtained compound D6 was 75.92 mg (yield: 90%).

LC retention time of compound D6: 4.325 minutes (HPLC analysis conditions: method 4)
LCMS (ESI) retention time of compound D6: 4.092 minutes, m/z = 773 $[M+Na]^+$ (LCMS analysis conditions: method 4)

Elongation reaction of compound B31 and compound A29 (amino acid residue at cyclized position B)

The results of the synthesis of compound D6 using HATU or COMU as the condensation reagent, and MeCN as the solvent are shown in Table 4. The experimental operation was in accordance with the case in which PyOxim was used as the condensation reagent, and Me-THP was used as the solvent (Example B-46-1). TM in the table is a target material (compound D6). The production amount of target material (TM) and epimer was shown as Area% ratio in the HPLC measurement (HPLC analysis condition: method 4). The yield was calculated by HPLC analysis using a standard preparation.

[Table 4]

| Example No. | Condensation reagent (equivalent) | Base (equivalent) | Solvent (v/w) | Equivalents of C-terminus and N-terminus | | Reaction time (h) | Conversion rate (%) | TM / epimer (%) | | Yield (%) |
|---|---|---|---|---|---|---|---|---|---|---|
| | | | | B31 | A29 | | | TM | Epimer | |
| B-46-1 | PyOxim (3.0) | DIPEA (5.0) | MeTHP (5) | 1.0 | 1.1 | 4 | 96.44 | 97.34 | 2.66 | 90.0 |
| B-46-2 | HATU (3.8) | DIPEA (4.6) | MeCN (200) | 1.0 | 1.0 | 2 | 72.44 | 95.37 | 4.63 | 61.9 |
| B-46-3 | COMU (3.8) | DIPEA (4.6) | MeCN (200) | 1.0 | 1.0 | 2 | 81.08 | 97.32 | 2.68 | 68.6 |
| B-46-4 | COMU (3.8) | DIPEA (4.6) | MeCN (5) | 1.1 | 1.0 | 2 | 99.02 | 97.68 | 2.32 | 61.4 |

*Conversion rates of B-46-1 to B-46-3 were calculated based on C-terminus 1.0 eq (calculation expression 3 of reaction conversion rate). Conversion rate of B-46-4 was calculated based on N-terminus 1.0 eq (calculation expression 1 of reaction conversion rate).

Example C-1

Synthesis of compound C1: tert-butyl (2S)-2-[benzyloxycarbonyl(ethyl)amino]-3-(p-tolyl)propanoate

[Formula 119]

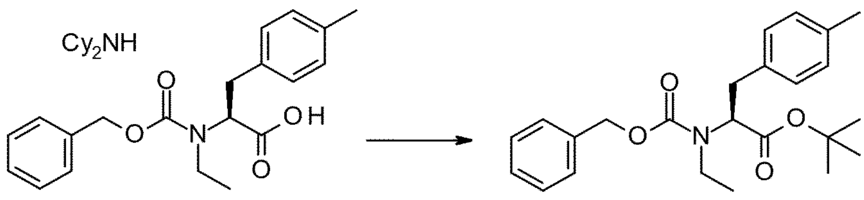

A27 C1

To a reaction vessel after nitrogen replacement, compound A27 (15.3 g), 2-MeTHF (110 mL), and 5% aqueous sodium hydrogen sulfate monohydrate solution (100 mL) were sequentially added, and the mixture was stirred at room temperature. After the aqueous layer was discharged, the resulting organic layer was washed with 5% aqueous sodium hydrogen sulfate monohydrate solution (100 mL × 2) and 10% aqueous NaCl solution (100 mL). The resulting organic layer was concentrated under reduced pressure at the external temperature of 40°C, and the resulting residue was added to the reaction vessel. Next, cyclohexane (80 mL) was added to the reaction vessel at room temperature. After tert-butyl 2,2,2-trichloroacetimidate (8.39 mL) and boron trifluoride diethyl ether complex (297 μL) were added, the mixture was then stirred at room temperature for 1 hour. The reaction mixture was sampled and prepared as a sample (sample preparation method 1), and it was confirmed by HPLC analysis that the reaction conversion rate was 99.9% (calculation expression 1 of reaction conversion rate). The resulting slurry was filtered and the solid residue was washed with cyclohexane. The resulting filtrate was washed with 10% aqueous citric acid solution (80 mL×7), 5% aqueous sodium carbonate solution (80 mL × 2), and 10% aqueous NaCl solution (80 mL). The resulting organic layer was dehydrated with sodium sulfate and filtered to remove sodium sulfate. The resulting filtrate was concentrated under reduced pressure at the external temperature of 40°C to obtain a residue (7.66 g) containing compound C1.
LC retention time of compound C1: 4.663 minutes (HPLC analysis conditions: method 1)

Example C-2

Synthesis of compound C2: tert-butyl (2S)-2-(ethylamino)-3-(p-tolyl)propanoate

[Formula 120]

C1

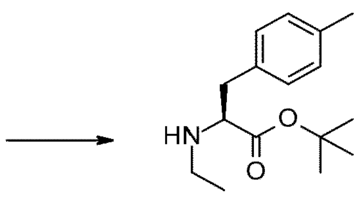

C2

The residue (6.70 g) containing compound C1 obtained in Example C-1 was added to a reaction vessel. Next, 2-MeTHF (53 mL) and 5% Pd/C (3.74 g, 50% aqueous content) were sequentially added at room temperature. The inside of the reaction vessel was replaced with hydrogen, and the mixture was stirred at room temperature for 3.5 hours. The reaction mixture was sampled and prepared as a sample (sample preparation method 1), and it was confirmed by HPLC analysis that the reaction conversion rate was 99.1% (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF (25 mL × 2). The resulting filtrate and solution were combined and concentrated under reduced pressure at the external temperature of 40°C to obtain a residue (4.33 g) containing compound C2.
LC retention time of compound C2: 2.714 minutes (HPLC analysis conditions: method 1)

Example C-3

Synthesis of compound C3: tert-butyl (2S)-2-[[(2S)-2-[benzyloxycarbonyl(methyl)amino]butanoyl]-ethyl-amino]-3-(p-tolyl)propanoate

[Formula 121]

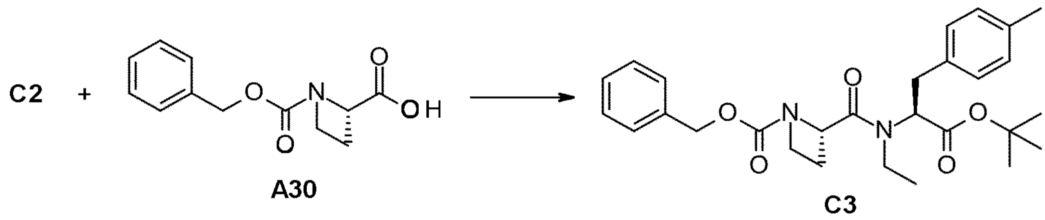

The residue (4.33 g) containing compound C2 obtained in Example C-2 and compound A30 (5.83 g) were added to a reaction vessel, and the reaction vessel was subjected to nitrogen replacement. Next, 2-MeTHF(50 mL), DIPEA(14.4 mL), and T3P (50 w/w% 2-MeTHF solution, 30.8 mL) were sequentially added, and then the mixture was stirred at room temperature for 1 hour. The reaction mixture was sampled and prepared as a sample (sample preparation method 1), and it was confirmed by HPLC analysis that the reaction conversion rate was 99.7% (calculation expression 1 of reaction conversion rate). 5% aqueous sodium carbonate solution (50 mL) was added, and the mixture was stirred for 10 minutes. After the aqueous layer was discharged, the resulting organic layer was washed with 5% aqueous sodium carbonate solution (50 mL × 2), 5% aqueous sodium hydrogen sulfate monohydrate solution (50 mL × 2), and saturated aqueous NaCl solution (50 mL). The resulting organic layer was dehydrated with sodium sulfate and filtered to remove sodium sulfate. The resulting filtrate was concentrated under reduced pressure at the external temperature of 40°C to obtain a solution (7.98 g) containing compound C3.

LC retention time of compound C3: 4.443 minutes (HPLC analysis conditions: method 1)
LCMS (ESI) retention time of compound C3: 4.339 minutes, m/z = 481.26 $[M+H]^+$ (LCMS analysis conditions: method 1)

Example C-4

Synthesis of compound C4: tert-butyl (2S)-2-[ethyl-[(2S)-2-(methylamino)butanoyl]amino]-3-(p-tolyl)propanoate

[Formula 122]

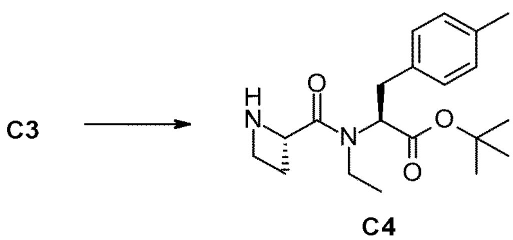

The residue (6.93 g) containing compound C3 obtained in Example C-3 was added to a reaction vessel. Next, 2-MeTHF (44 mL) and 5% Pd/C (3.08 g, 50% aqueous content) were sequentially added at room temperature. The inside of the reaction vessel was replaced with hydrogen, and the mixture was stirred at room temperature for 2 hours. The inside of the reaction vessel was replaced again with hydrogen, and the mixture was stirred at room temperature for an additional 2 hours. The reaction mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF (20

mL × 2). The resulting filtrate and the wash solution were combined and concentrated under reduced pressure at the external temperature of 40°C to obtain a residue (4.88 g) containing compound C4.
LC retention time of compound C4: 2.918 minutes (HPLC analysis conditions: method 1)

Example C-5

Synthesis of compound C5: tert-butyl (2S)-2-[[(2S)-2-[[(2S)-2-[benzyloxycarbonyl(methyl)amino]propanoyl]-methyl-amino]butanoyl]-ethyl-amino]-3-(p-tolyl)propanoate

[Formula 123]

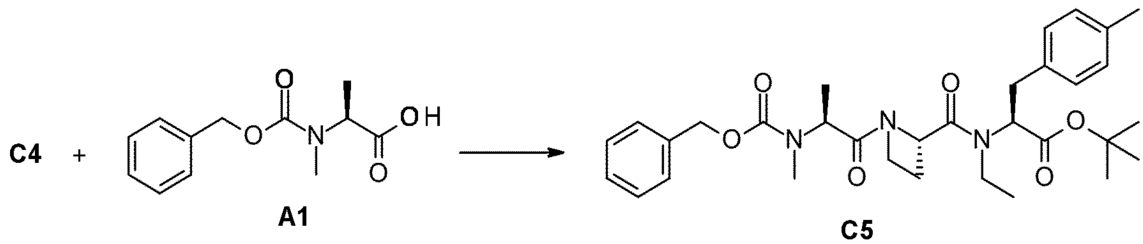

The residue (4.87 g) containing compound C4 obtained in Example C-4 and compound A1 (4.21 g) were added to a reaction vessel, and the reaction vessel was subjected to nitrogen replacement. Next, 2-MeTHF (43 mL), DIPEA (11.2 mL), and T3P (50 w/w% 2-MeTHF solution, 22.2 mL) were sequentially added, and then the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). 5% aqueous sodium carbonate solution (50 mL) was added, and the mixture was stirred for 10 minutes. After the aqueous layer was discharged, the resulting organic layer was washed with 5% aqueous sodium carbonate solution (50 mL × 2), 5% aqueous sodium hydrogen sulfate monohydrate solution (50 mL × 2), and again 5% aqueous sodium carbonate solution (50 mL × 5). The resulting organic layer was dehydrated with sodium sulfate and filtered to remove sodium sulfate. The resulting filtrate was concentrated under reduced pressure at the external temperature of 40°C to obtain a residue (3.64 g) containing compound C5.

LC retention time of compound C5: 4.445 minutes (HPLC analysis conditions: method 1)
LCMS (ESI) retention time of compound C5: 4.374 minutes, m/z = 566.25 $[M+H]^+$ (LCMS analysis conditions: method 1)

Example C-6

Synthesis of compound C6: tert-butyl (2S)-2-[methyl-[(2S)-2-[methyl-[(2S)-2-(methylamino)propanoyl]mino]butanoyl] amino]-3-(p-tolyl)propanoate

[Formula 124]

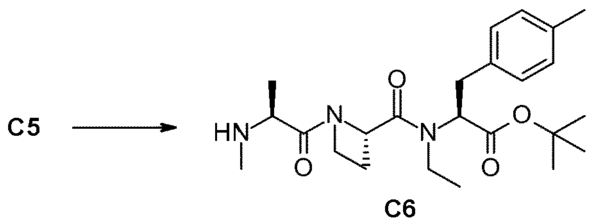

The residue (3.13 g) containing compound C5 obtained in Example C-5 was added to a reaction vessel. Next, 2-MeTHF (17 mL) and 5% Pd/C (1.19 g, 50% aqueous content) were sequentially added at room temperature. The inside of the reaction vessel was replaced with hydrogen, and the mixture was stirred at room temperature for 2 hours. The reaction

mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF (8.0 mL × 2). The resulting filtrate and the wash solution were combined and concentrated under reduced pressure at the external temperature of 40°C to obtain a residue (2.62 g) containing compound C6.
LC retention time of compound C6: 2.892 minutes (HPLC analysis conditions: method 1)

Example C-7

Synthesis of compound C7: tert-butyl (2S)-2-[[(2S)-1-[(2S)-2-[[(2S,3S)-2-(benzyloxycarbonylamino)-3-methyl-pentanoyl]-methyl-amino]propanoyl]azetidine-2-carbonyl]-ethyl-amino]-3-(p-tolyl)propanoate

[Formula 125]

C6 +

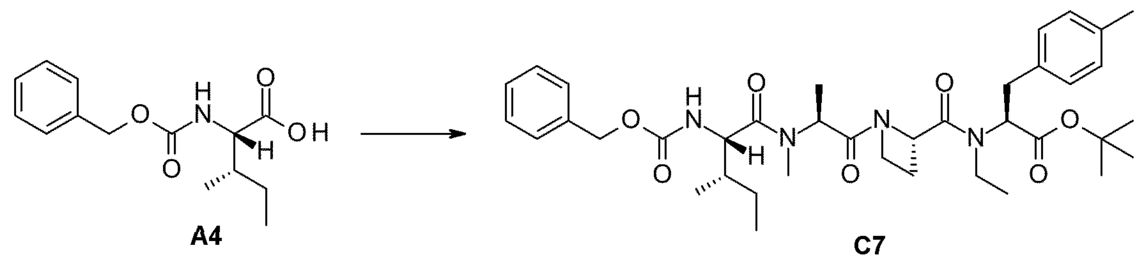

A4

C7

The residue (2.61 g) containing compound C6 obtained in Example C-6 and compound A4 (1.91 g) were added to a reaction vessel, and the reaction vessel was subjected to nitrogen replacement. Next, 2-MeTHF (9.2 mL), toluene (9.7 mL), MeCN (1.6 mL), and DIPEA (6.39 mL) were sequentially added at room temperature. HATU (3.60 g) was added with stirring, and then the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). N-methylimidazole (0.5 mL) was added to the reaction vessel, and 5% aqueous sodium carbonate solution (20 mL) was further added with stirring, and then the mixture was stirred for 1 hour. Next, 2.5% aqueous ammonia solution (20 mL) was added, and the mixture was stirred for 15 minutes. After the aqueous layer was discharged, the resulting organic layer was washed with 2.5% aqueous ammonia solution (30 mL), 5% aqueous sodium hydrogen sulfate monohydrate solution (30 mL × 2), and 3% aqueous dipotassium hydrogen phosphate solution (30 mL). The resulting organic layer was dehydrated with sodium sulfate and filtered to remove sodium sulfate. The resulting filtrate was concentrated under reduced pressure at the external temperature of 40°C to obtain a residue (4.14 g) containing compound C7.

LC retention time of compound C7: 4.701 minutes (HPLC analysis conditions: method 1)
LCMS (ESI) retention time of compound C7: 4.226 minutes, m/z = 679.47 $[M+H]^+$ (LCMS analysis conditions: method 2)

Example C-8

Synthesis of compound C8: tert-butyl (2S)-2-[ethyl-[(2S)-1-[(2S)-2-[methyl-[(2S,3S)-3-methyl-2-[1(2S)-4-methyl-2-[methyl(2-trimethylsilylethoxycarbonyl)amino]pentanoyl]amino]pentanoyl]amino]propanoyl]azetidine-2-carbonyl]amino]-3-(p-tolyl)propanoate

[Formula 126]

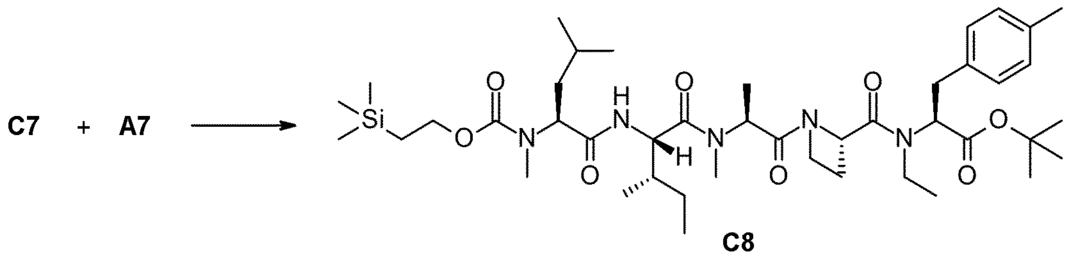

C8

The residue (3.15 g) containing compound C7 obtained in Example C-7 and isopropyl acetate (5.4 mL) were added to a reaction vessel. Next, the residue (2.54 g) containing compound A7 obtained in Example A-4, toluene (1.5 mL), acetone (15 mL), NMM (3.06 mL), and 5% Pd/C (1.00 g, 50% aqueous content) were sequentially added at room temperature. The inside of the reaction vessel was replaced with hydrogen, and the mixture was stirred at room temperature for 2 hours. The reaction mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, and then Pd/C was filtered under reduced pressure using filter paper and a filter. The reaction vessel, and the filter paper and the filter were washed with 2-MeTHF (50 mL). The resulting filtrate and the wash solution were combined and concentrated under reduced pressure at the external temperature of 40°C. The resulting residue was dissolved in MeTHF (40 mL) and 5% potassium carbonate (40 mL) and DMAP (586 mg) were added with stirring, and then the mixture was stirred for 2.5 hours. Next, 5% aqueous potassium hydrogen sulfate solution (40 mL) was added, and the mixture was stirred for 30 minutes. After the aqueous layer was discharged, the resulting organic layer was washed with 5% aqueous potassium hydrogen sulfate solution (40 mL) and 5% aqueous potassium carbonate solution (40 mL × 3). The resulting organic layer was dehydrated with sodium sulfate and filtered to remove sodium sulfate. The resulting filtrate was concentrated under reduced pressure at the external temperature of 40°C to obtain a residue (2.94 g) containing compound C8.

LC retention time of compound C8: 5.566 minutes (HPLC analysis conditions: method 1)
LCMS (ESI) retention time of compound C8: 5.337 minutes, m/z = 816.43 $[M+H]^+$ (LCMS analysis conditions: method 1)

Example C-9

Synthesis of compound C9: tert-butyl (2S)-2-[ethyl-[(2S)-1-[(2S)-2-[methyl-[(2S,3S)-3-methyl-2-[[(2S)-4-methyl-2-(methylamino)pentanoyl]amino]pentanoyl]amino]propanoyl]azetidine-2-carbonyl]amino]-3-(p-tolyl)propanoate

[Formula 127]

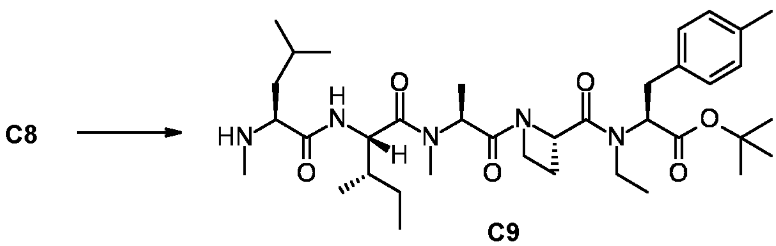

C9

The residue (2.21 g) containing compound C8 obtained in Example C-8, and 2-MeTHF (5.0 mL) were added at room temperature to a reaction vessel, and the reaction vessel was subjected to nitrogen replacement. Next, the external temperature of the reaction vessel was set to 50°C, and 1 M TBAF (6.76 mL) was added with stirring, and then the mixture was stirred at the external temperature of 50°C for 2 hours. The reaction mixture was sampled and prepared as a sample (sample preparation method 1), and it was confirmed by HPLC analysis that the reaction conversion rate was 99.9% or more (calculation expression 1 of reaction conversion rate). The external temperature setting was set to 0°C, and IPAc (10 mL) and 5% aqueous potassium carbonate solution (10 mL) were added with stirring, and then stirred at room temperature for 15 minutes. After the aqueous layer was discharged, the resulting organic layer was washed with 5% aqueous potassium carbonate solution (10 mL × 2). The resulting organic layer was dehydrated with sodium sulfate and filtered to

remove sodium sulfate. The resultant was concentrated under reduced pressure at the external temperature of 40°C to obtain a residue (1.71 g) containing compound C9.

LC retention time of compound C9: 3.582 minutes (HPLC analysis conditions: method 1)
LCMS (ESI) retention time of compound C9: 3.249 minutes, m/z = 672.49 $[M+H]^+$ (LCMS analysis conditions: method 1)

Example C-10

Synthesis of compound C10: tert-butyl (2S)-2-[[(2S)-1-[(2S)-2-[[(2S,3S)-2-[[(25)-2-[[(3S)-3-[[(2S)-2-[[1-[[(2S)-1-[(2S)-2-(benzyloxycarbonylamino)-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methylpentanoyl]-methyl-amino]propanoyl]azetidine-2-carbonyl]-ethyl-amino]-3-(p-tolyl)propanoate

[Formula 128]

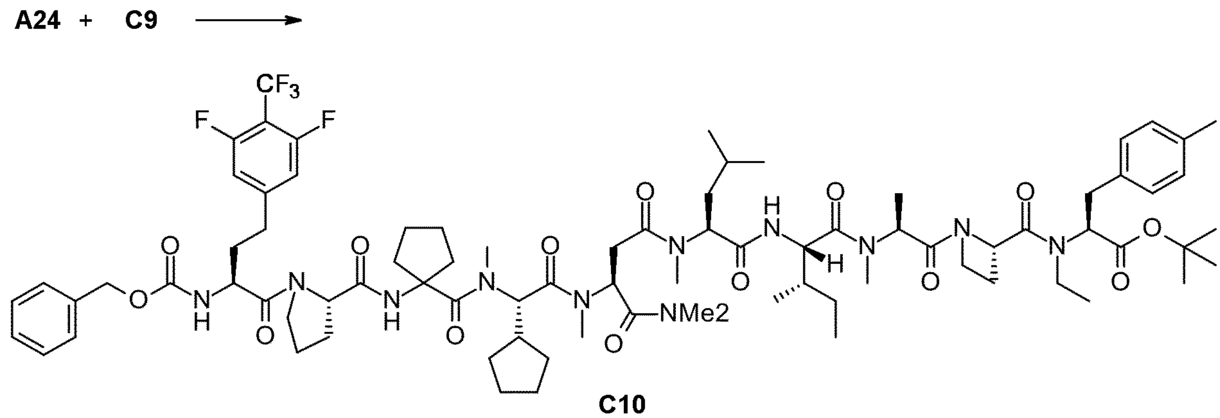

C10

The solution (4.01 g) containing compound A24 obtained in Example A-16 was added to a reaction vessel. The residue (1.34 g) containing compound C9 obtained in Example C-9, 2-MeTHF (36 mL), DMF (9.2 mL), and DIPEA (1.77 mL) were sequentially added at room temperature, and the reaction vessel was subjected to nitrogen replacement. Next, HATU (1.59 g) was added with stirring, and then the mixture was stirred at room temperature for 20 hours. Subsequently, the residue (0.15 g) containing compound C9 obtained in Example C-9 and HATU (0.83 g) were added, and the mixture was stirred for 3 hours. The reaction mixture was sampled and prepared as a sample (sample preparation method 1), and it was confirmed by HPLC analysis that the reaction conversion rate was 98.1% (calculation expression 1 of reaction conversion rate). To the reaction vessel, 2.5% aqueous ammonia solution (32 mL) was added, and the mixture was stirred for 30 minutes. After the aqueous layer was discharged, the resulting organic layer was washed with 10% aqueous sodium hydrogen sulfate monohydrate solution (28 mL × 3) and 5% aqueous potassium carbonate solution (28 mL × 2). The resulting organic layer was dehydrated with sodium sulfate and filtered to remove sodium sulfate. The resulting filtrate was concentrated under reduced pressure at the external temperature of 40°C, and the resulting residue was purified by silica gel column chromatography (eluent: ethyl acetate/heptane 80 : 20 to 100 : 0). The eluate containing the target material was concentrated under reduced pressure at 40°C, and the eluent was removed to obtain a residue (1.97 g) containing compound C10.

LC retention time of compound C10: 22.077 minutes (HPLC analysis conditions: method 3)
LCMS (ESI) retention time of compound C10: 22.67 minutes, m/z = 1575.142 $[M+H]^+$ (LCMS analysis conditions: method 3)

Example C-11

Synthesis of compound C11: tert-butyl (2S)-2-[[(2S)-1-[(2S)-2-[[(2S,3S)-2-[[(2S)-2-[[(3S)-3-[[(2S)-2-[[1-[[(2S)-1-[(2S)-2-amino-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-tolyl)propanoate

[Formula 129]

C10

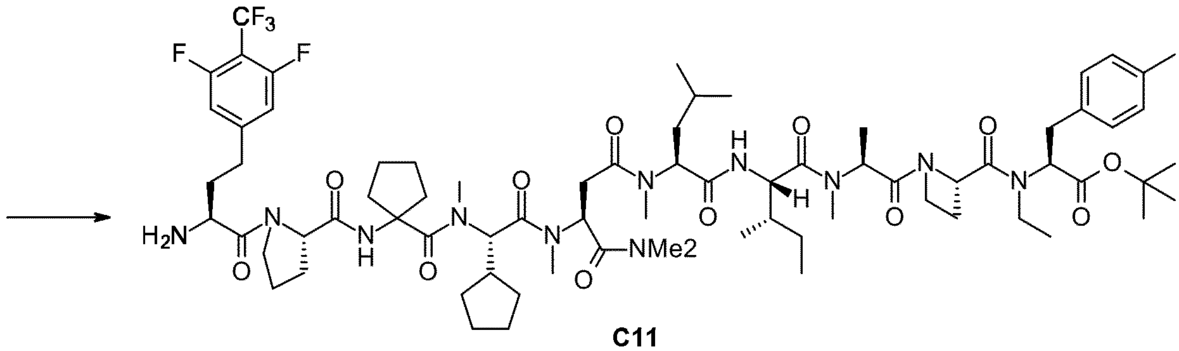

C11

The residue (1.44 g) containing compound C10 obtained in Example C-10 was added to a reaction vessel. Next, THF (15 mL) and 5% Pd/C (211 mg, 50% aqueous content) were sequentially added at room temperature. The inside of the reaction vessel was replaced with hydrogen, and the mixture was stirred at room temperature. The inside of the reaction vessel was replaced again with hydrogen after 2 and 4 hours, 5% Pd/C (205 mg, 50% aqueous content) was added after 5 hours, and the mixture was stirred for an additional 6 hours. The reaction mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with THF (10 mL × 2). The resulting filtrate and the wash solution were combined and concentrated under reduced pressure at the external temperature of 40°C to obtain a residue (1.42 g) containing compound C11.
LC retention time of compound C11: 4.628 minutes (HPLC analysis conditions: method 2)

Example C-12

Synthesis of compound C13: tert-butyl (2S)-2-[[(2S)-1-[(2S)-2-[[(2S,3S)-2-[[(2S)-2-[[(3S)-3-[[(2S)-2-[[1-[[(2S)-1-[(2S)-2-[[2-[benzyloxycarbonyl(methyl)aminolacetyl]amino]-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-tolyl)propanoate

[Formula 130]

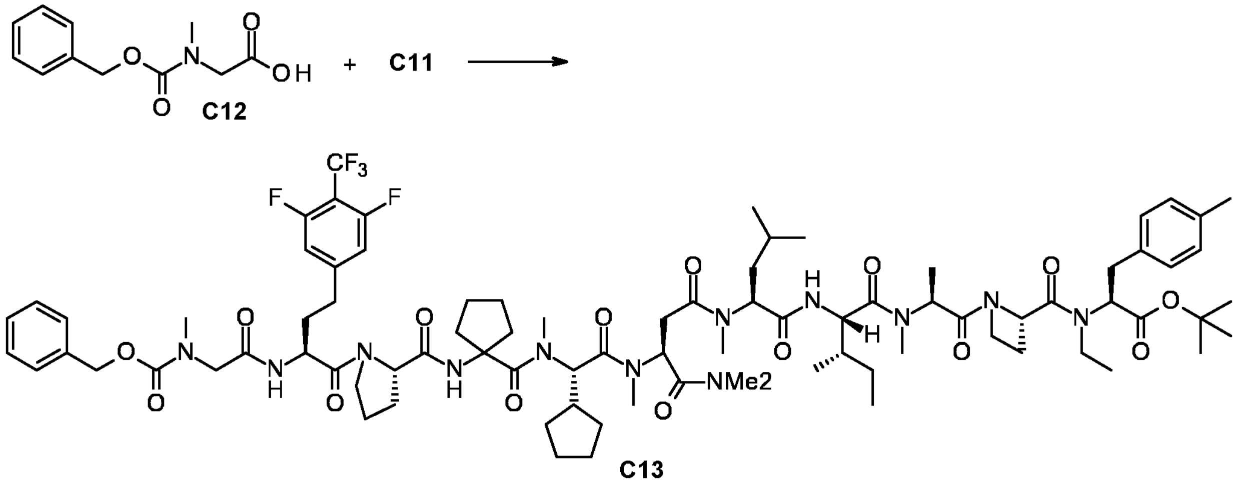

C12 + C11

C13

The residue (1.42 g) containing compound C11 obtained in Example C-11 and compound C12 (0.271 g) were added to a reaction vessel. 2-MeTHF (8.6 mL), DMF (2.9 mL), and DIPEA (0.964 mL) were sequentially added at room temperature, and the reaction vessel was subjected to nitrogen replacement. Next, HATU (0.863 g) was added with stirring, and then the mixture was stirred at room temperature for 3 hours. The reaction mixture was sampled and prepared as a sample (sample preparation method 1), and it was confirmed by HPLC analysis that the reaction conversion rate was 97.2% (calculation expression 1 of reaction conversion rate). To the reaction vessel, 2.5% aqueous ammonia solution (10 mL) was added, and the mixture was stirred for 15 minutes. After the aqueous layer was discharged, the resulting organic layer was washed with 10% aqueous sodium hydrogen sulfate monohydrate solution (10 mL × 3) and 5% aqueous potassium carbonate solution (10 mL × 2). The resulting organic layer was dehydrated with sodium sulfate and filtered to remove sodium sulfate. The resulting filtrate was concentrated under reduced pressure at the external temperature of 40°C, and the resulting residue was purified by silica gel column chromatography (eluent: ethyl acetate/methanol 100 : 0 to 90 : 10). The eluate containing the target material was concentrated under reduced pressure at the external temperature of 40°C, and the eluent was removed to obtain a residue (1.04 g) containing compound C13.

LC retention time of compound C13: 21.380 minutes (HPLC analysis conditions: method 3)
LCMS (ESI) retention time of compound C13: 22.04 minutes, m/z = 1668.111 $[M+Na]^+$ (LCMS analysis conditions: method 3)

Example C-13

Synthesis of compound C14: (2S)-2-[[(2S)-1-[(2S)-2-[[(2S,3S)-2-[[(2S)-2-[[(3S)-3-[[(2S)-2-[[1-[[(2S)-1-[(2S)-2-[[2-[benzyloxycarbonyl(methyl)amino]acetyl]amino]-4-[3,5-difluoro-4-(trifluoromethyl)phenyl]butanoyl]pyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-tolyl)propanoic acid

[Formula 131]

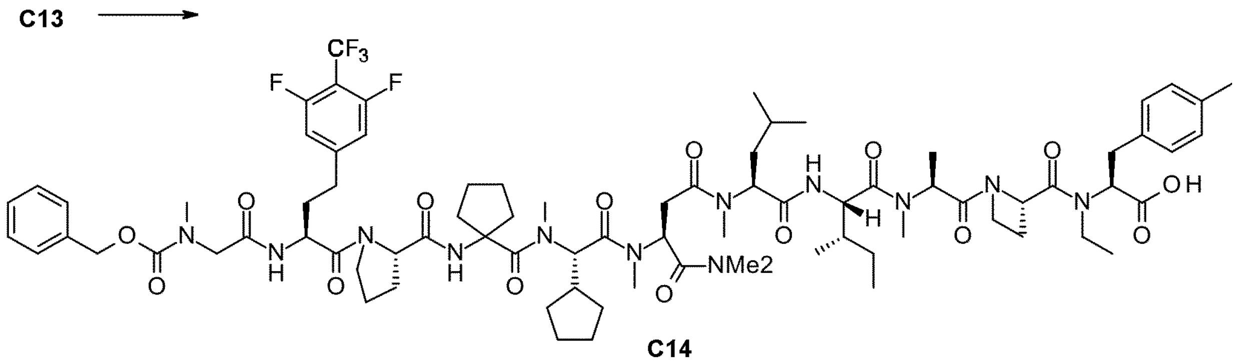

C14

The residue (0.895 g) containing compound C13 obtained in Example C-12 was added to a reaction vessel. 2-MeTHF (9.0 mL) and HMDS (0.570 mL) were sequentially added at room temperature, and the reaction vessel was subjected to nitrogen replacement. Next, the external temperature of the reaction vessel was set to 0°C, and TMSOTf (0.393 mL) was added with stirring, and then the mixture was stirred at room temperature for 2 hours. The external temperature was set again to 0°C, and HMDS (0.570 mL) and TMSOTf (0.393 mL) were added with stirring, and then the mixture was stirred at room temperature for 5 hours. The reaction mixture was sampled and prepared as a sample (sample preparation method 1), and it was confirmed by HPLC analysis that the reaction conversion rate was 99.0% (calculation expression 1 of reaction conversion rate). The external temperature was set to 0°C, and 5% aqueous sodium hydrogen carbonate solution (15 mL) was added to the reaction vessel, and the mixture was stirred at room temperature for 30 minutes. The aqueous layer was discharged, and then the resulting organic layer was washed with 5% aqueous sodium dihydrogen phosphate solution (15 mL) and 10% aqueous NaCl solution (15 mL). The resulting organic layer was dehydrated with sodium sulfate and filtered to remove sodium sulfate. The resulting filtrate was concentrated under reduced pressure at the external temperature of 40°C to obtain a residue (0.874 g) containing compound C14.
LC retention time of compound C14: 18.926 minutes (HPLC analysis conditions: method 3)

Example C-14

Synthesis of compound C15: (2S)-2-[[(2S)-1-[(2S)-2-[[(2S,3S)-2-[[(2S)-2-[[(3S)-3-[[(2S)-2-cyclopentyl-2-[[1-[[(2S)-1-[(2S)-4-[3,5-difluoro-4-(trifluoromethyl)phenyl]-2-[[2-(methylamino)acetyl]amino]butanoyl]pyrrolidine-2-carbonyl]amino]cyclopentanecarbonyl]-methyl-amino]acetyl]-methyl-amino]-4-(dimethylamino)-4-oxo-butanoyl]-methyl-amino]-4-methyl-pentanoyl]amino]-3-methyl-pentanoyl]methyl-amino]propanoyl]azetidine-2-carbonyl]ethyl-amino]-3-(p-tolyl)propanoic acid

[Formula 132]

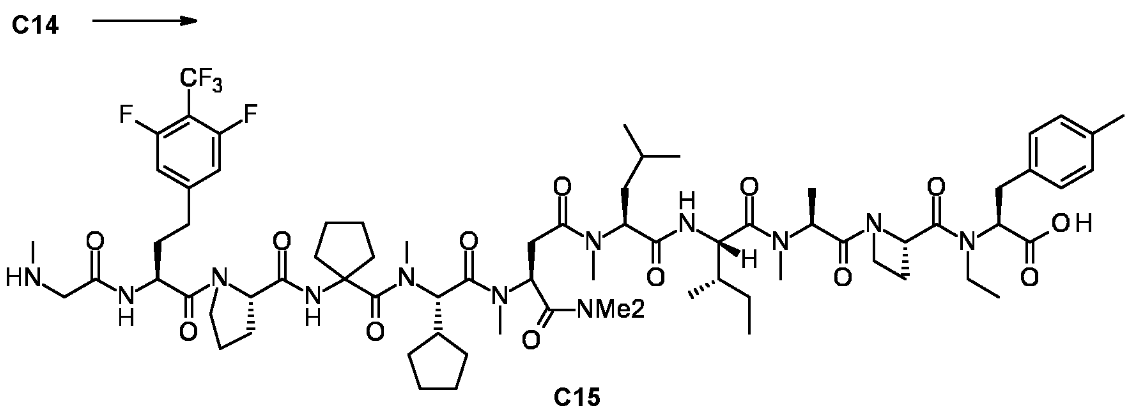

C15

The residue (0.875 g) containing compound C14 obtained in Example C-13 was added to a reaction vessel. Next, THF (6.0 mL) and 5% Pd/C (237 mg, 50% aqueous content) were sequentially added at room temperature. The inside of the reaction vessel was replaced with hydrogen, and the mixture was stirred at room temperature for 2 hours. The reaction

mixture was sampled and prepared as a sample (sample preparation method 1), and it was confirmed by HPLC analysis that the reaction conversion rate was 99.9% or more (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with THF (3.0 mL). The resulting filtrate and the wash solution were combined and concentrated under reduced pressure at the external temperature of 40°C. The resulting residue was purified by silica gel column chromatography (eluent: dichloromethane/methanol 100 : 0 to 70 : 30). The eluate containing the target material was concentrated under reduced pressure at the external temperature of 40°C, and the eluent was removed to obtain a residue (0.452 g) containing compound C15.

LC retention time of compound C15: 13.418 minutes (HPLC analysis conditions: method 3)
LCMS (ESI) retention time of compound C15: 13.93 minutes, m/z = 1456.181 $[M+H]^+$ (LCMS analysis conditions: method 3)

Example C-15

Synthesis of compound C16: (2S)-2-[[(2S)-2-[benzyloxycarbonyl(methyl)amino]butanoyl]-ethyl-amino]-3-(p-tolyl)propanoic acid

[Formula 133]

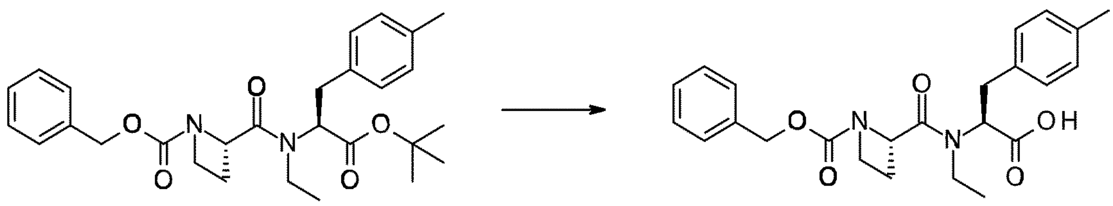

C3 C16

Compound C3 (7.35 g, content 89.84%) was added to a reaction vessel. 2-MeTHF (66 mL) and HMDS (14.40 mL) were sequentially added at room temperature, and the reaction vessel was subjected to nitrogen replacement. Next, the external temperature of the reaction vessel was set to 0°C, and TMSOTf (10.13 mL) was added with stirring, and then the mixture was stirred at 0°C for 1 hour, and further stirred at 15°C for 6 hours. The external temperature was set to 0°C, and 5% aqueous sodium hydrogen carbonate solution (66 mL) was added to the reaction vessel, and the mixture was stirred at room temperature for 2 hours and the aqueous layer was discharged. To the aqueous layer, 1 N aqueous hydrochloric acid solution (33 mL) was added, and 2 N aqueous hydrochloric acid solution (9.9 mL) was further added. The aqueous layer was then extracted with 2-MeTHF (66 mL). The resulting organic layer was concentrated under reduced pressure at the external temperature of 40°C. The resulting residue was purified by silica gel column chromatography (eluent: dichloromethane/methanol 100 : 0 to 80 : 20). The resultant was concentrated under reduced pressure at the external temperature of 40°C, and the eluent was removed to obtain a residue containing compound C16. Furthermore, the obtained residue was purified by silica gel column chromatography (eluent: ethyl acetate/methanol 95 : 5 to 90 : 10, then dichloromethane/methanol 90 : 10 to 80 : 20). The eluate containing the target material was concentrated under reduced pressure at the external temperature of 40°C, and the eluent was removed to obtain a residue containing compound C16. The amount of the obtained compound C16 was 4.25 g (content 76.03%, yield 55%).

LC retention time of compound C16: 3.501 minutes (HPLC analysis conditions: method 4)
LCMS(ESI) of compound C16: retention time: 4.588 minutes, m/z = 425.2 $[M+H]^+$ (LCMS analysis conditions: method 4)

Example C-16-1

Synthesis of compound A31: benzyl (2S)-2-[[(1S)-2-[(2-tert-butoxy-2-oxo-ethyl)-methyl-amino]-2-oxo-1-(p-tolylmethyl) ethyl]-ethyl-carbamoyl]azetidine-1-carboxylate

[Formula 134]

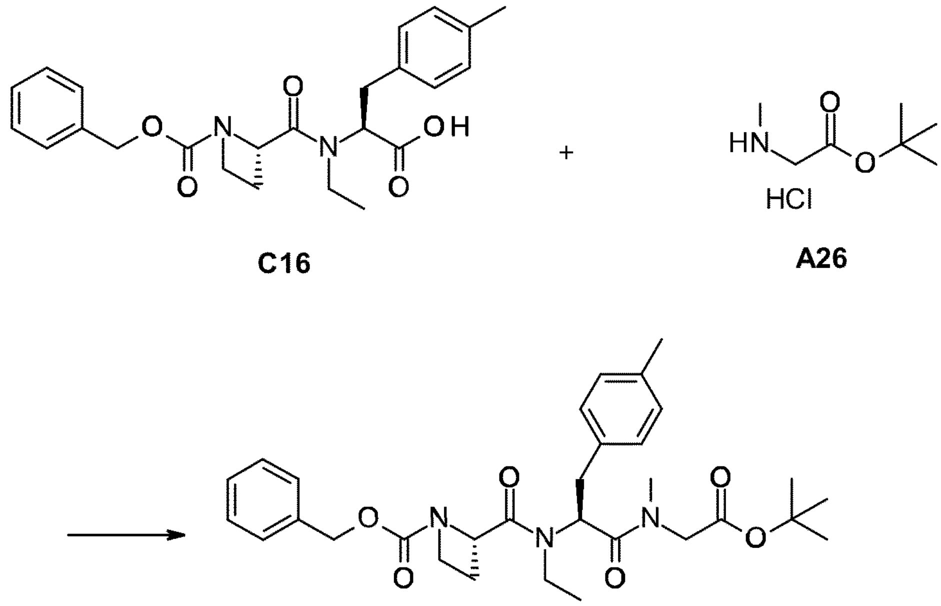

C16

A26

A31

Compound C16 (27.11 mg, content 76.03%) and compound A26 (9.18 mg) were added to the reaction vessel, and the reaction vessel was subjected to nitrogen replacement. Next, dimethyl carbonate (0.10 mL) and DIPEA (47.4 μL) were sequentially added. HATU (72.24 mg) was added with stirring at the external temperature of 0°C, and then the mixture was stirred at 25°C for 4 hours. As a result of HPLC analysis of the reaction solution using a standard preparation, the amount of the obtained compound A31 was 24.91 mg (yield: 93%).

LC retention time of compound A31: 4.217 minutes (HPLC analysis conditions: method 4)
Chiral LC retention time of compound A31: 15.605 minutes (HPLC analysis conditions: method 9)
LCMS (ESI) retention time of compound A31: 5.380 minutes, m/z = 552.2 $[M+H]^+$ (LCMS analysis conditions: method 4)

Elongation reaction of compound C16 and compound A26 (amino acid residue at cyclized position C)

The results of the synthesis of compound A31 using HATU or COMU as the condensation reagent, and dimethyl carbonate or MeCN as the solvent are shown in Table 5. The experimental operation was in accordance with the case in which HATU was used as the condensation reagent, and dimethyl carbonate was used as the solvent (Example C-16-1). TM in the table is a target material (compound A31). The production amount of the target material (TM) and epimer was shown as Area% ratio in the HPLC measurement (HPLC analysis condition: method 9). The yield was calculated by HPLC analysis using a standard preparation.

[Table 5]

| Example No. | Condensation reagent (equivalent) | Base (equivalent) | Solvent (v/w) | Equivalents of C-terminus and N-terminus | | Reaction time (h) | Conversion rate (%) | TM / epimer (%) | | Yield (%) |
|---|---|---|---|---|---|---|---|---|---|---|
| | | | | C16 | A26 | | | TM | Epimer | |
| C-16-1 | HATU (3.8) | DIPEA (5.6) | dimethyl carbonate (5) | 1.0 | 1.0 | 2 | 93.81 | 96.87 | 3.13 | 93.0 |
| C-16-2 | HATU (3.8) | DIPEA (5.6) | dimethyl carbonate (200) | 1.0 | 1.0 | 2 | 81.60 | 90.34 | 9.66 | 92.9 |
| C-16-3 | HATU (3.8) | DIPEA (5.6) | MeCN (5) | 1.0 | 1.0 | 2 | 93.63 | 98.86 | 1.14 | 91.8 |
| C-16-4 | HATU (3.8) | DIPEA (5.6) | MeCN (200) | 1.0 | 1.0 | 2 | 93.72 | 95.43 | 4.57 | 85.0 |
| C-16-5 | COMU (3.8) | DIPEA (5.6) | dimethyl carbonate (5) | 1.0 | 1.0 | 2 | 92.34 | 98.77 | 1.23 | 73.3 |
| C-16-6 | COMU (3.8) | DIPEA (5.6) | dimethyl carbonate (200) | 1.0 | 1.0 | 2 | 83.48 | 91.23 | 8.77 | 77.7 |

Example D-1

Compound D1: Synthesis of N-benzyloxycarbonyl-L-proline tert-butyl

[Formula 135]

A19

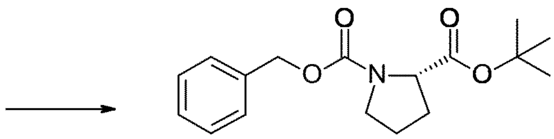

D1

Compound A19 (4.03 g) was added to a reaction vessel. Next, dichloromethane (20 mL) and cyclohexane (20 mL) were added to the reaction vessel at room temperature. After tert-butyl 2,2,2-trichloroacetimidate (5.8 mL) and boron trifluoride diethyl ether complex (0.33 mL) were added, the mixture was stirred at room temperature for 3 hours. The reaction mixture was sampled and prepared as a sample (sample preparation method 1) and subjected to HPLC analysis which confirmed that the reaction conversion rate was 80.3% (calculation expression 1 of reaction conversion rate). After tert-butyl 2,2,2-trichloroacetimidate (1.2 mL) and boron trifluoride diethyl ether complex (0.33 mL) were added, the mixture was stirred at room temperature for 0.5 hours, and the reaction solution was stood still for 16 hours, then concentrated under reduced pressure to remove dichloromethane. The resulting slurry was filtered and the solid residue was washed with cyclohexane. The resulting filtrate was washed with 10% aqueous citric acid solution (10 mL × 5), 5% aqueous sodium carbonate solution (20 mL × 3), and saturated aqueous NaCl solution (10 mL). The resulting organic layer was dehydrated with sodium sulfate and filtered to remove sodium sulfate, and then the resulting filtrate was concentrated under reduced pressure at the external temperature of 40°C to obtain 4.89 g of the crude product of compound D1.

LC retention time of compound D1: 3.695 minutes (HPLC analysis conditions: method 1)
LCMS (ESI) retention time of compound D1: 3.815 minutes, m/z = 328 $[M+Na]^+$ (LCMS analysis conditions: method 1)

Example D-2

Compound D2: Synthesis of L-proline tert-butyl

[Formula 136]

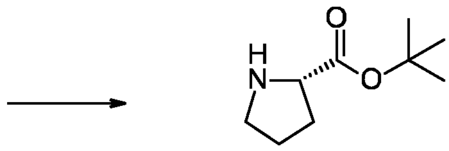

D1

D2

The compound D1 (2.22 g) obtained in Example D-1 was added to a reaction vessel. Next, 2-MeTHF (22 mL) and 5% Pd/C (1.08 g, 50% aqueous content) were sequentially added at room temperature. The inside of the reaction vessel was replaced with hydrogen, and the mixture was stirred at room temperature for 2 hours. The reaction mixture was sampled and prepared as a sample (sample preparation method 1) and subjected to HPLC analysis which confirmed that the reaction conversion rate was 99.9% or more (calculation expression 2 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF. The resulting solution was concentrated under reduced pressure to obtain a residue (1.27 g) containing compound D2.
LC retention time of compound D2: 1.974 minutes (HPLC analysis conditions: method 1)

Example D-3

Compound B20: Synthesis of tert-butyl (2S)-1-[(2S)-2-(benzyloxycarbonylamino)-4-[3,5-difluoro-4-(trifluoromethyl)phenyl]butanoyl]pyrrolidine-2-carboxylate

[Formula 137]

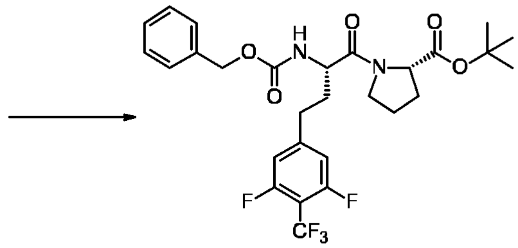

A22 + D2

B20

The residue containing compound D2 obtained in Example D-2 and compound A22 (5.16 g) were added at room temperature to a reaction vessel, and the reaction vessel was subjected to nitrogen replacement. Next, 2-MeTHF (33 mL), DIPEA (5.5 mL), and T3P (10.8 mL) were added, and then the mixture was stirred at room temperature for 2 hours. The reaction mixture was sampled and prepared as a sample (sample preparation method 5) and subjected to HPLC analysis which confirmed that the reaction conversion rate was 99.9% or more (calculation expression 1 of reaction conversion rate). 5% aqueous potassium carbonate solution (12 mL) was added with stirring, then N-methylimidazole (0.57 mL) was further added. The mixture was stirred for 2 hours and 30 minutes, and then an aqueous layer was discharged. The resulting organic layer was washed with 5% aqueous potassium carbonate solution (12 mL × 2), 5% aqueous sodium hydrogen sulfate monohydrate solution (18 mL × 3), and a mixed solution of 5% aqueous potassium carbonate solution and acetonitrile (volume ratio 2.4 : 1, 17 mL × 3). Heptane (30 mL) was added to the resultant organic layer, which was then washed with a mixed solution of 5% aqueous potassium carbonate and acetonitrile (volume ratio 1.2 : 1, 22 mL × 3). MTBE (10 mL) was added to the resultant organic layer, which was then washed with a mixed solution of 5% aqueous potassium carbonate and acetonitrile (volume ratio 1.2 : 1, 22 mL × 8). The resulting organic layer was dehydrated with sodium sulfate and filtered to remove sodium sulfate, and then the resulting filtrate was concentrated under reduced pressure at the external temperature of 40°C. The resulting residue was purified by silica gel column chromatography (eluent: heptane/ethyl acetate 92 : 8 to 48 : 52) to obtain 2.63 g of compound B20.

LC retention time of compound B20: 4.481 minutes (HPLC analysis conditions: method 1)
LCMS (ESI) retention time of compound B20: 4.547 minutes, m/z = 571 $[M+H]^+$ (LCMS analysis conditions: method 1)

Example D-4

Compound B21: Synthesis of tert-butyl (2S)-1-[(2S)-2-amino-4-[3,5-difluoro-4-(trifluoromethyl)phenyl]butanoyl]pyrrolidine-2-carboxylate

[Formula 138]

B20

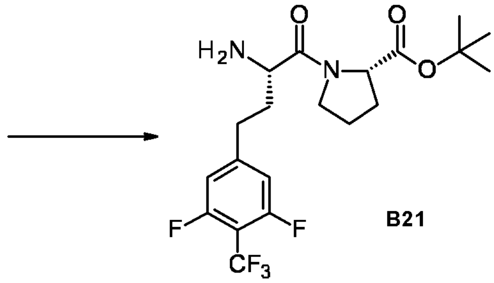

B21

The compound B20 (1.0 g) obtained in Example D-3 was added to a reaction vessel. Next, 2-MeTHF (8 mL) and 5% Pd/C (0.29 g, 50% aqueous content) were sequentially added at room temperature. The inside of the reaction vessel was replaced with hydrogen, and the mixture was stirred at room temperature for 1.5 hours. The reaction mixture was sampled and prepared as a sample (sample preparation method 1) and subjected to HPLC analysis which confirmed that the reaction conversion rate was 99.9% or more (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF. The resulting solution was concentrated under reduced pressure and then diluted with 2-MeTHF to obtain a solution containing compound B21.

LC retention time of compound B21: 3.107 minutes (HPLC analysis conditions: method 1)
LCMS (ESI) retention time of compound B21: 2.990 minutes, m/z = 437 $[M+H]^+$ (LCMS analysis conditions: method 1)

Example D-5

Compound B25: Synthesis of tert-butyl (3S)-3-[[(2S)-2-[(1-(benzyloxycarbonylamino)cyclopentanecarbonyl)-methyl-amino]-2-cyclopentyl-acetyl]-methyl-amino]-4-(dimethylamino)-4-oxo-butanoate

[Formula 139]

A18

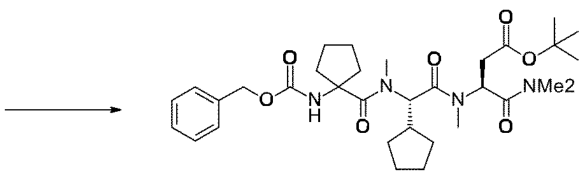

B25

The solution (3.61 g) containing compound A18 obtained in Example A-12 and DMF (10 mL) were added to a reaction vessel. The external temperature of the reaction vessel was set to 0°C, and N-carbobenzoxyoxysuccinimide (0.84 g) was added with stirring, and the mixture was stirred for an additional 6 hours and then stood still for 18 hours. The reaction mixture was sampled and prepared as a sample (sample preparation method 1) and subjected to HPLC analysis which confirmed that the reaction conversion rate was 99.0% (calculation expression 1 of reaction conversion rate). MeTHF (30 mL), MTBE (10 mL) and 5% aqueous sodium carbonate solution (15 mL) were added, and the mixture was stirred. After the underlayer was discharged, the resulting organic layer was washed with 5% aqueous sodium carbonate solution (15 mL × 3), 5% aqueous sodium hydrogen sulfate monohydrate solution (15 mL × 2), and saturated aqueous NaCl solution (15 mL). MeTHF (15 mL) was added to the organic layer, and the organic layer was washed with 5% aqueous sodium carbonate solution (15 mL). The obtained organic layer was dehydrated with sodium sulfate and filtered to remove

sodium sulfate. To the resulting filtrate, heptane was added, and then the resulting solid compound B25 was recovered by filtration. The amount of the obtained compound B25 was 1.61 g.

LC retention time of compound B25: 4.422 minutes (HPLC analysis conditions: method 1)
LCMS (ESI) retention time of compound B25: 4.340 minutes, m/z = 637 $[M+Na]^+$ (LCMS analysis conditions: method 1)

Example D-6

Compound B26: Synthesis of (3S)-3-[[(25)-2-[(1-(benzyloxycarbonylamino)cyclopentanecarbonyl)-methyl-amino]-2-cyclopentyl-acetyl]-methyl-amino]-4-(dimethylamino)-4-oxo-butanoic acid

[Formula 140]

B25

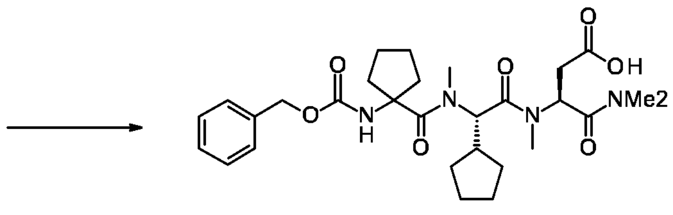

B26

The compound B25 (0.65 g) obtained in Example D-5 was added to a reaction vessel, and the reaction vessel was subjected to nitrogen replacement. Next, dichloromethane (5 mL), isopropyl acetate (6.5 mL) and HMDS (0.55 mL) were added to the reaction vessel. The external temperature of the reaction vessel was set to 0°C, and TMSOTf (0.38 mL) was added with stirring, and then the mixture was stirred for 2 hours. The reaction mixture was sampled and prepared as a sample (sample preparation method 1) and subjected to HPLC analysis which confirmed that the reaction conversion rate was 99.9% or more (calculation expression 1 of reaction conversion rate). Still at the external temperature of 0°C, MeTHF (10 mL), 5% aqueous dipotassium hydrogen phosphate solution (0.5 mL), and 5% aqueous sodium dihydrogen phosphate solution (3 mL) were sequentially added. After separating the organic layer and the aqueous layer, saturated aqueous NaCl solution (2 mL) was added to the aqueous layer, and the mixture was extracted with MeTHF (10 mL × 2). The resulting all organic layers were mixed and washed with saturated aqueous NaCl solution (5 mL). The resulting organic layer was dehydrated with sodium sulfate and filtered to remove sodium sulfate, and DIPEA (0.81 mL) was added. Then, the resulting solution was concentrated under reduced pressure at the external temperature of 40°C to obtain a residue containing compound B26.
LC retention time of compound B26: 3.459 minutes (HPLC analysis conditions: method 1)

Example D-7

Synthesis of compound A28: (tert-butyl 2-[[(2S)-2-[benzyloxycarbonyl(ethyl)amino]-3-(p-tolyl)propanoyl]-methyl-amino]acetate)

[Formula 141]

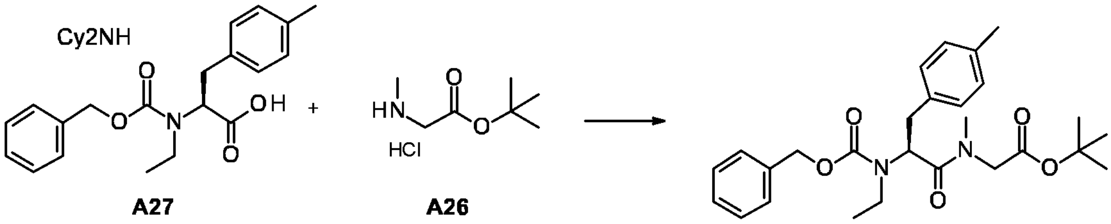

A27 A26 A28

To a reaction vessel after nitrogen replacement, compound A26 (270 g) and compound A27 (647 g), followed by 2-MeTHF (3.8 L), were added, and the mixture was stirred. The external temperature of the reaction vessel was set to 10°C and DIPEA (868 g) was added, and then T3P (50 w/w% 2-MeTHF solution, 1.89 kg) was added dropwise. The external temperature was set to 25°C, and the mixture was stirred for 5 hours. The reaction mixture was sampled and prepared as a sample (sample preparation method 2), and it was confirmed by HPLC analysis that the reaction conversion rate was 98.9% (calculation expression 3 of reaction conversion rate). The external temperature of the reaction vessel was set to 10°C, and 5% aqueous sodium carbonate solution (3.8 L) was added dropwise with stirring. The external temperature was set to 25°C, and the mixture was stirred for 30 minutes, and then the aqueous layer was discharged. The resulting organic layer was washed also at the external temperature of 25°C with 5% aqueous sodium hydrogen sulfate monohydrate solution (3.8 L) twice, and 5% aqueous sodium carbonate solution (3.8 L) once. The resulting organic layer was concentrated under reduced pressure at the external temperature of 40°C to obtain 1.30 kg of a solution containing compound A28.
LC retention time of compound A28: 4.500 minutes (HPLC analysis conditions: method 4)

Example D-8

Synthesis of compound A29: (tert-butyl 2-[[(2S)-2-fethylamino)-3-(p-tolyl)propanoyl]-methyl-amino]acetate)

[Formula 142]

A28

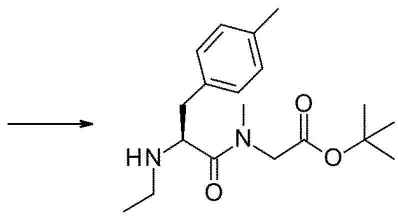

A29

To a reaction vessel after nitrogen replacement, the 2-MeTHF solution (1.28 kg) containing compound A28 obtained in Example D-7 and 2-MeTHF (2.8 L) were added, and then 5% Pd/C (258 g, 50% aqueous content) was added. The external temperature of the reaction vessel was set to 25°C, and the reaction vessel was pressurized with hydrogen until the internal pressure of the reaction vessel reached 0.18 MPaG. After 1 hour, no fluctuation in the internal pressure was confirmed, and then the reaction vessel was subjected to nitrogen replacement. The reaction mixture was sampled and prepared as a sample (sample preparation method 1), and it was confirmed by HPLC analysis that the reaction conversion rate was 99.9% (calculation expression 1 of reaction conversion rate). The reaction mixture was filtered under pressure. The reaction vessel and the filtering apparatus were washed with 2-MeTHF (1.8 L), and the filtrate and the wash solution were collected into a storage vessel as a storage solution. The resulting filtrate and the wash solution were concentrated under reduced pressure at the external temperature of 40°C to obtain a 2-MeTHF solution (955 g) containing compound A29.
LC retention time of compound A29: 2.389 minutes (HPLC analysis conditions: method 4)

Example D-9

Synthesis of compound A31: (benzyl (2S)-2-[[(1S)-2-[(2-tert-butoxy-2-oxo-ethyl)-methyl-amino]-2-oxo-1-(p-tolylmethyl)ethyl]-ethyl-carbamoyl]azetidine-1-carboxylate)

[Formula 143]

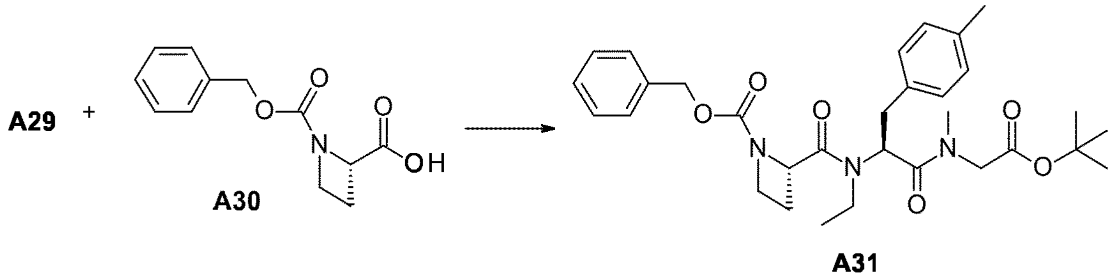

A29 + A30

A31

To a reaction vessel after nitrogen replacement, the 2-MeTHF solution (935 g) containing compound A29 obtained in Example D-8, compound A30 (420 g), and 2-MeTHF (3.0 L) were added with stirring. The external temperature of the reaction vessel was set to 10°C, DIPEA (770 g) was added with stirring, and then T3P (50 w/w% 2-MeTHF solution, 2.23 kg) was added dropwise. The external temperature of the reaction vessel was set to 25°C, and the reaction mixture was stirred for 2 hours. The reaction mixture was sampled and prepared as a sample (sample preparation method 1), and it was confirmed by HPLC analysis that the reaction conversion rate was 99.3% (calculation expression 1 of reaction conversion rate). The external temperature of the reaction vessel was set to 10°C, and 5% aqueous sodium carbonate solution (3.6 L) was added to the reaction mixture with stirring. The external temperature of the reaction vessel was set to 25°C and the mixture was stirred, and then the aqueous layer was discharged from the reaction vessel. The organic layer was washed with 5% aqueous sodium hydrogen sulfate monohydrate solution (3.6 L) twice, and then with 5% aqueous sodium carbonate solution (3.6 L). The resulting organic layer was concentrated under reduced pressure at the external temperature of 40°C to obtain 1.37 kg of a solution containing compound A31.
LC retention time of compound A31: 4.065 minutes (HPLC analysis conditions: method 4)

Example D-10

Synthesis of compound D3: (tert-butyl 2-[[(2S)-2-[[(2S)-azetidine-2-carbonyl-ethyl-amino]-3-(p-tolyl)propanoyl]-methyl-amino]acetate)

[Formula 144]

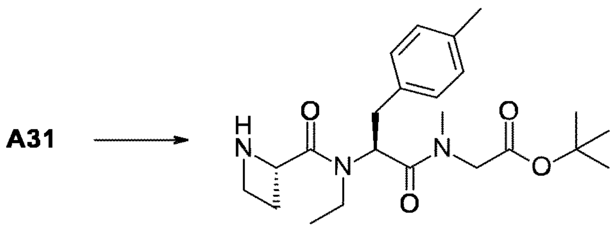

A31

D3

To a reaction vessel after nitrogen replacement, the 2-MeTHF solution (1.34 kg) containing compound A31 obtained in Example D-9 and 2-MeTHF (2.7 L) were added, and then 5% Pd/C (249 g, 50% aqueous content) was added. The external temperature of the reaction vessel was set to 25°C, and the reaction vessel was pressurized with hydrogen until the internal pressure of the reaction vessel reached 0.18 MPaG. After 40 hours, no fluctuation in the internal pressure was confirmed, and then the mixture was stirred for an additional 1 hour. The reaction mixture was sampled and prepared as a sample (sample preparation method 1), and it was confirmed by HPLC analysis that the reaction conversion rate was 99.7% (calculation expression 1 of reaction conversion rate). After replacing the inside of the reaction vessel with nitrogen, the reaction mixture was filtered under pressure. The reaction vessel and the filtering apparatus were washed with 2-MeTHF (1.8 L), and the filtrate and the wash solution were collected into a storage vessel as a storage solution. The resulting filtrate and the wash solution were concentrated under reduced pressure at the external temperature of 40°C to obtain a 2-MeTHF solution (1.28 kg) containing compound D3.
LC retention time of compound D3: 2.538 minutes (HPLC analysis conditions: method 4)

Example D-11

Synthesis of compound D4: (tert-butyl 2-[[(2S)-2-[[(2S)-1-[(2S)-2-[benzyloxycarbonyl(methyl)amino]propanoyl]azetidine-2-carbonyl]-ethyl-amino]-3-(p-tolyl)propanoyl]-methyl-amino]acetate)

[Formula 145]

D3 +

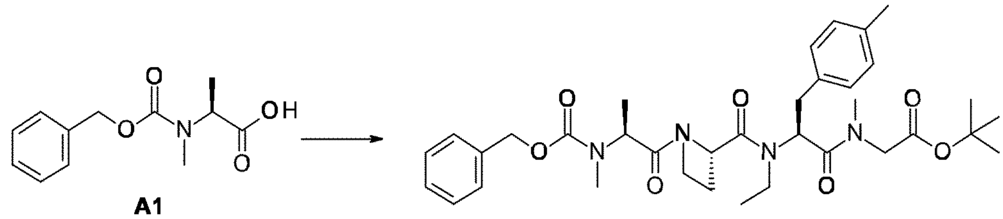

A1

D4

To a reaction vessel after nitrogen replacement, the 2-MeTHF solution (1.25 kg) containing compound D3 obtained in Example D-10, compound A1 (325 g), and 2-MeTHF (2.6 L) were sequentially added. The external temperature of the reaction vessel was set to 10°C, DIPEA (876 mL) was added with stirring, and then T3P (1.6 M 2-MeTHF solution, 1.71 kg) was added dropwise. The external temperature of the reaction vessel was set to 25°C, and the reaction mixture was stirred for 1 hour. The reaction mixture was sampled and prepared as a sample (sample preparation method 1), and it was confirmed by HPLC analysis that the reaction conversion rate was 96.4% (calculation expression 1 of reaction conversion rate). The external temperature of the reaction vessel was set to 10°C, and 5% aqueous sodium carbonate solution (3.4 L) was added to the reaction mixture with stirring. The external temperature of the reaction vessel was set to 25°C, the mixture was stirred, and then the aqueous layer was discharged. The organic layer was washed with 5% aqueous sodium hydrogen sulfate monohydrate solution (3.4 L) twice, and then with 5% aqueous sodium carbonate solution (3.4 L). The resulting organic layer was concentrated under reduced pressure at the external temperature of 40°C to obtain 1.87 kg of a 2-MeTHF solution containing compound D4.
LC retention time of compound D4: 4.004 minutes (HPLC analysis conditions: method 4)

Example D-12

Synthesis of compound D5: (tert-butyl 2-[[(2S)-2-[ethyl-[(2S)-1-[(2S)-2-(methylamino)propanoyl]azetidine-2-carbonyl]amino]-3-(p-tolyl)propanoyl]-methyl-amino]acetate)

[Formula 146]

D4

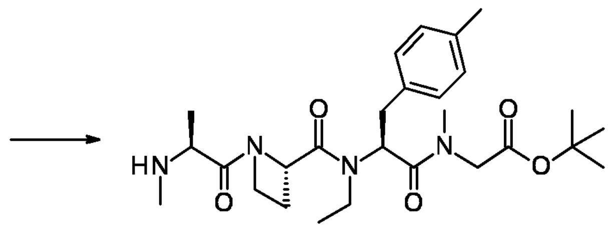

D5

To a reaction vessel after nitrogen replacement, the solution (1.83 kg) containing compound D4 obtained in Example D-11, 2-MeTHF (2.1 L), and 5% Pd/C (238 g, 50% aqueous content) were added. The external temperature of the reaction vessel was set to 25°C, and the reaction vessel was pressurized with hydrogen until the internal pressure of the reaction vessel reached 0.18 MPaG, and the mixture was stirred for 1 hour and 40 minutes. The reaction mixture was sampled and prepared as a sample (sample preparation method 1), and it was confirmed by HPLC analysis that the reaction conversion rate was 99.9% (calculation expression 1 of reaction conversion rate). After replacing the inside of the reaction vessel with nitrogen, the reaction mixture was filtered under pressure. The reaction vessel and the filtering apparatus were washed with 2-MeTHF (1.7 L), and then the resulting filtrate and the wash solution were concentrated under reduced pressure at the external temperature of 40°C to obtain a solution (1.36 kg) containing compound D5.

LC retention time of compound D5: 2.510 minutes (HPLC analysis conditions: method 4)

Example D-13

Synthesis of compound D6: (tert-butyl 2-[[(2S)-2-[[(2S)-1-[(2S)-2-[[(2S,3S)-2-(benzyloxycarbonylamino)-3-methyl-pentanoyl]-methyl-amino]propanoyl]azetidine-2-carbonyl]-ethyl-amino]-3-(p-tolyl)propanoyl]-methyl-amino]acetate)

[Formula 147]

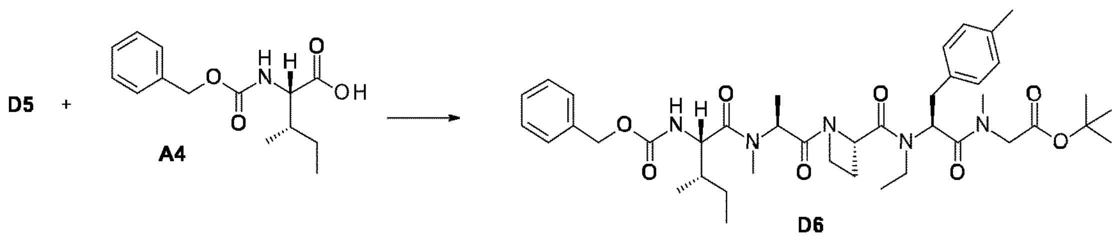

To a reaction vessel after nitrogen replacement, the solution (1.34 kg) containing compound D5 obtained in Example D-12, compound A4 (353 g), 2-MeTHF (0.78 L), toluene (1.7 L), and acetonitrile (0.55 L) were sequentially added at room temperature. The external temperature of the reaction vessel was set to 25°C, and DIPEA (845 mL) and HATU (629 g) were sequentially added at room temperature with stirring, and the mixture was stirred at 25°C for 2 hours. The reaction mixture was sampled and prepared as a sample (sample preparation method 1), and it was confirmed by HPLC analysis that the reaction conversion rate was 99.9% or more (calculation expression 1 of reaction conversion rate). N-methylimidazole (87 mL) was added to the reaction vessel, and 5% aqueous sodium carbonate solution (3.3 L) was further added with stirring, and then the mixture was stirred for 1 hour. Next, 2.5% aqueous ammonia solution (3.3 L) was added, and the mixture was stirred for 30 minutes, and then the aqueous layer was discharged. The resulting organic layer was washed with 2.5% aqueous ammonia solution (3.3 L), 10% aqueous sodium hydrogen sulfate monohydrate solution (3.3 L × 2), and 3% aqueous dipotassium hydrogen phosphate solution (3.3 L). The resulting organic layer was concentrated under reduced pressure at the external temperature of 40°C to obtain 2.30 kg of a solution containing compound D6. LC retention time of compound D6: 4.235 minutes (HPLC analysis conditions: method 4)

Example D-14

Synthesis of compound A7: ((2,3,4,5,6-pentafluorophenyl) (2S)-4-methyl-2-[methyl(2-trimethylsilylethoxycarbonyl)amino]pentanoate)

[Formula 148]

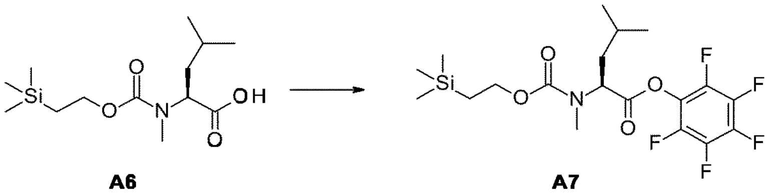

To a reaction vessel after nitrogen replacement, compound A6 (379 g), isopropyl acetate (2.5 L), and DMF (2.5 L) were sequentially added at room temperature. Pentafluorophenol (302 g) was added at room temperature with stirring. The external temperature of the reaction vessel was set to 0°C, and 1-(3-dimethylaminopropyl)-3-ethylcarbodiimide hydrochloride (314 g) was added to the reaction vessel. The reaction solution was warmed to 25°C over 1 hour, and the mixture was stirred for 2 hours and 10 minutes. The reaction mixture was sampled and prepared as a sample (sample preparation method 1), and it was confirmed by HPLC analysis that the reaction conversion rate was 99.6% (calculation expression 2 of reaction conversion rate). The external temperature of the reaction vessel was set to 0°C, and 0.5 M

aqueous hydrochloric acid solution (2.5 L) was added. The external temperature of the reaction vessel was set to 25°C, and the mixture was stirred for 10 minutes. After stirring was stopped, the aqueous layer was discharged from the reaction vessel. The resulting organic layer was washed with 0.5 M aqueous hydrochloric acid solution (2.5 L). To the resulting organic layer, 5% aqueous potassium carbonate solution (2.5 L), and then DMF (377 mL) was added. The reaction mixture was stirred for 10 minutes. After stirring was stopped, the aqueous layer was discharged from the reaction vessel. The resulting organic layer was washed with 5% aqueous potassium carbonate solution (2.5 L), and then 10% aqueous sodium chloride solution (2.5 L). The resulting organic layer was concentrated under reduced pressure at the external temperature of 40°C to obtain 1.15 kg of a solution containing compound A7.
LC retention time of compound A7: 6.175 minutes (HPLC analysis conditions: method 7)

Example D-15

Synthesis of compound D7: (tert-butyl 2-[[(2S)-2-[ethyl-[(2S)-1-[(2S)-2-[methyl-[(2S,3S)-3-methyl-2-[[(2S)-4-methyl-2-[methyl(2-trimethylsilylethoxycarbonyl)amino]pentanoyl]amino]pentanoyl]amino]propanoyl]azetidine-2-carbonyl]amino]-3-(p-tolyl)propanoyl]-methyl-amino]acetate)

[Formula 149]

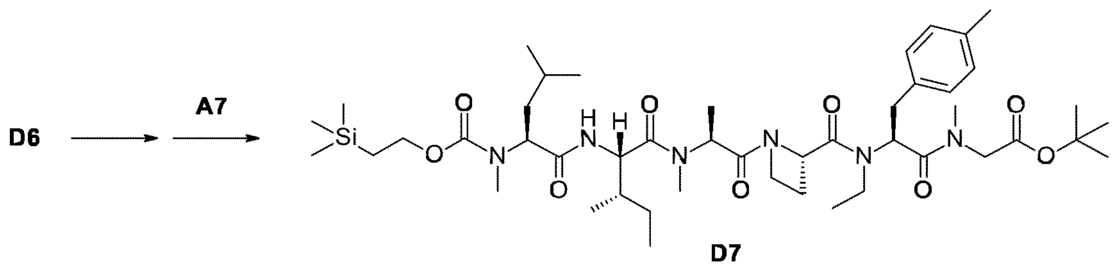


To a reaction vessel after nitrogen replacement, the solution containing compound D6 obtained in Example D-13 (574 g), the solution containing compound A7 obtained in Example D-14 (1.14 kg), isopropyl acetate (0.31 L), acetone (0.81 L), and N-methylmorpholine (0.36 L) were sequentially added at room temperature. The external temperature of the reaction vessel was set to 25°C and 5% Pd/C (116 g, 50% aqueous content) was added, and then the reaction vessel was pressurized with hydrogen until the internal pressure of the reaction vessel reached 0.18 MPaG. After 1 hour, no fluctuation in the internal pressure was confirmed. Then, the reaction vessel was pressurized with hydrogen to 0.18 MPaG, and the reaction mixture was further stirred for 1 hour. The reaction mixture was sampled and prepared as a sample (sample preparation method 1), and it was confirmed by HPLC analysis that the reaction conversion rate was 99.9% or more (calculation expression 1 of reaction conversion rate). After replacing the inside of the reaction vessel with nitrogen, the reaction mixture was filtered under pressure. The reaction vessel and the filtering apparatus were washed with 2-MeTHF (0.82 L), and then the filtrate and the wash solution were collected together, concentrated under reduced pressure at the external temperature of 40°C, and then the resulting residue was filtered. To a reaction vessel after nitrogen replacement, the above filtrate and 2-MeTHF (0.82 L) were added. The external temperature of the reaction vessel was set to 25°C, and 5% aqueous potassium carbonate (1.8 L) and 4-dimethylaminopyridine (66.8 g) were sequentially added while stirring. After stirring was stopped, the aqueous layer was discharged from the reaction vessel. The organic layer was washed with 5% aqueous potassium hydrogen sulfate solution (1.8 L × 2) and 5% aqueous potassium carbonate solution (1.8 L × 2). The resulting organic layer and the wash solution obtained by washing inside of the reaction vessel with 2-MeTHF (0.20 L) were prepared as a storage solution (first batch).

To a reaction vessel after nitrogen replacement, the solution containing compound D6 obtained in Example D-13 (574 g), the solution containing compound A7 obtained in Example D-14 (1.14 kg), isopropyl acetate (0.31 L), acetone (0.81 L), and N-methylmorpholine (0.36 L) were sequentially added at room temperature. The external temperature of the reaction vessel was set to 25°C and 5% Pd/C (116 g, 50% aqueous content) was added, and then the reaction vessel was pressurized with hydrogen until the internal pressure of the reaction vessel reached 0.18 MPaG. After 50 minutes, no fluctuation in the internal pressure was confirmed, and then the reaction mixture was sampled and prepared as a sample (sample preparation method 1), and it was confirmed by HPLC analysis that the reaction conversion rate was 99.9% or more (calculation expression 1 of reaction conversion rate). The reaction vessel was then pressurized with hydrogen to 0.18 MPaG, and the reaction mixture was further stirred for 1 hour. After replacing the inside of the reaction vessel with nitrogen, the reaction mixture was filtered under pressure. The reaction vessel and the filtering apparatus were washed

with 2-MeTHF (0.82 L), and then the filtrate and the wash solution were collected together, concentrated under reduced pressure at the external temperature of 40°C, and then the resulting residue was filtered. To a reaction vessel after nitrogen replacement, the above filtrate and 2-MeTHF (1.6 L) were added. The external temperature of the reaction vessel was set to 25°C, and 5% aqueous potassium carbonate (1.8 L) and 4-dimethylaminopyridine (66.7 g) were sequentially added while stirring. After stirring was stopped, the aqueous layer was discharged from the reaction vessel. The organic layer was washed with 5% aqueous potassium hydrogen sulfate solution (1.8 L × 2) and 5% aqueous potassium carbonate solution (1.8 L × 2). To the resulting organic layer, the above storage solution (first batch) was added, and the mixture was concentrated under reduced pressure at the external temperature of 40°C to obtain a solution (1.81 kg) containing compound D7.
LC retention time of compound D7: 5.964 minutes (HPLC analysis conditions: method 7)

Example D-16

Synthesis of compound D8: (tert-butyl 2-[[(2S)-2-[ethyl-[(2S)-1-[(2S)-2-[methyl-[(2S,3S)-3-methyl-2-[[(2S)-4-methyl-2-(methylamino)pentanoyl]amino]pentanoyl]amino]propanoyl]azetidine-2-carbonyl]amino]-3-(p-tolyl)propanoyl]-methylamino]acetate)

[Formula 150]

D7

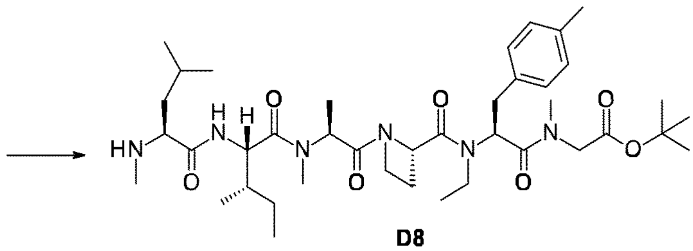

D8

To a reaction vessel after nitrogen replacement, the solution (1.79 kg) containing compound D7 obtained in Example D-15 and 2-MeTHF (1.9 L) were sequentially added at room temperature. The external temperature of the reaction vessel was set to 47°C, and tetrabutylammonium fluoride (1 M THF solution, 2.70 L) was added over 1 hour, and then the mixture was stirred for 1 hour. The reaction mixture was sampled and prepared as a sample (sample preparation method 1), and it was confirmed by HPLC analysis that starting material compound D7 was not detected (calculation expression 1 of reaction conversion rate). After stirring was stopped, isopropyl acetate (2.9 L) was added to the reaction vessel. The external temperature of the reaction vessel was set to 25°C, and 5% aqueous potassium carbonate solution (2.9 L) was added with stirring. After stirring was stopped, the aqueous layer was discharged from the reaction vessel. The resulting organic layer was washed with 5% aqueous potassium carbonate solution (2.9 L) twice. The resulting organic layer was concentrated under reduced pressure at the external temperature of 40°C to obtain a solution (1.87 kg) containing compound D8.
LC retention time of compound D8: 3.057 minutes (HPLC analysis conditions: method 4)

Example D-17

Compound D9: Synthesis of tert-butyl 2-[[(2S)-2-[[(2S)-1-[(2S)-2-[[(2S,3S)-2-[[(2S)-2-[[(3S)-3-[[(2S)-2-[[1-(benzyloxycarbonylamino)cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-tolyl)propanoyl]-methyl-amino]acetate

[Formula 151]

B26 + D8

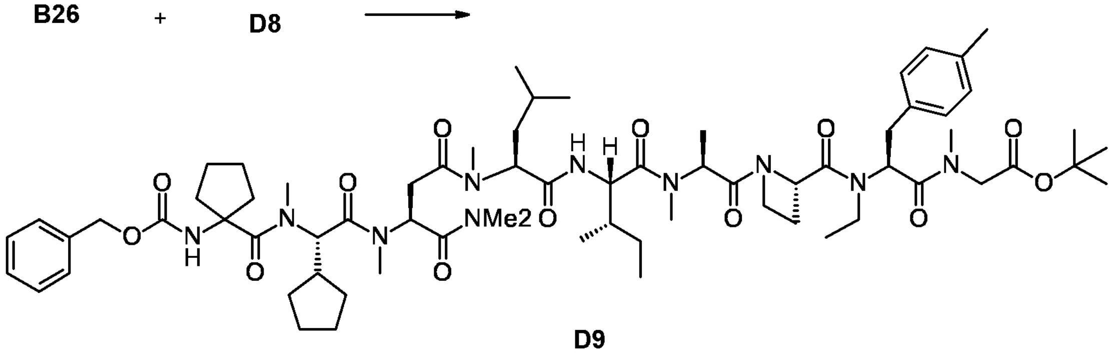

D9

The concentrated solution of compound B26 obtained in Example D-6 was added to a reaction vessel, and the reaction vessel was subjected to nitrogen replacement. Next, 2-MeTHF (4 mL), DMF (1.5 mL), DIPEA (1.0 mL), and the solution (2.16 g) of compound D8 obtained in Example D-16 were added. HATU (0.92 g) was added at room temperature, and the mixture was stirred for 1 hour. The reaction mixture was sampled and prepared as a sample (sample preparation method 5) and subjected to HPLC analysis which confirmed that the reaction conversion rate was 99.3% (calculation expression 3 of reaction conversion rate). The external temperature of the reaction vessel was set to 0°C, and N-methylimidazole (0.064 mL) and 5% aqueous sodium carbonate solution (10 mL) were added, and the mixture was stirred. After the underlayer was discharged, the resulting organic layer was washed with 5% aqueous sodium carbonate solution (10 mL), 2.5% ammonia water (10 mL × 2), 5% aqueous sodium hydrogen sulfate monohydrate solution (20 mL × 3), and 5% aqueous sodium carbonate solution (20 mL × 3). The resulting organic layer was dehydrated with sodium sulfate and filtered to remove sodium sulfate, and then the resulting filtrate was concentrated under reduced pressure at the external temperature of 40°C to obtain a residue (1.27 g) containing compound D9.

LC retention time of compound D9: 5.452 minutes (HPLC analysis conditions: method 1)
LCMS (ESI) retention time of compound D9: 21.57 minutes, m/z = 1306 $[M+Na]^+$ (LCMS analysis conditions: method 3)

Example D-18

Compound D10: Synthesis of 2-[[(2S)-2-[[(2S)-1-[(2S)-2-[[(2S,3S)-2-[[(2S)-2-[[(3S)-3-[[(2S)-2-[[1-(benzyloxycarbonylamino)cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-tolyl)propanoyl]-methyl-amino]acetic acid

[Formula 152]

D9

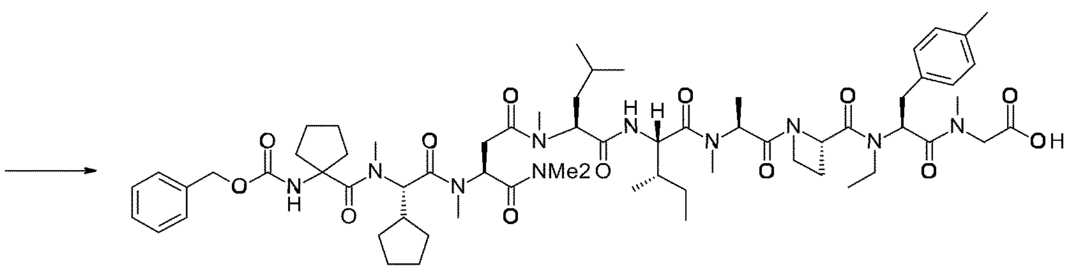

D10

The residue (0.90 g) containing compound D9 obtained in Example D-17 was added to a reaction vessel, and the reaction vessel was subjected to nitrogen replacement. Next, 2-MeTHF(9.0 mL) and HMDS(1.00 mL) were added. The external temperature of the reaction vessel was set to 0°C, and TMSOTf (0.724 mL) was added with stirring, and then the mixture was stirred for 1 hour. The reaction mixture was sampled and prepared as a sample (sample preparation method 1) and subjected to HPLC analysis which confirmed that the reaction conversion rate was 99.7% (calculation expression 1 of

reaction conversion rate). Still at the external temperature of 0°C, 5% aqueous dipotassium hydrogen phosphate solution (0.5 mL) and 5% aqueous sodium carbonate solution (3 mL) were sequentially added. Then, 85% phosphoric acid was added and the pH was adjusted to 5. After separating the organic layer and the aqueous layer, the aqueous layer was extracted with 2-MeTHF (10 mL × 3). The resulting all organic layers were mixed and washed with saturated aqueous NaCl solution (10 mL). The resulting organic layer was dehydrated with sodium sulfate and filtered to remove sodium sulfate, and then the obtained filtrate was concentrated under reduced pressure at the external temperature of 40°C to obtain a residue containing compound D10.
LC retention time of compound D10: 4.564 minutes (HPLC analysis conditions: method 1)

Example D-19

Compound D11: Synthesis of tert-butyl (2S)-1-[(2S)-2-[[2-[[(2S)-2-[[(2S)-1-[(2S)-2-[[(2S,3S)-2-[[(2S)-2-[[(3S)-3-[[(2S)-2-[[1-(benzyloxycarbonylamino)cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-tolyl)propanoyl]-methyl-amino]acetyl]amino]-4-[3,5-difluoro-4-(trifluoromethyl)phenyl]butanoyl]pyrrolidine-2-carboxylate

[Formula 153]

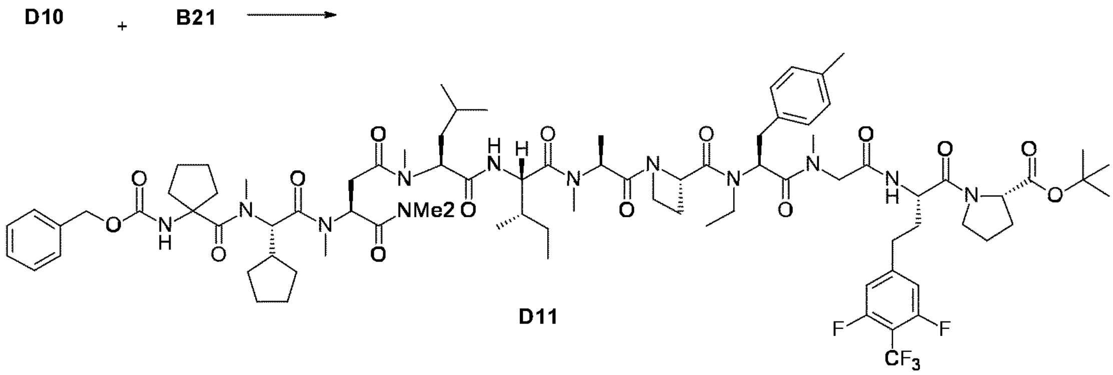

D11

The residue containing compound D10 obtained in Example D-18 was added to a reaction vessel, and the reaction vessel was subjected to nitrogen replacement. Next, 2-MeTHF (6.0 mL), MeCN (4.0 mL), DIPEA (0.737 mL), and the solution (3.60 mL) of compound B21 obtained in Example D-4 were added. HATU (0.667 g) was added at room temperature, and the mixture was stirred for 1 hour. The reaction mixture was sampled and prepared as a sample (sample preparation method 5) and subjected to HPLC analysis which confirmed that the reaction conversion rate was 99.0% (calculation expression 1 of reaction conversion rate). The external temperature was set to 0°C, and N-methylimidazole (0.0560 mL) and 5% aqueous sodium carbonate solution (10 mL) were added, and the mixture was stirred. After the underlayer was discharged, the resulting organic layer was washed with 5% aqueous sodium carbonate solution (10 mL), 2.5% ammonia water (10 mL × 2), 5% aqueous sodium hydrogen sulfate monohydrate solution (20 mL × 3), 5% aqueous sodium carbonate solution (20 mL), and saturated aqueous NaCl solution (10 mL). The resulting organic layer was dehydrated with sodium sulfate and filtered to remove sodium sulfate, and then the resulting filtrate was concentrated under reduced pressure at the external temperature of 40°C. The resulting residue was purified by silica gel column chromatography (eluent: dichloromethane/methanol 98 : 2 to 82 : 18) to obtain 0.761 g of compound D11.
LC retention time of compound D11: 21.550 minutes (HPLC analysis conditions: method 3)

Example D-20

Compound D12: Synthesis of (2S)-1-[(2S)-2-[[2-[[(2S)-2-[[(2S)-1-[(2S)-2-[[(2S,3S)-2-[[(2S)-2-[[(3S)-3-[[(2S)-2-[[1-(benzyloxycarbonylamino)cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-tolyl)propanoyl]-methyl-amino]acetyl]amino]-4-[3,5-difluoro-4-(trifluoromethyl)phenyl]butanoyl]pyridine-2-carboxylic acid

[Formula 154]

D11

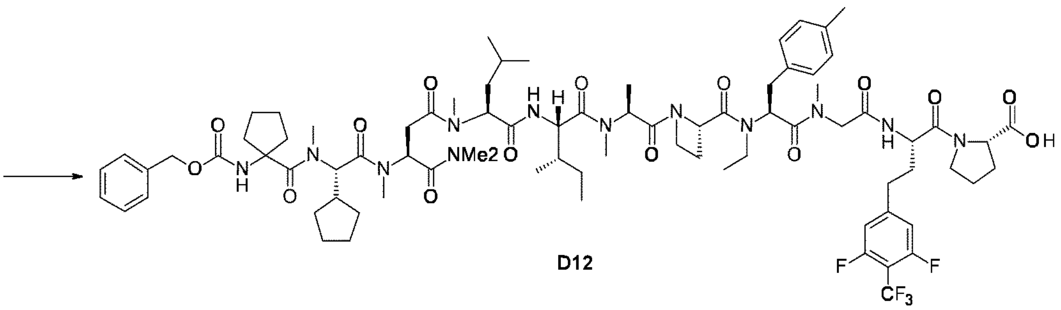

D12

The compound D11 (0.761 g) obtained in Example D-19 was added to a reaction vessel, and the reaction vessel was subjected to nitrogen replacement. Next, 2-MeTHF (7.0 mL) and HMDS (0.679 mL) were added. The external temperature of the reaction vessel was set to 0°C, and TMSOTf (0.485 mL) was added with stirring, and then the mixture was stirred for 2 hours. The reaction mixture was sampled and prepared as a sample (sample preparation method 1) and subjected to HPLC analysis which confirmed that the reaction conversion rate was 99.6% (calculation expression 1 of reaction conversion rate). Still at the external temperature of 0°C, 5% aqueous dipotassium hydrogen phosphate solution (0.5 mL) and 5% aqueous sodium dihydrogen phosphate solution (3 mL) were added. After separating the organic layer and the aqueous layer, the aqueous layer was extracted with 2-MeTHF (10 mL × 3). The resulting all organic layers were mixed and washed with saturated aqueous NaCl solution (10 mL). The resulting organic layer was dehydrated with sodium sulfate, filtered to remove sodium sulfate, and then concentrated under reduced pressure at the external temperature of 40°C. The resulting residue was diluted with EtOAc (80 mL) and then washed with 5% aqueous NaCl solution (10 mL × 3). The resulting organic layer was dehydrated with sodium sulfate and filtered to remove sodium sulfate, and then the resulting filtrate was concentrated under reduced pressure at the external temperature of 40°C to obtain a residue (0.766 g) containing compound D12.
LC retention time of compound D12: 19.111 minutes (HPLC analysis conditions: method 3)

Example D-21

Compound D13: Synthesis of (2S)-1-[(2S)-2-[[2-[[(2S)-2-[[(2S)-1-[(2S)-2-[[(2S,3S)-2-[[(2S)-2-[[(3S)-3-[[(2S)-2-[(1-aminocyclopentanecarbonyl)-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-tolyl)propanoyl]-methyl-amino]acetyl]amino-4-[3,5-difluoro-4-(trifluoromethyl)phenyl]butanoyl]pyrrolidine-2-carboxylic acid

[Formula 155]

D12 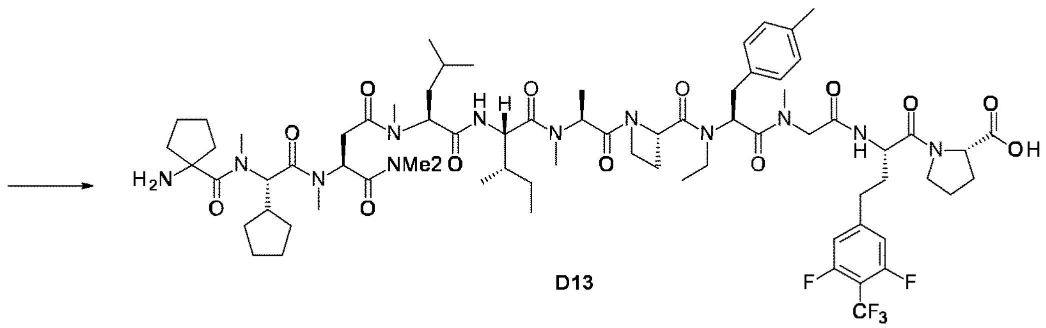

D13

The residue (0.766 g) containing compound D12 obtained in Example D-20 was added to a reaction vessel. Next, 2-MeTHF (10 mL) and 5% Pd/C (0.107 g, 50% aqueous content) were sequentially added at room temperature. The inside of the reaction vessel was replaced with hydrogen, and the mixture was stirred at room temperature for 5 hours. The reaction mixture was sampled and prepared as a sample (sample preparation method 1) and subjected to HPLC analysis which confirmed that the reaction conversion rate was 17.8% (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF, and the solutions were combined and concentrated under reduced pressure at the external temperature of 40°C. The resulting concentrated solution was added to the reaction vessel. Next, 2-MeTHF (10 mL) and 5% Pd/C (0.428 g, 50% aqueous content) were sequentially added at room temperature. The inside of the reaction vessel was replaced with hydrogen, and the mixture was stirred at room temperature for 6 hours. The reaction mixture was sampled and prepared as a sample (sample preparation method 1) and subjected to HPLC analysis which confirmed that the reaction conversion rate was 99.5% (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF, and the solutions were combined and concentrated under reduced pressure at the external temperature of 40°C. The resulting residue was purified by silica gel column chromatography (eluent: dichloromethane/methanol 96 : 4 to 56 : 44) to obtain D13 (0.502 g).

LC retention time of compound D13: 12.952 minutes (HPLC analysis conditions: method 3)
LCMS (ESI) retention time of compound D13: 13.34 minutes, m/z = 1456 $[M+H]^+$ (LCMS analysis conditions 3)

Example E-1

Compound E1: Preparation of (2S)-2-(benzyloxycarbonylamino)-4-[3,5-difluoro-4-(trifluoromethyl)phenyl]butanoic acid

[Formula 156]

A22 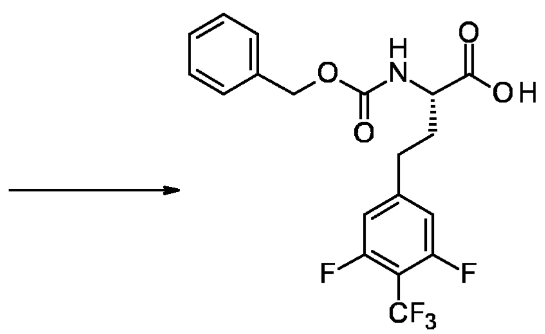

E1

Compound A22 (3.14 g) was added to a reaction vessel. Next, 2-MeTHF (50 mL) and 5% aqueous sodium hydrogen sulfate monohydrate solution (20 mL) were added, and the mixture was stirred. After the aqueous layer was

discharged, the resulting organic layer was washed with 5% aqueous sodium hydrogen sulfate monohydrate solution (20 mL × 4), water (10 mL × 2) and saturated aqueous NaCl solution (10 mL). The resulting organic layer was concentrated under reduced pressure at the external temperature of 40°C to obtain a solution (2.40 g) containing compound E1.

Example E-2

Compound E2: Synthesis of tert-butyl (2S)-2-(benzyloxycarbonylamino)-4-[3,5-difluoro-4-(trifluoromethyl)phenyl]butanoate

[Formula 157]

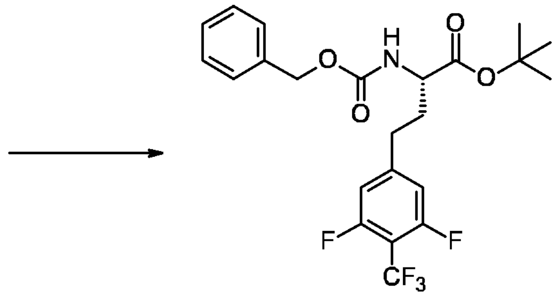

E1

E2

The solution (0.302 g) of compound E1 obtained in Example E-1 was added to a reaction vessel. Next, dichloromethane (0.90 mL) and cyclohexane (1.5 mL) were added at room temperature. tert-Butyl 2,2,2-trichloroacetimidate (0.257 mL) and boron trifluoride diethyl ether complex (0.0146 mL) were added. After the mixture was stirred at room temperature for 0.5 hours, the reaction mixture was sampled and prepared as a sample (sample preparation method 1) and subjected to HPLC analysis which confirmed that the reaction conversion rate was 94.7% (calculation expression 1 of reaction conversion rate), and served as a storage solution (first batch).

The solution (2.02 g) of compound E1 obtained in Example E-1 was added to a reaction vessel. Next, dichloromethane (6.1 mL) and cyclohexane (10 mL) were added to the reaction vessel at room temperature. After tert-butyl 2,2,2-trichloroacetimidate (1.74 mL) and boron trifluoride diethyl ether complex (0.0980 mL) were added, the mixture was stirred at room temperature for 2 hours. The reaction mixture was sampled and prepared as a sample (sample preparation method 1) and subjected to HPLC analysis which confirmed that the reaction conversion rate was 99.9% or more (calculation expression 1 of reaction conversion rate). The reaction mixture was mixed with the above storage solution and then filtered, and the solid residue was washed with cyclohexane. The resulting filtrate was washed with 10% aqueous citric acid solution (10 mL × 10), 5% aqueous sodium carbonate solution (10 mL × 4), and saturated aqueous NaCl solution (10 mL). The resulting organic layer was dehydrated with sodium sulfate and filtered to remove sodium sulfate, and then the resulting filtrate was concentrated under reduced pressure at the external temperature of 40°C to obtain 2.45 g of the crude product of compound E2.

LC retention time of compound E2: 4.469 minutes (HPLC analysis conditions: method 1)
LCMS (ESI) of compound E2: retention time: 4.563 min, m/z = 474 $[M+H]^+$ (LCMS analysis conditions 1)

Example E-3

Compound E3: Synthesis of tert-butyl (2S)-2- amino -4-[3,5-difluoro-4-(trifluoromethyl)phenyl]butanoate

[Formula 158]

E2

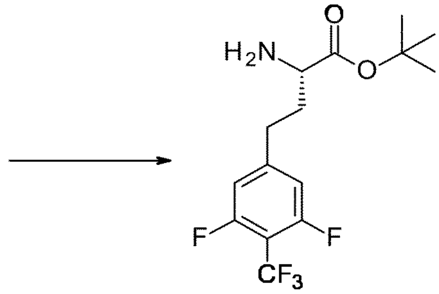

E3

The crude product (1.02 g) of compound E2 obtained in Example E-2 was added to a reaction vessel. Next, 2-MeTHF (10 mL) and 5% Pd/C (0.358 g, 50% aqueous content) were sequentially added at room temperature. The inside of the reaction vessel was replaced with hydrogen, and the mixture was stirred at room temperature for 1.5 hours. The reaction mixture was sampled and prepared as a sample (sample preparation method 1) and subjected to HPLC analysis which confirmed that the reaction conversion rate was 99.3% (calculation expression 2 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF. The resulting solution was concentrated under reduced pressure and analyzed by HPLC. The resulting solution was concentrated under reduced pressure and then diluted with 2-MeTHF to obtain a solution containing compound E3.

LC retention time of compound E3: 2.956 minutes (HPLC analysis conditions: method 1)
LCMS (ESI) of compound E3: retention time: 2.841 min, m/z = 340 $[M+H]^+$ (LCMS analysis conditions 1)

Example E-4

Compound E4: Synthesis of (3S)-3-[[(2S)-2-[[1-[[(2S)-1-benzyloxycarbonylpyrrolidine-2-carbonyl]amino]cyclopentanecarbonyl]-methyl-amino]-2-cyclopentyl-acetyl]-methyl-amino]-4-(dimethylamino)-4-oxo-butanoic acid

[Formula 159]

A20

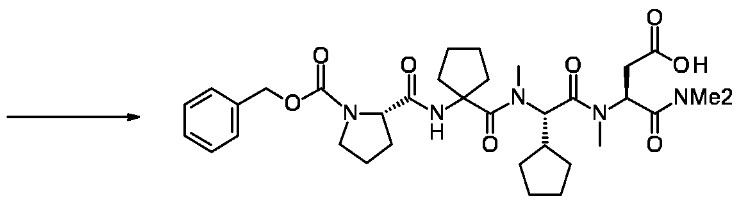

E4

The solution (5.75 g) of compound A20 obtained in Example A-13 was concentrated under reduced pressure, and the resulting residue was added to a reaction vessel which was then subjected to nitrogen replacement. Next, isopropyl acetate (7.0 mL) and HMDS (0.983 mL) were added to the reaction vessel. The external temperature of the reaction vessel was set to 0°C, and TMSOTf (0.678 mL) was added with stirring, and then the mixture was stirred for 1.5 hours. The reaction mixture was sampled and prepared as a sample (sample preparation method 1) and subjected to HPLC analysis which confirmed that the reaction conversion rate was 99.6% (calculation expression 1 of reaction conversion rate). Still at the external temperature of 0°C, 2-MeTHF (7.0 mL), 5% aqueous dipotassium hydrogen phosphate solution (0.50 mL), and 5% aqueous sodium dihydrogen phosphate solution (4.2 mL) were sequentially added. After separating the organic layer and the aqueous layer, saturated aqueous NaCl solution (2.0 mL) was added to the aqueous layer, and the mixture was extracted with 2-MeTHF (10 mL $\times$ 2). The resulting all organic layers were mixed and washed with saturated aqueous NaCl solution (5.0 mL). The resulting organic layer was dehydrated with sodium sulfate and filtered to remove sodium sulfate, and DIPEA (1.45 mL) was added. Then, the resulting solution was concentrated under reduced pressure at the external temperature of 40°C to obtain a residue containing compound E4. LC retention time of compound E4: 3.427 minutes (HPLC analysis conditions: method 1)

Example E-5

Compound E5: Synthesis of benzyl (2S)-2-[[1-[[(1S)-2-[[(1S)-3-[[(1S)-1-[[(1S,2S)-1-[[(1S)-2-[(2S)-2-[[(1S)-2-[(2-tert-butoxy-2-oxo-ethyl)-methyl-amino]-2-oxo-1-(p-tolylmethyl)ethyl]-ethyl-carbamoyl]azetidin-1-yl]-1-methyl-2-oxo-ethyl]-methyl-carbamoyl]-2-methylbutyl]carbamoyl]-3-methyl-butyl]-methyl-amino]-1-(dimethylcarbamoyl)-3-oxo-propyl]-methyl-amino]-1-cyclopentyl-2-oxo-ethyl]-methyl-carbamoyl]cyclopentyl]carbamoyl]pyrrolidine-1-carboxylate

[Formula 160]

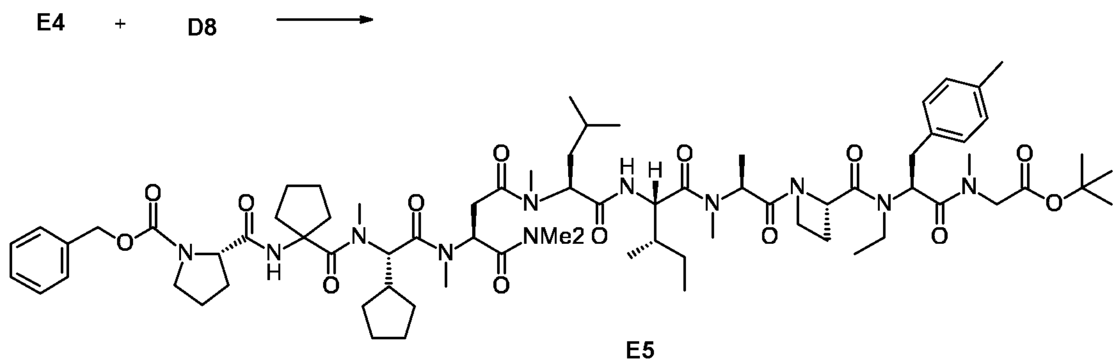

E5

The concentrated solution of compound E4 obtained in Example E-4 was added to a reaction vessel, and the reaction vessel was subjected to nitrogen replacement. Next, 2-MeTHF (7.0 mL), DMF (2.5 mL), DIPEA (1.8 mL), and the solution (3.87 g) of compound D8 obtained in Example D-16 were added. HATU (1.65 g) was added at room temperature, and the mixture was stirred for 40 minutes. The reaction mixture was sampled and prepared as a sample (sample preparation method 5) and subjected to HPLC analysis which confirmed that the reaction conversion rate was 99.9% (calculation expression 3 of reaction conversion rate). The external temperature of the reaction vessel was set to 0°C, and N-methylimidazole (0.115 mL) and 5% aqueous sodium carbonate solution (10 mL) were added, and the mixture was stirred. After the underlayer was discharged, the resulting organic layer was washed with 5% aqueous sodium carbonate solution (10 mL), 2.5% ammonia water (10 mL × 2), 5% aqueous sodium hydrogen sulfate monohydrate solution (20 mL×2, 10 mL×2), 10% aqueous citric acid solution (10 mL × 5), and saturated aqueous NaCl solution (10 mL). The resulting organic layer was dehydrated with sodium sulfate and filtered to remove sodium sulfate, and then the resulting filtrate was concentrated under reduced pressure at the external temperature of 40°C. The resulting residue was purified by silica gel column chromatography (eluent: dichloromethane/methanol 98 : 2 to 82 : 18) to obtain 1.24 g of compound E5.

LC retention time of compound E5: 5.361 minutes (HPLC analysis conditions: method 1)
LCMS (ESI) retention time of compound E5: 21.14 minutes, m/z = 1403 $[M+Na]^+$ (LCMS analysis conditions: method 3)

Example E-6

Compound E6: Synthesis of 2-[[(2S)-2-[[(2S)-1-[(2S)-2-[[(2S,3S)-2-[[(2S)-2-[[(3S)-3-[[(2S)-2-[[1-[[(2S)-1-benzyloxycarbonylpyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-toly])propanoyl]-methyl-amino]acetic acid

[Formula 161]

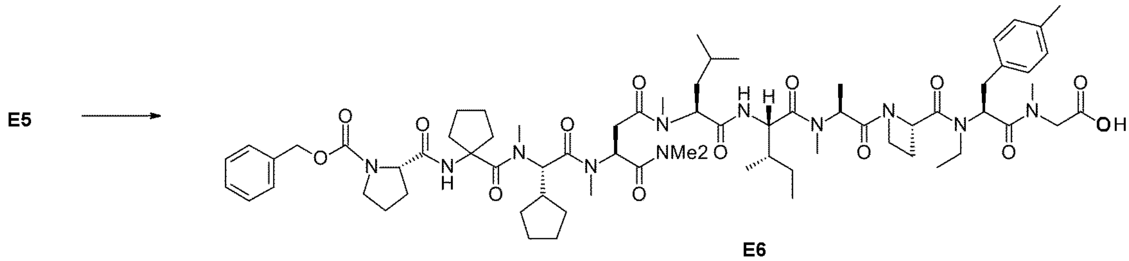

E6

The compound E5 (0.959 g) obtained in Example E-5 was added to a reaction vessel, and the reaction vessel was subjected to nitrogen replacement. Next, 2-MeTHF (10 mL) and HMDS (0.990 mL) were added. The external temperature of the reaction vessel was set to 0°C, and TMSOTf (0.715 mL) was added with stirring. The external temperature was then set to room temperature, and the mixture was stirred for 1 hour. The reaction mixture was sampled and prepared as a sample (sample preparation method 1) and subjected to HPLC analysis which confirmed that the reaction conversion rate was 99.2% (calculation expression 1 of reaction conversion rate). The external temperature was set to 0°C, 5% aqueous dipotassium hydrogen phosphate solution (0.50 mL), 5% aqueous sodium dihydrogen phosphate solution (4.0 mL), and saturated aqueous NaCl solution (2.0 mL) were sequentially added. After separating the organic layer and the aqueous layer, the aqueous layer was extracted with 2-MeTHF (10 mL × 2). The resulting all organic layers were mixed and washed with saturated aqueous NaCl solution (10 mL). The resulting organic layer was dehydrated with sodium sulfate and filtered to remove sodium sulfate, and then the resulting filtrate was concentrated under reduced pressure at the external temperature of 40°C to obtain a residue containing compound E6.
LC retention time of compound E6: 4.510 minutes (HPLC analysis conditions: method 1)

Example E-7

Compound E7: Synthesis of benzyl (2S)-2-[[1-[[(1S)-2-[[(1S)-3-[[(1S)-1-[[(1S,2S)-1-[[(1S)-2-[(2S)-2-[[(1S)-2-[[2-[[(1S)-1-tert-butoxycarbonyl-3-[3,5-difluoro-4-(trifluoromethyl)phenyl]propyl]amino]-2-oxo-ethyl]-methyl-amino]-2-oxo-1-(p-tolylmethyl)ethyl]-ethyl-carbamoyl]azetidin-1-yl]-1-methyl-2-oxo-ethyl]-methyl-carbamoyl]-2-methyl-butyl]carbamoyl]-3-methyl-butyl]-methyl-amino]-1-(dimethylcarbamoyl)-3-oxo-propyl]-methyl-amino]-1-cyclopentyl-2-oxo-ethyl]-methyl-carbamoyl]cyclopentyl]carbamoyl]pyrrolidine-1-carboxylate

[Formula 162]

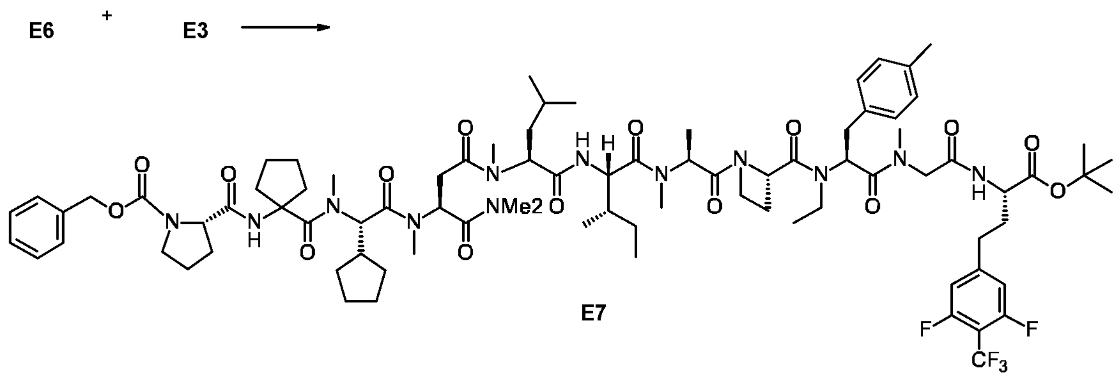

E7

The residue containing compound E6 obtained in Example E-6 was added to a reaction vessel, and the reaction vessel was subjected to nitrogen replacement. Next, MeCN (10 mL), DIPEA (0.558 mL), and the solution (8.06 mL) of compound E3 obtained in Example E-3 were added. HATU (1.00 g) was added at room temperature, and the mixture was stirred for 0.5 hours. The reaction mixture was sampled and prepared as a sample (sample preparation method 5) and subjected to HPLC analysis which confirmed that the reaction conversion rate was 81.9% (calculation expression 3 of reaction conversion rate). DIPEA (0.558 mL) and compound E3 (2.00 mL) were added, and then the mixture was stood still at room temperature for 15.5 hours. The reaction mixture was sampled and prepared as a sample (sample preparation

method 5) and subjected to HPLC analysis which confirmed that the reaction conversion rate was 81.7% (calculation expression 3 of reaction conversion rate). The external temperature was set to 0°C, and N-methylimidazole (0.0550 mL) and 5% aqueous sodium carbonate solution (10 mL) were added, and the mixture was stirred. After the underlayer was discharged, the resulting organic layer was washed with 5% aqueous sodium carbonate solution (10 mL), 2.5% ammonia water (20 mL × 2), 5% aqueous sodium hydrogen sulfate monohydrate solution (30 mL × 2), 5% aqueous sodium carbonate solution (20 mL), and saturated aqueous NaCl solution (10 mL). The resulting organic layer was dehydrated with sodium sulfate and filtered to remove sodium sulfate, and then the resulting filtrate was concentrated under reduced pressure at the external temperature of 40°C. The resulting residue was purified by silica gel column chromatography (eluent: dichloromethane/methanol 98 : 2 to 82 : 18) to obtain E7 (0.731 g).
LC retention time of compound E7: 21.328 minutes (HPLC analysis conditions: method 3)

Example E-8

Compound E8: Synthesis of (2S)-2-[[2-[[(2S)-2-[[(2S)-1-[(2S)-2-[[(2S,3S)-2-[[(2S)-2-[[(3S)-3-[[(2S)-2-[[1-[[(2S)-1-benzyloxycarbonylpyrrolidine-2-carbonyl]amino]cyclopentanecarbonyl]-methyl-amino]-2-cyclopentyl-acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-tolyl)propanoyl]-methyl-amino]acetyl]amino]-4-[3,5-difluoro-4-(trifluoromethyl)phenyl]butanoic acid

[Formula 163]

E7

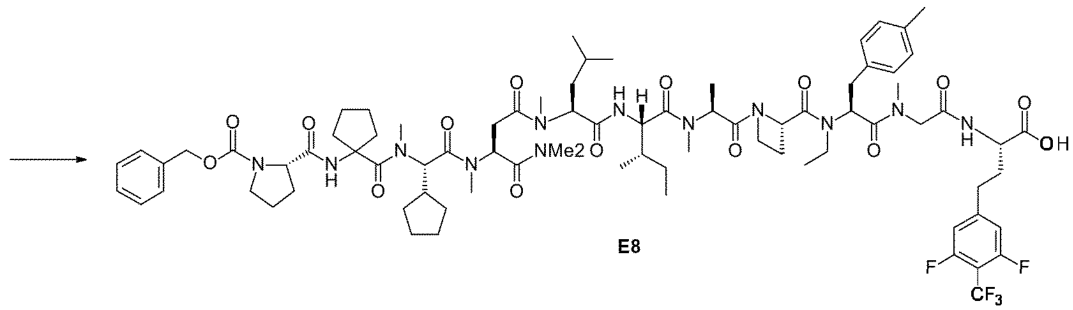

E8

The compound E7 (0.711 g) obtained in Example E-7 was added to a reaction vessel, and the reaction vessel was subjected to nitrogen replacement. Next, 2-MeTHF (7.0 mL) and HMDS (1.36 mL) were added. The external temperature of the reaction vessel was set to 0°C, and TMSOTf (0.780 mL) was added with stirring. The external temperature was set to room temperature, and the mixture was stirred for 1.5 hours. The reaction mixture was sampled and prepared as a sample (sample preparation method 1) and subjected to HPLC analysis which confirmed that the reaction conversion rate was 98.4% (calculation expression 1 of reaction conversion rate). The external temperature was then set to 0°C, 5% aqueous dipotassium hydrogen phosphate solution (0.50 mL), 5% aqueous sodium dihydrogen phosphate solution (3.0 mL), and saturated aqueous NaCl solution (2.0 mL) were sequentially added. After separating the organic layer and the aqueous layer, the aqueous layer was extracted with 2-MeTHF (10 mL × 3). The resulting all organic layers were mixed and washed with saturated aqueous NaCl solution (10 mL). The resulting organic layer was dehydrated with sodium sulfate and filtered to remove sodium sulfate, and then the resulting filtrate was concentrated under reduced pressure at the external temperature of 40°C. The resulting residue was purified by silica gel column chromatography (eluent: dichloromethane/methanol 96 : 4 to 76 : 24) to obtain 0.899 g of compound E8.
LC retention time of compound E8: 19.054 minutes (HPLC analysis conditions: method 3)

Example E-9

Compound E9: Synthesis of (2S)-2-[[2-[[(2S)-2-[[(2S)-1-[(2S)-2-[[(2S,3S)-2-[[(2S)-2-[[(3S)-3-[[(2S)-2-cyclopentyl-2-[methyl-[1-[[(2S)-pyrrolidine-2-carbonyl]amino]cyclopentanecarbonyl]amino]acetyl]-methyl-amino]-4-(dimethylamino)-4-oxo-butanoyl]-methyl-amino]-4-methyl-pentanoyl]amino]-3-methyl-pentanoyl]-methyl-amino]propanoyl]azetidine-2-carbonyl]-ethyl-amino]-3-(p-tolyl)propanoyl]-methyl-amino]acetyl]amino]-4-[3,5-difluoro-4-(trifluoromethyl)phenyl]butanoic acid

[Formula 164]

E8

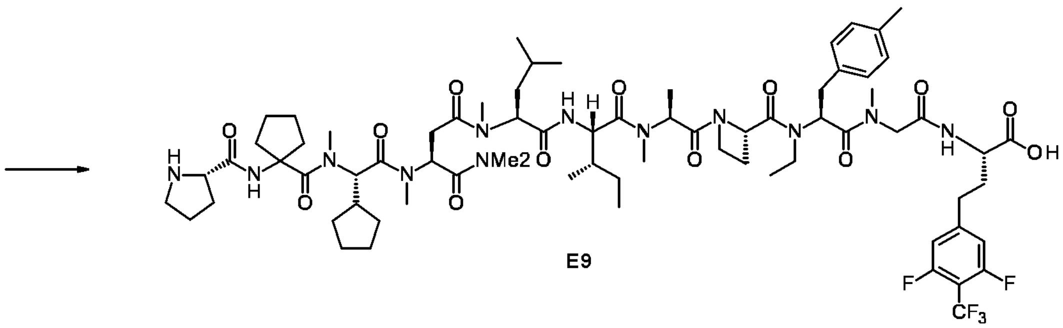

E9

The compound E8 (0.687 g) obtained in Example E-8 was added to a reaction vessel. Next, 2-MeTHF (10 mL) and 5% Pd/C (0.0954 g, 50% aqueous content) were sequentially added at room temperature. The inside of the reaction vessel was replaced with hydrogen, and the mixture was stirred at room temperature for 3 hours. The reaction mixture was sampled and prepared as a sample (sample preparation method 1) and subjected to HPLC analysis which confirmed that the reaction conversion rate was 95.2% (calculation expression 1 of reaction conversion rate). The inside of the reaction vessel was replaced with nitrogen, then the reaction mixture was filtered under reduced pressure using filter paper and a filter. The reaction vessel, and the filter paper and the filter were washed with 2-MeTHF, and the solutions were combined and concentrated under reduced pressure at the external temperature of 40°C. To the resulting concentrated solution, ethyl acetate (20 mL) was added, and the mixture was washed with water (10 mL × 3) and 5% aqueous NaCl solution (10 mL). The resulting organic layer was dehydrated with sodium sulfate and filtered to remove sodium sulfate, and then the resulting filtrate was concentrated under reduced pressure at the external temperature of 40°C. The resulting residue was purified by silica gel column chromatography (eluent: dichloromethane/methanol 96 : 4 to 66 : 34) to obtain 0.387 g of compound E9.

LC retention time of compound E9: 13.427 minutes (HPLC analysis conditions: method 3)
LCMS (ESI) of compound E9: retention time: 13.85 min, m/z = 1456 $[M+H]^+$ (LCMS analysis conditions 3)

Synthesis of compound 1: cyclization reaction of compound A36

Condensation reagents and solvents in cyclization reaction to compound 1 were investigated using compound A36 as the starting material. The cyclization reaction was monitored by HPLC analysis. The yield was calculated from Area% ratio by HPLC analysis using methyl benzoate as the internal standard substance.

[Formula 165]

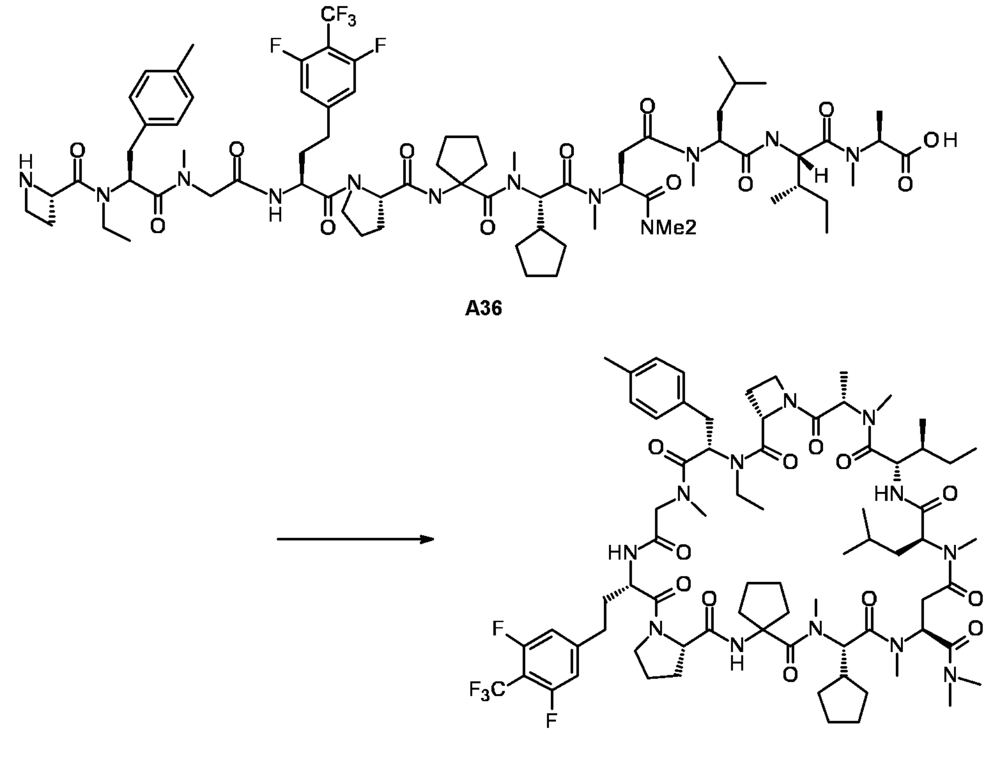

A36

1

[Formula 166]

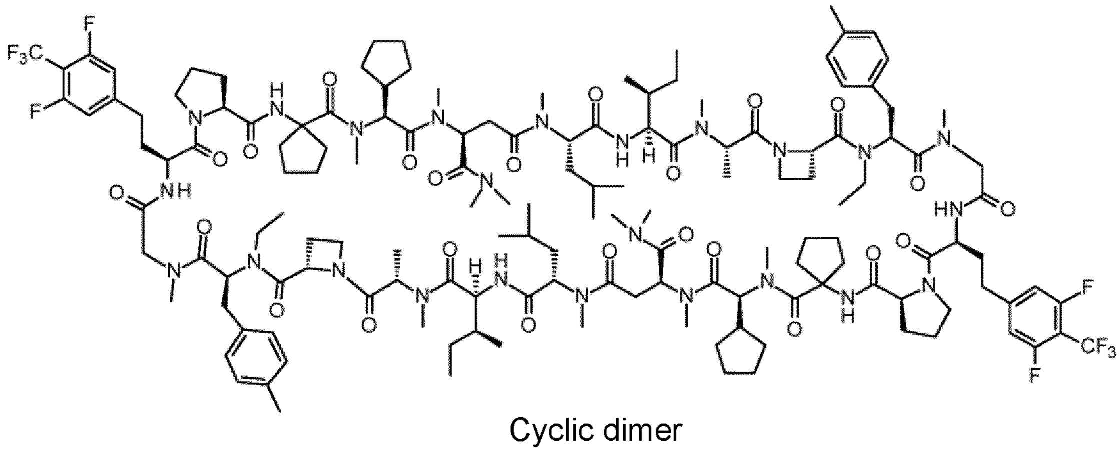

Cyclic dimer

[Formula 167]

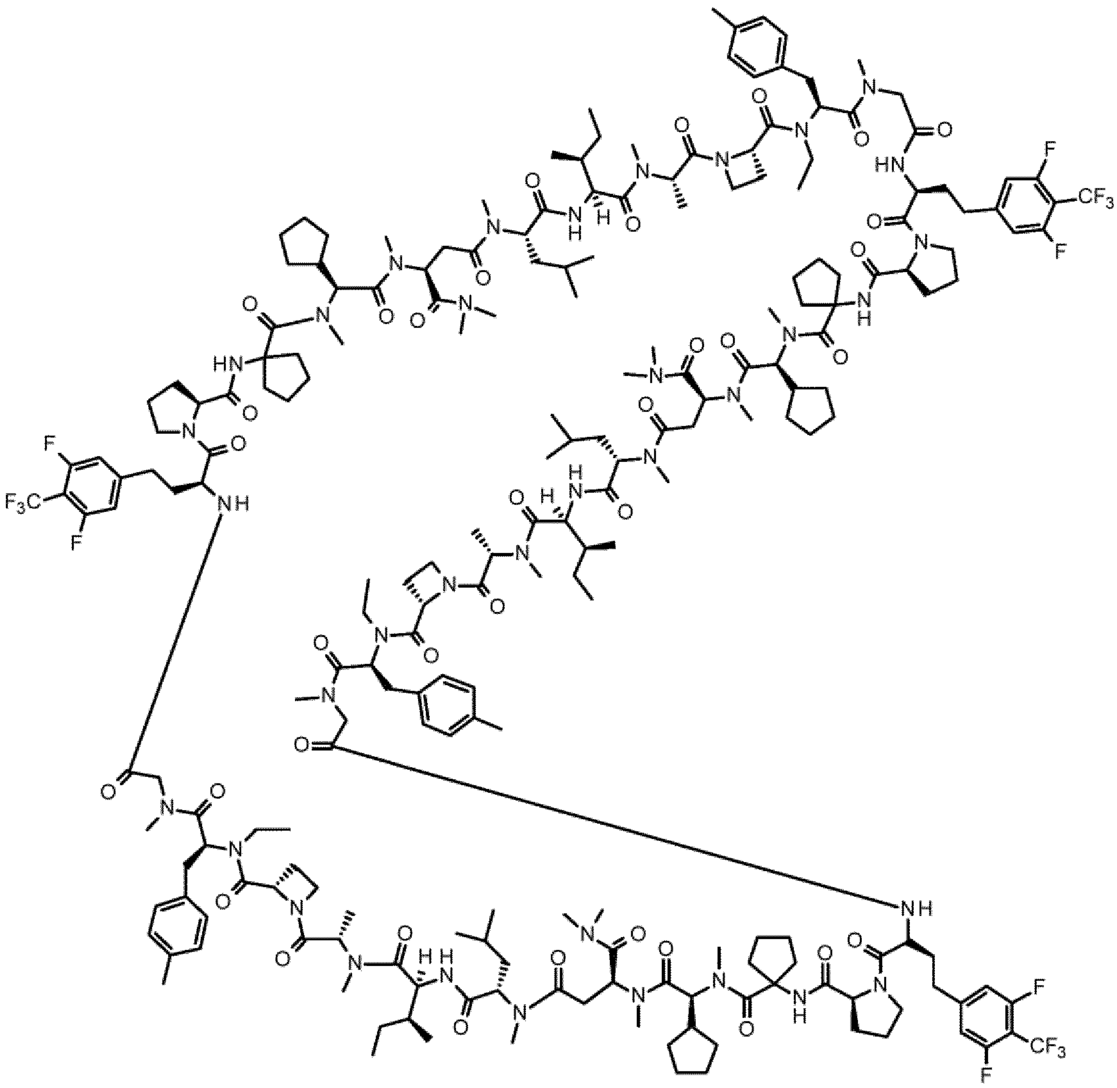

Cyclic trimer

Example 2.1.1

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide) (with use of COMU as condensation reagent, and acetonitrile as solvent)

Compound A36 (12.8 mg (6.84 μmol)) and an internal standard substance (methyl benzoate, 3.87 mg (26.4 μmol)) were weighed into a reaction vessel and dissolved in a solvent (acetonitrile, 2 mL (200 v/w)). DIPEA (5.49 μL (31.5 umol)) was added with stirring at room temperature. The external temperature of the reaction vessel was set to 25°C, and the condensation reagent (COMU, 11.2 mg (26.2 μmol)) was added, and then the mixture was stirred for 30 minutes. The reaction solution (50 μL) was diluted with a mixed solution (100 μL) of MeCN/propylamine (9 : 1) to prepare a solution for HPLC analysis. As the result of HPLC analysis using methyl benzoate as the internal standard substance, the yield was 84%.

LCMS (ESI) of compound 1: retention time: 18.81 minutes, m/z = 1439 $[M+H]^+$ (LCMS analysis conditions: method 3)
LCMS (ESI) of cyclic dimer (c-Dimer): retention time: 22.86 minutes, m/z = 2898 $[M+Na]^+$ (LCMS analysis conditions: method 3)
LCMS (ESI) of cyclic trimer (c-Trimer): retention time: 24.62 minutes, m/z = 2157 $[M+2H]^{2+}$ (LCMS analysis

conditions: method 3)

Operations were performed using the condensation reagent and the solvent shown in the table below in the same manner as the experiment of the synthesis of compound 1 in which COMU was used as the condensation reagent, and acetonitrile as the solvent, and consumption of starting material (SM, compound A36), production of target material (TM, compound 1) and formation of byproducts (cyclic dimer (c-Dimer) and cyclic trimer (c-Trimer)) were measured to investigate preferred reaction conditions. The table summarizes area% ratios (Area% ratio) of the starting material, target material, cyclic dimer, and cyclic trimer, and the yield of the target material (yield%).

[Table 6]

| Condensation reagent | Example No. | Solvent | Dropping method | Ratio of SM, TM, Epimer, c-Dimer and c-Trimer (%) | | | | | yield (%) |
|---|---|---|---|---|---|---|---|---|---|
| | | | | SM | TM | Epimer | c-Dimer | c-Trimer | |
| COMU | Example 2.1.1 | MeCN | normal | ND | 94.1 | 1.2 | 4.4 | 0.3 | 84 |
| | Example 2.1.2 | dimethyl carbonate | normal | ND | 92.8 | 1.8 | 5.1 | 0.3 | 68 |
| HATU | Example 2.1.3 | MeCN | normal | 0.1 | 92.0 | 1.8 | 5.7 | 0.4 | 57 |
| | Example 2.1.4 | dimethyl carbonate | normal | ND | 93.9 | 1.5 | 4.3 | 0.4 | 67 |
| DMT-MM | Example 2.1.5 | MeCN | normal | 0.1 | 90.9 | 1.0 | 7.4 | 0.6 | 74 |
| | Example 2.1.6 | dimethyl carbonate | normal | 0.1 | 92.1 | 0.6 | 6.7 | 0.6 | 69 |
| PyOxim | Example 2.1.7 | MeCN | normal | 1.6 | 89.8 | 0.6 | 6.8 | 1.3 | 61 |
| | Example 2.1.8 | dimethyl carbonate | normal | 0.9 | 94.1 | 1.2 | 3.8 | ND | 62 |
| PyBOP | Example 2.1.9 | MeCN | normal | ND | 88.4 | 3.9 | 7.0 | 0.8 | 84 |
| PyClop | Example 2.1.10 | MeCN | normal | 11.7 | 78.9 | 0.8 | 7.6 | 1.0 | 56 |

Example 2.2.1

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide) (with use of COMU as condensation reagent, and acetonitrile as solvent)

[Formula 168]

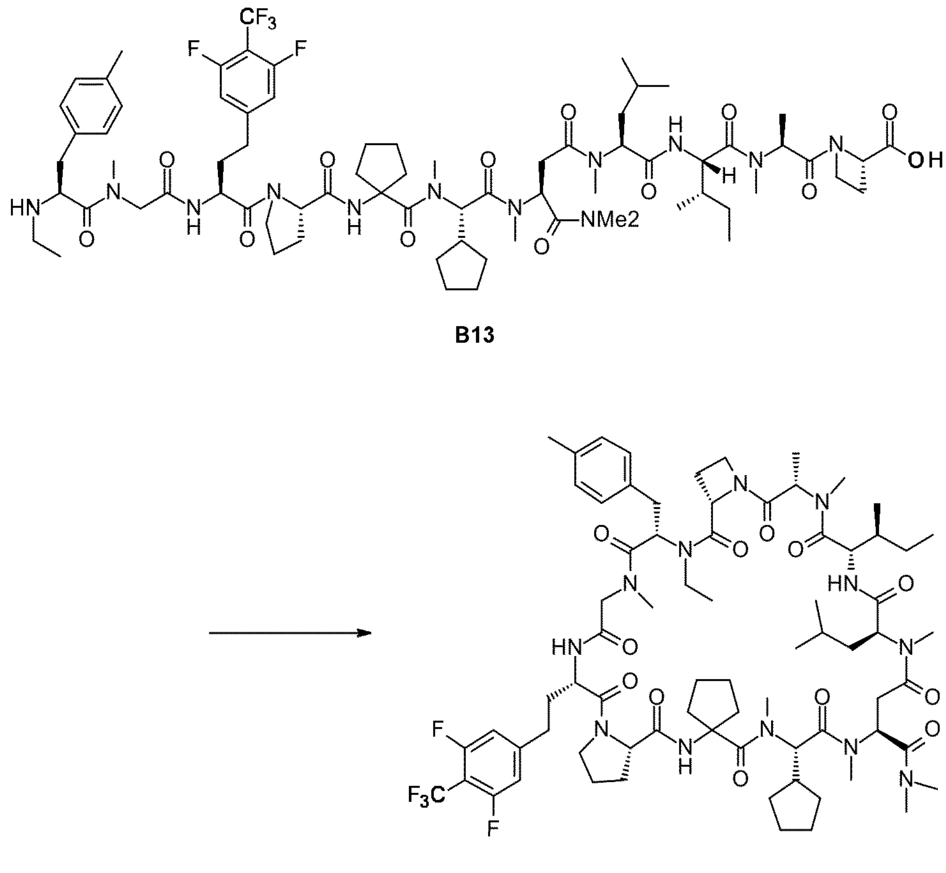

B13

1

Compound B13 (11.66 mg, content 88.5%) and an internal standard substance (methyl benzoate, 3.11 mg) were weighed into a reaction vessel and dissolved in acetonitrile (2 mL). DIPEA (5.69 μL) was added with stirring at room temperature. The external temperature of the reaction vessel was set to 25°C, and COMU (11.61 mg) was added, and then the mixture was stirred for 2 hours. The total amount of the reaction solution was diluted with a mixed solution (10 mL) of MeCN/propylamine (9 : 1) to prepare a solution for HPLC analysis. As the result of HPLC analysis using methyl benzoate as the internal standard substance, the yield was 86% (HPLC analysis conditions: method 3).

LCMS (ESI) of propylamide derivative of compound B13: retention time: 13.74 minutes, m/z = 1498 $[M+H]^+$ (LCMS analysis conditions: method 3)

[Formula 169]

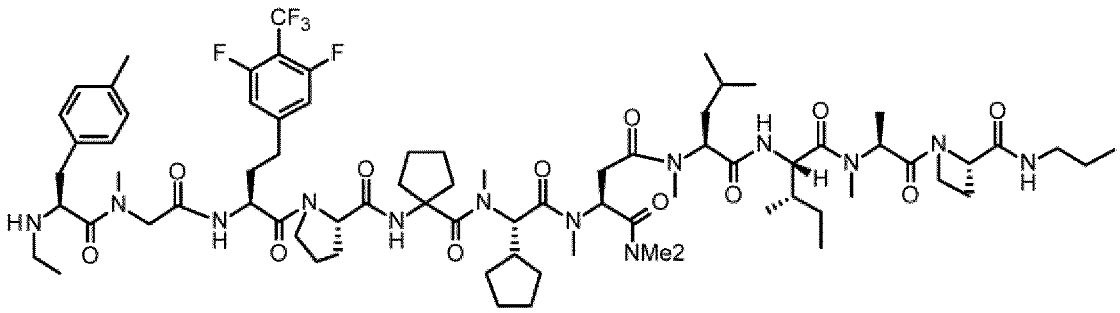

Propylamide derivative of compound B13

The cyclization reaction of Pseudo-High-Dilution was performed under the reaction conditions of Example 2.2.1. To a solution of the condensation reagent, a solution of the starting material and base was added dropwise for a long time.

Example 2.2.2

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-1111,4,7,10,13,16,19.22,25,28,31]undecaazacyclotetratriacontin-21,1'-cyclopentane]-15-carboxamide) (with use of COMU as condensation reagent, and acetonitrile as solvent, inverse dropwise addition)

Compound B13 (11.71 mg, content 88.5%) and an internal standard substance (methyl benzoate, 3.04 mg) were weighed into a reaction vessel and dissolved in acetonitrile (1 mL). DIPEA (5.69 μL) was added with stirring at room temperature to obtain a solution containing compound B13. To another reaction vessel after nitrogen replacement, COMU (11.64 mg) and acetonitrile (1 mL) were added. The external temperature of the reaction vessel containing the COMU solution was set to 25°C, and the solution containing compound B13 was added dropwise to the reaction mixture over 3 hours. Washing was performed with acetonitrile (0.05 mL), a rinse solution was added, and the mixture was stirred for 2 hours. The total amount of the reaction solution was diluted with a mixed solution (10 mL) of MeCN/propylamine (9 : 1) to prepare a solution for HPLC analysis. As the result of HPLC analysis using methyl benzoate as the internal standard substance, the yield was 83% (HPLC analysis conditions: method 3).

Cyclization reaction of compound B13 (Investigation of reaction conditions in Example 2.2.1)

The results of the synthesis of compound 1 using various condensation reagents and solvents are shown in Table 7. The experimental operation was in accordance with the case in which COMU was used as the condensation reagent, and acetonitrile was used as the solvent (Example 2.2.1). Regarding the dropping method in the table, the method of adding a condensation reagent to a solution of a starting material, a base and a solvent is "normal", and the method of dropping a solution of a starting material and a base to a solution of a condensation reagent for a long time is "inverse". SM in the table represents compound B13, and SM-NHPr represents propylamide derivative of compound B13. TM in the table is a target material (compound 1) and epimer is an epimer of compound 1. c-Dimer and c-Trimer are cyclic dimers and cyclic trimers, respectively, which are byproducts. Their generation amounts were shown as Area% ratio by HPLC measurement (HPLC analysis condition: method 3). The yield was calculated by HPLC analysis using methyl benzoate as the internal standard substance.

[Table 7]

| Solvent | Example No. | Condensation reagent | Dropping method | Ratio of SM, SM-NHPr, TM, Epimer, c-Dimer and c-Trimer (%) | | | | | | yield (%) |
|---|---|---|---|---|---|---|---|---|---|---|
| | | | | SM | SM-NHPr | TM | Epimer | c-Dimer | c-Trimer | |
| MeCN | Example 2.2.1 | COMU | normal | 0.01 | 0.08 | 97.9 | 0.5 | 1.5 | ND | 86 |
| | Example 2.2.2 | COMU | inverse | 0.06 | 0.07 | 99.2 | 0.6 | 0.2 | ND | 83 |
| | Example 2.2.3 | HATU | normal | 0.5 | 1.1 | 94.8 | 0.8 | 2.8 | ND | 94 |
| | Example 2.2.4 | PyBOP | normal | 0.2 | 81.2 | 18.4 | 0.1 | 0.1 | ND | 15 |
| | Example 2.2.5 | PyOxime | normal | 39.7 | 0.6 | 58.5 | 0.3 | 0.9 | ND | 44 |
| | Example 2.2.6 | PyClop | normal | 0.2 | 47.4 | 51.9 | ND | 0.5 | ND | 49 |
| | Example 2.2.7 | DMT-MM | normal | 1.4 | 0.1 | 96.6 | 0.7 | 1.1 | ND | 62 |
| | Example 2.2.8 | DEPBT | normal | 27.9 | 19.5 | 51.3 | 0.3 | 1.0 | ND | 47 |

(continued)

| Solvent | Example No. | Condensation reagent | Dropping method | Ratio of SM, SM-NHPr, TM, Epimer, c-Dimer and c-Trimer (%) | | | | | | yield (%) |
|---|---|---|---|---|---|---|---|---|---|---|
| | | | | SM | SM-NHPr | TM | Epimer | c-Dimer | c-Trimer | |
| | Example 2.2.9 | FDPP | normal | 4.2 | 45.0 | 50.6 | 0.1 | 0.03 | ND | 47 |
| | Example 2.2.10 | T3P | normal | 6.8 | 22.1 | 70.1 | 0.6 | 0.4 | ND | 63 |
| | Example 2.2.11 | BEP-BF4 | normal | 45.9 | 0.3 | 52.9 | ND | 0.9 | ND | 48 |
| dimethyl carbonate | Example 2.2.12 | HATU | normal | 6.9 | 0.6 | 90.7 | 0.7 | 1.1 | ND | 96 |
| | Example 2.2.13 | COMU | normal | ND | 0.7 | 96.3 | 0.8 | 2.3 | ND | 85 |
| 2-MeTHF | Example 2.2.14 | HATU | normal | 21.0 | 7.9 | 70.0 | 0.3 | 0.7 | ND | 52 |

Example 2.3.1

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide) (with use of HATU as condensation reagent, and dimethyl carbonate as solvent)

[Formula 170]

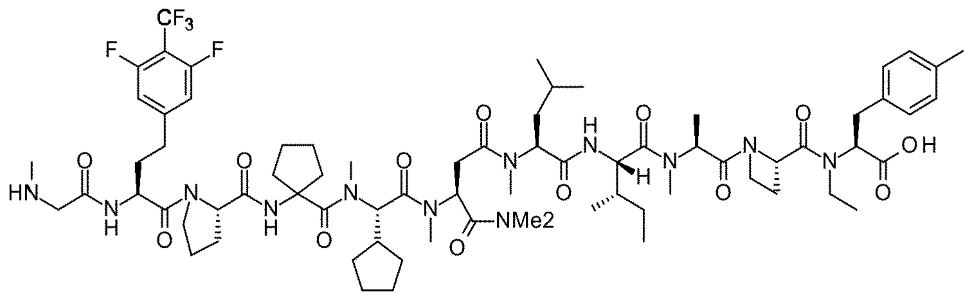

C15

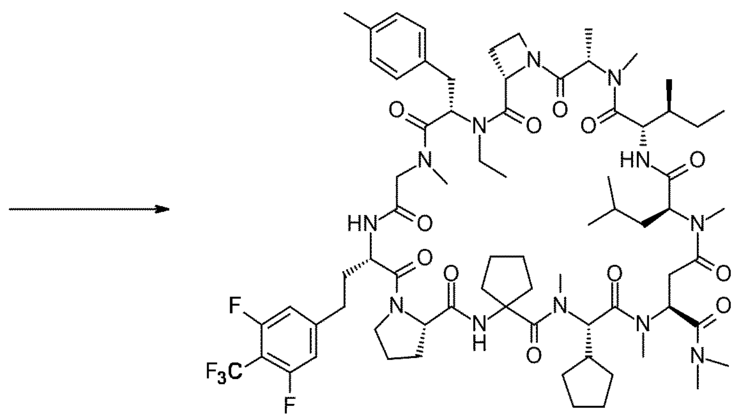

1

Compound C15 (11.22 mg, content 88.1%) and an internal standard substance (methyl benzoate, 3.03 mg) were weighed into a reaction vessel and dissolved in dimethyl carbonate (2 mL). DIPEA (5.69 μL) was added with stirring at room temperature. The external temperature of the reaction vessel was set to 25°C, and HATU (10.82 mg) was added, and then the mixture was stirred for 2 hours. The total amount of the reaction solution was diluted with a mixed solution (10 mL) of MeCN/propylamine (9 : 1) to prepare a solution for HPLC analysis. As the result of HPLC analysis using methyl benzoate as the internal standard substance, the yield was 84% (HPLC analysis conditions: method 3).

LCMS (ESI) of propylamide derivative of compound C15: retention time: 15.04 minutes, m/z = 1498 $[M+H]^+$ (LCMS analysis conditions: method 3)

[Formula 171]

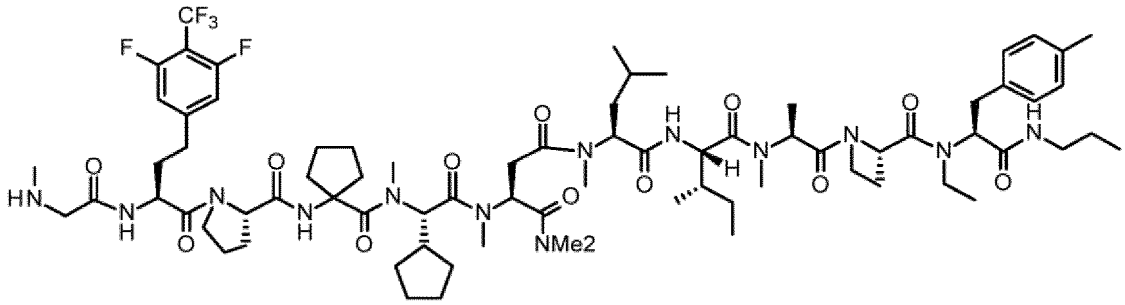

Propylamide derivative of compound C15

The cyclization reaction of Pseudo-High-Dilution was performed under the reaction conditions of Example 2.3.1. To a solution of the condensation reagent, a solution of the starting material and the base was added dropwise for a long time.

Example 2.3.2

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide) (with use of HATU as condensation reagent, and dimethyl carbonate as solvent, inverse dropwise addition)

Compound C15 (11.52 mg, content 88.1%) and an internal standard substance (methyl benzoate, 3.04 mg) were weighed into a reaction vessel and dissolved in dimethyl carbonate (1 mL). DIPEA (5.69 μL) was added with stirring at

room temperature to obtain a solution containing compound C15. To another reaction vessel after nitrogen replacement, HATU (10.52 mg) and dimethyl carbonate (1 mL) were added. The external temperature of the reaction vessel containing the HATU solution was set to 25°C, and the solution containing compound C15 was added dropwise to the reaction mixture over 3 hours. Washing was performed with dimethyl carbonate (0.05 mL), a rinse solution was added, and the mixture was stirred for 2 hours. The total amount of the reaction solution was diluted with a mixed solution (10 mL) of MeCN/propylamine (9 : 1) to prepare a solution for HPLC analysis. As the result of HPLC analysis using methyl benzoate as the internal standard substance, the yield was 82%

(HPLC analysis conditions: method 3).

Cyclization reaction of compound C15 (Investigation of reaction conditions in Example 2.3.1)

The results of the synthesis of compound 1 using various condensation reagents and solvents are shown in Table 8. The experimental operation was in accordance with the case in which HATU was used as the condensation reagent, and dimethyl carbonate was used as the solvent (Example 2.3.1). Regarding the dropping method in the table, the method of adding a condensation reagent to a solution of a starting material, a base and a solvent is "normal", and the method of dropping a solution of a starting material and a base to a solution of a condensation reagent for a long time is "inverse". SM in the table represents compound C15, and SM-NHPr represents propylamide derivative of compound C15. TM in the table is a target material (compound 1) and epimer is an epimer of compound 1. c-Dimer and c-Trimer are cyclic dimers and cyclic trimers, respectively, which are byproducts. Their generation amounts were shown as Area% ratio by HPLC measurement (HPLC analysis condition: method 3). The yield was calculated by HPLC analysis using methyl benzoate as the internal standard substance.

[Table 8]

| Solvent | Example No. | Condensation reagent | Dropping method | Ratio of SM, SM-NHPr, TM, Epimer, c-Dimer and c-Trimer (%) | | | | | | yield (%) |
|---|---|---|---|---|---|---|---|---|---|---|
| | | | | SM | SM-NHPr | TM | Epimer | c-Dimer | c-Trimer | |
| dimethyl carbonate | Example 2.3.1 | HATU | normal | 0.5 | 0.1 | 95.5 | 2.3 | 1.3 | 0.3 | 84 |
| | Example 2.3.2 | HATU | inverse | 0.2 | ND | 96.9 | 2.7 | 0.3 | 0.01 | 82 |
| | Example 2.3.3 | COMU | normal | 0.1 | 0.3 | 92.6 | 3.3 | 3.2 | 0.6 | 86 |
| MeCN | Example 2.3.4 | HATU | normal | 0.2 | 0.5 | 87.9 | 3.9 | 6.7 | 0.7 | 74 |
| | Example 2.3.5 | COMU | normal | ND | ND | 88.5 | 7.2 | 4.0 | 0.3 | 72 |
| | Example 2.3.6 | PyBOP | normal | 2.8 | 1.0 | 78.8 | 11.1 | 5.9 | 0.5 | 55 |
| | Example 2.3.7 | PyOxime | normal | 1.1 | ND | 84.5 | 8.0 | 5.8 | 0.7 | 54 |
| | Example 2.3.8 | PyClop | normal | 0.1 | 4.2 | 61.0 | 9.9 | 18.3 | 6.5 | 66 |
| | Example 2.3.9 | DMT-MM | normal | 0.5 | 0.8 | 86.1 | 10.8 | 1.3 | 0.5 | 63 |
| | Example 2.3.10 | DEPBT | normal | 4.0 | 0.1 | 71.8 | 21.1 | 2.8 | 0.2 | 59 |

Example 2.4.1

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide) (with use of COMU as condensation reagent, and dimethyl carbonate as solvent)

[Formula 172]

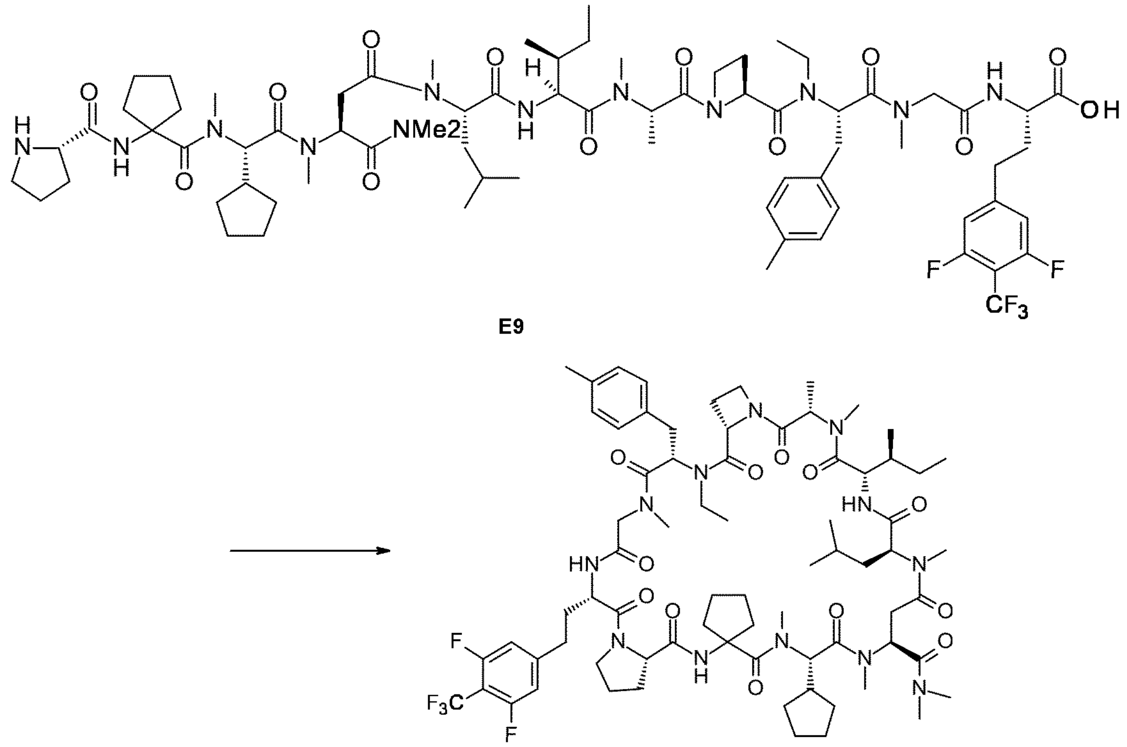

E9

1

Compound E9 (11.77 mg, content 86.0%) and an internal standard substance (methyl benzoate, 4.82 mg) were weighed into a reaction vessel and dissolved in dimethyl carbonate (2 mL). DIPEA (5.60 μL) was added with stirring at room temperature. The external temperature of the reaction vessel was set to 25°C, and COMU (11.96 mg) was added, and then the mixture was stirred for 2 hours. The total amount of the reaction solution was diluted with a mixed solution (10 mL) of MeCN/propylamine (9 : 1) to prepare a solution for HPLC analysis. As the result of HPLC analysis using methyl benzoate as the internal standard substance, the yield was 82% (HPLC analysis conditions: method 3).

LCMS (ESI) of propylamide derivative of compound E9: retention time: 14.63 minutes, m/z = 1498 $[M+H]^+$ (LCMS analysis conditions: method 3)

[Formula 173]

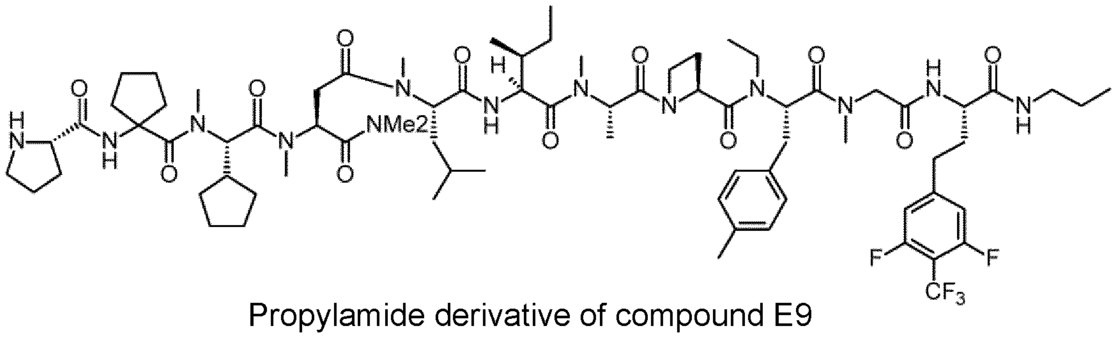

Propylamide derivative of compound E9

The cyclization reaction of Pseudo-High-Dilution was performed under the reaction conditions of Example 2.4.1. To a solution of the condensation reagent, a solution of the starting material and the base was added dropwise for a long time.

Example 2.4.2

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide) (with use of COMU as condensation reagent, and dimethyl carbonate as solvent,

inverse dropwise addition)

Compound E9 (12.34 mg, content 86.0%) and an internal standard substance (methyl benzoate, 4.89 mg) were weighed into a reaction vessel and dissolved in dimethyl carbonate (1 mL). DIPEA (5.84 μL) was added with stirring at room temperature to obtain a solution containing compound E9. To another reaction vessel after nitrogen replacement, COMU (13.77 mg) and dimethyl carbonate (1 mL) were added. The external temperature of the reaction vessel containing the COMU solution was set to 25°C, and the solution containing compound E9 was added dropwise to the reaction mixture over 3 hours. Washing was performed with dimethyl carbonate (0.05 mL), a rinse solution was added, and the mixture was stirred for 2 hours. The total amount of the reaction solution was diluted with a mixed solution (10 mL) of MeCN/propylamine (9 : 1) to prepare a solution for HPLC analysis. As the result of HPLC analysis using methyl benzoate as the internal standard substance, the yield was 88% (HPLC analysis conditions: method 3).

Cyclization reaction of compound E9 (Investigation of reaction conditions in Example 2.4.1)

The results of the synthesis of compound 1 using various condensation reagents and solvents are shown in Table 9. The experimental operation was in accordance with the case in which COMU was used as the condensation reagent, and dimethyl carbonate was used as the solvent (Example 2.4.1). Regarding the dropping method in the table, the method of adding a condensation reagent to a solution of a starting material, a base and a solvent is "normal", and the method of dropping a solution of a starting material and a base to a solution of a condensation reagent for a long time is "inverse". SM in the table represents compound E9, and SM-NHPr represents propylamide derivative of compound E9. TM in the table is a target material (compound 1) and epimer is an epimer of compound 1. c-Dimer and c-Trimer are cyclic dimers and cyclic trimers, respectively, which are byproducts. Their generation amounts were shown as Area% ratio by HPLC measurement (HPLC analysis condition: method 3). The yield was calculated by HPLC analysis using methyl benzoate as the internal standard substance.

[Table 9]

| Solvent | Example No. | Condensation reagent | Dropping method | Ratio of SM, SM-NHPr, TM, Epimer, c-Dimer and c-Trimer (%) | | | | | | yield (%) |
|---|---|---|---|---|---|---|---|---|---|---|
| | | | | SM | SM-NHPr | TM | Epimer | c-Dimer | c-Trimer | |
| dimethyl carbonate | Example 2.4.1 | COMU | normal | 0.14 | ND | 95.02 | 0.19 | 3.98 | 0.66 | 82 |
| | Example 2.4.2 | COMU | inverse | 0.2 | ND | 98.14 | 0.51 | 1.15 | ND | 88 |
| MeCN | Example 2.4.3 | HATU | normal | 0.16 | 0.15 | 73.18 | 1.11 | 20.5 | 4.91 | 54 |
| | Example 2.4.4 | COMU | normal | ND | ND | 91.93 | 0.24 | 6.89 | 0.94 | 72 |
| | Example 2.4.5 | PyBOP | normal | ND | 3.17 | 49.7 | 33.68 | 11.92 | 1.53 | 30 |
| | Example 2.4.6 | PyOxim | normal | ND | 0.21 | 87.58 | 0.22 | 10.13 | 1.85 | 66 |
| | Example 2.4.7 | PyClop | normal | ND | 30.7 | 49.3 | 7.68 | 10.77 | 1.55 | 25 |
| | Example 2.4.8 | DMT-MM | normal | ND | ND | 79.11 | 0.23 | 16.71 | 3.94 | 58 |
| 2-MeTHF | Example 2.4.9 | COMU | normal | 0.31 | ND | 89.38 | 5.99 | 3.59 | 0.73 | 82 |
| anisole | Example 2.4.10 | COMU | normal | 1.79 | ND | 86.88 | 0.35 | 9.46 | 1.52 | 64 |

Example 2.5.1

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide) (with use of COMU as condensation reagent, and dimethyl carbonate as solvent)

[Formula 174]

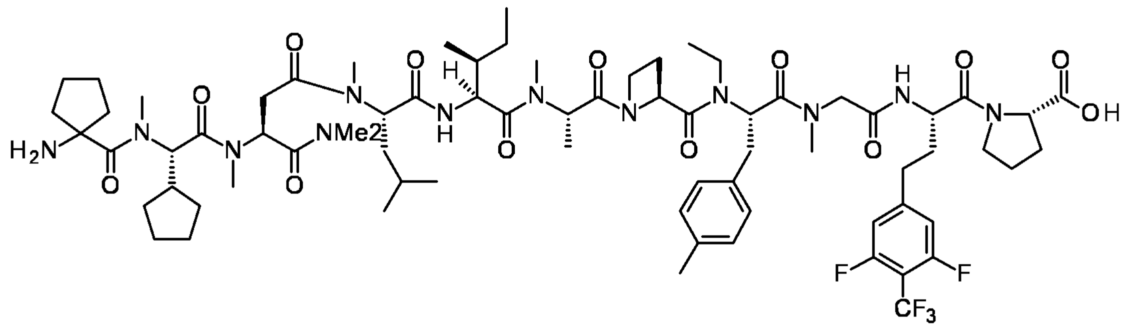

D13

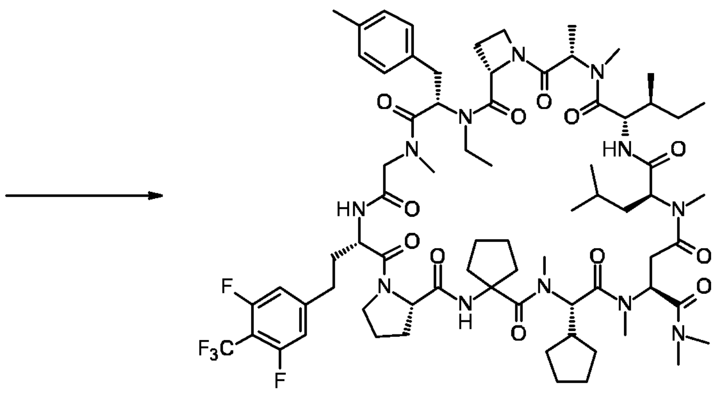

1

Compound D13 (10.90 mg, content 95.15%) and an internal standard substance (methyl benzoate, 4.74 mg) were weighed into a reaction vessel and dissolved in dimethyl carbonate (2 mL). DIPEA (5.70 μL) was added with stirring at room temperature. The external temperature of the reaction vessel was set to 25°C, and COMU (12.45 mg) was added, and then the mixture was stirred for 2 hours. The total amount of the reaction solution was diluted with a mixed solution (10 mL) of MeCN/propylamine (9 : 1) to prepare a solution for HPLC analysis. As the result of HPLC analysis using methyl benzoate as the internal standard substance, the yield was 71% (HPLC analysis conditions: method 3).

LCMS (ESI) of propylamide derivative of compound D13: retention time: 13.26 minutes, m/z = 1498 $[M+H]^+$ (LCMS analysis conditions: method 3)

[Formula 175]

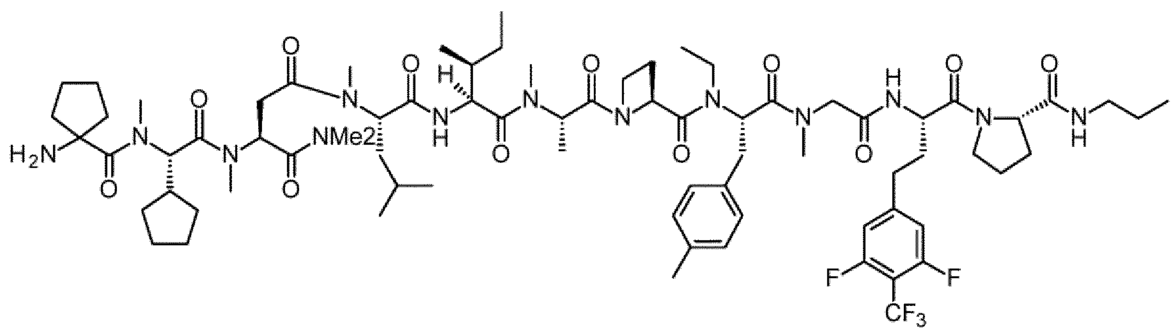

Propylamide derivative of compound D13

The cyclization reaction of Pseudo-High-Dilution was performed under the reaction conditions of Example 2.5.1. To a solution of the condensation reagent, a solution of the starting material and the base was added dropwise for a long time.

Example 2.5.2

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide) (with use of COMU as condensation reagent, and dimethyl carbonate as solvent, inverse dropwise addition)

Compound D13 (13.62 mg, content 95.15%) and an internal standard substance (methyl benzoate, 4.44 mg) were weighed into a reaction vessel and dissolved in dimethyl carbonate (1.3 mL). DIPEA (7.13 μL) was added with stirring at room temperature to obtain a solution containing compound D13. To another reaction vessel after nitrogen replacement, COMU (16.30 mg) and dimethyl carbonate (1.3 mL) were added. The external temperature of the reaction vessel containing the COMU solution was set to 25°C, and the solution containing compound D13 was added dropwise to the reaction mixture over 3 hours. Washing was performed with dimethyl carbonate (0.05 mL), a rinse solution was added, and the mixture was stirred for 2 hours. The total amount of the reaction solution was diluted with a mixed solution (10 mL) of MeCN/propylamine (9 : 1) to prepare a solution for HPLC analysis. As the result of HPLC analysis using methyl benzoate as the internal standard substance, the yield was 83% (HPLC analysis conditions: method 3).

Cyclization reaction of compound D13 (Investigation of reaction conditions in Example 2.5.1)

The results of the synthesis of compound 1 using various condensation reagents and solvents are shown in Table 10. The experimental operation was in accordance with the case in which COMU was used as the condensation reagent, and dimethyl carbonate was used as the solvent (Example 2.5.1). Regarding the dropping method in the table, the method of adding a condensation reagent to a solution of a starting material, a base and a solvent is "normal", and the method of dropping a solution of a starting material and a base to a solution of a condensation reagent for a long time is "inverse". SM in the table represents compound D13, and SM-NHPr represents propylamide derivative of compound D13. TM in the table is a target material (compound 1) and epimer is an epimer of compound 1. c-Dimer and c-Trimer are cyclic dimers and cyclic trimers, respectively, which are byproducts. Their generation amounts were shown as Area% ratio by HPLC measurement (HPLC analysis condition: method 3). The yield was calculated by HPLC analysis using methyl benzoate as the internal standard substance.

[Table 10]

| Solvent | Example No. | Condensation reagent | Dropping method | Ratio of SM, SM-NHPr, Epimer, TM, c-Dimer and c-Trimer (%) | | | | | | yield (%) |
|---|---|---|---|---|---|---|---|---|---|---|
| | | | | SM | SM-NHPr | Epimer | TM | c-Dimer | c-Trimer | |
| dimethyl carbonate | Example 2.5.1 | COMU | normal | 0.31 | 0.18 | 0.03 | 82.91 | 13.33 | 3.24 | 71 |
| | Example 2.5.2 | COMU | inverse | 0.67 | 0.37 | 0.07 | 96.73 | 2.06 | 0.09 | 83 |
| MeCN | Example 2.5.3 | HATU | normal | 0.26 | 0.25 | 0.03 | 77.15 | 18.57 | 3.73 | 58 |
| | Example 2.5.4 | COMU | normal | 0.60 | 0.28 | 0.03 | 84.52 | 12.49 | 2.08 | 71 |
| | Example 2.5.5 | PyBOP | normal | ND | 1.63 | 1.74 | 75.55 | 17.35 | 3.74 | 57 |
| | Example 2.5.6 | PyOxim | normal | 0.19 | 0.52 | 0.38 | 81.50 | 14.37 | 3.04 | 68 |
| | Example 2.5.7 | PyClop | normal | ND | 0.87 | 0.75 | 66.63 | 23.03 | 8.69 | 30 |
| | Example 2.5.8 | DMT-MM | normal | 0.25 | 4.90 | 0.15 | 84.06 | 9.30 | 1.33 | 66 |
| 2-MeTHF | Example 2.5.9 | COMU | normal | 0.25 | 0.47 | 0.02 | 78.28 | 16.91 | 4.06 | 64 |

(continued)

| Solvent | Example No. | Condensation reagent | Dropping method | Ratio of SM, SM-NHPr, Epimer, TM, c-Dimer and c-Trimer (%) | | | | | | yield (%) |
|---|---|---|---|---|---|---|---|---|---|---|
| | | | | SM | SM-NHPr | Epimer | TM | c-Dimer | c-Trimer | |
| anisole | Example 2.5.10 | COMU | normal | 0.38 | 0.11 | 0.08 | 82.62 | 12.01 | 2.64 | 70 |

Example 3 (Comparison of cyclized positions)

Comparative Example 3.1

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide) (cyclization reaction of compound F1)

[Formula 176]

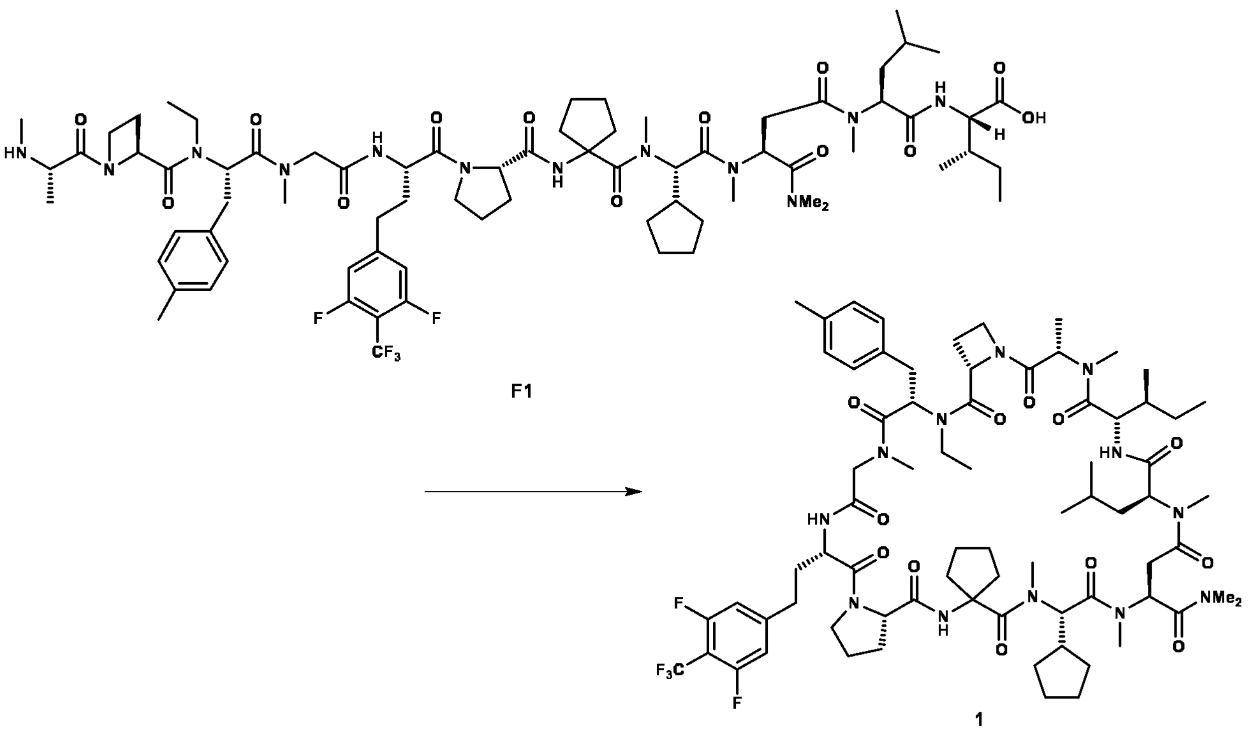

Compound F1 (15.03 mg, content 92.83%) was weighed into a reaction vessel and dissolved in MeCN (3.0 mL). DIPEA (8.28 μL) was added with stirring at room temperature. The external temperature of the reaction vessel was set to 25°C, and HATU (15.01 mg) was added, and then the mixture was stirred for 8 hours. The reaction solution (30 μL) was diluted with a mixed solution (60 μL) of MeCN/propylamine (9 : 1) to prepare a solution for HPLC analysis. The results of HPLC analysis are shown in Table 11 below. SM in the table represents a starting material (compound F1), TM represents a target material (compound 1), epimer represents an epimer of compound 1, c-Dimer represents a cyclic dimer, and c-Trimer represents a cyclic trimer. Their Area% ratios by HPLC measurement are shown. As the result, SM almost disappeared after 8 hours of reaction, and TM and Epimer were mainly formed, where the area ratio thereof was 38.4% and that of Epimer was 60.8%. Note that compound F1 was synthesized by existing methods using compound D4, compound A23, compound A4, and compound A6.

LCMS (ESI) of propylamide derivative of compound F1: retention time: 13.98 minutes, m/z = 1498 $[M+H]^+$ (LCMS analysis conditions: method 3)

[Formula 177]

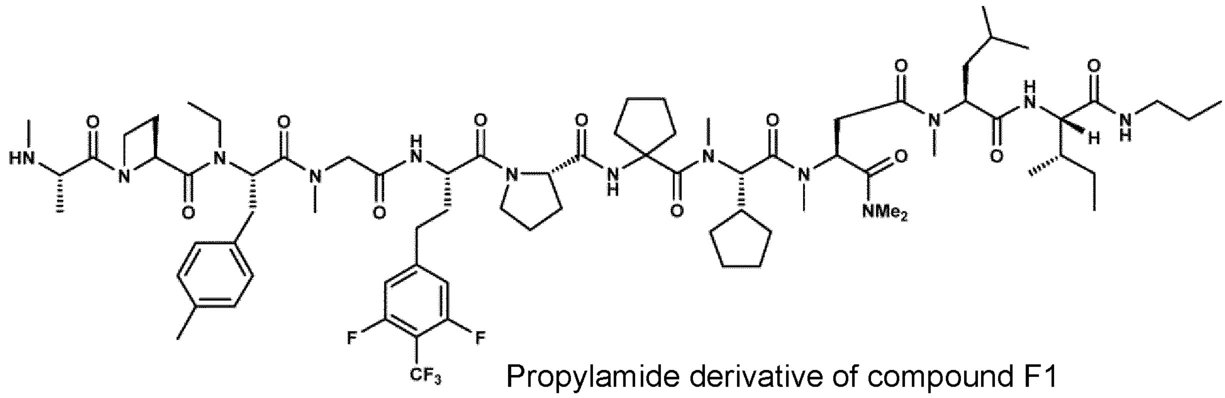

Propylamide derivative of compound F1

[Table 11]

| Ratio of SM, SM-NHPr, TM, Epimer, c-Dimer and c-Trimer (%) | | | | | |
|---|---|---|---|---|---|
| **SM** | **SM-NHPr** | **TM** | **Epimer** | **c-Dimer** | **c-Trimer** |
| **0.8** | **NA** | **38.4** | **60.8** | **0.2** | **NA** |

Example 3.2

Synthesis of compound 1: ((5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2.1-u]pyrrolo[2,1-i][1,4,7,10,13,16,19,22,25,28,31]undecaazacyclotetratriacontin-21,1'-cyclopentane]-15-carboxamide) (cyclization reaction of compound G1)

[Formula 178]

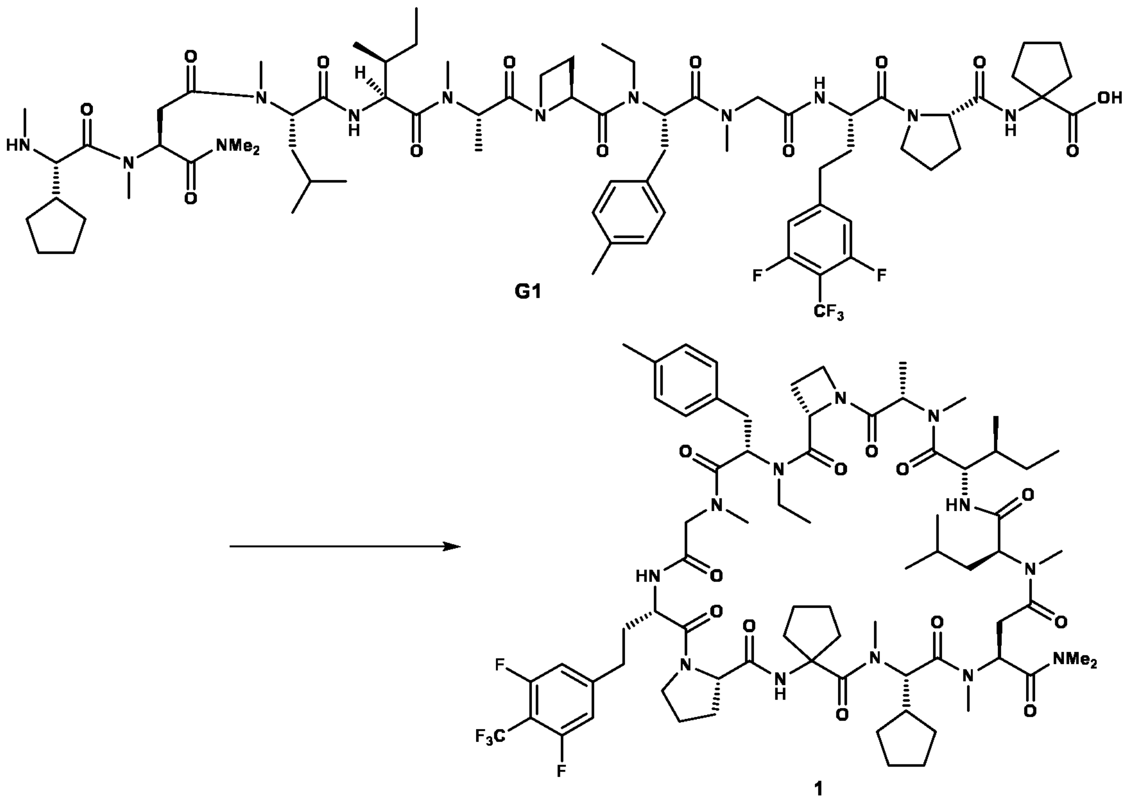

G1

1

Compound G1 (10.01 mg, content 90.79%) was weighed into a reaction vessel and dissolved in MeCN (1.56 mL). DIPEA (5.52 μL) was added with stirring at room temperature. The external temperature of the reaction vessel was set to 25°C, and a MeCN solution of HATU (0.059 M, 441 μL) was added, and then the mixture was stirred for 2 hours. The reaction solution (25 μL) was diluted with a mixed solution (125 μL) of MeCN/propylamine (9 : 1) to prepare a solution for HPLC analysis. The results of HPLC analysis are shown in Table 12 below. SM in the table represents a starting material (compound G1), TM represents a target material (compound 1), SM-NHPr represents propylamide derivative of compound G1, c-Dimer represents a cyclic dimer, and c-Trimer represents a cyclic trimer. Their Area% ratios by HPLC measurement are shown. As a result, the cyclization reaction almost did not proceed, and SM-NHPr was 99.8%. Note that compound G1 was synthesized by existing methods using compound A14, compound D7, compound A16 (or compound B24), compound A19 and compound A22.

LCMS (ESI) of propylamide derivative of compound G1: retention time: 13.70 minutes, m/z = 1498 $[M+H]^+$ (LCMS analysis conditions: method 3)

[Formula 179]

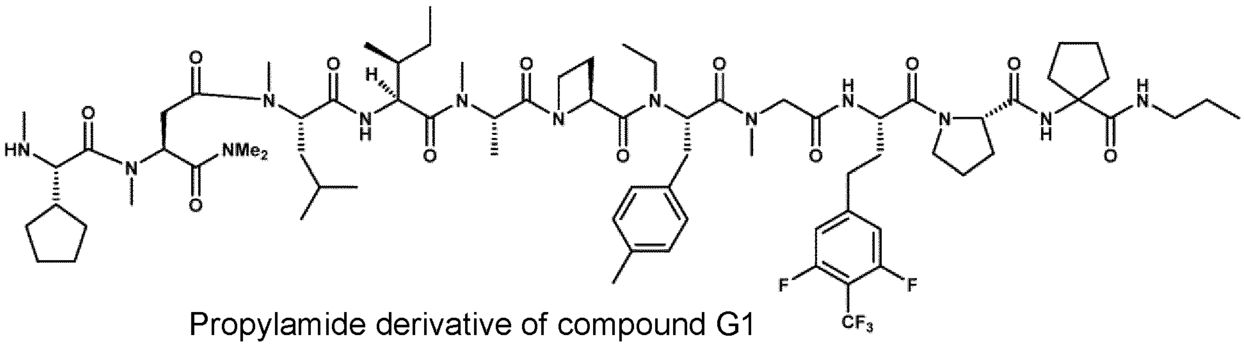

Propylamide derivative of compound G1

[Table 12]

| Ratio of SM, SM-NHPr, TM, c-Dimer and c-Trimer (%) | | | | |
|---|---|---|---|---|
| SM | SM-NHPr | TM | c-Dimer | c-Trimer |
| 0.5 | 96.3 | 3.2 | NA | NA |

[Industrial Applicability]

According to the present invention, provided are a method for producing a pharmaceutically useful cyclic peptide compound, or a salt thereof, or a solvate thereof, and a method for producing a peptide compound used in the production of the cyclic peptide compound, or a salt thereof, or a solvate thereof.

## Claims

1. A method for producing a cyclic peptide compound, or a salt thereof, or a solvate thereof, the method comprising:

    a step of linking an N-terminal amino acid residue of a peptide compound with a C-terminal amino acid residue of the peptide compound in a solvent, wherein
    at least one of the N-terminal amino acid residue and the C-terminal amino acid residue is a cyclic amino acid residue, and
    the solvent comprises one or more selected from the group consisting of acetonitrile, dimethyl carbonate, 2-MeTHF, and anisole.

2. A method for producing a cyclic peptide compound, or a salt thereof, or a solvate thereof, the method comprising:

    a step of linking an N-terminal amino acid residue of a peptide compound with a C-terminal amino acid residue of the peptide compound in a solvent, wherein
    the N-terminal amino acid residue and the C-terminal amino acid residue of the peptide compound are any of the following a) to e):

    a) the N-terminal amino acid residue is an N-substituted phenylalanine residue or an N-substituted phenylalanine derivative residue, and the C-terminal amino acid residue is a cyclic amino acid residue;
    b) the N-terminal amino acid residue is an $\alpha,\alpha$-di-substituted amino acid residue, and the C-terminal amino acid residue is a cyclic amino acid residue;
    c) the N-terminal amino acid residue is a cyclic amino acid residue, and the C-terminal amino acid residue is a homophenylalanine residue or a homophenylalanine derivative residue;
    d) the N-terminal amino acid residue is a cyclic amino acid residue, and the C-terminal amino acid residue is an N-substituted Ala residue; and
    e) the N-terminal amino acid residue is an N-substituted Gly residue, and the C-terminal amino acid residue is an N-substituted phenylalanine residue or an N-substituted phenylalanine derivative residue.

3. The method according to claim 2, wherein the solvent comprises one or more selected from the group consisting of acetonitrile, dimethyl carbonate, 2-MeTHF, and anisole.

4. The method according to any one of claims 1 to 3, wherein the linking the N-terminal amino acid residue of the peptide compound with the C-terminal amino acid residue of the peptide compound is linking an amino group of the N-terminal amino acid residue with a carboxyl group of the C-terminal amino acid residue.

5. The method according to any one of claims 1 to 4, wherein the linking step is performed in the presence of a condensation reagent.

6. The method according to claim 5, wherein the condensation reagent is one selected from the group consisting of HATU, COMU, DMT-MM, PyOxim, PyBOP, and PyClop.

7. The method according to any one of claims 1 to 6, wherein the linking step is performed by a liquid phase method.

**8.** The method according to any one of claims 1 and 3 to 7, wherein the N-terminal amino acid residue of the peptide compound is a cyclic amino acid residue and the C-terminal amino acid residue is a non-natural amino acid residue, or the N-terminal amino acid residue of the peptide compound is a non-natural amino acid residue and the C-terminal amino acid residue is a cyclic amino acid residue.

**9.** The method according to any one of claims 2 to 7, wherein the N-terminal amino acid residue and the C-terminal amino acid residue contained in the peptide compound are one selected from the following a') to e'):

a') the N-terminal amino acid residue is an EtPhe(4-Me) residue, and the C-terminal amino acid residue is an Aze(2) residue;
b') the N-terminal amino acid residue is a cLeu residue, and the C-terminal amino acid residue is a Pro residue;
c') the N-terminal amino acid residue is a Pro residue, and the C-terminal amino acid residue is a Hph(3,5-diF-4-$CF_3$) residue;
d') the N-terminal amino acid residue is a MeGly residue, and the C-terminal amino acid residue is an EtPhe(4-Me) residue; and
e') the N-terminal amino acid residue is an Aze(2) residue, and the C-terminal amino acid residue is a MeAla residue.

**10.** The method according to any one of claims 1 to 9, wherein the number of amino acid residues of the cyclic peptide compound is from 9 to 15.

**11.**

The method according to any one of claims 1 to 10, wherein the peptide compound is a linear peptide compound selected from the group consisting of:

[Formula 1]

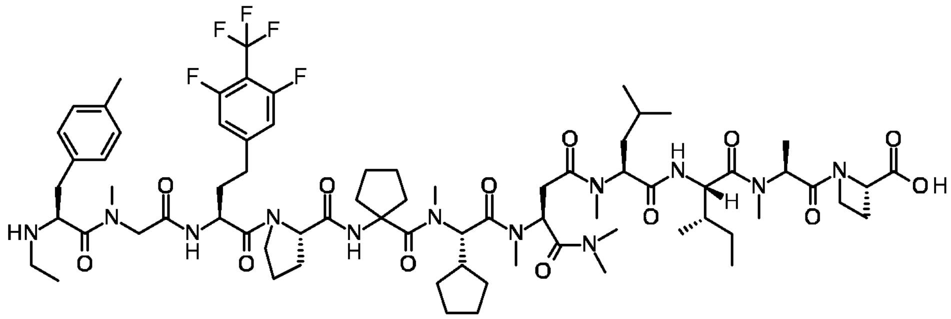

,

[Formula 2]

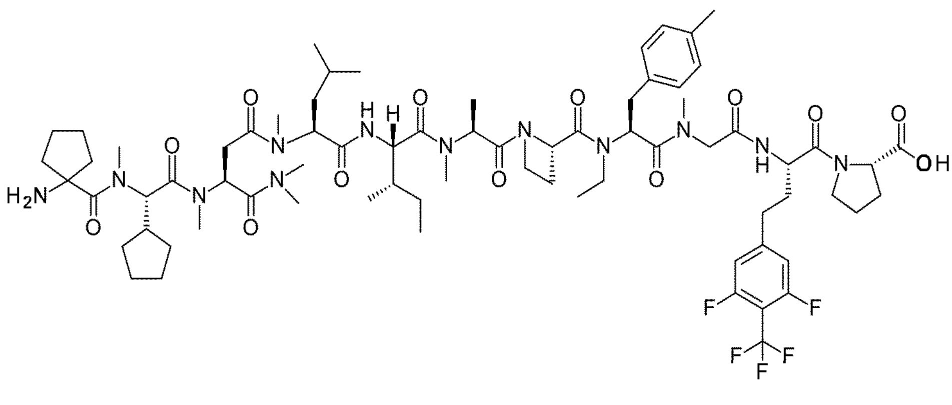

,

[Formula 3]

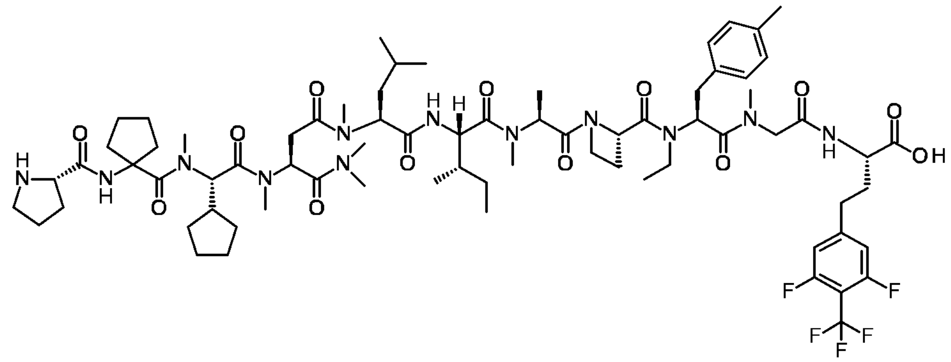

,

[Formula 4]

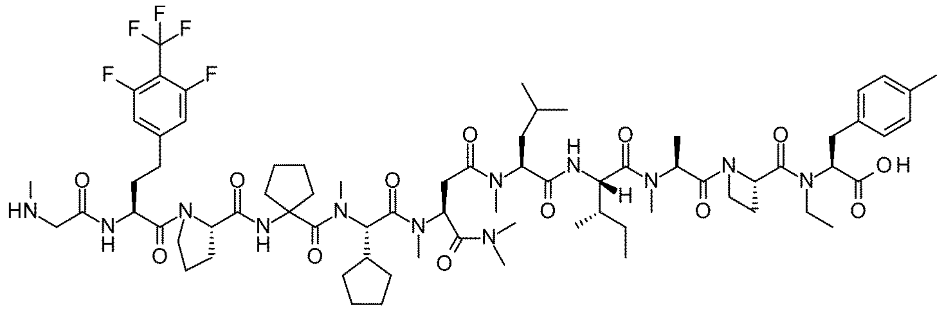

,

and

[Formula 5]

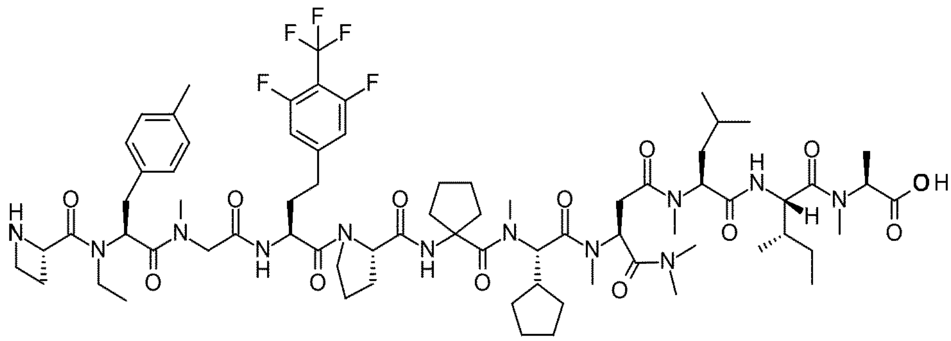

, or a salt thereof, or a solvate thereof.

**12.** The method according to any one of claims 1 to 11, wherein the cyclic peptide compound, or a salt thereof, or a solvate thereof is a solvate of the cyclic peptide compound.

**13.** The method according to claim 12, wherein the solvate of the cyclic peptide compound is a hydrate of the cyclic peptide compound.

**14.** The method according to any one of claims 1 to 13, wherein the cyclic peptide compound is represented by the following formula (2):

[Formula 6]

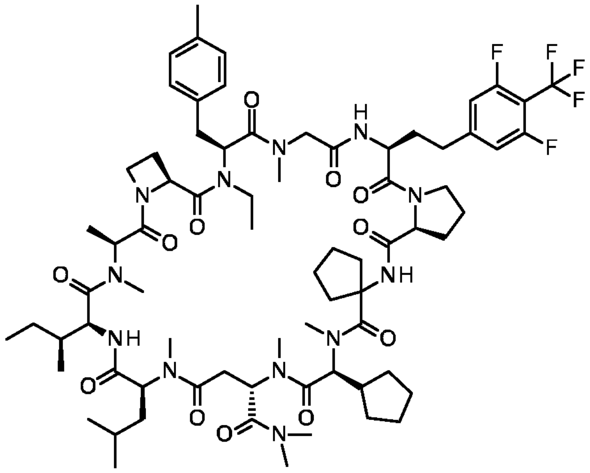

(2)

15. The method according to any one of claims 1 to 14, further comprising a step of isolating and/or purifying the cyclic peptide compound by crystallization to obtain a crystal of the cyclic peptide compound.

16. The method according to claim 15, wherein the crystal of the cyclic peptide compound is a non-solvate crystal or a solvate crystal of the cyclic peptide compound represented by the following formula (2):

[Formula 7]

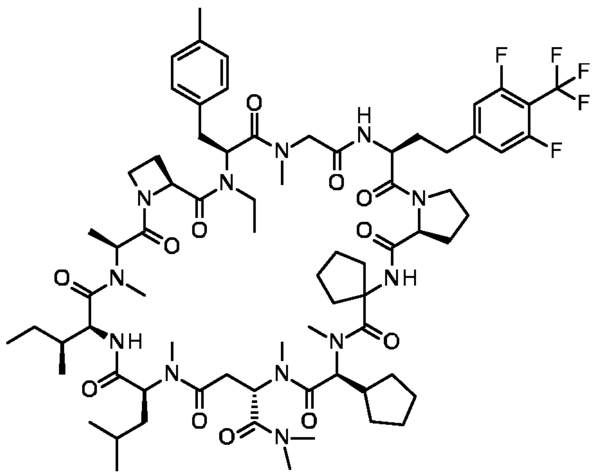

(2)

17. The method according to claim 16, wherein the crystal of the cyclic peptide compound is a solvate crystal.

18. The method according to claim 17, wherein the solvate crystal of the cyclic peptide compound is a hydrate crystal.

## INTERNATIONAL SEARCH REPORT

International application No.
**PCT/JP2023/037071**

**A. CLASSIFICATION OF SUBJECT MATTER**

***C07K 7/64***(2006.01)i; ***C07K 1/02***(2006.01)i
FI: C07K7/64; C07K1/02

According to International Patent Classification (IPC) or to both national classification and IPC

**B. FIELDS SEARCHED**

Minimum documentation searched (classification system followed by classification symbols)

C07K7/64; C07K1/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY/MEDLINE/EMBASE/BIOSIS (STN)、JSTPlus/JMEDPlus/JST7580 (JDreamIII)、PubMed、Japio-GPG/FX

**C. DOCUMENTS CONSIDERED TO BE RELEVANT**

| Category* | Citation of document, with indication, where appropriate, of the relevant passages | Relevant to claim No. |
|---|---|---|
| X | WO 2022/138598 A1 (CHUGAI SEIYAKU KABUSHIKI KAISHA) 30 June 2022 (2022-06-30)<br>synthesis examples 23, 24, examples 12-16 | 1-8, 10, 12-13 |
| Y | synthesis examples 23, 24, examples 12-16 | 1-18 |
| X | WO 2022/138891 A1 (CHUGAI SEIYAKU KABUSHIKI KAISHA) 30 June 2022 (2022-06-30)<br>claims 1, 16, paragraph [0197], examples 3, 4, 3-5 | 1, 4-8, 10, 12-13, 15 |
| Y | claims 1, 16, paragraph [0197], examples 3, 4, 3-5 | 1-18 |
| Y | WO 2021/090855 A1 (CHUGAI SEIYAKU KABUSHIKI KAISHA) 14 May 2021 (2021-05-14)<br>paragraphs [0863], [0867]-[1110], compound 1217 | 1-18 |
| A | WO 2021/090856 A1 (CHUGAI SEIYAKU KABUSHIKI KAISHA) 14 May 2021 (2021-05-14)<br>entire text, all drawings | 1-18 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 December 2023** | **09 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/037071**

**C. DOCUMENTS CONSIDERED TO BE RELEVANT**

| Category* | Citation of document, with indication, where appropriate, of the relevant passages | Relevant to claim No. |
|---|---|---|
| A | WO 2018/225851 A1 (CHUGAI SEIYAKU KABUSHIKI KAISHA) 13 December 2018 (2018-12-13)<br>entire text, all drawings | 1-18 |

Form PCT/ISA/210 (second sheet) (January 2015)

**INTERNATIONAL SEARCH REPORT**
**Information on patent family members**

International application No.
**PCT/JP2023/037071**

| Patent document cited in search report | Publication date (day/month/year) | Patent family member(s) | Publication date (day/month/year) |
|---|---|---|---|
| WO 2022/138598 A1 | 30 June 2022 | (Family: none) | |
| WO 2022/138891 A1 | 30 June 2022 | (Family: none) | |
| WO 2021/090855 A1 | 14 May 2021 | US 2023/0151060 A1<br>example 1, compound 1217<br>EP 4043478 A1 | |
| WO 2021/090856 A1 | 14 May 2021 | US 2023/0026641 A1<br>entire text, all drawings<br>EP 4056580 A1 | |
| WO 2018/225851 A1 | 13 December 2018 | US 2020/0277327 A1<br>entire text, all drawings<br>EP 3636656 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021090855 A


### Non-patent literature cited in the description


- *J. Peptide Res.*, 2005, vol. 65, 153-166
- *Org. Lett.*, 2013, vol. 15, 1155-1157
- *Org. Lett.*, 2012, vol. 14, 612-615
- *Chem. Rev.*, 1997, vol. 6, 2243-2266
- *Green Chem. Lett. Rev.*, 2021, vol. 14, 153-164
- Comprehensive Organic Transformations. **R. C. LAROCK**. A Guide to Functional Group Preparations
- **M. B. SMITH** ; **J. MARCH**. March's Advanced Organic Chemistry: Reactions, Mechanisms, and Structure
- Peptide Coupling Reagents, More than a Letter Soup. *Chem. Rev.*, 2011, vol. 111, 6557-6602
- Greene's Protective Groups in Organic Synthesis. 2014